(19) Europäisches Patentamt
European Patent Office
Office européen des brevets

(11) EP 4 269 446 B1

(12) EUROPEAN PATENT SPECIFICATION

(45) Date of publication and mention of the grant of the patent:
**26.06.2024 Bulletin 2024/26**

(21) Application number: **23187273.0**

(22) Date of filing: **14.08.2021**

(51) International Patent Classification (IPC):
***C07K 16/28*** *(2006.01)* ***A61P 35/00*** *(2006.01)*
***A61K 35/17*** *(2015.01)* ***C12N 5/0783*** *(2010.01)*
***C07K 16/24*** *(2006.01)* ***C07K 16/30*** *(2006.01)*
***C07K 16/32*** *(2006.01)* ***C07K 16/40*** *(2006.01)*
***C07K 16/42*** *(2006.01)*

(52) Cooperative Patent Classification (CPC):
**C07K 16/2809; C07K 16/2803; C07K 16/2818; C07K 16/2863; C07K 16/2878; C07K 16/30; C07K 16/32; C07K 16/40; C07K 16/4241;**
C07K 2317/21; C07K 2317/31; C07K 2317/34; C07K 2317/622; C07K 2317/73; C07K 2317/732;

(Cont.)

(54) **MULTISPECIFIC ANTI-TCR DELTA VARIABLE 1 ANTIBODIES**

MULTISPEZIFISCHE ANTI-TCR DELTA VARIABLE 1 ANTIKÖRPER

ANTICORPS MULTISPÉCIFIQUES ANTI-TCR DELTA VARIABLE 1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2020 PCT/GB2020/051959**
**17.02.2021 GB 202102222**
**17.02.2021 GB 202102224**

(43) Date of publication of application:
**01.11.2023 Bulletin 2023/44**

(62) Document number(s) of the earlier application(s) in accordance with Art. 76 EPC:
**23153152.6 / 4 218 932**
**21756070.5 / 4 010 082**

(73) Proprietor: **GammaDelta Therapeutics Limited**
**London W12 7FQ (GB)**

(72) Inventors:
- **UDEN, Mark**
  **London, W12 7FQ (GB)**
- **TUNA, Mihriban**
  **London, W12 7FQ (GB)**
- **MOUNT, Natalie**
  **London, W12 7FQ (GB)**
- **GOOD, Robert**
  **London, W12 7FQ (GB)**
- **FREEDMAN, Joshua**
  **London, W12 7FQ (GB)**
- **BHUMBRA, Shefali**
  **London, W12 7FQ (GB)**
- **NUSSBAUMER, Oliver**
  **London, W12 7FQ (GB)**
- **MEHTA, Raj Jaysukhlal**
  **London, W12 7FQ (GB)**

(74) Representative: **Heath, Abigail**
**Kilburn & Strode LLP**
**Lacon London**
**84 Theobalds Road**
**London WC1X 8NL (GB)**

(56) References cited:
**WO-A1-2020/060406** **WO-A1-2020/159368**
**WO-A1-2021/032963**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HANS-HEINRICH OBERG ET AL: "Bispecific antibodies enhance tumor-infiltrating T cell cytotoxicity against autologous HER-2-expressing high-grade ovarian tumors", JOURNAL OF LEUKOCYTE BIOLOGY, vol. 107, no. 6, 13 December 2019 (2019-12-13), pages 1081-1095, XP055740419, GB ISSN: 0741-5400, DOI: 10.1002/JLB.5MA1119-265R**
- **H.-H. OBERG ET AL: "Novel Bispecific Antibodies Increase T-Cell Cytotoxicity against Pancreatic Cancer Cells", CANCER RESEARCH, vol. 74, no. 5, 21 January 2014 (2014-01-21), pages 1349-1360, XP055268793, US ISSN: 0008-5472, DOI: 10.1158/0008-5472.CAN-13-0675**
- **DE BRUIN RENEE C G ET AL: "A bispecific nanobody approach to leverage the potent and widely applicable tumor cytolytic capacity of V gamma 9V delta 2-T cells", ONCOIMMUNOLOGY,, vol. 7, no. 1, 1 January 2018 (2018-01-01) , pages e1375641-1, XP009506483, ISSN: 2162-402X, DOI: 10.1080/2162402X.2017.1375641 [retrieved on 2017-10-20]**
- **DREW C. DENIGER ET AL: "Clinical Applications of Gamma Delta T Cells with Multivalent Immunity", FRONTIERS IN IMMUNOLOGY, vol. 5, 1 December 2014 (2014-12-01), XP055650678, DOI: 10.3389/fimmu.2014.00636**
- **CHITADZE GURANDA ET AL: "The Ambiguous Role of [gamma][delta] T Lymphocytes in Antitumor Immunity", TRENDS IN IMMUNOLOGY, ELSEVIER LTD. * TRENDS JOURNALS, GB, vol. 38, no. 9, 11 July 2017 (2017-07-11), pages 668-678, XP085170623, ISSN: 1471-4906, DOI: 10.1016/J.IT.2017.06.004**
- **DE WEERDT IRIS ET AL: "A Bispecific Single-Domain Antibody Boosts Autologous V?9V?2-T Cell Responses Toward CD1d in Chronic Lymphocytic Leukemia", CLINICAL CANCER RESEARCH , vol. 27, no. 6 15 March 2021 (2021-03-15), pages 1744-1755, XP055842279, US ISSN: 1078-0432, DOI: 10.1158/1078-0432.CCR-20-4576 Retrieved from the Internet: URL:https://clincancerres.aacrjournals.org /content/27/6/1744.full-text.pdf**


Remarks:

The complete document including Reference Table(s) and the Sequence Listing(s) can be downloaded from the EPO website

(52) Cooperative Patent Classification (CPC): (Cont.)
C07K 2317/74; C07K 2317/75; C07K 2317/92

## Description

### FIELD OF THE INVENTION

The present invention relates to multispecific, in particular bispecific, antibodies and fragments and variants thereof that specifically bind the T cell receptor of gamma delta T cells, and one other antigen.

### BACKGROUND OF THE INVENTION

The growing interest in T cell immunotherapy for cancer has focused on the evident capacity of subsets of CD8+ and CD4+ alpha beta (αβ) T cells to recognize cancer cells and to mediate host-protective functional potentials, particularly when de-repressed by clinically mediated antagonism of inhibitory pathways exerted by PD-1, CTLA-4, and other receptors. However, αβ T cells are MHC-restricted which can lead to graft versus host disease.

Gamma delta T cells (γδ T cells) represent a subset of T cells that express on their surface a distinct, defining γδ T-cell receptor (TCR). This TCR is made up of one gamma (γ) and one delta (δ) chain, each of which undergoes chain rearrangement but have a limited number of V genes as compared to αβ T cells. The main TRGV gene segments encoding Vγ are TRGV2, TRGV3, TRGV4, TRGV5, TRGV8, TRGV9 and TRGV11 and non-functional genes TRGV10, TRGV11, TRGVA and TRGVB. The most frequent TRDV gene segments encode Vδ1, Vδ2, and Vδ3, plus several V segments that have both Vδ and Vα designation (Adams et al., 296:30-40 (2015) Cell Immunol.). Human γδ T cells can be broadly classified based on their TCR chains, as certain γ and δ types are found on cells more prevalently, though not exclusively, in one or more tissue types. For example, most blood-resident γδ T cells express a Vδ2 TCR, commonly Vγ9Vδ2, whereas this is less common among tissue-resident γδ T cells such as those in the skin, which more frequently use the Vδ1 TCR paired with gamma chains, for example often paired with Vγ4 in the gut.

γδ T cells play a critical role in immune surveillance, recognising malignant or transformed cells (such as cancer cells) through a pattern of stress markers and then exerting potent and selective cytotoxicity. γδ T cells can therefore act as orchestrators of an immune response. Modulation of these cells *in situ* provides the potential to increase immunogenicity even in tumours with low mutational load which have proven challenging with other immunotherapies. Recognition of tumours by γδ T cells is not dependent on any single tumour antigen and modulators of γδ T cells therefore have potential in a range of disease indications, including both haematological and solid malignancies. The recognition mechanism of γδ T cells is not MHC restricted.

The authors of WO2019147735 hypothesise that some γδ cells have pro-tumour activity or inhibit the anticancer immune response mediated by αβ T cells. The authors postulate that γδ T cells are immunosuppressants and therefore suggest they should be depleted, inhibited or blocked in a cancer setting with the aid of antibodies.

However, despite the prevailing view that that anti-γδ antibodies will negatively modulate γδ cell function by blocking or killing such cells, it has been found that a positive correlation between γδ T cell infiltration and prognosis and/or survival in patients exists.

Compared to αβ TCR receptor/ligand interactions, understanding of vδ1 TCR receptor/ligand interactions are limited. In the absence of such understanding, antibodies which recognize vδ1 TCRs to date are mainly exploratory tools to probe this interaction. Such tools are typically crude, blocking antibodies which suggest TCR receptor/ligand interactions results in blocking, suppression or ablation of vδ1+ cells. For example, tool antibodies TS8.2 and TS-1 are employed as anti-γδ blocking antibodies in studies which suggest said antibodies reduce the cytotoxicity of vδ1 cells. These studies, combined with others, suggest use of such anti-vδ1 antibodies to favourably modulate the cytotoxicity of vδ1 cells in an *in situ* disease setting is inconceivable and there is therefore the need for antibodies which increase, not reduce, vδ1 cytotoxicity.

To exploit γδ T cells for immunotherapy requires either a means to expand the cells *in situ* or to harvest them and expand them ex *vivo* priorto re-infusion. The latter approach has previously been described using the addition of exogenous cytokines, for example see WO2017/072367 and WO2018/212808. Methods for expanding a patients' own γδ T cells has been described using pharmacologically modified forms of hydroxy-methyl but-2-enyl pyrophosphate (HMBPP) or clinically-approved aminobisphosphonates. By these approaches, over 250 cancer patients have been treated, seemingly safely, but with only rare incidences of complete remission. However, there is still a need for activating agents that have the proven capacity to expand large numbers of γδ T cells.

Further, a binding or activating agent capable of preferentially targeting or binding or recognizing or specifically modulating or increasing the numbers of Vδ1+ cells *in-situ* may be highly desirable as a medicament.

WO2020/060406 describes antibodies comprising a first binding moiety that is able to bind human CD1d and a second binding moiety that is able to bind the human Vγ9Vδ2-TCR. WO2020/159368 describes multispecific antibodies capable of binding human CD40 and a human Vγ9Vδ2 T cell receptor.

However, whilst medicaments exist that do potentially modulate Vδ2+ cells inclusive of the aminobisphosphonates such as Zometa® (zoledronic acid), said medicines are primarily designed to slow bone reabsorption. And regardless of said Vδ2+ modulation, there is a need to develop medicines specifically designed to bind, target, modulate, activate, or increase the numbers of Vδ1+ cells. This is because, for example, repeat Vδ2+ modulation can result in long-lasting and a progressively exhausted phenotype.

Further, and given the predominate tissue-resident nature of Vδ1+ cells, an ideal medicament capable of modulating Vδ1+ would also exhibit fewer 'off-target' undesirable effects and rapid renal clearance. Typically, said undesirable effects can manifest when employing small-molecule chemicals. For example, the aforementioned aminobisphosphonates shown capable of modulating the separate class of Vδ2+ cells (as a secondary effect versus primary modulating effect on bone) are associated with renal toxicity which manifests as deterioration of renal function and potential renal failure (e.g. Markowitz et al. (2003) Kidney Int. 64(1):281-289). Additional undesirable effects as listed by the European Medicine Agency for Zometa include anemia, hypersensitivity reactions, hypertension, arterial fibrillation, myalgia, general pain, malaise, blood urea increase, vomiting, joint swelling, chest pain, etc.

Further consideration must also be given to the *in situ* milieu in which vδ1+ cells find themselves. For example, it has previously been shown that non-haematopoietic, tissue-resident γδ T cells showed a strong proliferative response when first separated from tissue but only if they were not in direct cellular contact with autologous fibroblasts. It is found that the non-haematopoietic tissue-resident T cells (γδ T cells) must be separated from the non-haematopoietic cells, (e.g. stromal cells, particularly fibroblasts) in order to function. This is because direct contact of the lymphocytes with stromal or epithelial cells appears to inhibit expansion of tissue-resident γδ T cells. The observation that the pre-activated cells *in situ* exist in a further suppressed state is another reason vδ1 cells have not been considered a promising therapeutic target to date, Indeed, until the discoveries described herein it has not been conceived how one could favourably and selectively modulate these cells in situ, where blood and tissue vδ1+ cells are typically considered 'resting', 'pre-activated' or 'non-activated'.

Various formats of bispecific and multi-specific antibodies have been developed for a variety of therapeutic uses. Bi- and multi-specific antibodies can be divided into separate, although overlapping, classes based on the types of biological targets and modes of action. For example, such multispecific antibodies can be divided into classes such as cytotoxic effector cell redirectors (also known as bispecific, T-cell-recruiting antibodies, bispecific T-cell engagers, TCEs, or BiTEs) and dual immunomodulators (DIs).

TCEs are intended to enhance the patient's immune response to tumours by targeting T cells to tumour cells or vice versa, and work by targeting a first epitope of a T-cell receptor complex of a T-cell (usually CD3) and a second epitope, which is a cancer antigen or a cancer-associated antigen, such as a tumour associated antigen (TAA). Such antibodies colocalize tumour cells and T-cells to promote tumour cell killing. Examples of BiTEs include the CD3 x CD19 bispecific antibody blinatumomab, the CD3 x EpCAM bispecific antibody catumaxomab, and the CD3 x HER2 bispecific antibody ertumaxomab. TCEs such as BiTEs are generally provided in an scFv format, although other formats have been provided. For example, BiKEs are similar to BiTEs, but they target CD16 on NK cells, rather than CD3.

The 'T-cell receptor has been described as the most intricate receptor structure of the mammalian immune system. It comprises a transmembrane multi-protein receptor complex comprises a T-cell receptor in close proximity to a number of CD3 chains. For example, in mammals, a typical such complex comprises a T-cell receptor, a CD3γ chain, a, CD3δ chain, and two CD3ε chains. These chains associate with the T-cell receptor (TCR) alongside a ζ-chain (zeta-chain) which combined then generate typical activation signals in T lymphocytes. However alternative complexes have also been reported. For example, T-cell receptor complexes comprising a T-cell receptor and a zeta chain homodimer have been described. Additional co-receptors such as CD4 and CD8 can also aid TCR function.

Regardless of receptor complex composition, it is well established that said complexes translate cell surface binding events to intracellular phosphorylation signaling cascade. These phosphorylation events culminate in the activation of transcription factors such as NFAT and NFkB that lead to increased expression of cytokines and effector proteins such as granzymes and perforin. However, whilst the use of such TCEs to treat cancer remain a compelling concept, to date and even after 30 years of concerted efforts to advance TCEs in early clinical development, many of such bispecific antibodies have exhibited lackluster safety, efficacy and manufacturability profiles. Indeed, as of January 2020, blinatumomab remains the only approved TCE not then withdrawn. This TCE multispecific antibody fragment binds the T-cell receptor complex on a first binding arm and a CD19 target on a second binding arm.

Bispecific, T-cell-recruiting antibodies are discussed in Lejeune et al., 2020, Front Immunol., 11:762. However, the existing bispecific antibodies in this category, in particular those that recruit T-cells via CD3 binding, have significant off-target effects that result in severe adverse effects, given the potency of the CD3 antigen as signal transducer and its ubiquity in a patient's T-cell population. Hence for CD3 targeting bispecific examples such as Catumaxomab (now withdrawn), systemic delivery (e.g. intravenous) is not a realistic possibility. Instead, more contained delivery such as intra-operative, intra-peritoneal, intra-abdominal etc. is more often contemplated. This thereby limits optionality and use of said bispecifics as medicaments. Indeed, even for effector-attenuated anti-CD3 antibodies (i.e. a CD3 targeting T-cell complex engager but not a bispecific), the associated toxicity makes I.V. delivery challenging. For example, to limit exposure and reduce toxicity, the anti-CD3 antibody Foralumab is now most often being contemplated for oral delivery (e.g. in treatment of gut disease).

It is often stated that many of the current setbacks observed with such TCEs in early clinical trials are due to the high-affinity T-cell complex binding domains employed. Further, it has been proposed that this is because those designing these TCEs had not given due consideration to the low affinity of natural TCR-complex binding events they were hampered by severe dose-limiting toxicities resulting in prohibitively narrow therapeutic windows. Related to this it has been highlighted that many early TCE drug developers relied on three anti-CD3 T-cell complex binding domains derived from OKT3, SP34, and UCHT1. And these original binding domains all bind with a relatively high affinity in the single to low double-digit nM range equating to roughly to 1,000-fold higher affinity than a natural binding event. In turn it has been proposed that this can result in profoundly different (and often unfavourable) effects on the activation of T-cells compared to natural binding of the T-cell receptor complex. For example, TCE developers using platforms based on the higher affinity OKT3 may be confounded by the fact the OKT3 is apoptotic to T-cells in the presence of IL-2.

For these reasons it has become apparent that lower affinity T-cell complex binding is an important consideration for determining the design parameters of T-cell engaging bispecific antibody therapeutics.

Another issue when designing said TCEs, is the need to attenuate Fc function. Indeed, typically TCEs require the complete suppression of the Fc-mediated effector functions in order to maximize therapeutic efficacy and to minimize off-target toxicity because binding of Fc to Fc gamma receptor (FcγR) leads to activation of immune effector cells. In reality, the majority of the CD3-targeting bispecific antibodies currently in clinical practice have Fc domains with reduced binding activity to FcyR or are bispecific fragments intentionally without the Fc region. It would generally be expected that a TCE with unattenuated Fc function would induce an antibody-dependent cell-mediated cytotoxicity (ADCC) effect and thereby deplete the population of γδ T-cells recognized by the antibody. However, and again, by attenuating such functionality to avoid toxicity/safety complexities, one may also attenuate a potentially important efficacy angles too e.g. by engaging CD16+ or CD32+ or CD64+ immune cells, or by reducing half-life of the bispecific (e.g. if employing smaller bispecific antibody fragments such as BITEs). Methods of reducing the interaction of the FcyR and the TCE (such as using an IgG format designed to reduce said interaction) would be expected to reduce Fc-mediated immobilization of the TCE and reduce TCR clustering by cross-linking with the immobilized TCE.

To address some but not all of these complications, many companies such as Xencor (Pasadena, CA), Macrogenics (Gaithersburg, MD) and Genentech (San Francisco, CA) have more recently reported reducing the binding affinity of the T-cell receptor complex binding arms in their respective TCE platforms. However, reducing the affinities of said binding may result in less effective efficacy and less optionality in terms of TCE design and functionality. For example, it is now demonstrated that affinity of the binding domains in such TCEs drives distribution profile in vivo. Specifically, it is typically observed that TCE distribution is biased towards its highest affinity target. Hence, by reducing the affinity of a TCE binding domain to the T-cell complex, it is typical to then bias distribution away from T-cells; the very cells needed to drive efficacy of such TCEs. It is partly for such reasons that TCE therapeutic windows have been termed 'prohibitively narrow'.

Hence there is a need for improved TCE-type medicaments that address these problems with existing therapies and candidate therapies, including a platform that affords more optionality and does not rely on Fc-domain deactivation or attenuation, or on specific affinities to the TCR complex, and provides a safer therapeutic with reduced adverse side effects

Dual immunomodulator antibodies (DIs) are antibodies that bind two distinct immunomodulatory targets. Immunomodulatory targets include, for example, the immune checkpoint inhibitors PD-L1, PD-1, OX40, CTLA-4, LAG-3, TIM-3, TIGIT and VISTA (see, for example, Qin et al., 2019, Molecular cancer, 18:155). Typical example compounds in this class combine the targeting of two immunomodulatory signaling pathways. Examples of such DIs typically comprise at least two binding domains wherein both domains respectively target epitopes on two separate target proteins or complexed proteins present on immune cells such as T-cells. To date, it has not been conceived or indeed considered possible to generate a DI which targets at least two immunomodulatory targets wherein one such target is to the TCR δ1 chain, to help overcome the immunosuppressive tumour microenvironment and turn it from "cold" to "hot", promoting the accumulation of proinflammatory cytokines and T cell infiltration, and tumour cell killing. Existing DI approaches are being studied in an attempt to reduce or overcome the high toxicity associated with combination therapies (as well as improve efficacy over combination treatments), but many require careful monitoring and there has been mixed success rates. There remains a need to provide alternative immunomodulatory approaches for the treatment of conditions such as cancer, and the present invention provides an entirely new and alternative approach to DI antibodies never before contemplated.

Hence there is a need for improved medicaments, in particular multi- and bi-specific antibodies, for the treatment of infections, autoimmune conditions, and cancer.

## SUMMARY OF THE INVENTION

The subject matter for which protection is sought is as defined in the appended set of claims. The description may contain additional technical information, which although not part of the claimed invention, is provided to place the invention in a broader technical context and to illustrate possible related technical developments.

The present invention relates to multispecific antibodies that target γδ T-cells and another antigen. Accordingly, in a first aspect of the invention there is provided a multispecific antibody or fragment thereof that specifically binds a first target epitope, wherein the first target epitope is an epitope of a variable delta 1 (Vδ1) chain of a γδ T cell receptor (TCR); and a second target epitope, wherein the second epitope is an epitope of OX40.

The multispecific antibodies of the present disclosure can be divided into two classes. The first are multispecific antibodies that are T-cell engagers. The second are multispecific antibodies that are dual immunomodulators. The multispecific antibodies of the invention are dual immodulators.

In an aspect of the invention there is provided a multispecific antibody that specifically binds a first target epitope, wherein the first target epitope is an epitope of the variable delta 1 (Vδ1) chain of a γδ T cell receptor (TCR); and a second target epitope, wherein the second target epitope is an epitope of an immunomodulatory antigen, wherein the second epitope is an epitope of OX40.

In a further aspect of the disclosure, there is provided a polynucleotide sequence encoding a multispecific antibody or fragment thereof of the invention.

In a further aspect of the disclosure, there is provided an expression vector comprising a polynucleotide sequence of the invention. There is also provided a host cell comprising a polynucleotide sequence as of the disclosure or an expression vector of the disclosure. There is also provided a method for producing any multispecific antibody or fragment thereof of the invention, comprising culturing a host cell of the disclosure in a cell culture medium.

In a further aspect of the invention, there is provided a composition comprising the multispecific antibody or fragment thereof of the invention. There is also provided a pharmaceutical composition comprising the multispecific antibody or fragment thereof of the invention and a pharmaceutically acceptable diluent or carrier.

In a further aspect of the disclosure, there is provided a kit comprising a multispecific antibody or fragment of the invention or a pharmaceutical composition of the invention, optionally comprising instructions for use and/or an additional therapeutically active agent.

In a further aspect of the invention there is provided a multispecific antibody or fragment of the invention, or a pharmaceutical composition of the invention for use in a method of treating a disease or disorder in a subject. A method of modulating an immune response in a subject is also disclosed, comprising administering to the subject a multispecific antibody or fragment of the invention, or a pharmaceutical composition of the invention.

In a further aspect of the disclosure, there is provided a method of preparing a pharmaceutical composition comprising providing an antibody prepared according to a method of preparing a multispecific antibody or fragment of the invention and co-formulating the multispecific antibody with at least one or more pharmaceutically acceptable diluents or carriers.

In a further aspect of the disclosure, there is provided a method of generating a multispecific antibody of the invention, comprising the steps of selecting a first monospecific antibody which specifically binds a first target epitope, wherein the first target epitope is an epitope of the variable delta 1 (Vδ1) chain of a γδ T cell receptor (TCR); and combining the antibody or antigen-binding fragment thereof of said first antibody with an antibody or fragment thereof comprising a binding domain targeting a second epitope to generate the recombinant multispecific antibody.

In a still further aspect of the disclosure, there is provided a multispecific antibody or fragment thereof of the invention, or a pharmaceutical composition of the invention, or a kit of the disclosure, for use in medicine. There is also disclosed the use of a multispecific antibody or fragment thereof of the invention in the manufacture of a medicament.

## BRIEF DESCRIPTION OF THE FIGURES

**Figure 1: ELISA Detection of Directly Coated Antigen with Anti-Vδ1Ab (REA173, Miltenyi Biotec).** Detection was seen only with those antigens which contain the Vδ1 domain. Leucine zipper (LZ) format seems more potent than Fc format which is consistent with cell-based flow competition assay (data not shown).

**Figure 2: Polyclonal phage DELFIA data for DV1 selections.** (A) Heterodimer selections: heterodimeric LZ TCR format in round 1 and 2, with deselections on heterodimeric LZ TCR in both rounds. (B) Homodimer selections: round 1 performed using homodimeric Fc fusion TCR with deselection on human IgG1 Fc followed by round 2 on heterodimeric LZ TCR with deselection on heterodimeric LZ TCR. Each graph contains two bars for each target to represent selections from different libraries.

**Figure 3: IgG capture:** left) Sensorgrams of interaction of anti-L1 IgG with L1, right) steady state fits, if available. All experiments were performed at room temperature on MASS-2 instrument. Steady state fitting according to Langmuir 1:1 binding.

**Figure 4:** Results of TCR Downregulation Assay for clones 1245_P01_E07, 1252_P01_C08, 1245_P02_G04, 1245_P01_B07 and 1251_P02_C05 (A) or clones 1139_P01_E04, 1245_P02_F07, 1245_P01_G06 1245_P01_G09, 1138_P01_B09, 1251_P02_G10 and 1252_P01_C08 (B).

**Figure 5:** Results of T cell degranulation Assay for clones 1245_P01_E07, 1252_P01_C08, 1245_P02_G04, 1245_P01_B07 and 1251_P02_C05 (A) or clones 1139_P01_E04, 1245_P02_F07, 1245_P01_G06, 1245_P01_G09, 1138_P01_B09, and 1251_P02_G10 (B).

**Figure 6:** Results of Killing Assay (THP-1 flow-based assay) for clones 1245_P01_E07, 1252_P01_C08, 1245_P02_G04, 1245_P01_B07 and 1251_P02_C05 (A) or clones 1139_P01_E04, 1245_P02_F07, 1245_P01_G06, 1245_P01_G09, 1138_P01_B09 and 1251_P02_G10 (B).

**Figure 7: Total cell counts during Experiment 1 of Example 10.** Samples were cultured with varying concentration of anti-Vδ1 antibodies described herein and compared to samples cultured with comparator antibodies or controls. Graphs show total cell counts at **(A)** day 7, **(B)** day 14 and **(C)** day 18.

**Figure 8: Analysis of** Vδ1 **T cells during Experiment 1 of Example 10.** Graphs show **(A)** percentage of Vδ1 T cells, (B) Vδ1 T cell count and (C) Vδ1 fold change in the samples at day 18.

**Figure 9: Total cell counts during Experiment 2 of Example 10.** Samples were cultured with varying concentration of anti-Vδ1 antibodies described herein and compared to samples cultured with comparator antibodies or controls. Graphs show total cell counts at **(A)** day 7, **(B)** day 11, **(C)** day 14 and (D) day 17.

**Figure 10: Analysis of Vδ1 T cells during Experiment 2 of Example 10.** Graphs show **(A)** percentage of Vδ1 T cells, **(B)** Vδ1 T cell count and **(C)** Vδ1 fold change in the samples at day 17.

**Figure 11: Cell composition analysis.** The cell types present in the samples (including non-Vδ1 cells) were measured on day 17 of Experiment 2. Cells were harvested and analysed by flow cytometry for surface expression of Vδ1, Vδ2 and αβTCR. The percentage values are also provided in **Table 7.**

**Figure 12: SYTOX-flow killing assay results.** Cell functionality was tested using the SYTOX-flow killing assay and results are presented for **(A)** Experiment 1 at day 14 using cells in a 10:1 Effector-to-Target (E:T) ratio, and **(B)** Experiment 2 at day 17 (post freeze-thaw) using cells at a 1:1 and 10:1 E:T ratio.

**Figure 13: Total cell count post freeze-thaw.** Graph shows the total cell counts after 7 days of culturing cells post freeze-thaw for cultures contacted with B07, C08, E07, G04 or OKT-3 antibodies prior to freezing.

**Figure 14: Monitoring cell expansion.** Total cell counts were monitored until day 42 for cells cultured post freeze-thaw.

**Figure 15: Binding equivalence studies on modifiedanti-Vδ1 antibodies.**

**Figure 16: Anti-Vδ1 antibody binding equivalence studies on human germline Vδ1 antigen and a polymorphic variant thereof.**

**Figure 17: Anti-Vδ1 antibody conferred increases in Vδ1+ cell cytokine secretion levels.** Tissue-derived γδ T cells incubated with the antibodies as indicated. **(A)** The levels of TNF-alpha observed **(B)** The levels of IFN-gamma observed.

**Figure 18: Anti-Vδ1 antibody conferred increases in Vδ1+ cell Granzyme B levels/activity** Cancer cells co-cultured with tissue-derived γδ T cells for one hour at a set 1:20 T:E ratio and with the antibodies as indicated. Results highlight the quantities of Granzyme B detected in the cancer cells at the end of co-culture.

**Figure 19: Anti-Vδ1 antibody conferred modulation and proliferation of immune cells in human tissue.** Human skin punch-biopsies (from five different donors) incubated for 21-days in culture with the antibodies as indicated. **(A)** The number of viable pan-γδ+ cells. **(B)** The number of viable Vδ1+ cells. **(C)** The percentage of viable, double-positive Vδ1+ CD25+ cells.

**Figure 20: Anti-Vδ1 antibody conferred modulation and proliferation of tumour-infiltrating-lymphocyte (TILs) in human tumours.** Studies on renal cell carcinoma (RCC) +/- antibodies (A) Fold-increase in TIL Vδ1+ cells. (B) Total numbers of TIL Vδ1+ cells. (C) Example gating strategy (D) Comparative cell-surface phenotypic profile of TIL Vδ1+ cells. (E) Analysis of the TIL Vδ1-negative gated fraction.

**Figure 21: Anti-Vδ1 antibody conferred enhancement of Vδ1+ mediated cytotoxicity, and diseased-cell-specific cytotoxicity.** Cytotoxicity/potency-assays in model systems comprising a triculture of Vδ1+ effector cells, THP-1 monocytic cancer cells, and non-diseased, healthy primary monocytes. **(A)** Quantification of THP-1 and monocyte cell numbers in triple co-culture with γδ T-cells in the presence of anti-Vδ1 mAbs or controls. **(B)** A bar chart representation highlighting the window between diseased-cell specific killing and non-diseased healthy: Left-hand bar chart; fold-increase in killing of diseased-cells (THP-1) versus killing of non-diseased cells (primary human monocytes); Right-hand bar chart; same data but represented as percent-enhanced killing versus controls **(C)** Tabulated results summarizing the percent improvement in potency of Vδ1+ effector cell killing of THP-1 target cells +/- mAbs. **(D)** Tabulated results of EC50 values as calculated from Figure (A) represented as γδ T-cell numbers required to confer 50% THP-1 cell killing.

**Figure 22: Multi-specific antibody conferred enhancement of Vδ1+ effector cell mediated cytotoxicity.** The targeting of a tissue-centric disease associated antigen: **(A-D)** Example co-culture of Vδ1+ effector cells with A-431 cancer cells +/- multi-specific antibodies comprising anti-Vδ1 x anti-TAA (EGFR) bispecific binding moieties wherein the anti-Vδ1 VL+VH binding domain (to the first target) is combined with the CH1-CH2-CH3 domain of an anti-EGFR binding moiety (to the second target). **(E-H)** Example co-culture of Vδ1+ effector cells with A-431 cancer cells +/- multi-specific antibodies comprising anti-Vδ1 x anti-TAA (EGFR) bispecific binding moieties wherein the anti-Vδ1 binding domain (to the first target) comprises a full-length antibody (VH-CH1-CH2-CH3/VL-CL) then combined with an anti-EGFR cetuximab-derived scFv binding moiety (to the second target). **(I-J)** Alternative approach to representing the data: Percentage improvement conferred by multi-specific antibodies upon Vδ1+ effector cell cytotoxicity towards EGFR+ cells relative to component parts.

**Figure 23: Multi-specific antibody conferred enhancement of Vδ1+ mediated cytotoxicity and diseased-cell-specific cytotoxicity.** The targeting of a hemopoietic disease associated antigen (A) E:T ratios required to induce 50% Raji cell killing (B) Percentage improvement with addition of a Vδ1-CD19 multi-specific antibodies

**Figure 24: Anti-Vδ1/CD19 bispecific antibodies exhibit high affinity binding to human Vδ1 and cyno Vδ1, comparable to the parental monoclonal Vδ1 mAb.** Surface Plasmon Resonance (SPR) analysis was performed with Vδ1/CD19 bispecific to assess binding to human Vδ1 and CD19 antigen. The bispecific antibody binds both human and cyno Vδ1, with only a small reduction in affinity for both antigens.

**Figure 25: Vδ1-CD19 Bispecific T cell Engagers enhance CD19+ target cell cytotoxicity and γδT-cell activation while sparing healthy CD19+ B-cells.** (A) Expression of CD19 on cancerous NALM-6, Raji, B-cell and Vδ1 γδT-cells determined by flow cytometry. (B-D) Effect of Anti-Vδ1-CD19 BiTEs on NALM-6 cells (B), Raji cells (C) and B-cell cytotoxicity (D). Antibodies were titrated in the presence of 1:1 E:T ratio with Vδ1 γδT-cells for 12 hours. Percent live cells were calculated by high content confocal microscopy and normalized to live cell counts in the absence of Vδ1 γδT-cells. (E) Bar chart representing the percent number of live cells after 12 hours co-culture of Vδ1 γδT-cells either NALM-6, Raji or B-cells in the presence of Vδ1 BiTEs and controls as presented in (B), (C) and (D). (F-G) Effect of Vδ1-CD19 BiTEs on Vδ1 TCR surface expression in the presence of NALM-6 target cells (F) and healthy B-cells (G) as determined by flow cytometry after four hours co-culture. (H) Bar chart representing the maximum percent TCR downregulation as presented in (F) and (G) at the top concentrations (3μg/ml). (I-J) Effect of Vδ1-CD19 bispecifics on CD107a upregulation on Vδ1 cell surface in the presence of NALM-6 target cells (I) and healthy B-cells (J) as determined by flow cytometry after four hours co-culture. (K) CD107a upregulation in B-cells and NALM-6 cells. All data indicates mean ± standard deviation and is representative of n=3. Blinatumomab (CD3-CD19 BiTE) was included as a control in all assays.

**Figure 26: Affinity matured Vδ1 clones in a bispecific format bind to Her2$^+$ target cells and exhibit enhanced binding to Vδ1 γδT-cells, and enhanced cytotoxicity of Her2$^+$ target cells.** (A) Cell surface expression of Her2 and Vδ1 on breast cancer cell lines and Vδ1 γδT-cells (B). (C-E) Binding of Vδ1-Her2 bispecific antibodies and Her2 mAb control (Trastuzumab) to Her2+ (SK-BR-3 (C), BT-474 (D)), Her2$^-$ (MDA-MD-231 (E)) and Vδ1+ cells (F). (G-I) Percent live cells remaining after 24 hours co-culture in a 1:1 E:T ratio with Vδ1 γδT-cells and SK-BR-3 cells (G), BT-474 (H), and MDA-MB-231 cells (I) in the presence of Vδ1-Her2 bispecific antibodies. (J) Bar chart representing the percent increase in cytotoxicity after 24 hours Vδ1 γδT-cells Vδ1-Her2 bispecific antibodies.

**Figure 27: Anti- Vδ1/EGFR bispecific antibodies exhibit high affinity binding to human EGFR and a human Vδ1-binding affinity comparable to their parental mAbs.** (A, B) Surface Plasmon Resonance (SPR) analysis was performed with Vδ1/EGFR bispecific to assess binding to human Vδ1 (A) and human EGFR antigen (B). The parental mAbs, cetuximab and negative control mAbs were included for comparison purposes.

**Figure 28: Vδ1/EGFR bispecific antibody binds to EGFR+ A431 target cells and Vδ1 γδ T-cells. (A-D)** Cell surface expression of EGFR on A431 cell line and primary Vδ1 γδT-cells. **(E,F)** The level of binding by the anti-Vδ1/EGFR bispecific antibodies to A431 cell line or primary Vδ1 γδ T-cells. Target cells were stained with varying concentrations of antibody, followed by a fluorescent anti-human IgG detection antibody. All incubation steps were performed at 4°C and mAb binding was determined using flow cytometry to measure the median level of fluorescence. Logarithmic four parameter dose-response curves were fitted using GraphPad Prism 9.

**Figure 29: Vδ1/EGFR bispecific antibodies induces EGFR-specific T cell activation and degranulation leading to increased γδ T cell-mediated cytotoxicity of A431 target cells.**
**(A)** Cell surface expression of γδTCR on primary Vδ1 γδ T-cells following culture with bispecific antibodies for 24 hours in the presence or absence of A431 cells. **(B, C)** The number of viable A431 cells **(B)** and activation status of primary Vδ1 γδ T-cells following co-culturing at 1:1 ratio alongside varying concentrations of antibody for 24 hours. Viability was measured by viability dye and activation status using a CD25 antibody. **(D)** Degranulation of primary Vδ1 γδ T-cells following co-culture with A431 cells at 1:1 ratio alongside varying concentrations of antibody for four hours. Degranulation was determined by adding fluorophore-conjugated anti-CD107α antibody directly into the cell-antibody mix at the start of the co-culture. **(E)** The number of viable A431 cells following 24-hours co-culture with 10 pM of antibody and varying quantity of primary Vδ1 γδ T-cells. **(A-E)** In all cases, fluorescence was determined using flow cytometry to measure the median level of fluorescence. Logarithmic four parameter dose-response curves were fitted using GraphPad Prism 9. Data is represented as mean ± SD of two biological replicates

**Figure 30: CD3 downregulation. (A)** shows the vδ1 TCR MFI upon antibody stimulation as an indication of mAb target engagement. **(B)** shows the MFI of CD3 expression on positively gated vδ1 cells. Stimulation with the vδ1 antibody engaged vδ1 cells and resulted in down-regulation of both vδ1 and CD3 on vδ1 cells.

**Figure 31: Vδ1xFAPα bispecific antibodies enhance Vδ1 γδT-cell activation and lysis of FAPα$^+$ fibroblasts. (A)** shows binding kinetics of anti-Vδ1, anti FAPα monoclonal and anti-Vδ1xFAPα bispecific antibodies for recombinant human Vδ1 and FAPα as determined by surface plasmon resonance (SPR). (B,C) Binding of anti-Vδ1 and anti FAPα antibodies to FAPα$^+$ fibroblasts **(B)** and Vδ1 γδT-cells **(C). (D,E)** Effect of anti-Vδ1xFAPα bispecific antibodies and monoclonal controls, on Vδ1 TCR downregulation on Vδ1 γδT-cells in the absence **(D)** or presence of **FAPα$^+$** fibroblasts **(E). (F-G)** Effect of anti-Vδ1xFAPα bispecific antibodies and monoclonal controls, on CD107a upregulation on Vδ1 γδT-cells in the absence **(F)** or presence of **FAPα$^+$** fibroblasts **(G). (H)** Effect of anti-Vδ1xFAPα bispecific antibody and monoclonal controls on fibroblast lysis by Vδ1 γδT-cells after 24 hours co-culture. Percent live cells were calculated by high content confocal microscopy and normalized to live cell counts in the absence of Vδ1 γδT-cells.

**Figure 32: Vδ1xMSLN bispecific antibodies enhance Vδ1 γδT-cell activation and lysis of MSLN$^+$ target cells. (A)** binding kinetics of anti-Vδ1, anti-MSLN monoclonal and anti-Vδ1xMSLN bispecific antibodies for recombinant human Vδ1 and MSLN as determined by surface plasmon resonance (SPR). **(B,C)** Binding of anti-Vδ1 and anti-MSLN antibodies to **MSLN$^+$** HeLa cells **(B)** and Vδ1 γδT-cells **(C). (D,E)** Effect of anti-Vδ1xMSLN bispecific and monoclonal antibodies, on Vδ1 TCR downregulation on Vδ1 γδT-cells in the absence **(D)** or presence of **MSLN$^+$** OVCAR-3 cells **(E). (F,G)** Effect of anti-Vδ1xMSLN bispecific antibodies and monoclonal controls, on CD107a upregulation on Vδ1 γδT-cells in the absence **(F)** or presence of **MSLN$^+$** OVCAR-3 cells (G). **(H)** Effect of anti-Vδ1xMSLN bispecific antibody and monoclonal controls on HeLa cell lysis by Vδ1 γδT-cells after 24 hours co-culture. Percent live cells were calculated by high content confocal microscopy and normalized to live cell counts in the absence of Vδ1 γδT-cells.

**Figure 33: Vδ1xPD-1 bispecific antibodies enhance activation of Vδ1 γδT-cells and inhibit checkpoint blockade of PD-1$^+$ T-cells. A)** SPR analysis of anti-Vδ1, anti-PD-1, anti-RSVIgGcontrolxanti-PD-1 and anti-Vδ1xanti-PD-1 bispecific antibodies binding to recombinant human Vδ1 and PD-1. **B)** Dual binding of anti-Vδ1 and anti-PD-1 bispecific antibodies to recombinant human PD-1 and Vδ1 as determined by SPR. C) Expression of PD-1 on CD4 and CD8 T-cells activated with anti-CD3/anti-CD28 dynabeads. **D)** Binding of anti-Vδ1 and anti-PD-1 antibodies to PD-1$^+$ activated CD4 and CD8 T-cells and Vδ1 γδT-cells. **E)** Effect of anti-Vδ1xPD-1 bispecific antibodies and monoclonal controls, on Vδ1 TCR downregulation on Vδ1 γδT-cells in the absence or presence of PD-1$^+$ CD4 T-cells. **F)** EC50 of anti-Vδ1xPD-1 bispecific antibodies on Vδ1 TCR downregulation on Vδ1 γδT-cells in the presence of absence of PD-1$^+$ CD4 T-cells. **G)** Effect of Vδ1 crosslinked anti-Vδ1xPD-1 bispecific antibody on PD-1$^+$ T-cell activation.

**Figure 34: Vδ1x4-1BB bispecific antibodies enhance activation of Vδ1 γδT-cells and 4-1BB$^+$ T-cells. A)** SPR analysis of anti-Vδ1, anti-4-1BB, anti-RSVIgGcontrolxanti-4-1 BB and anti-Vδ1xanti-4-1BB bispecific antibodies binding to recombinant human Vδ1 and 4-1BB. **B)** Dual binding of anti-Vδ1 and anti-4-1BB bispecific antibodies to recombinant human 4-1 BB and Vδ1 as determined by SPR. **C)** Expression of 4-1BB on CD4 and CD8 T-cells activated with anti-CD3/anti-CD28 dynabeads. **D)** Binding of anti-Vδ1 and anti-4-1BB antibodies to 4-1BB$^+$ activated CD8 T-cells and Vδ1 γδT-cells. **E-F)** Effect of anti-Vδ1x4-1BB bispecific antibodies and monoclonal controls, on Vδ1 TCR downregulation on Vδ1 γδT-cells in the absence **(E)** or presence of 4-1BB$^+$ CD8 T-cells **(F). G)** Effect of Vδ1 crosslinked anti-Vδ1x4-1BB bispecific antibody on 4-1BB$^+$ T-cell activation.

**Figure 35: Vδ1xOX40 bispecific antibodies enhance activation of Vδ1 γδT-cells and OX40$^+$ T-cells. A)** SPR analysis of anti-Vδ1, anti-OX40, anti-RSVIgGcontrolxanti-OX40 and anti-Vδ1xanti-OX40 bispecific antibodies binding to recombinant human Vδ1 and OX40. **B)** Dual binding of anti-Vδ1 and anti-OX40 bispecific antibodies to recombinant human OX40 and Vδ1 as determined by SPR. **C)** Expression of OX40 on CD4 and CD8 T-cells activated with anti-CD3/anti-CD28 dynabeads. **D)** Binding of anti-Vδ1 and anti-OX40 antibodies to OX40$^+$ activated CD4 T-cells and Vδ1 γδT-cells. **E-F)** Effect of anti-Vδ1xOX40 bispecific antibodies and monoclonal controls, on Vδ1 TCR downregulation on Vδ1 γδT-cells in the absence **(E)** or presence of OX40$^+$ CD4 T-cells **(F). G)** Effect of Vδ1 crosslinked anti-Vδ1xOX40 bispecific antibody on **OX40$^+$** T-cell activation.

**Figure 36: Vδ1xTIGIT bispecific antibodies enhance activation of Vδ1 γδT-cells and inhibit checkpoint blockade of TIGIT$^+$ T-cells. A)** SPR analysis of anti-Vδ1, anti-TIGIT, anti-RSVIgGcontrolxanti-TIGIT and anti-Vδ1xanti-TIGIT bispecific antibodies binding to recombinant human Vδ1 and TIGIT. **B)** Dual binding of anti-Vδ1 and anti-TIGIT bispecific antibodies to recombinant human TIGIT and Vδ1 as determined by SPR. **C)** Expression of TIGIT on CD4 and CD8 T-cells activated with anti-CD3/anti-CD28 dynabeads. **D)** Binding of anti-Vδ1 and anti-TIGIT antibodies to TIGIT$^+$ activated CD4 and CD8 T-cells and Vδ1 γδT-cells. **E)** Effect of anti-Vδ1xTIGIT bispecific antibodies and monoclonal controls, on Vδ1 TCR downregulation on Vδ1 γδT-cells in the absence or presence of TIGIT$^+$ CD8 T-cells. **F)** EC50 of anti-Vδ1xTIGIT bispecific antibodies on Vδ1 TCR downregulation on Vδ1 γδT-cells in the presence or absence of TIGIT$^+$ CD8 T-cells. **G)** Effect of Vδ1 crosslinked anti-Vδ1xTIGIT bispecific antibody on TIGIT$^+$ T-cell activation.

**Figure 37: ADCC reporter bioassay shows no ADCC as a result of the anti-vδ1 antibodies.** Target cells, ie. γδ cells, were incubated with the ADCC bioassay effector cells in presence of anti-vδ1 antibodies, anti-vδ1 LAGA antibodies (Fc disabled), and RSV Isotype control. Luminescence signal was recorded as relative light units (RLU) and fold induction was calculated as described in the methods. N=2 γδ donors (performed in technical duplicates) for "anti-vδ1 antibody", "anti-vδ1 LAGA antibody", "RSV", "OKT3". N=1 Raji cell lines forthe "Rituximab + Rajis" condition (in technical duplicates) and n=1 γδ donors for the "anti-vδ1 antibody + Effector" and "anti-vδ1 LAGA antibody + Effector" conditions (performed in technical duplicate and singlicate, respectively). Effector:target ratio at 3:1.

**Figure 38: Vδ1-CD19 Bispecific T cell Engagers enhance CD19+ target cell cytotoxicity and γδT-cell activation while sparing healthy CD19+ B-cells. A-F)** Effect of anti-Vδ1xCD19 and CD3xCD19 bispecific antibodies on γδ T-cell or αβ T-cell mediated lysis of Raji cells or healthy primary B-cells. Percent positive Raji or healthy primary B-cells were determined at 24hours by confocal microscopy in tri-cultures of: Vδ1 γδT-cells with Raji cells **(A)** and primary healthy B-cells **(B);** tricultures of aβT-cells with Raji cells **(C)** and primary health B-cells **(D);** or quad cultures of Vδ1 γδT-cells and aβT-cells, with Raji cells **(E)** and primary healthy B-cells **(F). G-I)** Quantification of IL-17A secretion from γδT-cells or aβ T-cells 24hours post stimulation by anti-Vδ1xCD19 and CD3xCD19 bispecific antibodies. Cell culture supernatants from cocultures of Raji cells, primary B-cells and: γδT-cells **(G);** or αβ T-cells **(H);** or γδT-cells and αβ T-cells (**I**), were collected and IL-17A secretion determined by MSD. Dotted lines represent the lowest level of quantification.

## DETAILED DESCRIPTION OF THE INVENTION

The present invention provides a new class of multispecific antibodies that target a variable delta 1 (Vδ1) chain of a γδ T cell receptor (TCR) and a second antigen, wherein the second antigen is OX40. The second antigen, which is OX40, may be, for example, an immunomodulatory antigen, and thus the antibody may be a dual immunomodulatory antibody.

The TCEs of the present invention provide several advantages over the TCEs of the prior art. In particular, the TCEs may overcome many of the challenges associated with TCEs of the prior art by targeting the T-cell receptor complex via an entirely novel and discrete mechanism. Indeed, by specifically targeting (and activating) the T-cell receptor complex solely via binding to an epitope on the TRDV1 domain, a number of advantages are realized including:

- Engaging only a sub-set of T-cells rather than *all* T-cells (e.g. engagement of T-regs in a cancer setting may be undesirable);
- Engaging only a sub-set of T-cells (TRDV1+ T-cells) that are predominantly 'tissue-resident' and whose presence often positively correlates with good prognosis in a cancer/tumour setting;
- Activating the T-cell receptor complex via TRDV1 engagement thereby affording more optionality (e.g. increasing affinity of this binding domain). For example, by developing recombinant TCEs which engage a T-Cell Receptor complex solely via the TRDV1 domain rather than via CD3, increased affinity may drive more favourable functionality. For example, high-affinity TRDV1-binding TCEs may activate but not exhaust T-cells; and/or
- Engaging the TCE complex via said novel means and via recombinant TRDV1 binding domains may result in less deleterious effects and so reduce the need to attenuate Fc functionality in said TCE moieties. This then may afford additional optionality, for example by allowing TRDV1 TCEs to engage TRDV1+ cells via one binding domain, engaging a second cell type (such as a cancerous cell) by a second binding arm, and engaging other effector cells such as CD16+ or CD32+ or CD64+ immune cells via a functioning Fc domain.

Hence through the discoveries as described herein, the present inventors have generated a novel class of recombinant TCEs. Specifically, the present inventors have discovered a new class of TCEs which engage the T-cell receptor via a TRDV1 domain rather than other domains in said T-cell receptor signalling complexes. More specifically the present inventors have discovered a new class of TCEs which engage this complex via an activating epitope on TRDV1 and which may be bound at higher affinities without potentially conferring some of the previously reported deleterious effects associated with high-affinity T-cell receptor complex engagement. Further this new class of TCEs may engage in such a way which may allow for wild-type Fc functionality too, thereby affording additional efficacy potential too.

The DIs of the present invention provide a completely novel method of dual immunomodulatory target engagement that is entirely unique, providing a huge possible range of novel therapies requiring immunomodulation, such as cancer. The DI platform of the present invention provides a new class of therapeutics that may provide an important alternative or improvement to the existing DI approaches. Previously it has not be contemplated that a TRDV1-specific binding function as described herein could be incorporated into a DI format.

The multispecific antibodies of the invention may also display improved properties compared to equivalent monospecific antibodies. For example, the multispecific antibodies of the invention may also display improved properties compared to monospecific antibodies having the same antigen binding domains as the component parts of the multi-specific antibodies. In some embodiments, for example, the recombinant multispecific antibody confers increased gamma delta T-cell mediated cytotoxicity towards a diseased cell expressing the second epitope compared to the cytotoxicity conferred by an equivalent amount of said first monospecific antibody. The multispecific antibodies of the invention may also display improved cytotoxicity towards diseased cells whilst still sparing healthy cells.

**Definitions**

Unless defined otherwise, all technical and scientific terms used herein have the meaning commonly understood by a person skilled in the art to which this invention belongs. As used herein, the following terms have the meanings ascribed to them below.

Gamma delta ($\gamma\delta$) T cells represent a small subset of T cells that express on their surface a distinct, defining T Cell Receptor (TCR). This TCR is made up of one gamma ($\gamma$) and one delta ($\delta$) chain. Each chain contains a variable (V) region, a constant (C) region, a transmembrane region and a cytoplasmic tail. The V region contains an antigen binding site. There are two major sub-types of human $\gamma\delta$ T cells: one that is dominant in the peripheral blood and one that is dominant in non-haematopoietic tissues. The two sub-types may be defined by the type of $\delta$ and/or $\gamma$ present on the cells. For example, $\gamma\delta$ T cells that are dominant in peripheral blood primarily express the delta variable 2 chain (V$\delta$2). $\gamma\delta$ T cells that are dominant in non-haematopoietic tissues (i.e. are tissue-resident) primarily express the delta variable 1 chain. References to "V$\delta$1 T cells" or "V$\delta$1+ T cells" referto $\gamma\delta$ T cells with a V$\delta$1 chain, *i.e.* V$\delta$1+ cells.

References to "delta variable 1" may also referred to as V$\delta$1 or Vd1, and a nucleotide encoding a TCR chain containing this region or the TCR protein complex comprising this region may be referred to as "TRDV1". Antibodies or fragments thereof which interact with the V$\delta$1 chain of a $\gamma\delta$ TCR, are all effectively antibodies or fragments thereof which bind to V$\delta$1 and may referred to as "anti-TCR delta variable 1 antibodies or fragments thereof' or "anti-V$\delta$1 antibodies

or fragments thereof' or "anti-TRDV1 antibodies or fragments thereof' or "anti-TRDV1 antibodies or fragments thereof".

Additional references are made herein to other delta chains such as the "delta variable 2" chain. These can be referred to in a similar manner. For example, delta variable 2 chains can be referred to as Vδ2, while a nucleotide encoding a TCR chain containing this region or the TCR protein complex comprising this region may be referred to as "TRDV2". In preferred embodiments antibodies or fragments thereof which interact with the Vδ1 chain of a γδ TCR, do not interact with other delta chains such as Vδ2. In the invention, the antibodies are specific to TRDV1 and do not bind to TRDV2 (SEQ ID NO: 174) or other antigens, such as TRDV3 (SEQ ID NO: 175).

References to "gamma variable chains" are also made herein. These may be referred to as γ-chains or Vγ, while a nucleotide encoding a TCR chain containing this region or the TCR protein complex comprising this region may be referred to as TRGV. For example, TRGV4 refers to Vγ4 chain. In a preferred embodiments, antibodies or fragments thereof which interact with the Vδ1 chain of a γδ TCR, do not interact with gamma chains such as Vγ4 (TRGV4, SEQ ID NO: 173). In preferred embodiments, the antibodies also do not bind or interact with other domains found within a γδ TCR, such as TRDJ, TRDC, TRGJ or TRGC.

The term "T-cell receptor complex" is the complex of proteins comprising the "T-cell receptor" (or "TCR") found on the surface of T-cells responsible for recognising a variety of antigens. The T-cell receptor complex comprises either the alpha and beta chains of the T-cell receptor, or in the case of gamma delta T cells, the gamma and delta chains of the T-cell receptor, and up to 6 additional chains or more, such as CD3δ, CD3y, CD3ε and CD3ζ, although the precise makeup of T-cell receptor complexes can vary. The T-cell receptor complex mediates intracellular signalling in the T-cell, which may lead to T-cell activation.

The term "antibody" includes any antibody protein construct comprising at least one antibody variable domain comprising at least one antigen-binding site (ABS). Antibodies include, but are not limited to, immunoglobulins of types IgA, IgG, IgE, IgD, IgM (as well as subtypes thereof). The overall structure of Immunoglobulin G (IgG) antibodies assembled from two identical heavy (H)-chain and two identical light (L)-chain polypeptides is well established and highly conserved in mammals (Padlan (1994) Mol. Immunol. 31:169-217).

A conventional antibody or immunoglobulin (Ig) is a protein comprising four polypeptide chains: two heavy (H) chains and two light (L) chains. Each chain is divided into a constant region and a variable domain. The heavy (H) chain variable domains are abbreviated herein as VH, and the light (L) chain variable domains are abbreviated herein as VL. These domains, domains related thereto and domains derived therefrom, may be referred to herein as immunoglobulin chain variable domains. The VH and VL domains (also referred to as VH and VL regions) can be further subdivided into regions, termed "complementarity determining regions" ("CDRs"), interspersed with regions that are more conserved, termed "framework regions" ("FRs"). The framework and complementarity determining regions have been precisely defined (Kabat et al. Sequences of Proteins of Immunological Interest, Fifth Edition U.S. Department of Health and Human Services, (1991) NIH Publication Number 91-3242). There are also alternative numbering conventions for CDR sequences, for example those set out in Chothia et al. (1989) Nature 342: 877-883 or as summarized by IMGT.org In a conventional antibody, each VH and VL is composed of three CDRs and four FRs, arranged from amino-terminus to carboxy-terminus in the following order: FR1, CDR1, FR2, CDR2, FR3, CDR3, FR4. The conventional antibody tetramer of two heavy immunoglobulin chains and two light immunoglobulin chains is formed with the heavy and the light immunoglobulin chains inter-connected by *e.g.* disulphide bonds, and the heavy chains similarly connected. The heavy chain constant region includes three domains, CH1, CH2 and CH3. The light chain constant region is comprised of one domain, CL. The variable domain of the heavy chains and the variable domain of the light chains are binding domains that interact with an antigen. The constant regions of the antibodies typically mediate the binding of the antibody to host tissues or factors, including various cells of the immune system (*e.g.* effector cells) and the first component (C1q) of the classical complement system.

A fragment of the antibody (which may also referred to as "antibody fragment", "immunoglobulin fragment", "antigen-binding fragment" or "antigen-binding polypeptide") as used herein refers to a portion of an antibody (or constructs that contain said portion) that specifically binds to the target, the delta variable 1 (Vδ1) chain of a γδ T cell receptor (e.g. a molecule in which one or more immunoglobulin chains is not full length, but which specifically binds to the target). Examples of binding fragments encompassed within the term antibody fragment include:

(i) a Fab fragment (a monovalent fragment consisting of the VL, VH, CL and CH1 domains);
(ii) a F(ab')2 fragment (a bivalent fragment consisting of two Fab fragments linked by a disulphide bridge at the hinge region);
(iii) a Fd fragment (consisting of the VH and CH1 domains);
(iv) a Fv fragment (consisting of the VL and VH domains of a single arm of an antibody);
(v) a single chain variable fragment, scFv (consisting of VL and VH domains joined, using recombinant methods, by a synthetic linker that enables them to be made as a single protein chain in which the VL and VH regions pair to form monovalent molecules);
(vi) a VH (an immunoglobulin chain variable domain consisting of a VH domain);

(vii) a VL (an immunoglobulin chain variable domain consisting of a VL domain);
(viii) a domain antibody (dAb, consisting of either the VH or VL domain);
(ix) a minibody (consisting of a pair of scFv fragments which are linked via CH3 domains); and
(x) a diabody (consisting of a noncovalent dimer of scFv fragments that consist of a VH domain from one antibody connected by a small peptide linker a VL domain from another antibody).

Fragments of multispecific antibodies referred to herein are antigen-binding fragments. More specifically, the antibody fragments of the multispecific antibodies bind the same antigens as bound by the full multispecific antibody. For example, a fragment of a multispecific antibody that specifically binds TRDV1 and a second antigen also specifically binds TRDV1 and the same second antigen.

"Human antibody" refers to antibodies having variable and constant regions derived from human germline immunoglobulin sequences. Human subjects administered with said human antibodies do not generate cross-species antibody responses (for example termed HAMA responses - human-anti-mouse antibody) to the primary amino acids contained within said antibodies. Said human antibodies may include amino acid residues not encoded by human germline immunoglobulin sequences (*e.g.* mutations introduced by random or site-specific mutagenesis or by somatic mutation), for example in the CDRs and in particular CDR3. However, the term is not intended to include antibodies in which CDR sequences derived from the germline of another mammalian species, such as a mouse, have been grafted onto human framework sequences. Human antibodies that are prepared, expressed, created or isolated by recombinant means, such as antibodies expressed using a recombinant expression vector transfected into a host cell, antibodies isolated from a recombinant, combinatorial human antibody library, antibodies isolated from an animal (e.g. a mouse) that is transgenic for human immunoglobulin genes or antibodies prepared, expressed, created or isolated by any other means that involves splicing of human immunoglobulin gene sequences to other DNA sequences, may also be referred to as "recombinant human antibodies".

Substituting at least one amino acid residue in the framework region of a non-human immunoglobulin variable domain with the corresponding residue from a human variable domain is referred to as "humanisation". Humanisation of a variable domain may reduce immunogenicity in humans.

"Specificity" refers to the number of different types of antigens or antigenic determinants to which a particular antibody or fragment thereof can bind. The specificity of an antibody is the ability of the antibody to recognise a particular antigen as a unique molecular entity and distinguish it from another. An antibody that "specifically binds" to an antigen or an epitope is a term well understood in the art. A molecule is said to exhibit "specific binding" if it reacts more frequently, more rapidly, with greater duration and/or with greater affinity with a particular target antigen or epitope, than it does with alternative targets. An antibody "specifically binds" to a target antigen or epitope if it binds with greater affinity, avidity, more readily, and/or with greater duration than it binds to other substances.

The antibodies of the present invention are multispecific antibodies. A "multispecific antibody" is an antibody that is capable of binding a plurality of different epitopes simultaneously or sequentially. Generally, the epitopes will not be on the same antigen. Hence a multispecific antibody has the capability to selectively bind to epitopes present on different antigens via a plurality of different binding domains. This contrasts with conventional mono-specific antibodies which do not have this capability. Rather, a "monospecific antibody" only has binding specific for one antigen, although they may have multiple binding sites for that one antigen (e.g. the valency of a full human IgG antibody is 2, and the valency of other antibodies may be higher, but if the antibody only recognises one antigen, it is still classed as a monospecific antibody). Hence, the multispecific antibodies of the invention bind multiple different antigens simultaneously and/or sequentially.

In preferred embodiments of the invention, the antibodies are bispecific antibodies. A "bispecific antibody" is an antibody that is capable of binding two different epitopes simultaneously and/or sequentially. Generally, the epitopes will not be on the same antigen. Hence bispecific antibodies have the capability to selectively bind to two different epitopes present on two different antigens via two different binding domains. This contrasts with conventional mono-specific antibodies which do not have this capability. Hence, the bispecific antibodies of the invention bind two different antigens simultaneously and/or sequentially.

"Affinity", represented by the equilibrium constant for the dissociation of an antigen with an antigen-binding polypeptide (KD), is a measure of the binding strength between an antigenic determinant and an antigen-binding site on the antibody (or fragment thereof): the lesser the value of the KD, the stronger the binding strength between an antigenic determinant and the antigen-binding polypeptide. Alternatively, the affinity can also be expressed as the affinity constant (KA), which is 1/KD. Affinity can be determined by known methods, depending on the specific antigen of interest. For example, KD may be determined by surface plasmon resonance.

Any KD value less than $10^{-6}$ is considered to indicate binding. Specific binding of an antibody, or fragment thereof, to an antigen or antigenic determinant can be determined in any suitable known manner, including, for example, Scatchard analysis and/or competitive binding assays, such as radioimmunoassays (RIA), enzyme immunoassays (EIA) and sandwich competition assays, equilibrium dialysis, equilibrium binding, gel filtration, ELISA, surface plasmon resonance, or spectroscopy (*e.g.* using a fluorescence assay) and the different variants thereof known in the art.

"Avidity" is the measure of the strength of binding between an antibody, or fragment thereof, and the pertinent antigen. Avidity is related to both the affinity between an antigenic determinant and its antigen binding site on the antibody and the number of pertinent binding sites present on the antibody.

*"In situ"* means in the natural or original place, instead of being moved to another place. For example, an *in situ* Vδ1+ cell in a patient refers to a vδ1 cell *in vivo,* as opposed to an *in vitro* or *ex vivo* cell.

"Human tissue Vδ1+ cells," and "haemopoietic and blood Vδ1+ cells" and "tumour infiltrating lymphocyte (TIL) Vδ1+ cells," are defined as Vδ1+ cells contained in or derived from either human tissue or the haemopoietic blood system or human tumours respectively. All said cell types can be identified by their (i) location or from where they are derived and (ii) their expression of the Vδ1+ TCR.

"Modulating antibodies" are antibodies that confer a measurable change including, but not limited to, a measurable change in cell cycle, and/or in cell number, and/or cell viability, and/or in one or more cell surface markers, and/or in the secretion of one or more secretory molecules (e.g., cytokines, chemokines, leukotrienes, etc.), and/or a function (such as cytotoxicity towards a target cell or diseased cell), upon contacting or binding to a cell expressing the target to which the antibody binds. A method of "modulating" a cell, or population thereof, refers to a method wherein in at least one measurable change in said cell or cells, or secretion therefrom, is triggered to generate one or more "modulated cells".

An "immune response" is a measurable change in at least one cell, or one cell-type, or one endocrine pathway, or one exocrine pathway, of the immune system (including but not limited to a cell-mediated response, a humoral response, a cytokine response, a chemokine response) upon addition of a modulating antibody.

An "immune cell" is defined as a cell of the immune system including, but not limited to, CD34+ cells, B-Cells, CD45+ (lymphocyte common antigen) cells, Alpha-Beta T-cells, Cytotoxic T-cells, Helper T-cells, Plasma Cells, Neutrophils, Monocytes, Macrophages, Red Blood Cells, Platelets, Dendritic Cells, Phagocytes, Granulocytes, Innate lymphoid cells, Natural Killer (NK) cells and Gamma Delta T-cells. Typically, immune cells are classified with the aid of combinatorial cell surface molecule analysis (e.g., via flow cytometry) to identify or group or cluster to differentiate immune cells into sub-populations. These can be then still further sub-divided with additional analysis. For example, CD45+ lymphocytes can further sub-divided into vδ positive populations and vδ negative populations.

"Model systems" are biological models or biological representations designed to aid in the understanding of how a medicine such as an antibody or fragment thereof may function as a medicament in the amelioration of a sign or symptom of disease. Such models typically include the use of *in vitro, ex vivo,* and *in vivo* diseased cells, non-diseased cells, healthy cells, effector cells, and tissues etc., and in which the performance of said medicaments are studied and compared.

"Diseased cells" exhibit a phenotype associated with the progression of a disease such as a cancer, an infection such as a viral infection, or an inflammatory condition or inflammatory disease. For example, a diseased cell may be a tumour cell, an autoimmune tissue cell or a virally infected cell. Accordingly said diseased cells may be defined as tumorous, or virally infected, or inflammatory.

"Healthy cells" refers to normal cells that are not diseased. They may also be referred to as "normal" or "non-diseased" cells. Non-diseased cells include non-cancerous, or non-infected, or non-inflammatory cells. Said cells are often employed alongside relevant diseased cells to determine the diseased cell specificity conferred by a medicament and/or better understand the therapeutic index of a medicament.

"Diseased-cell-specificity" is a measure of how effective an effector cell or population thereof, (such as, for example, a population of Vδ1+ cells) is at distinguishing and killing diseased cells, such as cancer cells, whilst sparing non-diseased or healthy cells. This potential can be measured in model systems and may involve comparing the propensity of an effector cell, or a population of effector cells, to selectively kill or lyse diseased cells versus the potential of said effector cell/s to kill or lyse non-diseased or healthy cells. Said diseased-cell-specificity can inform the potential therapeutic index of a medicament.

"Enhanced diseased-cell specificity" describes a phenotype of an effector cell such as, for example, a Vδ1+ cell, or population thereof, which has been modulated to further increase its capacity to specifically kill diseased cells. This enhancement can be measured in a variety of ways inclusive of fold-change, or percentage increase, in diseased-cell killing specificity or selectivity.

"ADCC" or "antibody-dependent cell-mediated cytotoxicity" describes an immune response to cells coated with antibodies bound to the surface antigens of the cell. It is a cell-mediated process, whereby an immune effector cell (such as a NK cell, for example) recognise cell bound antibodies, triggering degranulation and lysis of the target cell. Typically, this is mediated via Fc-Fcy interactions. The Fc region of the cell-bound antibody recruits effector cells expressing Fcy receptors (eg. NK cells), leading to effector cell degranulation and death of the target cell.

"Fc enabled" refers to an antibody that comprises a functional Fc region (fragment crystallizable region), i.e. a Fc region that has not been disabled by mutation or otherwise. Fc enabled antibodies demonstrate unattenuated Fc function. The Fc enabled antibody may comprise human IGHC heavy chain sequence as listed by IMGT that has not been modified or engineered or constructed to reduce binding to one or more Fc gamma receptors. For example, via IGHC hinge mutation or by construction of an antibody comprising heavy chain constant domains which are chimeric or hybrid for IgG1/IgG2A or IgG1/IgG4 IGHC sequences. Suitably, the antibody or fragment thereof (*i.e.* polypeptide) of the invention is isolated. An "isolated" polypeptide is one that is removed from its original environment. The term "isolated" may be used to refer to an antibody that is substantially free of other antibodies having different antigenic specificities (*e.g.* an isolated antibody that specifically binds Vδ1, or a fragment thereof, is substantially free of antibodies that specifically bind antigens other than Vδ1). The term "isolated" may also be used to refer to preparations where the isolated antibody is sufficiently pure to be administered therapeutically when formulated as an active ingredient of a pharmaceutical composition, or at least 70-80% (w/w) pure, more preferably, at least 80-90% (w/w) pure, even more preferably, 90-95% pure; and, most preferably, at least 95%, 96%, 97%, 98%, 99%, or 100% (w/w) pure.

Suitably, the polynucleotides used in the present invention are isolated. An "isolated" polynucleotide is one that is removed from its original environment. For example, a naturally-occurring polynucleotide is isolated if it is separated from some or all of the coexisting materials in the natural system. A polynucleotide is considered to be isolated if, for example, it is cloned into a vector that is not a part of its natural environment or if it is comprised within cDNA.

The antibody or fragment thereof may be a "functionally active variant" which also includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide. By way of non-limiting example, said functionally active variants may still function when the frameworks containing the CDRs are modified, when the CDRs themselves are modified, when said CDRs are grafted to alternate frameworks, or when N- or C-terminal extensions are incorporated. Further, CDR containing binding domains may be paired with differing partner chains such as those shared with another antibody. Upon sharing with so called 'common' light or 'common' heavy chains, said binding domains may still function. Further, said binding domains may function when multimerized. Further, 'antibodies or fragments thereof may also comprise functional variants wherein the VH or VL or constant domains have been modified away or towards a different canonical sequence (for example as listed at IMGT.org) and which still function.

For the purposes of comparing two closely-related polypeptide sequences, the "% sequence identity" between a first polypeptide sequence and a second polypeptide sequence may be calculated using NCBI BLAST v2.0, using standard settings for polypeptide sequences (BLASTP). For the purposes of comparing two closely-related polynucleotide sequences, the "% sequence identity" between a first nucleotide sequence and a second nucleotide sequence may be calculated using NCBI BLAST v2.0, using standard settings for nucleotide sequences (BLASTN).

Polypeptide or polynucleotide sequences are said to be the same as or "identical" to other polypeptide or polynucleotide sequences, if they share 100% sequence identity over their entire length. Residues in sequences are numbered from left to right, i.e. from N- to C- terminus for polypeptides; from 5' to 3' terminus for polynucleotides.

In some embodiments, any specified % sequence identity of a sequence is calculated without the sequences of all 6 CDRs of the antibody. For example, the anti-Vδ1 antibody or antigen-binding fragment thereof may comprise a variable heavy chain region sequence having at least 70%, 75%, 80%, 85%, 90%, 91%, 92%, 93%, 94%, 95%, 96%, 97%, 98% or 99% to a specified variable heavy chain region sequence and/or a variable light chain region sequence having at least 70%, 75%, 80%, 85%, 90%, 91%, 92%, 93%, 94%, 95%, 96%, 97%, 98% or 99% identity to a specified variable light chain region sequence, wherein any amino acid variations occur only in the framework regions of the variable heavy and light chain region sequences. In such embodiments, the anti-Vδ1 antibody or antigen-binding fragment thereof having certain sequence identities retain the complete heavy and light chain CDR1, CDR2 and CDR3 sequences of the corresponding anti-Vδ1 antibody or antigen-binding fragment thereof.

A "difference" between sequences refers to an insertion, deletion or substitution of a single amino acid residue in a position of the second sequence, compared to the first sequence. Two polypeptide sequences can contain one, two or more such amino acid differences. Insertions, deletions or substitutions in a second sequence which is otherwise identical (100% sequence identity) to a first sequence result in reduced % sequence identity. For example, if the identical sequences are 9 amino acid residues long, one substitution in the second sequence results in a sequence identity of 88.9%. If first and second polypeptide sequences are 9 amino acid residues long and share 6 identical residues, the first and second polypeptide sequences share greater than 66% identity (the first and second polypeptide sequences share 66.7% identity).

Alternatively, for the purposes of comparing a first, reference polypeptide sequence to a second, comparison polypeptide sequence, the number of additions, substitutions and/or deletions made to the first sequence to produce the second sequence may be ascertained. An "addition" is the addition of one amino acid residue into the sequence of the first polypeptide (including addition at either terminus of the first polypeptide). A "substitution" is the substitution of one amino acid residue in the sequence of the first polypeptide with one different amino acid residue. Said substitution may be conservative or non-conservative. A "deletion" is the deletion of one amino acid residue from the sequence of the first polypeptide (including deletion at either terminus of the first polypeptide).

Using the three letter and one letter codes the naturally occurring amino acids may be referred to as follows: glycine (G or Gly), alanine (A or Ala), valine (V or Val), leucine (L or Leu), isoleucine (I or Ile), proline (P or Pro), phenylalanine (F or Phe), tyrosine (Y or Tyr), tryptophan (W or Trp), lysine (K or Lys), arginine (R or Arg), histidine (H or His), aspartic acid (D or Asp), glutamic acid (E or Glu), asparagine (N orAsn), glutamine (Q or Gln), cysteine (C or Cys), methionine (M or Met), serine (S or Ser) and Threonine (T or Thr). Where a residue may be aspartic acid or asparagine, the symbols Asx or B may be used. Where a residue may be glutamic acid or glutamine, the symbols Glx or Z may be used. Where a residue may be any amino acid the symbol Xaa or X may be used. References to aspartic acid include aspartate, and glutamic acid include glutamate, unless the context specifies otherwise.

A "conservative" amino acid substitution is an amino acid substitution in which an amino acid residue is replaced with another amino acid residue of similar chemical structure and which is expected to have little influence on the function, activity or other biological properties of the polypeptide. Such conservative substitutions suitably are substitutions in which one amino acid within the following groups is substituted by another amino acid residue from within the same group:

| Group | Amino acid residue |
|---|---|
| Non-polar aliphatic | Glycine |
| | Alanine |
| | Valine |
| | Methionine |
| | Leucine |
| | Isoleucine |
| Aromatic | Phenylalanine |
| | Tyrosine |
| | Tryptophan |
| Polar uncharged | Serine |
| | Threonine |
| | Cysteine |
| | Proline |
| | Asparagine |
| | Glutamine |
| Negatively charged | Aspartate |
| | Glutamate |
| Positively charged | Lysine |
| | Arginine |
| | Histidine |

Suitably, a hydrophobic amino acid residue is a non-polar amino acid. More suitably, a hydrophobic amino acid residue is selected from V, I, L, M, F, W or C. In some embodiments, a hydrophobic amino acid residue is selected from glycine, alanine, valine, methionine, leucine, isoleucine, phenylalanine, tyrosine, or tryptophan.

As used herein, numbering of polypeptide sequences and definitions of CDRs and FRs are as defined according to the EU and/or IMGT numbering system, as indicated in context. A "corresponding" amino acid residue between a first and second polypeptide sequence is an amino acid residue in a first sequence affinity which shares the same position according to the EU and/or IMGT numbering system, as indicated in context, with an amino acid residue in a second sequence, whilst the amino acid residue in the second sequence may differ in identity from the first. Suitably corresponding residues will share the same number (and letter) if the framework and CDRs are the same length according to EU or IMGT definition. Alignment can be achieved manually or by using, for example, a known computer algorithm for sequence alignment such as NCBI BLAST v2.0 (BLASTP or BLASTN) using standard settings.

References herein to an "epitope" refer to the portion of the target which is specifically bound by the antibody or fragment thereof. Epitopes may also be referred to as "antigenic determinants". An antibody binds "essentially the same epitope" as another antibody when they both recognize identical or sterically overlapping epitopes. Commonly used methods to determine whether two antibodies bind to identical or overlapping epitopes are competition assays, which can be configured in a number of different formats (*e.g.* well plates using radioactive or enzyme labels, or flow cytometry on antigen-expressing cells) using either labelled antigen or labelled antibody. An antibody binds "the same epitope" as another antibody when they both recognize identical epitopes (i.e. all contact points between the antigen and the antibody are the same). For example, an antibody may bind the same epitope as another antibody when all contact points across a specified region of an antigen are identified as the same with aid of a characterization method such as antibody/antigen cross-linking-coupled MS , HDX, X-ray crystallography, cryo-EM, or mutagenesis.

Further, with aid of such characterization methods, it is also possible to characterize antibodies which bind essentially the same epitope by recognizing some but not all of the identical contact points. Specifically, such antibodies may share a sufficient number of identical contact points in a specified antigenic region to deliver a broadly equivalent technical effect and/or equivalent antigen interaction selectivity. Additionally, in some instances whereby antibodies recognize essentially the same epitope and confer a broadly equivalent technical effect and/or interaction selectivity, it can also be useful to define the epitope binding footprint by the totality of antigen contacts inclusive of the most N-terminal antigen contact point through to the most C-terminal antigen contact point.

Epitopes found on protein targets may be defined as "linear epitopes" or "conformational epitopes". Linear epitopes are formed by a continuous sequence of amino acids in a protein antigen. Conformational epitopes are formed of amino acids that are discontinuous in the protein sequence, but which are brought together upon folding of the protein into its three-dimensional structure.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g. bacterial vectors having a bacterial origin of replication and episomal mammalian and yeast vectors). Other vectors (e.g. non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g. replication defective retroviruses, adenoviruses and adeno-associated viruses), which serve equivalent functions, and also bacteriophage and phagemid systems. The term "recombinant host cell" (or simply "host cell"), as used herein, is intended to refer to a cell into which a recombinant expression vector has been introduced. Such terms are intended to refer not only to the particular subject cell but to the progeny of such a cell, for example, when said progeny are employed to make a cell line or cell bank which is then optionally stored, provided, sold, transferred, or employed to manufacture an antibody or multispecific antibody or fragment thereof as described herein.

References to "subject", "patient" or "individual" refer to a subject, in particular a mammalian subject, to be treated. Mammalian subjects include humans, non-human primates, farm animals (such as cows), sports animals, or pet animals, such as dogs, cats, guinea pigs, rabbits, rats or mice. In some embodiment, the subject is a human. In alternative embodiments, the subject is a non-human mammal, such as a mouse.

The term "sufficient amount" means an amount sufficient to produce a desired effect. The term "therapeutically effective amount" is an amount that is effective to ameliorate a symptom of a disease or disorder. A therapeutically effective amount can be a "prophylactically effective amount" as prophylaxis can be considered therapy.

A disease or disorder is "ameliorated" if the severity of a sign or symptom of the disease or disorder, the frequency with which such a sign or symptom is experienced by a subject, or both, is reduced.

As used herein, "treating a disease or disorder" means reducing the frequency and/or severity of at least one sign or symptom of the disease or disorder experienced by a subject.

"Cancer," as used herein, refers to the abnormal growth or division of cells. Generally, the growth and/or life span of a cancer cell exceeds, and is not coordinated with, that of the normal cells and tissues around it. Cancers may be benign, pre-malignant or malignant. Cancer occurs in a variety of cells and tissues, including the oral cavity (e.g., mouth, tongue, pharynx, etc.), digestive system (e.g., esophagus, stomach, small intestine, colon, rectum, liver, bile duct, gall bladder, pancreas, etc.), respiratory system (e.g., larynx, lung, bronchus, etc.), bones, joints, skin (e.g., basal cell, squamous cell, meningioma, etc.), breast, genital system, (e.g., uterus, ovary, prostate, testis, etc.), urinary system (e.g., bladder, kidney, ureter, etc.), eye, nervous system (e.g., brain, etc.), endocrine system (e.g., thyroid, etc.), and hematopoietic system (e.g., lymphoma, myeloma, leukemia, acute lymphocytic leukemia, chronic lymphocytic leukemia, acute myeloid leukemia, chronic myeloid leukemia, etc.).

As used herein, the term "about" when used herein includes up to and including 10% greater and up to and including 10% lower than the value specified, suitably up to and including 5% greater and up to and including 5% lower than the value specified, especially the value specified. The term "between", includes the values of the specified boundaries.

**Multispecific antibodies or fragments thereof**

Provided herein are multispecific (suitably bispecific) antibodies or fragments thereof capable of specifically binding to the delta variable 1 chain (Vδ1) of a γδ T Cell Receptor (TCR), and another antigen. The invention relates to the use of said multispecific antibodies as medicaments for administration to a subject to be treated.

In one embodiment, the multispecific antibody or fragment thereof is an scFv, Fab, Fab', F(ab')2, Fv, variable domain (*e.g.* VH or VL), diabody, minibody or monoclonal antibody. In a further embodiment, the antibody or fragment thereof is an scFv.

Multispecific antibodies of the invention can be of any class, *e.g.* IgG, IgA, IgM, IgE, IgD, or isotypes thereof, and can comprise a kappa or lambda light chain. In one embodiment, the multispecific antibody is an IgG antibody, for example, at least one of isotypes, IgG1, IgG2, IgG3 or IgG4. In a further embodiment, the multispecific antibody may be in a format, such as an IgG format, that has been modified to confer desired properties, such as having the Fc mutated to reduce effector function, extend half-life, alter ADCC, or improve hinge stability. Such modifications are well known in the art.

In one embodiment, the multispecific antibody or fragment thereof is human. Thus, the multispecific antibody or fragment thereof may be derived from a human immunoglobulin (Ig) sequence. The CDR, framework and/or constant region of the antibody (or fragment thereof) may be derived from a human Ig sequence, in particular a human IgG sequence. The CDR, framework and/or constant region may be substantially identical for a human Ig sequence, in particular a human IgG sequence. An advantage of using human multispecific antibodies is that they are low or non-immunogenic in humans.

A multispecific antibody or fragment thereof can also be chimeric, for example a mouse-human antibody chimera.

Alternatively, the multispecific antibody or fragment thereof is derived from a non-human species, such as a mouse. Such non-human antibodies can be modified to increase their similarity to antibody variants produced naturally in humans, thus the antibody or fragment thereof can be partially or fully humanised. Therefore, in one embodiment, the antibody or fragment thereof is humanised.

**Multispecific antibody sequences**

The isolated anti-Vδ1 antibodies, or fragments thereof, of the invention may be described with reference to their CDR sequences that confer TRDV1 binding. However, the present invention is not limited to such sequences, since the invention is based on the entirely new principle of targeting TRDV1 using a multispecific antibody, for example to bind TRDV1 in a TCE platform or in a DI platform. Similarly, the present invention is not limited to specific sequences of domains that confer binding to the second antigen, wherein the second antigen is OX40.

According to one aspect of the invention, there is provided an isolated multispecific anti-Vδ1 antibody or fragment thereof, which comprises one or more of:

a CDR3 comprising a sequence having at least 80% sequence identity with any one of SEQ ID NOs: 2-25;
a CDR2 comprising a sequence having at least 80% sequence identity with any one of SEQ ID NOs: 26-37 and SEQ ID NOs: 160-171; and/or
a CDR1 comprising a sequence having at least 80% sequence identity with any one of SEQ ID NOs: 38-61.

According to one aspect of the invention, there is provided an isolated anti-Vδ1 multispecific antibody or fragment thereof, which comprises a CDR3 comprising a sequence having at least 80% sequence identity with any one of SEQ ID NOs: 2-25. In one embodiment, the multispecific antibody or fragment thereof comprises a CDR2 comprising a sequence having at least 80% sequence identity with any one of SEQ ID NOs: 26-37 and SEQ ID NOs: 160-171 . In one embodiment, the multispecific antibody or fragment thereof comprises a CDR1 comprising a sequence having at least 80% sequence identity with any one of SEQ ID NOs: 38-61.

In one embodiment, the multispecific antibody or fragment thereof comprises a CDR3 comprising a sequence having at least 85%, 90%, 95%, 97%, 98% or 99% sequence identity with any one of SEQ ID NOs: 2-25. In one embodiment, the multispecific antibody or fragment thereof comprises a CDR2 comprising a sequence having at least 85%, 90%, 95%, 97%, 98% or 99% sequence identity with any one of SEQ ID NOs: 26-37 and SEQ ID NOs: 160-171. In one embodiment, the multispecific antibody or fragment thereof comprises a CDR1 comprising a sequence having at least 85%, 90%, 95%, 97%, 98% or 99% sequence identity with any one of SEQ ID NOs: 38-61.

In one embodiment, the multispecific antibody or fragment thereof comprises a CDR3 consisting of a sequence having at least 85%, 90%, 95%, 97%, 98% or 99% sequence identity with any one of SEQ ID NOs: 2-25. In one embodiment, the multispecific antibody or fragment thereof comprises a CDR2 consisting of a sequence having at least 85%, 90%, 95%, 97%, 98% or 99% sequence identity with any one of SEQ ID NOs: 26-37 and SEQ ID NOs: 160-171. In one embodiment, the multispecific antibody or fragment thereof comprises a CDR1 consisting of a sequence having at least 85%, 90%, 95%, 97%, 98% or 99% sequence identity with any one of SEQ ID NOs: 38-61.

According to a further aspect of the invention, there is provided a multispecific antibody or fragment thereof, which comprises a VH region comprising a CDR3 comprising a sequence having at least 80% sequence identity with any one of SEQ ID NOs: 2-13 and/or a VL region comprising a CDR3 comprising a sequence having at least 80% sequence identity with any one of SEQ ID NOs: 14-25. According to a further aspect of the invention, there is provided a multispecific antibody or fragment thereof, which comprises a VH region comprising a CDR3 consisting of a sequence having at least 80% sequence identity with any one of SEQ ID NOs: 2-13 and/or a VL region comprising a CDR3 consisting of a sequence having at least 80% sequence identity with any one of SEQ ID NOs: 14-25.

According to a particular aspect of the invention, there is provided a multispecific antibody or fragment thereof, which comprises a VH region comprising a CDR3 comprising a sequence having at least 80% sequence identity with any one of SEQ ID NOs: 2-7, in particular 2-6, such as 2, 3 or 4 and/or a VL region comprising a CDR3 comprising a sequence having at least 80% sequence identity with any one of SEQ ID NOs: 14-19, in particular 14-18, such as 14, 15 or 16. According to another aspect of the invention, there is provided a multispecific antibody or fragment thereof, which comprises a VH region comprising a CDR3 consisting of a sequence having at least 80% sequence identity with any one of SEQ ID NOs: 2-7, in particular 2-6, such as 2, 3 or 4 and/or a VL region comprising a CDR3 consisting of a sequence having at least 80% sequence identity with any one of SEQ ID NOs: 14-19, in particular 14-18, such as 14, 15 or 16.

According to a particular aspect of the invention, there is provided a multispecific antibody or fragment thereof, which comprises a VH region comprising a CDR3 comprising a sequence having at least 80% sequence identity with any one of SEQ ID NOs: 8-13, in particular 8, 9, 10 or 11 and/or a VL region comprising a CDR3 comprising a sequence having at least 80% sequence identity with any one of SEQ ID NOs: 20-25, in particular 20, 21, 22 or 23. According to another aspect of the invention, there is provided a multispecific antibody or fragment thereof, which comprises a VH region comprising a CDR3 consisting of a sequence having at least 80% sequence identity with any one of SEQ ID NOs: 8-13, in particular 8, 9, 10 or 11 and/or a VL region comprising a CDR3 consisting of a sequence having at least 80% sequence identity with any one of SEQ ID NOs: 20-25, in particular 20, 21, 22 or 23.

According to a further aspect of the invention, there is provided a multispecific antibody or fragment thereof, which comprises a VH region comprising a CDR3 comprising a sequence having at least 90% sequence identity with any one of SEQ ID NOs: 2-13 and/or a VL region comprising a CDR3 comprising a sequence having at least 90% sequence identity with any one of SEQ ID NOs: 14-25. According to a further aspect of the invention, there is provided a multispecific antibody or fragment thereof, which comprises a VH region comprising a CDR3 consisting of a sequence having at least 90% sequence identity with any one of SEQ ID NOs: 2-13 and/or a VL region comprising a CDR3 consisting of a sequence having at least 90% sequence identity with any one of SEQ ID NOs: 14-25.

According to a particular aspect of the invention, there is provided a multispecific antibody or fragment thereof, which comprises a VH region comprising a CDR3 comprising a sequence having at least 90% sequence identity with any one of SEQ ID NOs: 2-7, in particular 2-6, such as 2, 3, 4 or 5 and/or a VL region comprising a CDR3 comprising a sequence having at least 90% sequence identity with any one of SEQ ID NOs: 14-19, in particular 14-18, such as 14, 15, 16 or 17. According to another aspect of the invention, there is provided a multispecific antibody or fragment thereof, which comprises a VH region comprising a CDR3 consisting of a sequence having at least 90% sequence identity with any one of SEQ ID NOs: 2-7, in particular 2-6, such as 2, 3, 4 or 5 and/or a VL region comprising a CDR3 consisting of a sequence having at least 90% sequence identity with any one of SEQ ID NOs: 14-19, in particular 14-18, such as 14, 15, 16 or 17.

According to a particular aspect of the invention, there is provided a multispecific antibody or fragment thereof, which comprises a VH region comprising a CDR3 comprising a sequence having at least 90% sequence identity with any one of SEQ ID NOs: 8, 9, 10 or 11 and/or a VL region comprising a CDR3 comprising a sequence having at least 90% sequence identity with any one of SEQ ID NOs: 20, 21, 22 or 23. According to another aspect of the invention, there is provided a multispecific antibody or fragment thereof, which comprises a VH region comprising a CDR3 consisting of a sequence having at least 90% sequence identity with any one of SEQ ID NOs: 8, 9, 10 or 11 and/or a VL region comprising a CDR3 consisting of a sequence having at least 90% sequence identity with any one of SEQ ID NOs: 20, 21, 22 or 23.

According to a further aspect of the invention, there is provided a multispecific antibody or fragment thereof, which comprises a VH region comprising a CDR3 comprising a sequence having at least 95% sequence identity with any one of SEQ ID NOs: 2-13 and/or a VL region comprising a CDR3 comprising a sequence having at least 95% sequence identity with any one of SEQ ID NOs: 14-25. According to a further aspect of the invention, there is provided a multispecific antibody or fragment thereof, which comprises a VH region comprising a CDR3 consisting of a sequence having at least 95% sequence identity with any one of SEQ ID NOs: 2-13 and/or a VL region comprising a CDR3 consisting of a sequence having at least 95% sequence identity with any one of SEQ ID NOs: 14-25.

According to a particular aspect of the invention, there is provided a multispecific antibody or fragment thereof, which comprises a VH region comprising a CDR3 comprising a sequence having at least 95% sequence identity with any one of SEQ ID NOs: 2-7, in particular 2-6, such as 2, 3, 4 or 5 and/or a VL region comprising a CDR3 comprising a sequence having at least 95% sequence identity with any one of SEQ ID NOs: 14-19, in particular 14-18, such as 14, 15, 16 or 17. According to another aspect of the invention, there is provided a multispecific antibody or fragment thereof, which comprises a VH region comprising a CDR3 consisting of a sequence having at least 95% sequence identity with any one of SEQ ID NOs: 2-7, in particular 2-6, such as 2, 3, 4 or 5 and/or a VL region comprising a CDR3 consisting of a sequence having at least 95% sequence identity with any one of SEQ ID NOs: 14-19, in particular 14-18, such as 14, 15, 16 or 17.

According to a particular aspect of the invention, there is provided a multispecific antibody or fragment thereof, which comprises a VH region comprising a CDR3 comprising a sequence having at least 95% sequence identity with any one of SEQ ID NOs: 8, 9, 10 or 11 and/or a VL region comprising a CDR3 comprising a sequence having at least 95% sequence identity with any one of SEQ ID NOs: 20, 21, 22 or 23. According to another aspect of the invention, there is provided a multispecific antibody or fragment thereof, which comprises a VH region comprising a CDR3 consisting of a sequence having at least 95% sequence identity with any one of SEQ ID NOs: 8, 9, 10 or 11 and/or a VL region comprising a CDR3 consisting of a sequence having at least 95% sequence identity with any one of SEQ ID NOs: 20, 21, 22 or 23.

According to a further aspect of the invention, there is provided a multispecific antibody or fragment thereof, which comprises a VH region comprising a CDR3 comprising a sequence having at least 80% sequence identity with any one of SEQ ID NOs: 2-13 and a VL region comprising a CDR3 comprising a sequence having at least 80% sequence identity with any one of SEQ ID NOs: 14-25. According to a further aspect of the invention, there is provided a multispecific antibody or fragment thereof, which comprises a VH region comprising a CDR3 consisting of a sequence having at least 80% sequence identity with any one of SEQ ID NOs: 2-13 and a VL region comprising a CDR3 consisting of a sequence having at least 80% sequence identity with any one of SEQ ID NOs: 14-25.

Embodiments which refer herein to "at least 80%" or "80% or greater", will be understood to include all values equal to or greater than 80%, such as 85%, 90%, 95%, 97%, 98%, 99% or 100% sequence identity. In one embodiment, the antibody or fragment of the invention comprises at least 85%, such as at least 90%, at least 95%, at least 97%, at least 98% or at least 99% sequence identity to the specified sequence.

Instead of percentage sequence identity, the embodiments may also be defined with one or more amino acid changes, for examples one or more additions, substitutions and/or deletions. In one embodiment, the sequence may comprise up to five amino acid changes, such as up to three amino acid changes, in particular up to two amino acid changes. In a further embodiment, the sequence may comprise up to five amino acid substitutions, such as up to three amino acid substitutions, in particular up to one or two amino acid substitutions. For example, CDR3 of the multispecific antibody or fragment thereof of the present invention comprises or more suitably consists of a sequence having no more than 2, more suitably no more than 1 substitution(s) compared to any one of SEQ ID NOs: 2-25.

Suitably any residues of CDR1, CDR2 or CDR3 differing from their corresponding residues in SEQ ID NO: 2-61 and SEQ ID NOs: 160-171 are conservative substitutions with respect to their corresponding residues. For example, any residues of CDR3 differing from their corresponding residues in SEQ ID NOs: 2-25 are conservative substitutions with respect to their corresponding residues.

In one embodiment, the multispecific antibody or fragment thereof comprises:

(i) a VH region comprising a CDR3 comprising a sequence having at least 80% sequence identity with any one of SEQ ID NOs: 2-13;
(ii) a VH region comprising a CDR2 comprising a sequence having at least 80% sequence identity with any one of SEQ ID NOs: 26-37;
(iii) a VH region comprising a CDR1 comprising a sequence having at least 80% sequence identity with any one of SEQ ID NOs: 38-49;
(iv) a VL region comprising a CDR3 comprising a sequence having at least 80% sequence identity with any one of SEQ ID NOs: 14-25;
(v) a VL region comprising a CDR2 comprising a sequence having at least 80% sequence identity with any one of SEQ ID NOs: 160-171; and/or
(vi) a VL region comprising a CDR1 comprising a sequence having at least 80% sequence identity with any one of SEQ ID NOs: 50-61.

In one embodiment, the multispecific antibody or fragment thereof comprises a heavy chain with:

(i) a VH region comprising a CDR3 comprising a sequence having at least 80% sequence identity with any one of SEQ ID NOs: 2-13;

(ii) a VH region comprising a CDR2 comprising a sequence having at least 80% sequence identity with any one of SEQ ID NOs: 26-37; and

(iii) a VH region comprising a CDR1 comprising a sequence having at least 80% sequence identity with any one of SEQ ID NOs: 38-49.

In one embodiment, the multispecific antibody or fragment thereof comprises a light chain with:

(i) a VL region comprising a CDR3 comprising a sequence having at least 80% sequence identity with any one of SEQ ID NOs: 14-25;

(ii) a VL region comprising a CDR2 comprising a sequence having at least 80% sequence identity with any one of SEQ ID NOs: 160-171; and

(iii) a VL region comprising a CDR1 comprising a sequence having at least 80% sequence identity with any one of SEQ ID NOs: 50-61.

In one embodiment, the multispecific antibody or fragment thereof comprises (orthe component (i.e. binding domain) of the multispecific antibody conferring TRDV1-binding consists of) a VH region comprising a CDR3 comprising a sequence having at least 80% sequence identity with any one of SEQ ID NOs: 2, 3, 4, 5 or 6, such as 2, 3, 4 or 5, in particular 2, 3 or 4. In one embodiment, the multispecific antibody or fragment thereof comprises (or the component of the multispecific antibody conferring TRDV1-binding consists of) a VH region comprising a CDR2 comprising a sequence having at least 80% sequence identity with any one of SEQ ID NOs: 26, 27, 28, 29 or 30, such as 26, 27, 28 or 29, in particular 26, 27 or 28. In one embodiment, the multispecific antibody or fragment thereof comprises (or the component of the multispecific antibody conferring TRDV1-binding consists of) a VH region comprising a CDR1 comprising a sequence having at least 80% sequence identity with any one of SEQ ID NOs: 38, 39, 40, 41 or 42, such as 38, 39, 40 or 41, in particular 38, 39 or 40.

In one embodiment, the multispecific antibody or fragment thereof comprises (or the component of the multispecific antibody conferring TRDV1-binding consists of) a VH region comprising a CDR3 comprising a sequence having at least 80% sequence identity with any one of SEQ ID NOs: 8, 9, 10 or 11. In one embodiment, the multispecific antibody or fragment thereof comprises (or the component of the multispecific antibody conferring TRDV1-binding consists of) a VH region comprising a CDR2 comprising a sequence having at least 80% sequence identity with any one of SEQ ID NOs: 32, 33, 34 or 35. In one embodiment, the multispecific antibody or fragment thereof comprises (or the component of the multispecific antibody conferring TRDV1-binding consists of) a VH region comprising a CDR1 comprising a sequence having at least 80% sequence identity with any one of SEQ ID NOs: 44, 45, 46 or 47.

In one embodiment, the VH region comprises a CDR3 comprising a sequence of SEQ ID NO: 2, a CDR2 comprising a sequence of SEQ ID NO: 26, and a CDR1 comprising a sequence of SEQ ID NO: 38. In one embodiment, the CDR3 consists of a sequence of SEQ ID NO: 2, the CDR2 consists of a sequence of SEQ ID NO: 26, and the CDR1 consists of a sequence of SEQ ID NO: 38.

In one embodiment, the VH region comprises a CDR3 comprising a sequence of SEQ ID NO: 3, a CDR2 comprising a sequence of SEQ ID NO: 27, and a CDR1 comprising a sequence of SEQ ID NO: 39. In one embodiment, the CDR3 consists of a sequence of SEQ ID NO: 3, the CDR2 consists of a sequence of SEQ ID NO: 27, and the CDR1 consists of a sequence of SEQ ID NO: 39.

In one embodiment, the VH region comprises a CDR3 comprising a sequence of SEQ ID NO: 4, a CDR2 comprising a sequence of SEQ ID NO: 28, and a CDR1 comprising a sequence of SEQ ID NO: 40. In one embodiment, the CDR3 consists of a sequence of SEQ ID NO: 4, the CDR2 consists of a sequence of SEQ ID NO: 28, and the CDR1 consists of a sequence of SEQ ID NO: 40.

In one embodiment, the VH region comprises a CDR3 comprising a sequence of SEQ ID NO: 5, a CDR2 comprising a sequence of SEQ ID NO: 29, and a CDR1 comprising a sequence of SEQ ID NO: 41. In one embodiment, the CDR3 consists of a sequence of SEQ ID NO: 5, the CDR2 consists of a sequence of SEQ ID NO: 29, and the CDR1 consists of a sequence of SEQ ID NO: 41.

In one embodiment, the VH region comprises a CDR3 comprising a sequence of SEQ ID NO: 6, a CDR2 comprising a sequence of SEQ ID NO: 30, and a CDR1 comprising a sequence of SEQ ID NO: 42. In one embodiment, the CDR3 consists of a sequence of SEQ ID NO: 6, the CDR2 consists of a sequence of SEQ ID NO: 30, and the CDR1 consists of a sequence of SEQ ID NO: 42.

In one embodiment, the VH region comprises a CDR3 comprising a sequence of SEQ ID NO: 8, a CDR2 comprising a sequence of SEQ ID NO: 32, and a CDR1 comprising a sequence of SEQ ID NO: 44. In one embodiment, the CDR3 consists of a sequence of SEQ ID NO: 8, the CDR2 consists of a sequence of SEQ ID NO: 32, and the CDR1 consists of a sequence of SEQ ID NO: 44.

In one embodiment, the VH region comprises a CDR3 comprising a sequence of SEQ ID NO: 9, a CDR2 comprising a sequence of SEQ ID NO: 33, and a CDR1 comprising a sequence of SEQ ID NO: 45. In one embodiment, the CDR3 consists of a sequence of SEQ ID NO: 9, the CDR2 consists of a sequence of SEQ ID NO: 33, and the CDR1 consists of a sequence of SEQ ID NO: 45.

In one embodiment, the VH region comprises a CDR3 comprising a sequence of SEQ ID NO: 10, a CDR2 sequence of SEQ ID NO: 34, and a CDR1 sequence of SEQ ID NO: 46. In one embodiment, the CDR3 consists of a sequence of SEQ ID NO: 10, the CDR2 consists of a sequence of SEQ ID NO: 34, and the CDR1 consists of a sequence of SEQ ID NO: 46.

In one embodiment, the VH region comprises a CDR3 comprising a sequence of SEQ ID NO: 11, a CDR2 sequence of SEQ ID NO: 35, and a CDR1 sequence of SEQ ID NO: 47. In one embodiment, the CDR3 consists of a sequence of SEQ ID NO: 11, the CDR2 consists of a sequence of SEQ ID NO: 35, and the CDR1 consists of a sequence of SEQ ID NO: 47.

In one embodiment, the multispecific antibody or fragment thereof comprises (or the component of the multispecific antibody conferring TRDV1-binding consists of) a VL region comprising a CDR3 comprising a sequence having at least 80% sequence identity with any one of SEQ ID NOs: 14-25, such as SEQ ID NOs: 14, 15, 16, 17 or 18 such as 14, 15, 16 or 17, in particular 14, 15 or 16. In one embodiment, the multispecific antibody or fragment thereof comprises (or the component of the multispecific antibody conferring TRDV1-binding consists of) a VL region comprising a CDR2 comprising a sequence having at least 80% sequence identity with any one of SEQ ID NOs: 160-171 , such as SEQ ID NOs: 160, 161, 162, 163 or 164, such as 160, 161, 162 or 163, in particular 160, 161 or 162. In one embodiment, the multispecific antibody or fragment thereof comprises (or the component of the multispecific antibody conferring TRDV1 - binding consists of) a VL region comprising a CDR1 comprising a sequence having at least 80% sequence identity with any one of SEQ ID NOs: 50-61, such as SEQ ID NOs: 50, 51, 52, 53 or 54, such as 50, 51, 52 or 53, in particular 50, 51 or 52.

In one embodiment, the VL region comprises a CDR3 comprising a sequence of SEQ ID NO: 14, a CDR2 comprising a sequence of SEQ ID No: 160, and a CDR1 comprising a sequence of SEQ ID NO: 50. In one embodiment, the CDR3 consists of a sequence of SEQ ID NO: 14, the CDR2 consists of a sequence of SEQ ID No: 160, and the CDR1 consists of a sequence of SEQ ID NO: 50.

In one embodiment, the VL region comprises a CDR3 comprising a sequence of SEQ ID NO: 15, a CDR2 comprising a sequence of SEQ ID No: 161, and a CDR1 comprising a sequence of SEQ ID NO: 51. In one embodiment, the CDR3 consists of a sequence of SEQ ID NO: 15, the CDR2 consists of a sequence of SEQ ID No: 161, and the CDR1 consists of a sequence of SEQ ID NO: 51.

In one embodiment, the VL region comprises a CDR3 comprising a sequence of SEQ ID NO: 16, a CDR2 comprising a sequence of SEQ ID No: 162, and a CDR1 comprising a sequence of SEQ ID NO: 52. In one embodiment, the CDR3 consists of a sequence of SEQ ID NO: 16, the CDR2 consists of a sequence of SEQ ID No: 162, and the CDR1 consists of a sequence of SEQ ID NO: 52.

In one embodiment, the VL region comprises a CDR3 comprising a sequence of SEQ ID NO: 17, a CDR2 comprising a sequence of SEQ ID No: 163, and a CDR1 comprising a sequence of SEQ ID NO: 53. In one embodiment, the CDR3 consists of a sequence of SEQ ID NO: 17, the CDR2 consists of a sequence of SEQ ID No: 163, and the CDR1 consists of a sequence of SEQ ID NO: 53.

In one embodiment, the VL region comprises a CDR3 comprising a sequence of SEQ ID NO: 18, a CDR2 comprising a sequence of SEQ ID No: 164, and a CDR1 comprising a sequence of SEQ ID NO: 54. In one embodiment, the CDR3 consists of a sequence of SEQ ID NO: 18, the CDR2 consists of a sequence of SEQ ID No: 164, and the CDR1 consists of a sequence of SEQ ID NO: 54.

In one embodiment, the multispecific antibody or fragment thereof comprises (or the component of the multispecific antibody conferring TRDV1-binding consists of) a VL region comprising a CDR3 comprising a sequence having at least 80% sequence identity with any one of SEQ ID NOs: 20, 21, 22 or 23. In one embodiment, the multispecific antibody or fragment thereof comprises (or the component of the multispecific antibody conferring TRDV1-binding consists of) a VL region comprising a CDR2 comprising a sequence having at least 80% sequence identity with any one of SEQUENCES: 166, 167, 168 or 169. In one embodiment, the multispecific antibody or fragment thereof comprises (or the component of the multispecific antibody conferring TRDV1-binding consists of) a VL region comprising a CDR1 comprising a sequence having at least 80% sequence identity with any one of SEQ ID NOs: 56, 57, 58 or 59.

In one embodiment, the VL region comprises a CDR3 comprising a sequence of SEQ ID NO: 20, a CDR2 comprising a sequence of SEQ ID No: 166, and a CDR1 comprising a sequence of SEQ ID NO: 56. In one embodiment, the CDR3 consists of a sequence of SEQ ID NO: 20, the CDR2 consists of a sequence of SEQ ID No: 166, and the CDR1 consists of a sequence of SEQ ID NO: 56.

In one embodiment, the VL region comprises a CDR3 comprising a sequence of SEQ ID NO: 21, a CDR2 comprising a sequence of SEQ ID No: 167, and a CDR1 comprising a sequence of SEQ ID NO: 57. In one embodiment, the CDR3 consists of a sequence of SEQ ID NO: 21, the CDR2 consists of a sequence of SEQ ID No: 167, and the CDR1 consists of a sequence of SEQ ID NO: 57.

In one embodiment, the VL region comprises a CDR3 comprising a sequence of SEQ ID NO: 22, a CDR2 comprising a sequence of SEQ ID No: 168, and a CDR1 comprising a sequence of SEQ ID NO: 58. In one embodiment, the CDR3 consists of a sequence of SEQ ID NO: 22, the CDR2 consists of a sequence of SEQ ID No: 168, and the CDR1 consists of a sequence of SEQ ID NO: 58.

In one embodiment, the VL region comprises a CDR3 comprising a sequence of SEQ ID NO: 23, a CDR2 comprising a sequence of SEQ ID No: 169, and a CDR1 comprising a sequence of SEQ ID NO: 59. In one embodiment, the CDR3 consists of a sequence of SEQ ID NO: 23, the CDR2 consists of a sequence of SEQ ID No: 169, and the CDR1 consists of a sequence of SEQ ID NO: 59.

In one embodiment, the VH region comprises a CDR3 comprising a sequence of SEQ ID NO: 2, a CDR2 comprising a sequence of SEQ ID NO: 26, a CDR1 comprising a sequence of SEQ ID NO: 38, and the VL region comprises a CDR3 comprising a sequence of SEQ ID NO: 14, a CDR2 comprising a sequence of SEQ ID No: 160, and a CDR1 comprising a sequence of SEQ ID NO: 50. In one embodiment, the HCDR3 consists of a sequence of SEQ ID NO: 2, the HCDR2 consists of a sequence of SEQ ID NO: 26, the HCDR1 consists of a sequence of SEQ ID NO: 38, the LCDR3 consists of a sequence of SEQ ID NO: 14, the LCDR2 consists of a sequence of SEQ ID No: 160, and the LCDR1 consists of a sequence of SEQ ID NO: 50.

In one embodiment, the VH region comprises a CDR3 comprising a sequence of SEQ ID NO: 3, a CDR2 comprising a sequence of SEQ ID NO: 27, a CDR1 comprising a sequence of SEQ ID NO: 39, and the VL region comprises a CDR3 comprising a sequence of SEQ ID NO: 15, a CDR2 comprising a sequence of SEQ ID No: 161, and a CDR1 comprising a sequence of SEQ ID NO: 51. In one embodiment, the HCDR3 consists of a sequence of SEQ ID NO: 3, the HCDR2 consists of a sequence of SEQ ID NO: 27, the HCDR1 consists of a sequence of SEQ ID NO: 39, the LCDR3 consists of a sequence of SEQ ID NO: 15, the LCDR2 consists of a sequence of SEQ ID No: 161, and the LCDR1 consists of a sequence of SEQ ID NO: 51.

In one embodiment, the VH region comprises a CDR3 comprising a sequence of SEQ ID NO: 4, a CDR2 comprising a sequence of SEQ ID NO: 28, a CDR1 comprising a sequence of SEQ ID NO: 40, and the VL region comprises a CDR3 comprising a sequence of SEQ ID NO: 16, a CDR2 comprising a sequence of SEQ ID No: 162, and a CDR1 comprising a sequence of SEQ ID NO: 52. In one embodiment, the HCDR3 consists of a sequence of SEQ ID NO: 4, the HCDR2 consists of a sequence of SEQ ID NO: 28, the HCDR1 consists of a sequence of SEQ ID NO: 40, the LCDR3 consists of a sequence of SEQ ID NO: 16, the LCDR2 consists of a sequence of SEQ ID No: 162, and the LCDR1 consists of a sequence of SEQ ID NO: 52.

In one embodiment, the VH region comprises a CDR3 comprising a sequence of SEQ ID NO: 5, a CDR2 comprising a sequence of SEQ ID NO: 29, a CDR1 comprising a sequence of SEQ ID NO: 41, and the VL region comprises a CDR3 comprising a sequence of SEQ ID NO: 17, a CDR2 comprising a sequence of SEQ ID No: 163, and a CDR1 comprising a sequence of SEQ ID NO: 53. In one embodiment, the HCDR3 consists of a sequence of SEQ ID NO: 5, the HCDR2 consists of a sequence of SEQ ID NO: 29, the HCDR1 consists of a sequence of SEQ ID NO: 41, the LCDR3 consists of a sequence of SEQ ID NO: 17, the LCDR2 consists of a sequence of SEQ ID No: 163, and the LCDR1 consists of a sequence of SEQ ID NO: 53.

In one embodiment, the VH region comprises a CDR3 comprising a sequence of SEQ ID NO: 6, a CDR2 comprising a sequence of SEQ ID NO: 30, a CDR1 comprising a sequence of SEQ ID NO: 42, and the VL region comprises a CDR3 comprising a sequence of SEQ ID NO: 18, a CDR2 comprising a sequence of SEQ ID No: 164, and a CDR1 comprising a sequence of SEQ ID NO: 54. In one embodiment, the HCDR3 consists of a sequence of SEQ ID NO: 6, the HCDR2 consists of a sequence of SEQ ID NO: 30, the HCDR1 consists of a sequence of SEQ ID NO: 42, the LCDR3 consists of a sequence of SEQ ID NO: 18, the LCDR2 consists of a sequence of SEQ ID No: 164, and the LCDR1 consists of a sequence of SEQ ID NO: 54.

In one embodiment, the VH region comprises a CDR3 comprising a sequence of SEQ ID NO: 7, a CDR2 comprising a sequence of SEQ ID NO: 31, a CDR1 comprising a sequence of SEQ ID NO: 43, and the VL region comprises a CDR3 comprising a sequence of SEQ ID NO: 19, a CDR2 comprising a sequence of SEQ ID No: 165, and a CDR1 comprising a sequence of SEQ ID NO: 55. In one embodiment, the HCDR3 consists of a sequence of SEQ ID NO: 7, the HCDR2 consists of a sequence of SEQ ID NO: 31, the HCDR1 consists of a sequence of SEQ ID NO: 43, the LCDR3 consists of a sequence of SEQ ID NO: 19, the LCDR2 consists of a sequence of SEQ ID No: 165, and the LCDR1 consists of a sequence of SEQ ID NO: 55.

In one embodiment, the VH region comprises a CDR3 comprising a sequence of SEQ ID NO: 8, a CDR2 comprising a sequence of SEQ ID NO: 32, a CDR1 comprising a sequence of SEQ ID NO: 44, and the VL region comprises a CDR3 comprising a sequence of SEQ ID NO: 20, a CDR2 comprising a sequence of SEQ ID No: 166, and a CDR1 comprising a sequence of SEQ ID NO: 56. In one embodiment, the HCDR3 consists of a sequence of SEQ ID NO: 8, the HCDR2 consists of a sequence of SEQ ID NO: 32, the HCDR1 consists of a sequence of SEQ ID NO: 44, the LCDR3 consists of a sequence of SEQ ID NO: 20, the LCDR2 consists of a sequence of SEQ ID No: 166, and the LCDR1 consists of a sequence of SEQ ID NO: 56.

In one embodiment, the VH region comprises a CDR3 comprising a sequence of SEQ ID NO: 9, a CDR2 comprising a sequence of SEQ ID NO: 33, a CDR1 comprising a sequence of SEQ ID NO: 45, and the VL region comprises a CDR3 comprising a sequence of SEQ ID NO: 21, a CDR2 comprising a sequence of SEQ ID No: 167, and a CDR1 comprising a sequence of SEQ ID NO: 57. In one embodiment, the HCDR3 consists of a sequence of SEQ ID NO: 9, the HCDR2 consists of a sequence of SEQ ID NO: 33, the HCDR1 consists of a sequence of SEQ ID NO: 45, the LCDR3 consists of a sequence of SEQ ID NO: 21, the LCDR2 consists of a sequence of SEQ ID No: 167, and the LCDR1 consists of a sequence of SEQ ID NO: 57.

In one embodiment, the VH region comprises a CDR3 comprising a sequence of SEQ ID NO: 10, a CDR2 comprising a sequence of SEQ ID NO: 34, a CDR1 comprising a sequence of SEQ ID NO: 46, and the VL region comprises a CDR3 comprising a sequence of SEQ ID NO: 22, a CDR2 comprising a sequence of SEQ ID No: 168, and a CDR1 comprising a sequence of SEQ ID NO: 58. In one embodiment, the HCDR3 consists of a sequence of SEQ ID NO: 10, the HCDR2 consists of a sequence of SEQ ID NO: 34, the HCDR1 consists of a sequence of SEQ ID NO: 46, the LCDR3 consists of a sequence of SEQ ID NO: 22, the LCDR2 consists of a sequence of SEQ ID No: 168, and the LCDR1 consists of a sequence of SEQ ID NO: 58.

In one embodiment, the VH region comprises a CDR3 comprising a sequence of SEQ ID NO: 11, a CDR2 comprising a sequence of SEQ ID NO: 35, a CDR1 comprising a sequence of SEQ ID NO: 47, and the VL region comprises a CDR3 comprising a sequence of SEQ ID NO: 23, a CDR2 comprising a sequence of SEQ ID No: 169, and a CDR1 comprising a sequence of SEQ ID NO: 59. In one embodiment, the HCDR3 consists of a sequence of SEQ ID NO: 11, the HCDR2 consists of a sequence of SEQ ID NO: 35, the HCDR1 consists of a sequence of SEQ ID NO: 47, the LCDR3 consists of a sequence of SEQ ID NO: 23, the LCDR2 consists of a sequence of SEQ ID No: 169, and the LCDR1 consists of a sequence of SEQ ID NO: 59.

In one embodiment, the VH region comprises a CDR3 comprising a sequence of SEQ ID NO: 12, a CDR2 comprising a sequence of SEQ ID NO: 36, a CDR1 comprising a sequence of SEQ ID NO: 48, and the VL region comprises a CDR3 comprising a sequence of SEQ ID NO: 24, a CDR2 comprising a sequence of SEQ ID No: 170, and a CDR1 comprising a sequence of SEQ ID NO: 60. In one embodiment, the HCDR3 consists of a sequence of SEQ ID NO: 12, the HCDR2 consists of a sequence of SEQ ID NO: 36, the HCDR1 consists of a sequence of SEQ ID NO: 48, the LCDR3 consists of a sequence of SEQ ID NO: 24, the LCDR2 consists of a sequence of SEQ ID No: 170, and the LCDR1 consists of a sequence of SEQ ID NO: 60.

In one embodiment, the VH region comprises a CDR3 comprising a sequence of SEQ ID NO: 13, a CDR2 comprising a sequence of SEQ ID NO: 37, a CDR1 comprising a sequence of SEQ ID NO: 49, and the VL region comprises a CDR3 comprising a sequence of SEQ ID NO: 25, a CDR2 comprising a sequence of SEQ ID No: 171, and a CDR1 comprising a sequence of SEQ ID NO: 61. In one embodiment, the HCDR3 consists of a sequence of SEQ ID NO: 13, the HCDR2 consists of a sequence of SEQ ID NO: 37, the HCDR1 consists of a sequence of SEQ ID NO: 49, the LCDR3 consists of a sequence of SEQ ID NO: 25, the LCDR2 consists of a sequence of SEQ ID No: 171, and the LCDR1 consists of a sequence of SEQ ID NO: 61.

In one embodiment, the multispecific antibody or fragment thereof comprises one or more CDR sequences as described in **Table 3.** In a further embodiment, the multispecific antibody or fragment thereof comprises one or more (such as all) CDR sequences of clone 1252_P01_C08 as described in **Table 3.** In an alternative embodiment, the multispecific antibody or fragment thereof comprises one or more (such as all) CDR sequences of clone 1245_P01_E07 as described in **Table 3.** In an alternative embodiment, the multispecific antibody or fragment thereof comprises one or more (such as all) CDR sequences of clone 1245_P02_G04 as described in **Table 3.** In an alternative embodiment, the multispecific antibody or fragment thereof comprises one or more (such as all) CDR sequences of clone 1245_P02_B07 as described in **Table 3.** In an alternative embodiment, the multispecific antibody or fragment thereof comprises one or more (such as all) CDR sequences of clone 1251_P02_C05 as described in **Table 3.** In an alternative embodiment, the multispecific antibody or fragment thereof comprises one or more (such as all) CDR sequences of clone 1139_P01_E04 as described in **Table 3.** In an alternative embodiment, the multispecific antibody or fragment thereof comprises one or more (such as all) CDR sequences of clone 1245_P02_F07 as described in **Table 3.** In an alternative embodiment, the multispecific antibody or fragment thereof comprises one or more (such as all) CDR sequences of clone 1245_P01_G06 as described in **Table 3.** In an alternative embodiment, the multispecific antibody or fragment thereof comprises one or more (such as all) CDR sequences of clone 1245_P01_G09 as described in **Table 3.** In an alternative embodiment, the multispecific antibody or fragment thereof comprises one or more (such as all) CDR sequences of clone 1138_P01_B09 as described in **Table 3.** In an alternative embodiment, the multispecific antibody or fragment thereof comprises one or more (such as all) CDR sequences of clone 1251_P02_G10 as described in **Table 3.**

Suitably the VH and VL regions recited above each comprise four framework regions (FR1-FR4). In one embodiment, the multispecific antibody or fragment thereof comprises a framework region (e.g. FR1, FR2, FR3 and/or FR4) comprising a sequence having at least 80% sequence identity with the framework region in any one of SEQ ID NOs: 62-85. In one embodiment, the multispecific antibody or fragment thereof comprises a framework region (e.g. FR1, FR2, FR3 and/or FR4) comprising a sequence having at least 90%, such as at least 95%, 97% or 99% sequence identity with the framework region in any one of SEQ ID NOs: 62-85. In one embodiment, the multispecific antibody or fragment thereof comprises a framework region (e.g. FR1, FR2, FR3 and/or FR4) comprising a sequence in any one of SEQ ID NOs: 62-85. In one embodiment, the multispecific antibody or fragment thereof comprises a framework region (e.g. FR1, FR2, FR3 and/or FR4) consisting of a sequence in any one of SEQ ID NOs: 62-85.

The antibodies described herein may be defined by full light chain and/or heavy chain variable sequences that confer TRDV1 binding. Therefore, according to a further aspect of the invention, there is provided an isolated multispecific anti-Vδ1 antibody or fragment thereof, which comprises an amino acid sequence having at least 80% sequence identity with any one of SEQ ID NOs: 62-85. According to a further aspect of the invention, there is provided an isolated multispecific anti-Vδ1 antibody or fragment thereof, in which the component of the multispecific antibody conferring TRDV1-binding consists of an amino acid sequence having at least 80% sequence identity with any one of SEQ ID NOs: 62-85.

In one embodiment, the multispecific antibody or fragment thereof comprises a VH region comprising an amino acid sequence having at least 80% sequence identity with any one of SEQ ID NOs: 62-73. In one embodiment, the multispecific antibody or fragment thereof comprises a VH region consisting of an amino acid sequence having at least 80% sequence identity with any one of SEQ ID NOs: 62-73. In a further embodiment, the VH region comprises an amino acid sequence having at least 80% sequence identity with any one of SEQ ID NOs: 62, 63, 64, 65 or 66, such as 62, 63, 64 or 65, in particular 62, 63 or 64. In a further embodiment, the VH region consists of an amino acid sequence having at least 80% sequence identity with any one of SEQ ID NOs: 62, 63, 64, 65 or 66, such as 62, 63, 64 or 65, in particular 62, 63 or 64. In a further embodiment, the VH region comprises an amino acid sequence having at least 80% sequence identity with any one of SEQ ID NOs: 68, 69, 70, 71, 72 or 73, such as 68, 69, 70 or 71. In a further embodiment, the VH region consists of an amino acid sequence having at least 80% sequence identity with any one of SEQ ID NOs: 68, 69, 70, 71, 72 or 73, such as 68, 69, 70 or 71.

In one embodiment, the multispecific antibody or fragment thereof comprises a VL region comprising an amino acid sequence having at least 80% sequence identity with any one of SEQ ID NOs: 74-85. In one embodiment, the multispecific antibody or fragment thereof comprises a VL region consisting of an amino acid sequence having at least 80% sequence identity with any one of SEQ ID NOs: 74-85. In a further embodiment, the VL region comprises an amino acid sequence having at least 80% sequence identity with any one of SEQ ID NOs: 74, 75, 76, 77 or 78, such as 74, 75, 76 or 77, in particular 74, 75, or 76. In a further embodiment, the VL region consists of an amino acid sequence having at least 80% sequence identity with any one of SEQ ID NOs: 74, 75, 76, 77 or 78, such as 74, 75, 76 or 77, in particular 74, 75, or 76. In a further embodiment, the VL region comprises an amino acid sequence having at least 80% sequence identity with any one of SEQ ID NOs: 80, 81, 82, 83, 84 or 85, such as 80, 81, 82 or 83. In a further embodiment, the VL region consists of an amino acid sequence having at least 80% sequence identity with any one of SEQ ID NOs: 80, 81, 82, 83, 84 or 85, such as 80, 81, 82 or 83.

In a further embodiment, the multispecific antibody or fragment thereof comprises a VH region comprising an amino acid sequence having at least 80% sequence identity with any one of SEQ ID NOs: 62-73 and a VL region comprising an amino acid sequence having at least 80% sequence identity with any one of SEQ ID NOs: 74-85. In a further embodiment, the multispecific antibody or fragment thereof comprises a VH region consisting of an amino acid sequence having at least 80% sequence identity with any one of SEQ ID NOs: 62-73 and a VL region consisting of an amino acid sequence having at least 80% sequence identity with any one of SEQ ID NOs: 74-85.

In a further embodiment, the multispecific antibody or fragment thereof comprises a VH region comprising an amino acid sequence having at least 95% sequence identity with any one of SEQ ID NOs: 62-73 and a VL region comprising an amino acid sequence having at least 95% sequence identity with any one of SEQ ID NOs: 74-85. In a further embodiment, the multispecific antibody or fragment thereof comprises a VH region consisting of an amino acid sequence having at least 95% sequence identity with any one of SEQ ID NOs: 62-73 and a VL region consisting of an amino acid sequence having at least 95% sequence identity with any one of SEQ ID NOs: 74-85.

In a further embodiment, the multispecific antibody or fragment thereof comprises a VH region comprising an amino acid sequence having at least 96% sequence identity with any one of SEQ ID NOs: 62-73 and a VL region comprising an amino acid sequence having at least 96% sequence identity with any one of SEQ ID NOs: 74-85. In a further embodiment, the multispecific antibody or fragment thereof comprises a VH region consisting of an amino acid sequence having at least 96% sequence identity with any one of SEQ ID NOs: 62-73 and a VL region consisting of an amino acid sequence having at least 96% sequence identity with any one of SEQ ID NOs: 74-85.

In one embodiment, the multispecific antibody or fragment thereof comprises a VH region comprising an amino acid sequence of SEQ ID NO: 63 (1252_P01_C08). In an alternative embodiment, the multispecific antibody or fragment thereof comprises a VH region comprising an amino acid sequence of SEQ ID NO: 62 (1245_P01_E07). In an alternative embodiment, the multispecific antibody or fragment thereof comprises a VH region comprising an amino acid sequence of SEQ ID NO: 64 (1245_P02_G04). In an alternative embodiment, the multispecific antibody or fragment thereof comprises a VH region comprising an amino acid sequence of SEQ ID NO: 68 (1139_P01_E04). In an alternative embodiment, the multispecific antibody or fragment thereof comprises a VH region comprising an amino acid sequence of SEQ ID NO: 69 (1245_P02_F07). In an alternative embodiment, the multispecific antibody or fragment thereof comprises a VH region comprising an amino acid sequence of SEQ ID NO: 70 (1245_P01_G06). In an alternative embodiment, the multispecific antibody or fragment thereof comprises a VH region comprising an amino acid sequence of SEQ ID NO:

71 (1245_P01_G09).

In one embodiment, the multispecific antibody or fragment thereof comprises a VH region consisting of an amino acid sequence of SEQ ID NO: 63 (1252_P01_C08). In an alternative embodiment, the multispecific antibody or fragment thereof comprises a VH region consisting of an amino acid sequence of SEQ ID NO: 62 (1245_P01_E07). In an alternative embodiment, the multispecific antibody or fragment thereof comprises a VH region consisting of an amino acid sequence of SEQ ID NO: 64 (1245_P02_G04). In an alternative embodiment, the multispecific antibody or fragment thereof comprises a VH region consisting of an amino acid sequence of SEQ ID NO: 68 (1139_P01_E04). In an alternative embodiment, the multispecific antibody or fragment thereof comprises a VH region consisting of an amino acid sequence of SEQ ID NO: 69 (1245_P02_F07). In an alternative embodiment, the multispecific antibody or fragment thereof comprises a VH region consisting of an amino acid sequence of SEQ ID NO: 70 (1245_P01_G06). In an alternative embodiment, the multispecific antibody or fragment thereof comprises a VH region consisting of an amino acid sequence of SEQ ID NO: 71 (1245_P01_G09).

In one embodiment, the multispecific antibody or fragment thereof comprises a VL region comprising an amino acid sequence of SEQ ID NO: 75 (1252_P01_C08). In an alternative embodiment, the multispecific antibody or fragment thereof comprises a VL region comprising an amino acid sequence of SEQ ID NO: 74 (1245_P01_E07). In an alternative embodiment, the multispecific antibody or fragment thereof comprises a VL region comprising an amino acid sequence of SEQ ID NO: 76 (1245_P02_G04). In an alternative embodiment, the multispecific antibody or fragment thereof comprises a VL region comprising an amino acid sequence of SEQ ID NO: 80 (1139_P01_E04). In an alternative embodiment, the multispecific antibody or fragment thereof comprises a VL region comprising an amino acid sequence of SEQ ID NO: 81 (1245_P02_F07). In an alternative embodiment, the multispecific antibody or fragment thereof comprises a VL region comprising an amino acid sequence of SEQ ID NO: 82 (1245_P01_G06). In an alternative embodiment, the multispecific antibody or fragment thereof comprises a VL region comprising an amino acid sequence of SEQ ID NO: 83 (1245_P01_G09).

In one embodiment, the multispecific antibody or fragment thereof comprises a VL region consisting of an amino acid sequence of SEQ ID NO: 75 (1252_P01_C08). In an alternative embodiment, the multispecific antibody or fragment thereof comprises a VL region consisting of an amino acid sequence of SEQ ID NO: 74 (1245_P01_E07). In an alternative embodiment, the multispecific antibody or fragment thereof comprises a VL region consisting of an amino acid sequence of SEQ ID NO: 76 (1245_P02_G04). In an alternative embodiment, the multispecific antibody or fragment thereof comprises a VL region consisting of an amino acid sequence of SEQ ID NO: 80 (1139_P01_E04). In an alternative embodiment, the multispecific antibody or fragment thereof comprises a VL region consisting of an amino acid sequence of SEQ ID NO: 81 (1245_P02_F07). In an alternative embodiment, the multispecific antibody or fragment thereof comprises a VL region consisting of an amino acid sequence of SEQ ID NO: 82 (1245_P01_G06). In an alternative embodiment, the multispecific antibody or fragment thereof comprises a VL region consisting of an amino acid sequence of SEQ ID NO: 83 (1245_P01_G09).

In one embodiment, the multispecific antibody or fragment thereof comprises a VH region comprising an amino acid sequence of SEQ ID NO: 63 (1252_P01_C08) and a VL region comprising an amino acid sequence of SEQ ID NO: 75 (1252_P01_C08). In an alternative embodiment, the multispecific antibody or fragment thereof comprises a VH region comprising an amino acid sequence of SEQ ID NO: 62 (1245_P01_E07) and a VL region comprising an amino acid sequence of SEQ ID NO: 74 (1245_P01_E07). In an alternative embodiment, the multispecific antibody or fragment thereof comprises a VH region comprising an amino acid sequence of SEQ ID NO: 64 (1245_P02_G04) and a VL region comprising an amino acid sequence of SEQ ID NO: 76 (1245_P02_G04). In an alternative embodiment, the multispecific antibody or fragment thereof comprises a VH region comprising an amino acid sequence of SEQ ID NO: 68 (1139_P01_E04) and a VL region comprising an amino acid sequence of SEQ ID NO: 80 (1139_P01_E04). In an alternative embodiment, the multispecific antibody or fragment thereof comprises a VH region comprising an amino acid sequence of SEQ ID NO: 69 (1245_P02_F07) and a VL region comprising an amino acid sequence of SEQ ID NO: 81 (1245_P02_F07). In an alternative embodiment, the multispecific antibody or fragment thereof comprises a VH region comprising an amino acid sequence of SEQ ID NO: 70 (1245_P01_G06) and a VL region comprising an amino acid sequence of SEQ ID NO: 82 (1245_P01_G06). In an alternative embodiment, the multispecific antibody or fragment thereof comprises a VH region comprising an amino acid sequence of SEQ ID NO: 71 (1245_P01_G06) and a VL region comprising an amino acid sequence of SEQ ID NO: 83 (1245_P01_G09).

In one embodiment, the multispecific antibody or fragment thereof comprises a VH region consisting of an amino acid sequence of SEQ ID NO: 63 (1252_P01_C08) and a VL region consisting of an amino acid sequence of SEQ ID NO: 75 (1252_P01_C08). In an alternative embodiment, the multispecific antibody or fragment thereof comprises a VH region consisting of an amino acid sequence of SEQ ID NO: 62 (1245_P01_E07) and a VL region consisting of an amino acid sequence of SEQ ID NO: 74 (1245_P01_E07). In an alternative embodiment, the multispecific antibody or fragment thereof comprises a VH region consisting of an amino acid sequence of SEQ ID NO: 64 (1245_P02_G04) and a VL region consisting of an amino acid sequence of SEQ ID NO: 76 (1245_P02_G04). In an alternative embodiment, the multispecific antibody or fragment thereof comprises a VH region consisting of an amino acid sequence of SEQ ID NO: 68 (1139_P01_E04) and a VL region consisting of an amino acid sequence of SEQ ID NO: 80 (1139_P01_E04). In an alternative embodiment, the multispecific antibody or fragment thereof comprises a VH region consisting of an amino acid sequence of SEQ ID NO: 69 (1245_P02_F07) and a VL region consisting of an amino acid sequence of SEQ ID NO: 81 (1245_P02_F07). In an alternative embodiment, the multispecific antibody or fragment thereof comprises a VH region consisting of an amino acid sequence of SEQ ID NO: 70 (1245_P01_G06) and a VL region consisting of an amino acid sequence of SEQ ID NO: 82 (1245_P01_G06). In an alternative embodiment, the multispecific antibody or fragment thereof comprises a VH region consisting of an amino acid sequence of SEQ ID NO: 71 (1245_P01_G06) and a VL region consisting of an amino acid sequence of SEQ ID NO: 83 (1245_P01_G09).

For fragments comprising both the VH and VL regions, these may be associated either covalently (e.g. via disulphide bonds or a linker) or non-covalently. The multispecific antibody fragment described herein may comprise an scFv, i.e. a fragment comprising a VH region and a VL region joined by a linker. In one embodiment, the VH and VL region are joined by a (e.g. synthetic) polypeptide linker. The polypeptide linker may comprise a $(Gly_4Ser)_n$ linker, where n = from 1 to 8, *e.g.* 2, 3, 4, 5 or 7. The polypeptide linker may comprise a $[(Gly_4Ser)_n(Gly_3AlaSer)_m]_p$ linker, where n = from 1 to 8, *e.g.* 2, 3, 4, 5 or 7, m = from 1 to 8, e.g. 0, 1, 2 or 3, and p = from 1 to 8, e.g. 1, 2 or 3. In a further embodiment, the linker comprises SEQ ID NO: 98. In a further embodiment, the linker consists of SEQ ID NO: 98.

In one embodiment, the multispecific antibody or fragment thereof comprises an amino acid sequence having at least 80% sequence identity with any one of SEQ ID NOs: 86-97. In a further embodiment, the multispecific antibody or fragment thereof comprises an amino acid sequence of any one of SEQ ID NOs: 86-97. In a yet further embodiment, the multispecific antibody or fragment thereof comprises an amino acid sequence of SEQ ID NO: 87 (1252_P01_C08). In an alternative embodiment, the multispecific antibody or fragment thereof comprises an amino acid sequence of SEQ ID NO: 86 (1245_P01_E07). In an alternative embodiment, the multispecific antibody or fragment thereof comprises an amino acid sequence of SEQ ID NO: 88 (1245_P02_G04). In an alternative embodiment, the multispecific antibody or fragment thereof comprises an amino acid sequence of SEQ ID NO: 92 (1139_P01_E04). In an alternative embodiment, the multispecific antibody or fragment thereof comprises an amino acid sequence of SEQ ID NO: 93 (1245_P02_F07). In an alternative embodiment, the multispecific antibody or fragment thereof comprises an amino acid sequence of SEQ ID NO: 94 (1245_P01_G06). In an alternative embodiment, the multispecific antibody or fragment thereof comprises an amino acid sequence of SEQ ID NO: 95 (1245_P01_G09).

In one embodiment, the component of the multispecific antibody conferring TRDV1-binding consists of an amino acid sequence having at least 80% sequence identity with any one of SEQ ID NOs: 86-97. In a further embodiment, the component of the multispecific antibody conferring TRDV1-binding consists of an amino acid sequence of any one of SEQ ID NOs: 86-97. In a yet further embodiment, the component of the multispecific antibody conferring TRDV1-binding consists of an amino acid sequence of SEQ ID NO: 87 (1252_P01_C08). In an alternative embodiment, the component of the multispecific antibody conferring TRDV1-binding consists of an amino acid sequence of SEQ ID NO: 86 (1245_P01_E07). In an alternative embodiment, the component of the multispecific antibody conferring TRDV1-binding consists of an amino acid sequence of SEQ ID NO: 88 (1245_P02_G04). In an alternative embodiment, the component of the multispecific antibody conferring TRDV1-binding consists of an amino acid sequence of SEQ ID NO: 92 (1139_P01_E04). In an alternative embodiment, the component of the multispecific antibody conferring TRDV1-binding consists of an amino acid sequence of SEQ ID NO: 93 (1245_P02_F07). In an alternative embodiment, the component of the multispecific antibody conferring TRDV1-binding consists of an amino acid sequence of SEQ ID NO: 94 (1245_P01_G06). In an alternative embodiment, the component of the multispecific antibody conferring TRDV1-binding consists of an amino acid sequence of SEQ ID NO: 95 (1245_P01_G09).

It will be understood by a person skilled in the art that scFv constructs may be designed and made inclusive of N-terminal and C-terminal modifications to aid with translation, purification and detection. For example, at the N-terminus of an scFv sequence, an additional methionine and/or alanine amino acid residue may be included ahead of the canonical VH sequences (e.g. starting QVQ or EVQ). At the C-terminus (i.e. C-terminal to the canonical mature VL domain sequence ending as per the IMGT definition), additional sequences may be included such as (i) a partial sequence of the constant domain and/or (ii) additional synthetic sequences inclusive of tags, such as His-tags and Flag-tags, to aid with purification and detection. In one embodiment, SEQ ID NO: 124 is added to the C-terminus of any one of SEQ ID NOs: 86, 88-90, 92-97. In one embodiment, SEQ ID NO: 125 is added to the C-terminus of any one of SEQ ID NOs: 86, 88-90, 92-97. In one embodiment, SEQ ID NO: 126 is added to the C-terminus of any one of SEQ ID NOs: 87 or 91. In one embodiment, SEQ ID NO: 127 is added to the C-terminus of any one of SEQ ID NOs: 87 or 91. It is well understood that said scFv N- or C-terminal sequences are optional and can be removed, modified or substituted if alternate scFv design, translation, purification or detection strategies are adopted.

As described herein, the antibodies may be in any format. In a preferred embodiment, the multispecific antibody is in an IgG1 format. Therefore, in one embodiment, the multispecific antibody or fragment thereof comprises an amino acid sequence having at least 80% sequence identity with any one of SEQ ID NOs: 111-122. In a further embodiment, the multispecific antibody or fragment thereof comprises an amino acid sequence of any one of SEQ ID NOs: 111-122. In a yet further embodiment, the multispecific antibody or fragment thereof comprises an amino acid sequence of SEQ ID NOs: 111-116, such as SEQ ID NOs: 111-113 and 116. In a yet further embodiment, the multispecific antibody or fragment thereof comprises an amino acid sequence of SEQ ID NOs: 117-122, such as SEQ ID NOs: 117-120. In a yet further embodiment, the multispecific antibody or fragment thereof comprises an amino acid sequence of SEQ ID NOs: 111, 112, 116-120, such as SEQ ID NOs: 111, 112 or 116, or SEQ ID NOs: 117-120.

In one embodiment, the component of the multispecific antibody conferring TRDV1-binding consists of an amino acid sequence having at least 80% sequence identity with any one of SEQ ID NOs: 111-122. In a further embodiment, the component of the multispecific antibody conferring TRDV1-binding consists of an amino acid sequence of any one of SEQ ID NOs: 111-122. In a yet further embodiment, the component of the multispecific antibody conferring TRDV1-binding consists of an amino acid sequence of SEQ ID NOs: 111-116, such as SEQ ID NOs: 111-113 and 116. In a yet further embodiment, the component of the multispecific antibody conferring TRDV1-binding consists of an amino acid sequence of SEQ ID NOs: 117-122, such as SEQ ID NOs: 117-120. In a yet further embodiment, the component of the multispecific antibody conferring TRDV1-binding consists of an amino acid sequence of SEQ ID NOs: 111, 112, 116-120, such as SEQ ID NOs: 111, 112 or 116, or SEQ ID NOs: 117-120.

In one embodiment, the multispecific antibody binds to the same, or essentially the same, TRDV1 epitope as, or competes with, an antibody or fragment thereof as defined herein. One can easily determine whether a multispecific antibody binds to the same TRDV1 epitope as, or competes for binding with, a reference anti-Vδ1 antibody by using routine methods known in the art. For example, to determine if a test antibody binds to the same TRDV1 epitope as a reference anti-Vδ1 antibody of the invention, the reference antibody is allowed to bind to a Vδ1 protein or peptide under saturating conditions. Next, the ability of a test multispecific antibody to bind to the Vδ1 chain is assessed. If the test multispecific antibody is able to bind to Vδ1 following saturation binding with the reference anti-Vδ1 antibody, it can be concluded that the test multispecific antibody binds to a different TRDV1 epitope than the reference anti-Vδ1 antibody. On the other hand, if the test multispecific antibody is not able to bind to the Vδ1 chain following saturation binding with the reference anti-Vδ1 antibody, then the test multispecific antibody may bind to the same TRDV1 epitope as the epitope bound by the reference anti-Vδ1 antibody of the invention. Of course, binding to the TRDV1 epitope refers to the component of the multispecific antibody that confers binding to the first target epitope, which is an epitope of TRDV1. Testing of binding to the same or different epitope may alternatively be conducted using the sequence of the multispecific antibodies provided herein, but in a monospecific format.

The present invention also includes multispecific anti-Vδ1 antibodies that compete for binding to Vδ1 with an antibody or fragment thereof as defined herein (either in a multi- or mono-specific format), or an antibody having the CDR sequences of any of the exemplary antibodies described herein. For example, competitive assays can be performed with the antibody of the present invention in order to determine what proteins, antibodies, and other antagonists compete for binding to the Vδ1 chain with the antibody of the present invention and/or share the epitope. These assays are readily known to those of skill in the art; they evaluate competition between antagonists or ligands for a limited number of binding sites on a protein, e.g. Vδ1. The antibody (or fragment thereof) is immobilized or insolubilized before or after the competition and the sample bound to the Vδ1 chain is separated from the unbound sample, for example, by decanting (where the antibody was pre-insolubilized) or by centrifuging (where the antibody was precipitated after the competitive reaction). Also, the competitive binding may be determined by whether the function is altered by the binding or lack of binding of the antibody to the protein, *e.g.* whether the antibody molecule inhibits or potentiates the enzymatic activity of, for example, a label. ELISA and other functional assays may be used, as known in the art and described herein.

Two antibodies bind to the same or overlapping epitope if each competitively inhibits (blocks) binding of the other to the target antigen. That is, a 1 -, 5-, 10-, 20- or 100-fold excess of one antibody inhibits binding of the other by at least 50% but preferably 75%, 90% or even 99% as measured in a competitive binding assay. Alternatively, two antibodies have the same epitope if essentially all amino acid mutations in the target antigen that reduce or eliminate binding of one antibody reduce or eliminate binding of the other.

Additional routine experimentation (*e.g.* peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

In some embodiments, the antibody or fragment thereof contains a modified effector function through alteration to the sugars linked to Asn 297 (EU numbering scheme). In a further said modification, Asn 297 is not fucosylated or exhibits reduced fucosylation (i.e., a defucosylated antibody or a non-fucosylated antibody). Fucosylation includes the addition of the sugar fucose to a molecule, for example, the attachment of fucose to N-glycans, O-glycans and glycolipids. Accordingly, in a defucosylated antibody, fucose is not attached to the carbohydrate chains of the constant region. The antibody may be modified to prevent or inhibit fucosylation of the antibody. Typically, glycosylation modifications involve expressing said antibody or fragment thereof in a host cell containing alternate glycosylation processing capabilities either through targeted engineering or through targeted or serendipitous host or clone selection (e.g. see Example 13). These and other effector modifications are discussed further in recent reviews such as by Xinhua Wang et al. (2018) Protein & Cell 9: 63-73 and by Pereira et al. (2018) mAbs 10(5): 693-711.

**Antibody sequence modifications**

The multispecific antibodies and fragments thereof may be modified using known methods. Sequence modifications to antibody molecules described herein, in particular to those parts of the multispecific antibodies conferring Vδ1 binding, can be readily incorporate by those skilled in the art. The following examples are non-limiting.

During antibody discovery and sequence recovery from phage libraries, desired antibody variable domains may be re-formatted into full length IgG by sub-cloning. To accelerate the process, variable domains are often transferred using restriction enzymes. These unique restriction sites may introduce additional/alternate amino acids and away from the canonical sequence (such canonical sequences may be found, for example, in the international ImMunoGeneTics [IMGT] information system, see http://www.imgt.org). These may be introduced as kappa or lambda light chain sequence modifications.

*Kappa light chain modifications*

The variable kappa light chain variable sequences may be cloned using restriction sites (e.g. Nhe1-Not1) during re-formatting into full length IgG. More specifically, at the kappa light chain N-terminus, an additional Ala-Ser sequence was introduced to support cloning. Preferably, this additional AS sequence is then removed during further development such to generate the canonical N-terminal sequence. Hence, in one embodiment, kappa light chain containing antibodies described herein do not contain an AS sequence at their N-termini, i.e. SEQ ID NOs: 74, 76-78 and 80-85 do not comprise the initial AS sequence. In a further embodiment, SEQ ID NOs: 74 and 76-78 do not comprise the initial AS sequence. It will be understood that this embodiment also applies to other sequences included herein which contain this sequence (e.g. SEQ ID NOs: 86, 88-90 and 92-97).

Additional amino acid changes may be made to support cloning. For example, for the antibodies described herein, at the kappa light-chain variable-domain/constant domain border a valine-to-alanine change was introduced to support cloning. This resulted in a kappa constant domain modification. Specifically, this results in the constant domain beginning RTAAAPS (from a NotI restriction site). Preferably, this sequence can be modified during further development to generate the canonical kappa light-chain constant regions which start with RTVAAPS. Hence, in one embodiment kappa light chain containing antibodies described herein contain a constant domain stating with the sequence RTV. Therefore, in one embodiment, sequence RTAAAPS of SEQ ID NOs: 111-114 and 117-122 is replaced with sequence RTVAAPS. For example, see Example 13 and SEQ ID NOs: 129, 130.

*Lambda light chain modifications*

Similar to the kappa example above, the lambda light chain variable domains may also be cloned by introducing restriction sites (*e.g.* Nhe1-Not1) during re-formatting into full length IgG. More specifically, at the lambda light chain N-terminus, an additional Ala-Ser sequence may be introduced to support cloning. Preferably, this additional AS sequence is then removed during further development such to generate the canonical N-terminal sequence. Hence, in one embodiment, lambda light chain containing antibodies described herein do not contain an AS sequence at their N-termini *i.e.* SEQ ID NOs: 75 and 79 do not comprise the initial AS sequence. It will be understood that this embodiment also applies to other sequences included herein which contain this sequence (*e.g.* SEQ ID NOs: 87, 91, 115 and 116). In one embodiment, SEQ ID NO: 75 does not contain the initial six residues, *i.e.* the ASSYEL sequence is removed.

As another example, for the antibodies described herein at the lambda light-chain variable-domain/constant domain border a lysine-to-alanine sequence change was introduced to support cloning. This resulted in a lambda constant domain modification. Specifically, this results in the constant domain beginning with GQPAAAPS (from a NotI restriction site). Preferably, this sequence can be modified during further development such to generate the canonical lambda light constant region which starts GQPKAAPS. Hence, in one embodiment, lambda light chain containing antibodies described herein contain a constant domain starting with the sequence GQPK. Therefore, in one embodiment, sequence GQPAAAPS of SEQ ID NO: 115 or 116 is replaced with sequence GQPKAAPS.

*Heavy chain modifications*

Typically, human variable heavy chain sequences start with either the basic glutamine (Q) or acidic glutamate (E). However, both such sequences are then known to convert to the acidic amino acid residue, pyro-glutamate (pE). The Q to pE conversion results in a charge change to the antibody, whilst an E to pE conversion does not change the charge of the antibody. Hence to avoid a variable charge-change over time one option is to modify a starting heavy chain sequence from Q to E in the first instance. Hence, in one embodiment, the heavy chain of antibody described herein contains a Q to E modification at the N-terminus. In particular, the initial residue of SEQ ID NOs: 62, 64 and/or 67-71 may be modified from Q to E. It will be understood that this embodiment also applies to other sequences included herein which contain this sequence (*e.g.* SEQ ID NOs: 86, 88, 91-97 and 111, 112, 115, 117-120). For example, see Example 13 and SEQ ID NOs: 129, 130.

Furthermore, the C-terminus of the IgG1 constant domain ends with PGK. However, the terminal basic lysine (K) is then often cleaved during expression (*e.g.* in CHO cells). This in turn results in charge change to the antibody through varied loss of the C-terminal lysine residue. Therefore, one option is to remove the lysine in the first instance resulting in a uniform and consistent heavy chain C-terminus sequence ending in PG. Hence, in one embodiment, the heavy chain of an antibody described herein has the terminal K removed from its C-terminus. In particular, the antibody of the invention may comprise any one of SEQ ID NOs: 111-122 where the terminal lysine residue has been removed. For example, see SEQ ID NO: 141.

*Optional allotype modifications*

During antibody discovery, specific human allotypes may be employed. Optionally, the antibodies can be switched to differing human allotypes during development. By way of non-limiting example, for the kappa chain there are three human allotypes designated Km1, Km1,2 and Km3 which define three Km alleles (using allotype numbering): Km1 correlates with valine 153 (IMGT V45.1) and leucine 191 (IMGT L101); Km1,2 correlates with alanine 153 (IMGT A45.1) and leucine 191 (IMGT L101); and Km3 correlates with alanine 153 (IMGT A45.1) and valine 191 (IMGT V101). Optionally, one can therefore modify a sequence from one allotype to another by standard cloning approaches. For example, a L191V (IMGT L101V) change will convert a Km1,2 allotype to a Km3 allotype. For further reference on such allotypes see Jefferis and Lefranc (2009) MAbs 1(4):332-8.

Hence in one embodiment a multispecific antibody described herein contains amino acid substitutions derived from another human allotype of the same gene. In a further embodiment, the antibody contains a L191V (IMGT L101V) substitution to the kappa chain to convert the c-domain from a km1,2 to a km3 allotype. For example, see Example 13 and SEQ ID NOs: 129, 130.

**Antibodies targeted to TRDV1 epitopes**

Provided herein are antibodies (or fragments thereof) which bind to an epitope of the Vδ1 chain of a γδ TCR (in addition to binding a second epitope, discussed elsewhere). Such binding may optionally have an effect on γδ TCR activity, such as activation. The antibodies of the invention are preferably specific for the Vδ1 chain of a γδ TCR, and do not bind epitopes of other antigens, such as the Vδ2 chain of a γδ TCR or the Vδ3 chain of a γδ TCR. The antibodies of the present invention may be considered agonistic antibodies, at least with respect to the agonistic effect conferred upon Vδ1 cells upon binding.

In one embodiment, the epitope may be an activating epitope of a γδ T cell. An "activating" epitope can include, for example, stimulating a TCR function, such as cell degranulation, TCR downregulation, cytotoxicity, proliferation, mobilisation, increased survival or resistance to exhaustion, intracellular signaling, cytokine or growth factor secretion, phenotypic change, or a change in gene expression. For example, the binding of the activating epitope may stimulate expansion (*i.e.* proliferation) of the γδ T cell population, preferably the Vδ1+ T cell population. Accordingly, these multi-specific antibodies can be used to modulate γδ T cell activation, and, thereby, to modulate the immune response. Therefore, in one embodiment, binding of the activating epitope downregulates the γδ TCR. In an additional or alternative embodiment, binding of the activating epitope activates degranulation of the γδ T cell. In a further additional or alternative embodiment, binding of the activating epitope promotes γδ T cell mediated killing.

In some embodiments, an activating epitope of TRDV1 is one that, upon being bound by an antibody, results in down-regulation of the receptor and optionally activates the Vδ1 cell. In some embodiments said down-regulation of the receptor also results in the down-regulation of associated CD3 molecules. In some embodiments, the activating epitope is one that upregulates expression of activatory markers on the Vδ1 cell, for example CD107a, CD25, CD69 and/or Ki67. In some embodiments, the activating epitope is one that upregulates expression of activatory markers on the Vδ1 cell, for example CD107 and CD25, and optionally CD69 and/or Ki67. In some embodiments, upregulation of the one or more activatory markers (such as CD107a) may be upregulation in the presence of cancer cells. In preferred embodiments of the invention, the multispecific antibodies bind activating epitopes of TRDV1, in particular via the TRDV1-binding domain.

As T-cell receptors are often complexed with other proteins, downregulation of the T-cell receptor via Vδ1 antibody binding may cause downregulation of other proteins associated with the T-cell receptor (i.e. the binding of the Vδ1 antibody causes down regulation of the T-cell receptor complex). For example, in some embodiments, an activating epitope of TRDV1 is one that upon binding, down-regulates the TCR/CD3 receptor complex. In this way, the antibodies of the invention may cause indirect downregulation of cell surface proteins that are not bound by the antibody, but are complexed to the T-cell receptor. Given T-cells expressing gamma delta 1 chains (i.e. Vδ1 cells) represent only a small number of the total T-cell population, the antibodies of the invention can be used to selectively (and indirectly) down-regulate proteins in the TCR complex, such as CD3, by only downregulating them in Vδ1 cells.

In some embodiments, a T-cell receptor complex activating epitope is one that upon activation, downregulates the T-cell receptor complex, whilst not downregulating CD3 molecules not associated with said TRDV1 TCR complex

The epitope is preferably comprised of at least one extracellular, soluble, hydrophilic or external portion of the Vδ1 chain of a γδ TCR.

In particular, the epitope does not comprise an epitope found in a hypervariable region of the Vδ1 chain of the γδ TCR, in particular CDR3 of the Vδ1 chain. In a preferred embodiment, the epitope is within the non-variable region of the Vδ1 chain of the γδ TCR. It will be appreciated that such binding allows for the unique recognition of the Vδ1 chain without the restriction to the sequences of the TCR which are highly variable (in particular CDR3). Various γδ TCR complexes which recognise antigen may be recognised in this way, solely by presence of the Vδ1 chain. As such, it will be appreciated that any Vδ1 chain-comprising γδ TCR may be recognised using the antibodies or fragments thereof as defined herein, irrespective of the specificity of the γδ TCR. In one embodiment, the epitope comprises one or more amino acid residues within amino acid regions 1-24 and/or 35-90 of SEQ ID NO: 1, *e.g.* the portions of the Vδ1 chain which are not part of the CDR1 and/or CDR3 sequences. In one embodiment, the epitope does not comprise amino acid residues within amino acid region 91-105 (CDR3) of SEQ ID NO: 1.

In some embodiments the epitope comprises amino acids in the TRDV-1 CDR2 sequence.

In a similar manner to the well characterised αβ T cells, γδ T cells utilize a distinct set of somatically rearranged variable (V), diversity (D), joining (J), and constant (C) genes, although γδ T cells contain fewer V, D, and J segments than αβ T cells. In one embodiment, the epitope bound by the antibodies (or fragments thereof) does not comprise an epitope found in the J region of the Vδ1 chain (e.g. one of the four J regions encoded in the human delta one chain germline: SEQ ID NO:152 (J1*0) or 153 (J2*0) or 154 (J3*0) or 155 (J4*0)) or in the C-region of the Vδ1 chain (e.g. SEQ ID NO:156 (C1*0) which contains the C-terminal juxtamembrane/transmembrane regions). In one embodiment, the epitope bound by the antibodies (or fragments thereof) does not comprise an epitope found in the N-terminal leader sequence of the Vδ1 chain (e.g. SEQ ID NO:150). The antibody or fragment may therefore only bind in the V region of the Vδ1 chain (e.g. SEQ ID NO: 151). Thus, in one embodiment, the epitope consists of an epitope in the V region of the γδ TCR (e.g. amino acid residues 1-90 of SEQ ID NO: 1).

Reference to the epitope are made in relation to the Vδ1 sequence derived from the sequence described in Luoma et al. (2013) Immunity 39: 1032-1042, and RCSB Protein Data Bank entries: 4MNH and 3OMZ, shown as SEQ ID NO: 1:

AQKVTQAQSSVSMPVRKAVTLNCLYETSWWSYYIFWYKQLPSKEMIFLIRQGSDEQNAKSGRYSVNFKK
AAKSVALTISALQLEDSAKYFCALGESLTRADKLIFGKGTRVTVEPNIQNPDPAVYQLRDSKSSDKSVCLF
TDFDSQTNVSQSKDSDVYITDKTVLDMRSMDFKSNSAVAWSNKSDFACANAFNNSIIPEDTFFPSPESS
(SEQ ID NO: 1)

SEQ ID NO: 1 represents a soluble TCR comprising a V region (also referred to as the variable domain), a D region, a J region and a TCR constant region. The V region comprises amino acid residues 1-90, the D region comprises amino acid residues 91-104, the J region comprises amino acid residues 105-115 and the constant region (derived from T-cell receptor alpha) comprises amino acid residues 116-209. Within the V region, CDR1 is defined as amino acid residues 25-34 of SEQ ID NO: 1, CDR2 is defined as amino acid residues 50-54 of SEQ ID NO: 1, and CDR3 is defined as amino acid residues 93-104 of SEQ ID NO: 1 (Xu et al., PNAS USA 108(6):2414-2419 (2011)).

Therefore, according to an aspect of the invention, there is provided an isolated antibody or fragment thereof, which binds to an epitope of a variable delta 1 (Vδ1) chain of a γδ T cell receptor (TCR) comprising one or more amino acid residues within amino acid regions:

(i) 3-20 of SEQ ID NO: 1; and/or
(ii) 37-77 of SEQ ID NO: 1.

In a further embodiment, antibodies or fragments thereof additionally recognize the polymorphic V region comprising amino acid residues 1-90 epitope of SEQ ID NO:128. Hence, amino acids 1-90 of SEQ ID NO:1 and the polymorphic germline variant sequence (amino acids 1-90 SEQ ID NO:128) may be considered interchangeable when defining epitopes described herein. Studies presented herein have demonstrated antibodies of the invention can recognize both variants of this germline sequence. By way of example, where it is stated that antibodies or fragments thereof as defined herein recognize epitopes comprising one or more amino acid residues within amino acid regions 1-24 and/or 35-90 of SEQ ID NO:1 this also refers to the same regions of SEQ ID NO:128; specifically amino acid regions 1-24 and/or 35-90 of SEQ ID NO:128.

In one embodiment, antibodies or fragments thereof recognize one or more amino acid residues within amino acid regions 1-90 of SEQ ID NO:1 and the equivalently located amino acids of regions 1-90 in SEQ ID NO:128. More specifically, in one embodiment antibodies or fragments thereof as defined herein recognize a human germline epitope wherein said germline encodes either an alanine (A) or valine (V) at position 71 of SEQ ID NO:1.

In one embodiment, the epitope comprises one or more, such as two, three, four, five, six, seven, eight, nine, ten or more amino acid residues within the described regions.

In a further embodiment, the epitope comprises one or more (such as 5 or more, such as 10 or more) amino acid residues within amino acid region 3-20 of SEQ ID NO: 1. In an alternative embodiment, the epitope comprises one or more (such as 5 or more, such as 10 or more) amino acid residues within amino acid region 37-77 of SEQ ID NO: 1 (such as amino acid region 50-54). In a yet further embodiment, the epitope comprises one or more (such as 5 or more, such as 10 or more) amino acid residues within amino acid region 3-20 (such as 5-20 or 3-17) and one or more (such as 5 or more, such as 10 or more) amino acid residues within amino acid region 37-77 (such as 62-77 or 62-69) of SEQ ID NO: 1.

It will be further understood that said antibody (or fragment thereof) does not need to bind to all amino acids within the defined range. Such epitopes may be referred to as linear epitopes. For example, an antibody which binds to an epitope comprising amino acid residues within amino acid region 5-20 of SEQ ID NO: 1 may only bind with one or more of the amino acid residues in said range, *e.g.* the amino acid residues at each end of the range (*i.e.* amino acids 5 and 20), optionally including amino acids within the range (*i.e.* amino acids 5, 9, 16 and 20).

In one embodiment, the epitope comprises at least one of amino acid residues 3, 5, 9, 10, 12, 16, 17, 20, 37, 42, 50, 53, 59, 62, 64, 68, 69, 72 or 77 of SEQ ID NO: 1. In further embodiments, the epitope comprises one, two, three, four, five, six, seven, eight, nine, ten, eleven or twelve amino acids selected from amino acid residues 3, 5, 9, 10, 12, 16, 17, 20, 37, 42, 50, 53, 59, 62, 64, 68, 69, 72 or 77 of SEQ ID NO: 1.

In one embodiment, the epitope comprises one or more amino acid residues within the following amino acid regions of SEQ ID NO: 1 (or SEQ ID NO:128, as described above):

(i) 3-17;
(ii) 5-20;
(iii) 37-53;
(iv) 50-64;
(v) 59-72;
(vi) 59-77;
(vii) 62-69; and/or
(viii) 62-77.

In a further embodiment, the epitope comprises one or more amino acid residues within amino acid regions: 5-20 and 62-77; 50-64; 37-53 and 59-72; 59-77; or 3-17 and 62-69, of SEQ ID NO: 1. In a further embodiment, the epitope consists of one or more amino acid residues within amino acid regions: 5-20 and 62-77; 50-64; 37-53 and 59-72; 59-77; or 3-17 and 62-69, of SEQ ID NO: 1.

In a further embodiment, the epitope comprises amino acid residues: 3, 5, 9, 10, 12, 16, 17, 62, 64, 68 and 69 of SEQ ID NO: 1, or suitably consists of amino acid residues: 3, 5, 9, 10, 12, 16, 17, 62, 64, 68 and 69 of SEQ ID NO: 1. In a further embodiment, the epitope comprises amino acid residues: 5, 9, 16, 20, 62, 64, 72 and 77 of SEQ ID NO: 1, or suitably consists of amino acid residues: 5, 9, 16, 20, 62, 64, 72 and 77 of SEQ ID NO: 1. In yet further embodiment, the epitope comprises the amino acid residues: 37, 42, 50, 53, 59, 64, 68, 69, 72, 73 and 77 of SEQ ID NO: 1, or suitably consists of amino acid residues: 37, 42, 50, 53, 59, 64, 68, 69, 72, 73 and 77 of SEQ ID NO: 1. In a further embodiment, the epitope comprises the amino acid residues: 50, 53, 59, 62 and 64 of SEQ ID NO: 1, or suitably consists of amino acid residues: 50, 53, 59, 62 and 64 of SEQ ID NO: 1. In a further embodiment, the epitope comprises amino acid residues: 59, 60, 68 and 72 of SEQ ID NO: 1, or suitably consists of amino acid residues: 59, 60, 68 and 72 of SEQ ID NO: 1.

In one embodiment, the epitope comprises one or more amino acid residues within amino acid regions 5-20 and/or 62-77 of SEQ ID NO: 1. In a further embodiment, the epitope consists of one or more amino acid residues within amino acid regions 5-20 and 62-77 of SEQ ID NO: 1. In an alternative further embodiment, the epitope comprises one or more amino acid residues within amino acid regions 5-20 or 62-77 of SEQ ID NO: 1. Antibodies or fragments thereof having such epitopes may have some or all of the sequences of 1245_P01_E07, or such antibodies or fragments thereof may be derived from 1245_P01_E07. For example, antibodies or fragments thereof having one or more CDR sequences of 1245_P01_E07 or one or both of the VH and VL sequences of 1245_P01_E07 may bind such epitopes.

In one embodiment, the epitope comprises one or more amino acid residues within amino acid region 50-64 of SEQ ID NO: 1. In a further embodiment, the epitope consists of one or more amino acid residues within amino acid region 50-64 of SEQ ID NO: 1. Antibodies or fragments thereof having such epitopes may have some or all of the sequences of 1252_P01_C08, or such antibodies or fragments thereof may be derived from 1252_P01_C08. For example, antibodies or fragments thereof having one or more CDR sequences of 1252_P01_C08 or one or both of the VH and VL sequences of 1252_P01_C08 may bind such epitopes.

In one embodiment, the epitope comprises one or more amino acid residues within amino acid regions 37-53 and/or 59-77 of SEQ ID NO: 1. In a further embodiment, the epitope consists of one or more amino acid residues within amino acid regions 37-53 and 59-77 of SEQ ID NO: 1. In an alternative further embodiment, the epitope comprises one or more amino acid residues within amino acid regions 37-53 or 59-77 of SEQ ID NO: 1. Antibodies or fragments thereof having such epitopes may have some or all of the sequences of 1245_P02_G04, or such antibodies or fragments thereof may be derived from 1245_P02_G04. For example, antibodies or fragments thereof having one or more CDR sequences of 1245_P02_G04 or one or both of the VH and VL sequences of 1245_P02_G04 may bind such epitopes.

In one embodiment, the epitope comprises one or more amino acid residues within amino acid region 59-72 of SEQ ID NO: 1. In a further embodiment, the epitope consists of one or more amino acid residues within amino acid region 59-72 of SEQ ID NO: 1. Antibodies or fragments thereof having such epitopes may have some or all of the sequences of 1251_P02_C05, or such antibodies or fragments thereof may be derived from 1251_P02_C05. For example, antibodies or fragments thereof having one or more CDR sequences of 1251_P02_C05 or one or both of the VH and VL sequences of 1251_P02_C05 may bind such epitopes.

In one embodiment, the epitope does not comprise amino acid residues within amino acid region 11-21 of SEQ ID NO: 1. In one embodiment, the epitope does not comprise amino acid residues within amino acid region 21-28 of SEQ ID NO: 1. In one embodiment, the epitope does not comprise amino acid residues within amino acid region 59 and 60 of SEQ ID NO: 1. In one embodiment, the epitope does not comprise amino acid residues within amino acid region 67-82 of SEQ ID NO: 1.

In one embodiment, the epitope is not the same epitope bound by a commercially available anti-Vδ1 antibody, such as TS-1 or TS8.2. As described in WO2017197347, binding of TS-1 and TS8.2 to soluble TCRs was detected when the δ1 chain included Vδ1 J1 and Vδ1 J2 sequences but not to the Vδ1 J3 chain, indicating that the binding of TS-1 and TS8.2 involved critical residues in the delta J1 and delta J2 region.

References to "within" herein include the extremities of the define range. For example, "within amino acid regions 5-20" refers to all of amino acid resides from and including residue 5 up to and including residue 20.

Various techniques are known in the art to establish which epitope is bound by an antibody. Exemplary techniques include, for example, routine cross-blocking assays, alanine scanning mutational analysis, peptide blot analysis, peptide cleavage analysis crystallographic studies and NMR analysis. In addition, methods such as epitope excision, epitope extraction and chemical modification of antigens can be employed. Another method that can be used to identify the amino acids within a polypeptide with which an antibody interacts is hydrogen/deuterium exchange detected by mass spectrometry (as described in Example 9). In general terms, the hydrogen/deuterium exchange method involves deuterium-labelling the protein of interest, followed by binding the antibody to the deuterium-labelled protein. Next, the protein/antibody complex is transferred to water and exchangeable protons within amino acids that are protected by the antibody complex undergo deuterium-to-hydrogen back-exchange at a slower rate than exchangeable protons within amino acids that are not part of the interface. As a result, amino acids that form part of the protein/antibody interface may retain deuterium and therefore exhibit relatively higher mass compared to amino acids not included in the interface. After dissociation of the antibody, the target protein is subjected to protease cleavage and mass spectrometry analysis, thereby revealing the deuterium-labelled residues which correspond to the specific amino acids with which the antibody interacts.

The multispecific antibodies and fragments thereof suitably specifically bind to both human TRDV1 (SEQ ID NO: 1 and the polymorphic variant of SEQ ID NO: 128) as well as cyno TRDV1 (SEQ ID NO: 172), via the TRDV1-binding domain.

## Antibodies targeted to epitopes of a second antigen

As the antibodies of the present invention are multispecific antibodies (preferably bispecific antibodies), they specifically bind a second antigen, wherein the second antigen is OX40, in addition to specifically binding TRDV1. The identity of the second antigen determines if the antibody is in one of two categories, as discussed herein: a T-cell engager (TCE) antibody or a dual immunomodulator (DI) antibody.

In embodiments of the disclosure relating to TCEs, the second antigen is a cancer antigen or a cancer-associated antigen. In such embodiments, the antibodies specifically bind a first target epitope, wherein the first target epitope is an epitope of the variable delta 1 (Vδ1) chain of a γδ T cell receptor (TCR); and a second target epitope, wherein the second target epitope is an epitope of a cancer antigen or cancer-associated antigen. The identities of other, non-claimed specific possible second antigens in this category are discussed elsewhere. However, in embodiments of the disclosure the second antigen can be any antigen expressed by a cancer cell that promotes the Vδ1-T cell mediated killing of said cancer cell (e.g. direct killing or via immune licensing effect of signaling to other immune cells upon tumour cell binding). Such Vδ1-cell mediated cancer cell killing is promoted by colocalizing the Vδ1-T cells and cancer cells, and activation of the Vδ1-T cells via binding of the multispecific antibody, in particular to an activating epitope of the Vδ1-T cell. The present invention exemplifies a completely novel platform for TCE-type antibodies.

In some embodiments, the second antigen is not an antigen of an ovarian carcinoma. In some embodiments, the second antigen is not an antigen of a Mov19+ ovarian carcinoma. In some embodiments, the multispecific (suitably bispecific) antibody does not specifically bind to Mov19+ ovarian carcinoma cells. In some embodiments, the multispecific (suitably bispecific) antibody does not specifically bind to alpha-folate receptor (alpha-FR). Alpha-FR is also known as folate receptor 1, FOLR1, folate receptor alpha, or FRα. It is encoded by the FOLR1 gene (UniProt accession no. P15328) and has the sequence of SEQ ID NO: 176. In some embodiments, the multispecific (suitably bispecific) antibody does not specifically bind to an epitope bound by the scFv MOV19.

In some embodiments, the antibody or antigen-binding fragment thereof is a bispecific antibody, wherein the second antigen is not alpha-folate receptor.

In some embodiments the multispecific antibody is a human recombinant antibody encoded by a recombinant nucleic acid open reading frame or frames expressed from a recombinant host cell. In some embodiments the multispecific antibody is not a rodent or other non-human antibody derived from B-cell fusion hybridoma technologies. In some embodiments the multispecific antibody does not comprise non-human IgG constant domain sequence found only in non-human animal species, such as sequence found in rodent-derived hybridomas.

In embodiments relating to DIs, the second antigen is an immunomodulatory antigen, wherein the second antigen is OX40. In such embodiments, the antibodies specifically bind a first target epitope, wherein the first target epitope is an epitope of the variable delta 1 (Vδ1) chain of a γδ T cell receptor (TCR); and a second target epitope, wherein the second target epitope is an immunomodulatory antigen, wherein the second antigen is OX40. An "immunomodulatory" antigen is an antigen that modulates (for example promotes) antibody- and/or cell-mediated immunity. An immunomodulatory antigen is one that is present on the cell surface of a T-cell. In embodiments of the invention in which the second antigen is an immunomodulatory antigen, the second antigen is not the T-cell receptor or a component of the T-cell receptor complexes. For example, in embodiments of the invention in which the second epitope is an epitope of an immunomodulatory antigen, the second epitope is not an epitope of TRDV1. In preferred embodiments of the invention in which the second epitope is an epitope of an immunomodulatory antigen, the second epitope is not an epitope of the T-cell receptor complex. For example, in some embodiments, the second epitope is not an epitope of CD3. Hence the antibodies of these embodiments are "dual immunomodulators" as they specifically bind to T-cells via TRDV1, and may additionally bind to T-cells via a second, different, epitope, wherein the epitope is not an epitope of the T-cell receptor complex. Example second antigens disclosed herein include, for example, the immune checkpoint inhibitors PD-L1, PD-1, OX40, CTLA-4, LAG-3, TIM-3, TIGIT and VISTA. For example, solid tumors recruit immunosuppressive cells such as myeloid derived suppressor cells (MDSCs), tumor-associated macrophages (TAMs), and regulatory T-cells (Tregs), all of which inhibit the activity of cytotoxic T-cells. Therefore, the most effective use of DIs in solid tumors will likely require the use of multispecific moieties to target T-cell modulating pathways in combination to help to overcome the immunosuppressive TME and render an immune excluded or immune desert "cold" tumor into an inflamed "hot" one. However, the present disclosure is not limited to specific second immunomodulatory antigens, since it presents a completely novel platform for DI-type antibodies.

In embodiments of the invention, the multispecific antibodies (suitably bispecific antibodies) of the invention, do not specifically bind (or directly interact with) CD3. In embodiments of the invention, the second antigen is not CD3.

## Antibody binding

The multispecific antibody or fragment thereof of the invention may bind to the Vδ1 chain of a γδ TCR with a binding affinity (KD) as measured by surface plasmon resonance of less than $1.5 \times 10^{-7}$ M *(i.e.* 150 nM). In a preferred embodiment, the KD is less than $1.5 \times 10^{-7}$ M *(i.e.* 150 nM). In a further embodiment, the KD is $1.3 \times 10^{-7}$ M (i.e. 130 nM) or less, such as $1.0 \times 10^{-7}$ M (*i.e.* 100 nM) or less. In a yet further embodiment, the KD is less than $5.0 \times 10^{-8}$ M (*i.e.* 50 nM), such as less than $4.0 \times 10^{-8}$ M (*i.e.* 40 nM), less than $3.0 \times 10^{-8}$ M (*i.e.* 30 nM) or less than $2.0 \times 10^{-8}$ M (i.e. 20 nM). For example, according to some aspects, there is provided a human multispecific antibody which binds to the Vδ1 chain of a γδ TCR with a binding affinity (KD) as measured by surface plasmon resonance of less than $1.5 \times 10^{-7}$ M (*i.e.* 150 nM).

In one aspect of the invention, there is provided a multispecific antibody or fragment thereof which binds to the Vδ1 chain of a γδ TCR with a binding affinity (KD) as measured by surface plasmon resonance of less than $4.0 \times 10^{-8}$ M (*i.e.* 40 nM), less than $3.0 \times 10^{-8}$ M (*i.e.* 30 nM) or less than $2.0 \times 10^{-8}$ M (*i.e.* 20 nM).

The multispecific antibody or fragment thereof of the invention may bind to the second antigen (or epitope of the second antigen) with a binding affinity (KD) as measured by surface plasmon resonance of less than 1.5 x $10^{-7}$ M (*i.e.* 150 nM). In a preferred embodiment, the KD is less than 1.5 x $10^{-7}$ M (*i.e.* 150 nM). In a further embodiment, the KD is 1.3 x $10^{-7}$ M (*i.e.* 130 nM) or less, such as 1.0 x $10^{-7}$ M (*i.e.* 100 nM) or less. In a yet further embodiment, the KD is less than 5.0 x $10^{-8}$ M (*i.e.* 50 nM), such as less than 4.0 x $10^{-8}$ M (*i.e.* 40 nM), less than 3.0 x $10^{-8}$ M (*i.e.* 30 nM) or less than 2.0 x $10^{-8}$ M (*i.e.* 20 nM). For example, according to some aspects, there is provided a human multispecific antibody which binds to the second antigen (or epitope of the second antigen) with a binding affinity (KD) as measured by surface plasmon resonance of less than 1.5 x $10^{-7}$ M (*i.e.* 150 nM).

In one aspect of the invention, there is provided a multispecific antibody or fragment thereof which binds to the second antigen (or epitope of the second antigen) with a binding affinity (KD) as measured by surface plasmon resonance of less than 4.0 x $10^{-8}$ M (*i.e.* 40 nM), less than 3.0 x $10^{-8}$ M (*i.e.* 30 nM) or less than 2.0 x $10^{-8}$ M (*i.e.* 20 nM).

In one embodiment, the binding affinity of the multispecific antibody or fragment thereof is established by coating the antibody or fragment thereof directly or indirectly (e.g. by capture with an anti-human IgG Fc) onto the surface of a sensor (e.g. an amine high capacity chip or equivalent), wherein the target bound by the antibody or fragment thereof (e.g. the Vδ1 chain of a γδ TCR) is flowed over the chip to detect binding. Suitably, a MASS-2 instrument (which may also be referred to as Sierra SPR-32) is used at 25 °C in PBS + 0.02 % Tween 20 running buffer at 30 μl/min.

Described herein are other assays which may be used to define antibody function. For example, the antibody or fragment thereof described herein may be assessed by γδ TCR engagement, e.g. measuring downregulation of the γδ TCR upon antibody binding. Surface expression of the γδ TCR following application of the antibody or fragment thereof (optionally presented on the surface of a cell) can be measured, e.g. by flow cytometry. The antibody or fragment thereof described herein may also be assessed by measuring γδ T cell degranulation. For example, expression of CD107a, a marker for cell degranulation, can be measured following application of the antibody or fragment thereof (optionally presented on the surface of a cell) to γδ T cells, e.g. by flow cytometry. The antibody or fragment thereof described herein may also be assessed by measuring γδ T cell killing activity (to test if the antibody has an effect on the killing activity of the γδ T cell). For example, target cells may be incubated with γδ T cells in the presence of the antibody or fragment thereof (optionally presented on the surface of a cell). Following incubation, the culture may be stained with a cell viability dye to distinguish between live and dead target cells. The proportion of dead cells can then be measured, e.g. by flow cytometry.

## Multi-specific antibodies

The antibodies of the present invention are bi-specific or multi-specific. Multi-specific antibodies generally may be specific for different epitopes of one target polypeptide or may be specific for more than one target polypeptide. However, in the present invention, the antibodies generally specifically bind two (or more) different antigens.

In embodiments of the invention, the second target epitope is an epitope of OX40.

In various embodiments, the second target epitope is an epitope of a cancer antigen or a cancer-associated antigen (for example a tumour-associated antigen). In various embodiments of the disclosure, the cancer antigen or cancer-associated antigen is one selected from AFP, AKAP-4, ALK, alphafetoprotein, Androgen receptor, B7H3, BAGE, BCA225, BCAA, Bcr-abl, beta-Catenin, beta-HCG, beta-human chorionic gonadotropin, BORIS, BTAA, CA 125, CA 15-3, CA 195, CA 19-9, CA 242, CA 27.29, CA 72-4, CA-50, CAM 17.1, CAM43, Carbonic anhydrase IX, carcinoembryonic antigen, CD22, CD33/IL3Ra, CD68\P1, CDK4, CEA, chondroitin sulfate proteoglycan 4 (CSPG4) , c-Met, CO-029, CSPG4, Cyclin B1, cyclophilin C-associated protein, CYP1B1, E2A-PRL, EGFR, EGFRvlll, ELF2M, EpCAM, EphA2, EphrinB2, Epstein Barr virus antigens EBVA , ERG (TMPRSS2ETS fusion gene), ETV6-AML, FAP, FGF-5, Fos-related antigen 1, Fucosyl GM1, G250, Ga733\EpCAM, GAGE-1, GAGE-2, GD2, GD3, glioma-associated antigen, GloboH, Glycolipid F77, GM3, GP 100, GP 100 (Pmel 17), H4-RET, HER-2/neu, HER-2/Neu/ErbB-2, high-molecular-weight melanoma-associated antigen (HMW-MAA), HPV E6, HPV E7, hTERT, HTgp-175, human telomerase reverse transcriptase, Idiotype, IGF-I receptor, IGF-II, IGH-IGK, insulin growth factor (IGF)-I, intestinal carboxyl esterase, K-ras, LAGE-1a, LCK, lectin-reactive AFP, Legumain, LMP2, M344, MA-50, Mac-2 binding protein, MAD-CT-1, MAD-CT-2, MAGE, MAGE A1, MAGE A3, MAGE-1, MAGE-3, MAGE-4, MAGE-5, MAGE-6, MART-1, MART-1/MelanA, M-CSF, melanoma-associated chondroitin sulfate proteoglycan (MCSP), Mesothelin, MG7-Ag, ML-IAP, MN-CA IX, MOV18, MUC1, Mum-1, hsp70-2, MYCN, MYL-RAR, NA17, NB/70K, neuron-glial antigen 2 (NG2), neutrophil elastase, nm-23H1, NuMa, NY-BR-1, NY-CO-1, NY-ESO, NY-ESO-1, NY-ESO-1, OY-TES1, p15, p16, p180erbB3, p185erbB2, p53, p53 mutant, Page4, PAX3, PAX5, PDGFR-beta, PLAC1, Polysialic Acid, prostate-carcinoma tumor antigen-1 (PCTA-1), prostate-specific antigen, prostatic acid phosphatase (PAP), Proteinase3 (PR1), PSA, PSCA, PSMA, RAGE-1, Ras, Ras-mutant, RCAS1, RGS5, RhoC, ROR1, RU1, RU2 (AS), SART3, SDCCAG16, sLe(a), Sperm protein 17, SSX2, STn, Survivin, TA-90, TAAL6, a TAG-72, telomerase, thyroglobulin, Tie 2, TIGIT, TLP, Tn, TPS, TRP-1, TRP-2, TRP-2, TSP-180, Tyrosinase, VEGF, VEGFR2, VISTA, WT1, XAGE 1, 43-9F, 5T4, and 791Tgp72.

In some embodiments of the disclosure, the second target epitope is in the ErbB subfamily. Erb-B1 (EGFR), Erb-B2 (HER2) are members of subclass I of the superfamily of receptor tyrosine kinases (RTKs). In some embodiments the second target epitope is an RTK. RTKs share a similar protein structure comprised of an extracellular ligand binding domain and a single transmembrane helix. They are then predominantly subdivided further into separate sub-classes via the nature of their intracellular tyrosine kinase domain (TKD) and a carboxyl (C-) terminal tail. The extracellular domain regions of RTKs exhibits a variety of conserved elements including immunoglobulin (Ig)-like or epidermal growth factor (EGF)-like domains. In some embodiments the second epitope is an epitope of a receptor tyrosine kinase. Examples of the RTK family include VEGFR2, EGFR, c-MET, IGF-I receptor, PDGFR-beta, CD115, CD117, CD140A, CD140B, CD167a, CD167b, CD172g, CD220, CD246, CD303 CD331, CD332, CD333 and CD340.

For example, in some embodiments of the disclosure the second target epitope is HER2 (human epidermal growth factor receptor 2). HER2 (also know as ErbB-2 or CD340) is a cancer-associated antigen and is an example of a receptor tyrosine kinase, in the ErbB subfamily. HER2 expression is low in healthy tissues, with up to 40-100 fold increases in expression in Her2+ cancers compared to normal tissues. Her2 overexpression is associated with many breast cancers, gastric cancers, espohageal cancers, ovarian cancers, endometrial cancers, NSCLCs and colorectal cancers. In many cancers, HER2 dimerises with other ErbB receptors and results in activation of various downstream signalling pathways, in turn leading to uncontrolled proliferation and apoptosis resistance. Overexpression of HER2 correlates with lower survival rates, and it is therefore a target to improve prognosis, as well as as a tumour marker. Monoclonal antibodies that specifically bind to epitopes of HER2 are well known in the art. For example, trastuzumab is a monoclonal antibody which binds specifically to an epitope of HER2.

In some embodiments of the disclosure, the second target epitope is EGFR. EGFR (epidermal growth factor receptor) is a cancer-associated antigen and is an example of a receptor tyrosine kinase, in the ErbB subfamily. EGFR is expressed in multiple organs and plays an important role in initiating signaling that directs the behaviour of epithelial cells and tumours of epithelial origin. EGFR-mediated signaling is also involved in controlling cell proliferation, migration, survival, and metastasis by regulating diverse cellular pathways. As with other receptor tyrosine kinases, mutations affecting EGFR activity or leading to EGFR upregulation are associated with many cancers. In fact, genetic alterations in EGFR are observed in up to 30% of solid tumours and are typically associated with poor prognosis. Disruption of EGFR signalling, by inhibiting binding of EGF to the extracellular domain or by inhibiting the intracellular tyrosine kinase activity can limit EGFR-expressing tumour growth. EGFR inhibitors can therefore be anti-cancer agents. Indeed, certain tumour cells are dependent on EGFR signaling and thus possess an "Oncogene addiction", which makes this receptor an attractive target for therapy. Monoclonal antibodies that specifically bind to epitopes of EGFR are well known in the art. For example, cetuximab is a monoclonal antibody which binds specifically to an epitope of EGFR.

In some embodiments of the disclosure, the second target epitope is an epitope of a B-lymphocyte antigen. Examples of antigens notably expressed on B-cells include CD1d, CD5, CD10, CD11b, CD19, CD20, CD21, CD22, CD23, CD24, CD32A, CD32B CD37,CD39, CD40, CD45, CD52, CD72, CD79a, CD79b, CD138, CD166, CD179A, CD179B, CD180, CD185, CD150, CD213a1 , CD213a2, CD217, CD244, CD255, CD229, CD232, CD267, CD268, CD269, CD274, CD277, CD279, CD290, CD300A, CD300C, CD305, CD307a, CD307b, CD307c, CD307d, CD307e, CD316, CD319, CD327, CD352, and CD361. For example, in some embodiments of the disclosure the second target epitope is CD19. CD19 (cluster of differentiation 19) is a cancer-associated antigen. CD19 is also an example of a Type I transmembrane glycoprotein in the immunoglobulin superfamily. In some embodiments the second target is an epitope present on a member of the immunoglobulin superfamily (IgSF). Examples of this family include, CD2, CD3, CD4, CD7, CD8, CD19, CD79A, CD79B, CD28, CD48, CD58, CD80, CD86, CD90, CD96, CD147, CD150, CD155, CD229, CD244, CD273, CD274, CD276. CD19 is widely expressed on B cells throughout their development, with the surface density of CD19 increasing as B cells mature. CD19 is involved in recruiting signalling proteins from the cytoplasm. CD19 is also involved in B cell receptor signalling pathways and is essential to the functioning of the B cell receptor. Its expression on B cells makes it a useful target against leukaemia and neoplastic lymphocytes, as well as a diagnostic biomarker for cancers arising from B cells. CD19 mutations can lead to reduced production of antibodies and immunodeficiency, therefore CD19 can also be targeted for autoimmune disease treatments. Monoclonal antibodies that specifically bind to epitopes of CD19 are well known in the art. For example, blinatumomab is a monoclonal antibody which binds specifically to an epitope of CD19.

In some embodiments of the disclosure, the second target epitope is present on a tumour stromal antigen. Examples of tumour stromal antigens include FAP alpha, CD29, CD44, CD73, CD105 and CD166. For example, in some embodiments of the disclosure the second target epitope is FAP$\alpha$ (Fibroblast activation protein $\alpha$). FAP$\alpha$ is a cancer- associated antigen also known as seprase or prolyl endopeptidase FAP. It is selectively expressed in the stroma of a range of epithelial carcinomas. FAP$\alpha$ is an example of a cell surface serine protease in the dipeptidyl peptidase family. FAP$\alpha$ is expressed by cancer associated fibroblasts (CAFs), which play an important role in the tumour microenvironment. Other molecules selectively expressed on CAFs include CD10, CD90, CD140A, and CD140B. In some embodiments the second epitope is an epitope present on a molecule selectively expressed on CAFs. Over 90% of epithelial cancers (breast, CRC skin and pancreatic cancers) are found to express FAP$\alpha$ on the surface of CAFs in the surrounding stroma. CAFs secrete the chemokine CXCL12 that binds to CXCR4 on T-cells and is immunosuppressive. FAP$\alpha$ is found to be expressed in aggressive melanoma cell lines and is significantly increased in patients with poor outcome and survival in breast cancer. FAPα has both collagenase and dipeptidase activities and promotes tumour growth, migration, invasion, metastasis and ECM degradation. Normal healthy adult tissues have no detectable FAPα expression outside areas of tissue remodelling or wound healing and therefore FAPα is a promising anti-cancer target due to its nearly exclusive expression in tumour stroma and the direct role of FAPα in various aspects of cancer progression. Monoclonal antibodies that specifically bind to epitopes of FAPα are well known in the art. For example, sibrotuzumab is a monoclonal antibody which binds specifically to an epitope of FAPα.

In some embodiments of the disclosure, the second target epitope is present on a cell-surface glycoprotein. Protein glycosylation is an important and common post-translational modification. More than 50% of human proteins are believed to be glycosylated to modulate the functionality of proteins. Aberrant glycosylation has been correlated to several diseases, such as inflammatory skin diseases, diabetes mellitus, cardiovascular disorders, rheumatoid arthritis, Alzheimer's and prion diseases, and cancer Examples of cell-surface glycoproteins include CD1a, CD1b, CD1c, CD1d, CD1e, CD3d, CD3e, CD3g, CD8a, CD8b, CD11a, CD21, CD36, CD42a, CD42b, CD42c, CD42d, CD43, CD66a, CD66f, CD177, CD235a, CD235b, CD236, CD238, CD243, CD227 and CD301. For example, in some embodiments of the disclosure the second target epitope is mesothelin (MSLN). MSLN is a cancer-associated antigen and is an example of a cell-surface glycoprotein. MSLN has limited expression in healthy cells (mesothelial cells lining the pleura, peritoneum, and pericardium) but is also expressed on a number of cancers (malignant mesothelioma and pancreatic, cholangiocarcinoma, ovarian and lung adenocarcinomas, malignant mesothelioma, pancreatic cancer, ovarian cancer, endometrial cancer, biliary cancer, gastric cancer, and paediatric acute myeloid leukaemia). MSLN is an example of a tumour-differentiation antigen. The physiological function of MSLN is unclear, but MSLN is a useful target for localisation of therapies to MSLN+ tumours, or can be exploited as a tumour marker. Monoclonal antibodies that specifically bind to epitopes of MSLN are well known in the art. For example, anetumab is a monoclonal antibody which binds specifically to an epitope of MSLN.

In various embodiments of the disclosure, the second target epitope is an epitope of an immunomodulator antigen. Immunomodulator antigens are antigens that modulate (activate or suppress) the immune system. In some embodiments, the immunomodulator antigen is a cell-surface protein (i.e. an antigen expressed on the surface of a cell, in particular an antigen expressed on the surface of an immune cell, such as lymphocytes, neutrophils, monocytes or macrophages). The immunomodulator antigen may be expressed on a Vδ1+ T-cell or may be expressed by a different cell, for example a CD4+ cell, a CD8+ cell, or a different immune cell. The immunomodulatory antigen may be selected from the group consisting of B7-1 (CD80), B7-2 (CD86), B7-DC (CD273), B7-H1 (CD274), B7-H2 (CD275), B7-H3 (CD276), B7-H4 (VTCN1), B7-H5 (VISTA), BTLA (CD272), 4-1BB (CD137), CD137L, CD24, CD27, CD28, CD38, CD40, CD40L (CD154), CD54, CD59, CD70, CTLA4 (CD152), CXCL9, GITR (CD357), HVEM (CD270), ICAM-1 (CD54), ICOS (CD278), LAG-3 (CD223), OX40 (CD134), OX40L (CD252), PD-1 (CD279), PD-L1 (CD274), TIGIT, CD314, CD334, CD335, CD337, and TIM-3 (CD366).

In some embodiments of the disclosure, the second target epitope is an epitope of a stimulatory immune checkpoint molecule. For example, in embodiments of the invention the second target epitope is OX40 (CD134). OX40 is an immunomodulator antigen and an example of a member of the TNFR superfamily (TNFRSF). In some embodiments the second target epitope is an epitope present on a TNFRSF molecule. These protein are a superfamily of cytokine receptors characterized by the ability to bind tumor necrosis factors (TNFs) via an extracellular cysteine-rich domain. Examples include CD18, CD27, CD30, CD40, CD95, CD120a, CD120b, CD134, CD137, CD265, CD268, CD269, CD270, CD271, CD357 and CD358. One such example OX40 (CD134) is a late-co-stimulatory immune checkpoint receptor expressed on CD4+ and CD8+ T-cells. OX40 is more highly expressed on CD4+ T cells and is not constitutively expressed on naive T-cells as expression of OX40 is dependent on full activation of the T-cell. When activated (e.g. by OX40L, OX40 promotes CD4+/CD8+ T-cell activation, survival and expansion of effector and memory T-cells, as well as supressing Treg activity. This supresses immune evasion by the tumour. As OX40 is a stimulatory target, therapies which target OX40 activate OX40 expressing immune cells to stimulate immune response (e.g. a CD48+ T-cell response) against the tumour. Monoclonal antibodies that specifically bind to epitopes of OX40 are well known in the art. For example, pogalizumab is a monoclonal antibody which binds specifically to an epitope of OX40.

In some embodiments, the second target epitope is an epitope present on a TNF superfamily member. For example, in some embodiments of the disclosure the second target epitope is an epitope of 4-1 BB (CD137). 4-1BB is an immunomodulator antigen and also an example of a member of the TNF superfamily. This is a protein superfamily of type II transmembrane proteins containing TNF homology domain which includes CD70, CD137, CD153, CD154, CD252, CD253, CD254, CD256, CD257, and CD258. 4-1BB (CD137) is an inducible, co-stimulatory immune checkpoint receptor expressed on T-cells, as well as NK cells, dendritic cells (DC), monocytes, neutrophils and B-cells. 4-1BB is a stimulatory antigen and is particularly expressed on activated CD8+ T-cells. In vitro 4-1BB stimulates expansion of CD4+ T-cells, CD8+ T-cells, macrophages and DCs as well as cytokine production. In vivo 4-1BB is biased towards CD8+ T-cell activation and demonstrates strong anti-tumour activity, as crosslinking of 4-1BB is shown to enhance T-cell proliferation, IL-2 secretion, survival and cytolytic activity. As 4-1BB is a stimulatory target, therapies which target 4-1BB activate 4-1BB expressing immune cells to stimulate immune response (e.g. a cytolytic CD8+ T-cell response) against the tumour. Monoclonal antibodies that specifically bind to epitopes of 4-1BB are well known in the art. For example, utomilumab is a monoclonal antibody which binds specifically to an epitope of 4-1BB.

In some embodiments of the disclosure, the second target epitope is an immune checkpoint inhibitor molecule. For example, in some embodiments of the disclosure the second target epitope is TIGIT (T cell immunoreceptor with Ig and ITIM domains). TIGIT is an immunomodulator antigen and is an example of an immune checkpoint inhibitor expressed on T-cells, including $\gamma\delta$ T cells, and NK cells. TIGIT plays a role in immune homeostasis and preventing autoimmunity by binding to its ligand (PVR/CD155) resulting in T-cell suppression. TIGIT is overexpressed on tumor infiltrated lymphocytes. Therapeutic blockade of TIGIT is desirable because it increases T-cell proliferaton, cytokine productions and degranulation. Monoclonal antibodies that specifically bind to epitopes of TIGIT are well known in the art. For example, tiragolumab is a monoclonal antibody which binds specifically to an epitope of TIGIT.

In some embodiments of the disclosure, the second target epitope is an immune checkpoint inhibitor molecule. For example, in some embodiments of the disclosure the second target epitope is PD-1 (programmed cell death protein 1). PD-1 is an immunomodulator antigen and an example of a cell surface receptor member of the immunoglobulin superfamily. PD-1 is an example of an immune checkpoint inhibitor expressed on activated CD4+/CD8+ T-cells, as well as other types of immune cells such as $\gamma\delta$ T cells, B cells and macrophage. The binding of PD-1 to its ligands results in inhibition of T-cell activation. Under normal circumstances this plays a role in immune homeostasis - protecting against autoimmunity by decreasing apoptosis in Tregs and increase apoptosis of antigen-specific T-cells. In cancer settings this results in immune escape for tumor cells by inactivating cytolytic CD8+ T-cells. Blockade of PD-1 is therefore a promising therapeutic target. Monoclonal antibodies that specifically bind to epitopes of PD-1 are well known in the art. For example, pembrolizumab is a monoclonal antibody which binds specifically to an epitope of PD-1.

In some embodiments of the disclosure, the second target epitope is a stimulatory immune checkpoint molecule. Stimulatory immune checkpoint molecules include, for example, OX40, OX40L, 4-1BB (CD137), CD137L, CD27, CD70, CD28, GITR, ICOS, CD40 and CD40L. In some embodiments of the disclosure, the second target epitope is one or more selected from OX40, OX40L, 4-1BB (CD137), CD137L, CD27, CD70, CD28, GITR, ICOS, CD40 and CD40L. The second target epitope is OX40.

In some embodiments of the disclosure, the second target epitope is an immune checkpoint inhibitor molecule. Immune checkpoint inhibitor molecules include, for example, TIGIT, CD155, PD-1, PD-L1, CTLA-4, B7-H3, B7-H4, BTLA, LAG-3, VISTA and TIM-3. In some embodiments of the disclosure, the second target epitope is one or more selected from TIGIT, CD155, PD-1, PD-L1, CTLA-4, B7-H3, B7-H4, BTLA, LAG-3, VISTA and TIM-3. In some embodiments of the disclosure, the second target epitope is one or more selected from TIGIT and PD-1.

References herein to an antigen being "on" a cell refer to antigens that are expressed on the cell surface membrane or are associated with the (extracellular side of) the cell surface membrane of such cells.

In various embodiments, the second target epitope is an epitope of a cluster of differentiation CD antigen. The cluster of differentiation (CD) nomenclature is a unifying system by which cell surface molecules are identified and named. Typically, cell surface proteins are not assigned a CD number until at least two monoclonal antibodies have been raised against said cell surface proteins. As such this system ensures all cell surface proteins assigned a CD number are tractable to being recognized and bound by specific monoclonal antibodies or fragment thereof. In various embodiments of the disclosure, the CD antigen is one selected from CD1a, CD1b, CD1c, CD1d, CD1e, CD2, CD3, CD3d, CD3e, CD3g, CD4, CD5, CD6, CD7, CD8, CD8a, CD8b, CD9, CD10, CD11a, CD11b, CD11c, CD11d, CD13, CD14, CD15, CD16, CD16a, CD16b, CD17, CD18, CD19, CD20, CD21, CD22, CD23, CD24, CD25, CD26, CD27, CD28, CD29, CD30, CD31, CD32A, CD32B, CD33, CD34, CD35, CD36, CD37, CD38, CD39, CD40, CD41, CD42, CD42a, CD42b, CD42c, CD42d, CD43, CD44, CD45, CD46, CD47, CD48, CD49a, CD49b, CD49c, CD49d, CD49e, CD49f, CD50, CD51, CD52, CD53, CD54, CD55, CD56, CD57, CD58, CD59, CD60a, CD60b, CD60c, CD61, CD62E, CD62L, CD62P, CD63, CD64a, CD65, CD65s, CD66a, CD66b, CD66c, CD66d, CD66e, CD66f, CD68, CD69, CD70, CD71, CD72, CD73, CD74, CD75, CD75s, CD77, CD79A, CD79B, CD80, CD81, CD82, CD83, CD84, CD85A, CD85B, CD85C, CD85D, CD85F, CD85G, CD85H, CD85I, CD85J, CD85K, CD85M, CD86, CD87, CD88, CD89, CD90, CD91, CD92, CD93, CD94, CD95, CD96, CD97, CD98, CD99, CD100, CD101, CD102, CD103, CD104, CD105, CD106, CD107, CD107a, CD107b, CD108, CD109, CD110, CD111, CD112, CD113, CD114, CD115, CD116, CD117, CD118, CD119, CD120, CD120a, CD120b, CD121a, CD121b, CD122, CD123, CD124, CD125, CD126, CD127, CD129, CD130, CD131, CD132, CD133, CD134, CD135, CD136, CD137, CD138, CD139, CD140A, CD140B, CD141, CD142, CD143, CD144, CDw145, CD146, CD147, CD148, CD150, CD151, CD152, CD153, CD154, CD155, CD156, CD156a, CD156b, CD156c, CD157, CD158, CD158A, CD158B1, CD158B2, CD158C, CD158D, CD158E1, CD158E2, CD158F1, CD158F2, CD158G, CD158H, CD158I, CD158J, CD158K, CD159a, CD159c, CD160, CD161, CD162, CD163, CD164, CD165, CD166, CD167a, CD167b, CD168, CD169, CD170, CD171, CD172a, CD172b, CD172g, CD173, CD174, CD175, CD175s, CD176, CD177, CD178, CD179a, CD179b, CD180, CD181, CD182, CD183, CD184, CD185, CD186, CD187, CD188, CD189, CD190, CD191, CD192, CD193, CD194, CD195, CD196, CD197, CDw198, CDw199, CD200, CD201, CD202b, CD203c, CD204, CD205, CD206, CD207, CD208, CD209, CD210, CDw210a, CDw210b, CD211, CD212, CD213a1, CD213a2, CD214, CD215, CD216, CD217, CD218a, CD218b, CD219, CD220, CD221, CD222, CD223, CD224, CD225, CD226, CD227, CD228, CD229, CD230, CD231, CD232, CD233, CD234, CD235a, CD235b, CD236, CD237, CD238, CD239, CD240CE, CD240D, CD241, CD242, CD243, CD244, CD245[17], CD246, CD247, CD248, CD249, CD250, CD251, CD252, CD253, CD254, CD255, CD256, CD257, CD258, CD259, CD260, CD261, CD262, CD263, CD264, CD265, CD266, CD267, CD268, CD269, CD270, CD271, CD272, CD273, CD274, CD275, CD276, CD277, CD278, CD279, CD280, CD281, CD282, CD283, CD284, CD285, CD286, CD287, CD288, CD289, CD290, CD291, CD292, CDw293, CD294, CD295, CD296, CD297, CD298, CD299, CD300A, CD300C, CD301, CD302, CD303, CD304, CD305, CD306, CD307, CD307a, CD307b, CD307c, CD307d, CD307e, CD308, CD309, CD310, CD311, CD312, CD313, CD314, CD315, CD316, CD317, CD318, CD319, CD320, CD321, CD322, CD323, CD324, CD325, CD326, CD327, CD328, CD329, CD330, CD331, CD332, CD333, CD334, CD335, CD336, CD337, CD338, CD339, CD340, CD344, CD349, CD351, CD352, CD353, CD354, CD355, CD357, CD358, CD360, CD361, CD362, CD363, CD364, CD365, CD366, CD367, CD368, CD369, CD370, and CD371.

**Healthy Cell Sparing**

While the mechanisms by which γδ T-cells recognize antigens and distinguish between healthy and diseased cells are not fully understood (Ming Heng and Madalene Heng, Antigen Recognition by yδ T-Cells. Madame Curie Bioscience Database [Internet], Austin (TX): Landes Bioscience; 2000-2013), the fact that γδ T-cells are able to distinguish between healthy cells and diseased cells and exhibit remarkable diseased cell polycytotoxicity (see non-limiting example cell types in Table 1) this means that they can be leveraged to provide improved medicaments with improved therapeutic windows. Further, by leveraging such γδ T-cell capabilities, there is provided an opportunity to treat disease while sparing healthy cells, by colocalizing γδ T-cells with diseased cells even when a particular cancer antigen, inflammatory antigen, or pathogen antigen is either not known, or is also present on healthy cells, in a particular patient.

**Table 1. Example cancer cells killed by polycytotoxic human Vδ1+ cells**

| | |
|---|---|
| Breast Cancer | |
| M-CSF7, T47D, MDA-MB-231 | Mahvi et al (1993) Cancer Imm. Immunother. (1993) 37:181 - 186 Dutta I et al (2017). Front. Immunol. 8:776 |
| Lung Cancer | |
| GLC1, N592 | Ferrarini et al (1996) Jn of Nat. Cancer Inst., Volume 88 (7) pp 436-441 |
| Pancreas Cancer | |
| panc89, QGP-1, PANC-1 | Maeurer et al (1996) JEM 183 (4) 1681-1696 Kitayama 1993 Clin Exp Imm 93 (3) 442-7 |
| Gastrointestinal Cancer | |
| HT29, HCT116, Y, SKCO1, Caco2, HCT116, Lovo, DLD-1, SW480 | Wu, et al. (2015), OncoImmunology, 4:3, e992749 Mikulak, et al (2019) JCI Insight. 4(24):e125884 Groh et al (1999) PNAS 96 (12) 6879-6884 |
| Neuroblastoma | |
| LAN1, KELLY | Fisher et al (2014) Clin Cancer Res; 20(22); 5720-32 |
| Melanoma | |
| A375 | Cordova (2012) Plos ONE 7 (11) e49878 |
| Ovarian Cancer | |
| OV-1063,<br>SW626 | Groh et al (1999) PNAS 96 (12) 6879-6884 |
| Liver Cancer | |
| HepG2 | Groh et al (1999) PNAS 96 (12) 6879-6884 |
| Cervical Cancer | |
| HeLa | Groh et al (1999) PNAS 96 (12) 6879-6884 |
| Prostate Cancer | |
| DV145, PC-3 | Groh et al (1999) PNAS 96 (12) 6879-6884 |

(continued)

| | |
|---|---|
| Multiple Myeloma | |
| ARH77, U266 | Knight et al (2012) Cytotherapy, 14:9, 1110-1118, |
| AML | |
| KG-1, KASUMI-1, OCI-AML3, U937, HL60, MV4 11, AML193, HEL, THP-1 | Lorenzo et al (2019) Canc Imm Res 7 (4) |
| CLL | |
| MEC-1 | Almeida et al (2016) Clin Can Res 22 (23) |

By way of one non-limiting example, recent studies with CD3xHER2 multi-specifics highlight the challenges of current or conventional approaches. Specifically, use of such conventional approaches can result in less favorable toxicity profiles. This is because like many other tumour associated antigens (TAAs), the HER2 antigen is not only expressed in cancers such as breast cancer but is also expressed on healthy tissues such as heart cells. Hence use of CD3xHER2 medicaments which engage and co-localize all T-cells with HER2 positive cells can result in less favorable therapy windows or therapeutic indices. This is because such medicaments will engage all T-cells of which the vast majority in circulation will be αβ T-cells (CD4+ positive, CD8+ positive etc.). And once αβ T-cells are co-localized with HER2 positive cells, such conventional αβ T-cells exhibit limited capabilities to spare HER2+ healthy cells and limited capabilities to kill only diseased HER2+ diseased cells. Consequently, and by way of this example, in cynomolgus studies whereby such CD3xHER2 bispecifics were administered, early euthanasia (even on the day of dosing) was required in some situations. Further, during this example study (see Staflin et al. (2020) JCI Insight 5(7): e133757) it was concluded that retargeting T cells to kill HER2-expressing cells may induce adverse effects on HER2-expressing tissues. It was noted that with exception of the liver, all affected or damaged tissues expressed HER2.

In a further non-limiting example, a second binding specificity may be to a tumour associated moiety also involved in controlling or regulating immune cell function. For example, the second specificity may be designed to target a so-called "checkpoint inhibitor" such as PD-L1 (CD274) or CD155. Once again, neither PDL-1 and CD155 are 100% disease specific. Both proteins can also be expressed on healthy cells. However, multi-specific antibodies designed to specifically co-localize Vδ1+ cells to either PD-L1 positive cells or CD155 positive cells may result in the selective killing PD-L1 or CD155 positive diseased or cancerous cells. Further targeting diseased-associated checkpoint inhibitors present on diseased cells such as cancer cells will not only co-localize Vδ1+ cells to such tumours but may also confer additional favourable effects, for example by modulating or dampening PD-1/PD-L1 or TIGIT/CD155 signalling which otherwise may negatively regulates T cell-mediated immune responses to the disease.

Hence instead of employing such conventional approaches, provided herein are multi-specific antibodies wherein at least one first binding specificity is able to bind Vδ1+ cells and at least one second binding specificity is able to bind targets present on diseased tissues and cells. The use of such multi-specific antibodies in this way may thereby result in the co-localization of Vδ1+ cells to diseased cells expressing the second target. Further, and given such disease associated targets are not often 100% disease specific, this approach of targeting and co-localizing Vδ1+ effector cells specifically, may be more preferred over conventional approaches. This is because Vδ1+ effector cells may be capable of recognizing stress patterns in diseased or infected cells and so able to selectively kill diseased cells whilst sparing healthy cells also expressing the same target.

The multispecific antibodies presented herein are therefore able to engage on the TCR of vδ1 cells but full activation does not occur unless tumour cells are also present. Full engagement of the presently presented antibodies on the TCR leads to partial downregulation and it is believed the vδ1 cells bound by the presently presented antibodies only become fully activated and become cytotoxic when in the presence of stressed cells such as tumour cells. This is shown, for example, in Figure 25, I, J, K and Figure 38 A-F. This represents another vital safety advantage for the approach presented herein, since off target cytotoxicity is reduced and the full potency of the multispecific antibodies to activate vδ1 cells is only unleashed in the presence of tumour cells, meaning healthy cells (even healthy cells expressing the second target antigen of the multispecific antibody) are spared.

One mechanism behind γδ T cells being able to detect stress signals on tumour cells is believed to be due to the NCRs (natural cytotoxicity receptors) they express. The NCRs are able to engage NCR ligands on tumour cells. A dual mechanism of activation may therefore be employed, wherein the γδ T cells are activated via TCR stimulation, including via NCRs, which can sense the tumour cells to enable full activation and cytotoxicity.

This contrasts with stimulation of αβ T cells via CD3, for example, wherein all stimulation is via the TCR. Such cells are therefore almost indiscriminate between healthy or transformed cells because they do not have mechanisms such as antigen presentation independent sensing of tumour cells, for example via NCRs. Therefore, if CD3 antibodies are Fc enabled they will attract other immune cells which can trigger a cascade of unpredictable and desirable events such as cytokine storms, exhaustion and even overactivation of immune cells leading to, for example, NK cells killing T cells etc. In the present approach, stimulation of γδ T cells with the presently presented multispecific antibodies do not lead to such concerns because γδ T cells) are able to distinguish between healthy cells and tumour cells, including via their NCR sensing mechanism and therefore selectively kill stressed cells such as cancer cells or virally infected cells due to this diseased cell specificity

In a further non-limiting example, a patient may have liver cancer, where no liver cancer specific antigen is known in the patient. In this instance, the second specificity of the multi-specific antibody can be to an epitope present on many or all liver cells, such as, for example, asialoglycoprotein receptor 1. This will then colocalize the γδ T-cells to the liver, where the γδ T-cells can kill the liver cancer cells, while sparing the healthy liver cells. This is shown, for example, in Figure 25, I, J, K and Figure 38 A-f. By way of a third non-limiting example, in a patient that has lung cancer, where no lung cancer antigen is known in the patient, the second specificity of the multi-specific antibody can be to an epitope on a normal lung cell, such as, for example, SP-1. This will colocalize the γδ T-cells to the lung, where the γδ T-cells can kill the lung cancer cells, while sparing the healthy lung cells. By way of a fourth non-limiting example, in a patient that has B cell lymphoma, where no B cell lymphoma antigen is known in the patient, the second specificity of the multi-specific antibody can be to an epitope on normal B cells, such as, for example, CD19. This will colocalize the γδ T-cells to B cells, where the γδ T-cells can kill the lymphoma cells, while sparing healthy B cells. Cell-specific antigens, cell-associated antigens, tissue-specific antigens, and tissue-associated antigens are well known in the art and any such antigen can be targeted by the second specificity of the multi-specific antibodies of the invention.

The second binding specificity may target an antigen on the same cell as Vδ1 or on a different cell of the same tissue type or of a different tissue type. In certain embodiments, the target epitope may be on a different cell including a different T-cell, a B-cell, a tumour cell, an autoimmune tissue cell or a virally infected cell. Alternatively, the target epitope may be on the same cell.

The multi-specific antibodies, or fragments thereof, can be made in any format, so long as the antibody, or fragment thereof, has multiple specificities. The multispecific (suitably bispecific) antibodies of the invention comprise at least two binding domains, a first binding domain and a second binding domain. The first binding domain confers binding specificity to a first target antigen (or epitope of a first target antigen), wherein the first target epitope is an epitope of the variable delta 1 (Vδ1 or TRDV1) chain of a γδ T cell receptor (TCR). The second binding domain confers binding specificity to a second target antigen (or epitope of a second target antigen), wherein the second target antigen is OX40. The second target antigen may be a cancer antigen or a cancer-associated antigen, or the second antigen may be an immunomodulatory antigen.

Examples of multi-specific antibody formats include, but are not limited to, CrossMab, DAF (two-in-one), DAF (four-in-one), DutaMab, DT-IgG, Knobs-in-holes (KIH), Knobs-in-holes (common light chain), Charge pair, Fab-arm exchange, SEEDbody, Triomab, LUZ-Y, Fcab, κλ-body, orthogonal Fab, DVD-IgG, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, Zybody, DVI-IgG(four-in-one), Nanobody, Nanoby-HAS, BiTE, Diabody, DART, TandAb, scDiabody, scDiabody-CH3, Diabody-CH3, Triple Body, Morrison formats, Miniantibody, Minibody, TriBi minibody, scFv-CH3 KIH, Fab-scFv, scFv-CH-CL-scFv, F(ab')2, F(ab)2-scFv2, scFv-KIH, Fab-scFv-Fc, Tetravalent HCAb, scDiabody-Fc, Diabody-Fc, Tandem scFv-Fc, Intrabody, Dock and Lock, ImmTAC, HSAbody, scDiabody-HAS, Tandem scFv-Toxin, IgG-IgG, ov-X-Body, duobody, $mab^2$ and scFv1-PEG-scFv2 (see Spiess et al. (2015) Molecular Immunology 67:95-106).

An antibody or fragment thereof as described herein may also be assessed by measuring its capacity for enhanced functionality in a multi-specific format such as a bispecific or trispecific format. Surprisingly through such studies it is possible to identify yet further functional improvements in the performance of the antibodies or fragments thereof as described herein (see Example 20 and 21).

Various antibody-derived multi-specific formats have been described previously and are typically built empirically from the component binding parts. Typically, once constructed, the performance of such multi-specific or multi-target binding formats as described herein may be measured in one or more of the aforementioned model systems (cell killing, cell proliferation, healthy cell sparing/diseased cell specific models etc). Optionally they are also compared to said component parts and other comparator molecules.

Whilst not being limited by this approach, in general when constructing antibodies as multi-specific antibodies, the binding domain modules to each target (first, second, third etc) are optional built from scFv, Fab, Fab', F(ab')2, Fv, variable domain (e.g. VH or VL), diabody, minibody or full length antibodies. For example, each said binding domain or module is created in one or more of the following non-limiting formats wherein binding domains comprising variable domains, and/or full length antibodies, and/or antibody fragments, are operatively linked in series to generate multi-specific antibodies.

Remarkably, multi-specific antibodies comprising at least one (first) binding domain targeting the Vδ1 chain of a γδ TCR as described herein are further enhanced when said first binding domain is formatted with a multi-specific antibody format comprising at least one second binding domain against either tissue ("solid") and haemopoietic ("liquid") disease or cell-type associated targets.

*Multi-specific antibodies - non-limiting examples:*

To outline the applicability of the approach a series of non-limiting example multi-specific antibodies were constructed. These multi-specific antibodies comprised at least one (first) binding domain targeting the Vδ1 chain of a γδ TCR and at least one (second) binding domain targeting a disease associated target:

A first example; Vδ1-EGFR multi-specific antibody:

For this example, one binding domain (to the first target) comprised intact antibody moieties; specifically, VH-CH1-CH2-CH3 and cognate VL-CL partners, whilst the second binding domain (to the second target) comprised an antibody fragment; specifically, a scFv format. The two binding modules were then fused with aid of a linker. The resulting bispecific format is sometimes termed a 'Morrison format'. In this instance a first binding domain targets the Vδ1 chain of a γδ TCR and a second binding domain targets EGFR (see Example 20).

A second example; Vδ1-EGFR multi-specific antibody:

For this example, one binding domain (to the first target) comprised an antibody variable domain (specifically comprising a VH and cognate VL domain) whilst the second binding domain (to the second target) comprises a binding domain within a heavy chain constant domain (CH1-CH2-CH3) (see also EP2546268 A1 Table 1 / EP3487885 A1). The resulting bispecific comprises a first binding domain targeting the Vδ1 chain of a γδ TCR and a second binding domain targeting EGF receptor (see Example 20).

A third example; Vδ1-CD19 multi-specific antibody:

For this example, one binding domain (to the first target) comprised intact antibody moieties specifically, VH-CH1-CH2-CH3 and cognate VL-CL partners, whilst the second binding domain (to the second target) comprised an antibody fragment; specifically, a scFv format. The two binding modules were then fused with aid of a linker. For this example, the resulting bispecific comprised a first binding domain targeting the Vδ1 chain of a γδ TCR and a second binding domain targeting CD19 (see Example 21).

A fourth example; a further Vδ1-CD19 multi-specific antibody

For this example, an additional multi-specific antibody specifically binding to both TRDV1 and CD19 was prepared and tested for antigen binding (including both human and cyno-TRDV1), Vδ1-cell activation and Vδ1-cell cytotoxicity (see Examples 22 and 23).

A fifth example; a Vδ1-Her2 multi-specific antibody

For this example, a multi-specific antibody specifically binding to both TRDV1 and Her2 was prepared and tested for antigen binding, Vδ1-cell activation and Vδ1-cell cytotoxicity (see Example 24).

A sixth example; a further Vδ1-EGFR multi-specific antibody

For this example, an additional multi-specific antibody specifically binding to both TRDV1 and EGFR was prepared and tested for antigen binding, Vδ1-cell activation and Vδ1-cell cytotoxicity (see Examples 26 and 27).

A seventh example; a Vb1-FAPa multi-specific antibody

For this example, one binding domain (to the first target) comprised intact antibody moieties specifically, VH-CH1-CH2-CH3 and cognate VL-CL partners, whilst the second binding domain (to the second target) comprised an antibody fragment; specifically, a scFv format. The two binding modules were then fused with aid of a linker. For this example, the resulting bispecific comprised a first binding domain targeting the Vδ1 chain of a γδ TCR and a second binding domain targeting FAPα (see Example 29).

An eighth example; a Vδ1-mesothelin multi-specific antibody

For this example, one binding domain (to the first target) comprised intact antibody moieties specifically, VH-CH1-CH2-CH3 and cognate VL-CL partners, whilst the second binding domain (to the second target) comprised an antibody fragment; specifically, a scFv format. The two binding modules were then fused with aid of a linker. For this example, the resulting bispecific comprised a first binding domain targeting the Vδ1 chain of a γδ TCR and a second binding domain targeting mesothelin (see Example 30).

A ninth example; a Vδ1-PD-1 multi-specific antibody

For this example, one binding domain (to the second target) comprised intact antibody moieties specifically, VH-CH1-CH2-CH3 and cognate VL-CL partners, whilst the second binding domain (to the first target) comprised an antibody fragment; specifically, a scFv format. The two binding modules were then fused with aid of a linker. For this example, the resulting bispecific comprised a first binding domain targeting PD-1 and a second binding domain targeting the Vδ1 chain of a γδ TCR (see Example 32).

A tenth example; a Vδ1-4-1BB multi-specific antibody

For this example, one binding domain (to the first target) comprised intact antibody moieties specifically, VH-CH1-CH2-CH3 and cognate VL-CL partners, whilst the second binding domain (to the second target) comprised an antibody fragment; specifically, a scFv format. The two binding modules were then fused with aid of a linker. For this example, the resulting bispecific comprised a first binding domain targeting the Vδ1 chain of a γδ TCR and a second binding domain targeting 4-1BB (see Example 33).

An eleventh example; a Vδ1-OX40 multi-specific antibody

For this example, one binding domain (to the first target) comprised intact antibody moieties specifically, VH-CH1-CH2-CH3 and cognate VL-CL partners, whilst the second binding domain (to the second target) comprised an antibody fragment; specifically, a scFv format. The two binding modules were then fused with aid of a linker. For this example, the resulting bispecific comprised a first binding domain targeting the Vδ1 chain of a γδ TCR and a second binding domain targeting OX40 (see Example 34).

A twelfth example; a Vδ1-TIGIT multi-specific antibody

For this example, one binding domain (to the second target) comprised intact antibody moieties specifically, VH-CH1-CH2-CH3 and cognate VL-CL partners, whilst the second binding domain (to the first target) comprised an antibody fragment; specifically, a scFv format. The two binding modules were then fused with aid of a linker. For this example, the resulting bispecific comprised a first binding domain targeting TIGIT and a second binding domain targeting the Vδ1 chain of a γδ TCR (see Example 35).

Remarkably in all said examples comprising at least one (first) binding domain targeting the Vδ1 chain of a γδ TCR at least one second domain targeting a second epitope enhanced functionality was observed versus the controls and component parts (see Examples 20 to 35 herein).

Collectively, these non-limiting examples highlight the flexibility of the multispecific antibodies or fragments thereof as described herein. These non-limiting examples outline that multi-specific antibody approach wherein antibodies of fragments thereof targeting the germline Vδ1 chain (amino acids 1-90 of SEQ ID NO:1) may be further enhanced by combining with second binding domains to form multi-specific antibodies. By way of non-limiting examples, multi-specific antibodies are provided herein with enhanced functionality and which contain binding domains comprising intact antibodies (VH-CH1-CH2-CH3 and VL-CL), and/or variable domains (VH and cognate VL or VH-CH1 and cognate VL-CL), and/or antibody fragments (scFv).

In one embodiment multi-specific antibody binding domains which target Vδ1 chain of a γδ TCR (the first target) may comprise (i) one or two or more antibody binding domains each comprising a heavy chain (VH-CH1-CH2-CH3) and a cognate light chain partner (VL-CL) and/or (ii) one or two or more antibody binding domains each comprising a heavy chain variable domain (VH, or VH-CH1) and a cognate light chain variable domain partner (VL, or VL-VC) and/or (iii) one or two or more antibody binding domains each comprising a CDR-containing antibody fragment.

In one embodiment there is provided a multi-specific antibody comprising at least one first antibody-derived binding domain targeting the Vδ1 chain of a γδ TCR and which is operatively linked to at least one second antibody binding domain targeting a second epitope, wherein the second epitope is an epitope of OX40. Optionally, said binding domains comprise at least one or more VH and cognate VL binding domain, or one or more VH-CH1-CH2-CH3 and cognate VL-CL binding domain, or one or more antibody fragment binding domains. Optionally, said second binding domain targets a second epitope associated with, or expressed on, the cell surface of a cell. Optionally, said second epitope is located on a cell surface polypeptide associated with a diseased cell or tumour cell or a virally infected cell or an autoimmune tissue cell. Said second epitope or epitopes are located on the disease and cell-type associated CD19, EGFR, Her2, FAPα, mesothelin, PD-1, 4-1BB, OX40 or TIGIT antigens. Optionally, said multi-specific antibody comprising at least one first antibody-derived binding domain targeting the Vδ1 chain of a γδ TCR is operatively linked to a second binding domain binding the EGF receptor and comprising one or more of the following heavy chain modifications in accordance with EU nomenclature; L358T and/or T359D and /or K360D and/or N361G and/or Q362P and/or N384T and/or G385Y and/or Q386G and/or D413S and/or K414Y and/or S415W and/or Q418Y and or Q419K.

Optionally, a multi-specific antibody comprising at least one first antibody-derived binding domain targeting the Vδ1 chain of a γδ TCR is operatively linked to a second binding domain comprising SEQ ID NO:147 or SEQ ID NO: 148 or SEQ ID NO:149 or SEQ ID NO:157 or functionally equivalent binding variants thereof and which target EGFR, CD19, Her2, FAPα, mesothelin, PD-1, 4-1BB, OX40 or TIGIT. Optionally, resulting multi-specific antibodies comprise SEQ ID NO: 140 or SEQ ID NO: 141 or SEQ ID NO:142 or SEQ ID NO: 144 or SEQ ID NO: 145 or SEQ ID NO: 146 or SEQ ID NO: 158 or SEQ ID NO: 159. Said entities are hereby included as non-limiting novel compositions to aid with understanding.

In one aspect of the invention multi-specific antibodies of the invention can be used in therapeutically effective amounts to treat a disease or disorder such to ameliorate at least one sign or symptom of a disease or disorder.

In one embodiment, there is provided a method of selecting or characterizing or comparing antibodies or fragment thereof as described herein which bind to the Vδ1 chain of a γδ TCR in a multi-specific antibody format wherein said multi-specific antibody is applied to Vδ1+ cells in order to measure the conferred effect by said multi-specific entity Vδ1+ cells (e.g. upon said Vδ1+ phenotype and/or cytotoxicity and/or diseased-cell specificity and/or enhancement thereof).

## Immunoconjugates

The multispecific antibodies or fragments thereof of the present invention may be conjugated to a therapeutic moiety, such as a cytotoxin or a chemotherapeutic agent. Such conjugates may be referred to as immunoconjugates. As used herein, the term "immunoconjugate" refers to an antibody which is chemically or biologically linked to another moiety, such as a cytotoxin, a radioactive agent, a cytokine, an interferon, a target or reporter moiety, an enzyme, a toxin, a peptide or protein or a therapeutic agent. The antibody may be linked to the cytotoxin, radioactive agent, cytokine, interferon, target or reporter moiety, enzyme, toxin, peptide or therapeutic agent at any location along the molecule so long as it is able to bind its target. Examples of immunoconjugates include antibody drug conjugates and antibody-toxin fusion proteins. In one embodiment, the agent may be a second different antibody to Vδ1. In certain embodiments, the antibody may be conjugated to an agent specific for a tumor cell or a virally infected cell. The type of therapeutic moiety that may be conjugated to the anti-Vδ1 antibody and will take into account the condition to be treated and the desired therapeutic effect to be achieved. In one embodiment, the agent may be a second antibody, or fragment thereof, that binds to a molecule other than Vδ1.

## Medicaments for modulating γδ T cells

The multispecific antibodies or fragments thereof as described herein may be used to modulate or useful for modulating delta variable 1 chain (Vδ1) T cells in a patient *in situ (i.e. in vivo),* via the TRDV1-binding domain. The multispecific antibodies or fragments thereof may be comprised in medicaments for such purposes.

Modulation of Vδ1 T cells may include:

- expansion of the Vδ1 T cells, e.g. by selectively increasing the number of Vδ1 T cells or promotion of survival of Vδ1 T cells;
- stimulation of the Vδ1 T cells, e.g. by increasing Vδ1 T cell potency, i.e. increasing target cell killing;
- prevention of Vδ1 T cell exhaustion, e.g. by increasing persistence of the Vδ1 T cells;
- degranulation of Vδ1 T cells;
- increase in NCR expression;
- immunomodulation of the Vδ1 T cells, e.g. by downregulation of Vδ1 TCR cell surface expression, i.e. by causing Vδ1 TCR internalisation or reduced expression of Vδ1 TCR protein, or blocking the Vδ1 TCR from binding; and/or
- downregulation of a TCR/CD3 complex

Unlike anti-Vδ1 antibodies of the prior art which focus on depletion of Vδ1 T-cells, the multispecific antibodies of the present invention are useful for the activation of Vδ1 T-cells via the TRDV1-binding domain. Although they may cause downregulation of the TCRs on T-cells to which they bind, they do not cause Vδ1 T-cell depletion, but rather they stimulate the T-cells and hence may be useful in therapeutic settings that would benefit from the activation of this compartment of T-cells. Activation of Vδ1 T-cells is evident through TCR downregulation, CD3 downregulation, changes in activation markers such as CD25 and Ki67 and degranulation marker CD107a. Activation of Vδ1 T-cell in turn triggers release of inflammatory cytokines such as INFγ and TNFα to promote immune licencing.

In one embodiment of the invention, there is provided a multispecific anti-Vδ1 antibody or antigen-binding fragment thereof, characterised in that it:

a. causes downregulation of TCRs on Vδ1 T-cells;
b. does not exhibit CDC or ADCC; and
c. does not deplete Vδ1 T-cells.

In some embodiments, the multispecific anti-Vδ1 antibody or antigen-binding fragments also stimulates Vδ1 T-cell proliferation.

T-cell depletion is the process of T cell death removal or reduction. References to the multispecific antibodies or antigen binding fragments not depleting the Vδ1 T cells refers to a depletion of less than about 30% or less than about 20% (preferably less than about 10%) of the viable Vδ1 T+ cell population when incubated by one or more of the antibodies of the invention as described herein, and as measured by any via suitable means in a controlled study (for example via controlled flow cytometry methodology or via other established controlled assays).

ADCC and CDC are mechanisms by which T-cell depletion may occur. Reference to the antibodies or antigen binding fragments herein not causing ADCC or CDC refers to a depletion of less than about 30% or less than about 20% (preferably less than about 10%) of the viable Vδ1 T+ cell population via ADCC and/or CDC when incubated by one or more of the antibodies of the invention as described herein, as measured by any via suitable means (for example via controlled flow cytometry methodology or via other established controlled assays).

In one embodiment, there is provided a multispecific anti-Vδ1 antibody or antigen-binding fragment thereof, characterised in that it does not induce secretion of IL-17A. IL-17A (Interleukin-17A) is a pro-tumorigenic cytokine which is produced by activated T-cells. IL-17A can enhance tumour growth and dampen the anti-cancer immune response. As shown in **Figure 38, G to I,** anti-vδ1 antibodies do not induce secretion of IL-17A, whereas anti-CD3 antibodies do. Reference to the antibodies or antigen binding fragments herein not inducing secretion of IL-17A refers to inducing less than about 30%, or less than about 20%, or less than about 10% of the IL-17A secretion induced by equivalent CD3 multispecific antibodies.

Some of the following sections relate to anti-Vδ1 antibodies provided in a monospecific format, whose use in multispecific (suitably bispecific) formats is specifically contemplated by the present invention. Suitably, the functional properties of the antibodies when provided in a monospecific format are shared by the multispecific antibodies of the invention that additionally specifically bind to a second antigen.

## Medicaments that modulate immune cell markers on Vδ1+ cells

The multispecific antibody or fragment thereof may modulate immune cell markers of Vδ1+ cells upon administration to a patient.

An multispecific antibody or fragment thereof described herein may also be assessed for its suitability for its therapeutic use by measuring γδ T modulation, and such an assessment may be carried out when the antibody is provided in a monospecific format. For example, by measuring a change in the levels of CD25 or CD69 or CD107a present on a Vδ1+ T-cell or cells in a model system. Such markers are often used as markers of lymphocyte modulation (e.g. proliferation or degranulation) and can be measured following application of an antibody or fragment thereof as described herein, e.g. by flow cytometry. Surprisingly, during such assessments (e.g. see e.g. Examples 7, 17, 18) it was observed that monospecific versions of the multispecific antibodies as described herein conferred measurably higher levels of CD25 or CD69 or CD107a levels on target Vδ1+ T-cells. Optionally, the change in phenotype of a Vδ1+ cell or population thereof tested in the model system can then be compared to the change in phenotype when an alternative comparator antibody is applied (e.g. OKT-3, TS8.2, etc.) to said equivalent γδ T cells.

Hence in one aspect of the disclosure, there is provided a method of assessing an antibody or fragment thereof which binds to the Vδ1 chain of a γδ TCR for therapeutic use comprising administering the antibody or fragment thereof to a cell population comprising Vδ1+ cells and determining the effect on the level of CD25 and/or CD69 and/or CD107a on the surface of the Vδ1+ cells. The effect on the level of CD25, CD69 and/or CD107a may be determined/measured over a period of time. It will be understood that the effect can be measured in comparison to the level of CD25 and/or CD69 and/or CD107a on the surface of the Vδ1+ cell when said antibody is not applied to said cell over the same period of time. In a further aspect of the disclosure there is provided a method of selecting or characterizing or comparing the antibodies or fragments thereof as described herein which bind to the Vδ1 chain of a γδ TCR by adding said antibodies to a cell population comprising Vδ1+ cells and then measuring the level (or expression) of CD25 or CD69 or CD107a on the surface of said Vδ1+ cells.

**Medicaments that modulate growth properties or numbers of Vδ1+ cells**

The multispecific antibody or fragment thereof may modulate the growth properties of Vδ1+ cells upon administration to a patient. For example, the multispecific antibody or fragment thereof may expand Vδ1+ cells.

An alternate approach to measuring γδ T proliferation may include measuring the change in relative number of Vδ1+ cells over time when applying an antibody or fragment thereof as described herein to model systems containing said cells. Surprisingly, during such assessments it was observed that antibodies as described herein when provided in a monospecific format where able to measurably increase the number of said Vδ1+ T-cells (e.g. see e.g. Example 10, 17 and 18), Optionally this change in number can then be compared to the change in number observed when an alternative comparator antibody is applied (e.g. anti-OKT3) to said model systems.

Hence in another aspect of the disclosure, there is provided a method of assessing an antibody or fragment thereof which binds to the Vδ1 chain of a γδ TCR comprising administering the antibody or fragment thereof to a cell population comprising Vδ1+ cells and determining the effect on the number of Vδ1+ cells in the population. The effect on cell number can be determined/measured over a period of time. It will be understood that the effect can be measured in comparison to the effect on cell numbers observed when said antibody is not applied to the cell population for the same period of time. In a further aspect of the disclosure there is provided a method of selecting or characterizing or comparing antibodies or fragment thereof as described herein which bind to the Vδ1 chain of a γδ TCR by applying said antibodies to a cell population comprising Vδ1+ cells and then measuring the number of said cells over time.

**Medicaments that modulate the proliferative capacity and numbers of Vδ1+ cells**

An ideal therapeutic multispecific antibody or fragment thereof as described herein which binds to the Vδ1 chain of a γδ TCR may be one that is capable of enhancing the proliferation of Vδ1+ cells *in vivo.* Such antibodies can then be employed as medicaments designed to specifically increase the Vδ1+ cell number in a subject or patient. For example:

*Cancer:*

Relative increases in the numbers of Vδ1+ cells have been reported as a positive prognostic indicator associated with improved outcomes for many cancer (for example see Gentles et al (2015) Nature Immunology 21: 938-945; Wu et al. (2019) Sci. Trans. Med. 11(513): eaax9364; Catellani et al. (2007) Blood 109(5): 2078-2085). In one embodiment, presented herein is a medicament capable of increasing the relative or absolute numbers of Vδ1+ cells *in situ* within in a cancer patient.

*Pathogenic/Parasitic/Viral Infections:*

Vδ1+ cell enrichment is observed during host defense against numerous acquired pathogenic/parasitic/viral infections. For recent general review see Zhao et al. (2018) Immunol. Res. 2018:5081634. Furthermore, increased numbers Vδ1+ are also considered protective against a variety of DNA and RNA viral infections. For example, increased numbers are also considered protective during CMV infections associated with allogeneic transplants (see van Dorp et al. (2011) Biology of Blood and Marrow Transplantation 17(2): S217). Additionally, Vδ+ cell numbers increase in patients with coronavirus infection (Poccia et al. (2006) J. Infect. Dis. 193(9): 1244-1249).

In another embodiment, presented herein is a medicament capable of increasing the relative or absolute numbers of Vδ1+ cells in a subject or patient harboring a pathogenic infection.

*Stem Cell Transplant:*

Increased numbers of Vδ1+ cells have also been associated with less disease relapse, fewer viral infections, higher overall and disease-free survival and favorable clinical outcomes in general during hematopoietic stem cell transplant (for example see Aruda et al. (2019) Blood 3(21): 3436-3448 and see Godder et al. (2007) Bone Marrow Transplantation 39: 751-757). Hence another embodiment, presented herein is a medicament capable of increasing the relative or absolute numbers of Vδ1+ cells in a subject as part of a treatment regimen supporting a stem cell transplant.

Consequently, a medicament capable of preferentially or specifically increasing the numbers of Vδ1+ cells *in-situ* is highly desirable.

## Medicaments that maintain or induce or increase Vδ1+ cell cytokine secretion

Cytokines are a large group of proteins, peptides or glycoproteins that are secreted by specific cells of immune system. They are a category of signaling molecules that mediate and regulate immunity, inflammation, and hematopoiesis. A number of cytokines have been implicated in ameliorating signs and symptoms of disease through either direct or indirect modulation of the tumour and cellular microenvironment, autoimmune tissue and associated microenvironment, or virally infected tissue or cellular environment. Exemplar pro-inflammatory cytokines include tumour necrosis factor-alpha (TNFα) and Interferon-gamma (IFNy).

However, many such cytokines exhibit unfavourable toxicity when dosed systemically. For example, whilst TNFα can induce the haemorrhagic necrosis of transplanted tumours, and has been reported to exert synergic anti-tumour effects when combined with other chemotherapeutic drugs, various clinical trials with systemic recombinant human TNFα (rhTNFα) have highlighted significant dose limiting side effects inclusive of hypotension, rigors, phlebitis, thrombocytopenia, leucopenia and hepatotoxicity, fever, fatigue, nausea/vomiting, malaise and weakness, headache, chest tightness, low back pain, diarrhoea and shortness of breath.

Use of recombinant IFNγ also faces similar systemic toxicity challenges. For example, whilst in a cancer setting IFNγ can exert favorable pleiotropic effects including MHC class I and II upregulation to stimulate anti-tumour immunity, increasing T-cell infiltration, conferring anti-angiogenesis effects, inducing chemokine / cytokine secretion, and exerting direct cancer cell anti-proliferative effects, adverse side-effects are also observed. These include fever, headache, chills, fatigue, diarrhoea, nausea, vomiting, anorexia, transient increases in hepatic transaminase, and transient decreases in granulocyte and leucocyte counts.

For a recent review on both the potential and limitation of systemic recombinant TNFα and IFNγ see Shen et al. (2018) Cell Prolif. 51(4): e12441.

Hence there is a need for more *in situ* controlled, more localized, more tissue or cell specific production of such cytokines. For example, more controlled expression or induction of pro-inflammatory cytokines is proposed as one approach whereby "cold" tumours can be turned "hot". Hot tumours are also sometimes termed "T-cell-inflamed" because of an increase in the number or density of CD45+ T-cells also observed. See Bonaventura et al. (2019) Front. Immunol. 10: 168 for a recent review.

For such reasons, an ideal therapeutic multispecific antibody or fragment thereof as described herein which binds to the Vδ1 chain of a γδ TCR may be one that can maintain or enhance or induce the secretion of cytokines in Vδ1+ cells *in vivo*. Such antibodies can then be employed as medicaments designed to specifically increase or induce cytokines in a subject or patient and in a more localized, less systemic manner and one which better correlates with the distribution of Vδ1+cells in said subject or patient.

Remarkably, when monospecific versions of the multispecific antibodies as described herein which bind to the the Vδ1 chain of a γδ TCR are applied to Vδ1+ cells, a significantly higher level of secreted cytokines are observed. More specifically, and as a non-limiting example, a significant higher level of TNFα and IFNγ is observed. See e.g. Example 15.

Hence in another aspect of the disclosure, there is provided a method of assessing an antibody or multispecific antibody or fragment thereof which binds to the Vδ1 chain of a γδ TCR comprising administering the antibody or fragment thereof to a cell population comprising Vδ1+ cells and determining the amount of at least one cytokine produced by the cell population. The amount of cytokine produced can be determined/measured over a period of time and optionally compared to the amount observed when said antibody is not applied to the cell population for the same period of time. In one embodiment, the observed level of cytokine produced when the antibody is administered to the cell population is more than about 10%, more than about 20%, more than about 30%, more than about 50%, more than about 100%, more than about 150%, more than about 200%, more than about 250%, more than about 300%, more than about 350%, more than about 400%, more than about 450%, more than about 500%, more than about 1000%, relative to the level of cytokine produced when the antibody is not applied. In a further aspect of the invention, the cytokine is a pro-inflammatory cytokine. In a further aspect of the invention, the cytokine is the TNF-α cytokine. In a further aspect of the invention, is IFN-γ cytokine.

In a further aspect of the disclosure there is provided a method of selecting or characterizing or comparing antibodies or multispecific antibodies or fragments thereof as described herein which bind to the Vδ1 chain of a γδ TCR by applying said antibodies to a cell population comprising Vδ1+ cells and then measuring the level of at least one cytokine generated. In a further aspect of the invention the cytokine measured is TNF-α cytokine and/or IFN-γ cytokine.

In a further aspect of the disclosure, there is provided a method of assessing an antibody or multispecific antibody or fragment thereof which binds to the Vδ1 chain of a γδ TCR by applying said antibody or fragment thereof to a cell population comprising Vδ1+ cells and measuring the effect of the antibody on modulating a colder or cold tumour to become a hotter or hot tumour by determining the quantity of proinflammatory cytokines produced and/or the number or density of CD45+ T-cells present in the tumour or tumour microenvironment.

## Medicaments that maintain or induce or increase Vδ1+ cell Granzyme B activity

Granzyme B is a serine protease commonly found in the granules of natural killer cells (NK cells) and cytotoxic T cells. It is secreted by these cells along with the pore forming protein perforin to mediate apoptosis in target cells, such as diseased cells.

When Vδ1+ cells are incubated in co-cultures with target diseased cells (such as cancer cells) in model systems, levels of Granzyme B levels and activity can be measured in the target diseased cells ahead of lysis. Remarkably when an antibody or fragment thereof as described herein which binds to the Vδ1 chain of a γδ TCR is then applied to such co-cultures of Vδ1+ cells and cancer cells in such model systems, higher Granzyme B levels and activity are then observed in the diseased cancer cells ahead of cell death (see e.g. Example 16).

Hence in another aspect of the invention, there is provided a method for assessing an antibody or fragment thereof which binds to the Vδ1 chain of a γδ TCR comprising administering the antibody or fragment thereof to a co-culture comprising Vδ1+ cells and diseased cells (such as cancer cells) and measuring the effect on the amount of Granzyme B produced by the diseased cells in the co-culture. The amount of cytokine produced can be determined/measured over a period of time and optionally compared to the amount observed when said antibody is not applied to said co-cultures for the same period of time. In one embodiment, the level of Granzyme B measured when said antibody is applied to said co-culture is more than about 10%, more than about 20%, more than about 30%, more than about 40%, more than about 50%, more than about 70%, more than about 80%, more than about 90%, more than about 100%, more than about 200%, relative to the Granzyme B level observed when said antibody is not applied.

In a further aspect of the invention there is provided a method of selecting or characterizing or comparing antibodies or fragment thereof as described herein which bind to the Vδ1 chain of a γδ TCR by applying said antibodies to a co-culture comprising Vδ1+ cells and diseased cells and then measuring the quantity or activity of Granzyme B in the diseased cell.

## Medicaments that expand polyclonal Vδ1+ cell populations

An ideal multispecific antibody medicament may also be one designed to ensure the expanding Vδ1+ cells do not become too clonally focused at the hypervariable CDR3 sequence level. Hence an ideal antibody medicament may be designed such to avoid inducing proliferation Vδ1+ cells by binding to specific or 'private' δ1+ CDR3 sequence paratopes. Rather, the antibody may bind via conserved germline sequences present on all Vδ1+ T cell receptors and in a gamma-chain independent manner, rather than bind to sequences presented only a sub-set of Vδ1+ cells.

Hence an ideal antibody medicament may stimulate the expansion Vδ1+ cells to generate a plurality of Vδ1+ cells containing a mixture of CDR3 sequences. This in turn would result in an *in vivo* expanded heterogenous polyclonal population of Vδ1+ cells displaying different CDR3 sequences on delta variable 1 chains. Remarkably, during analysis of expanded Vδ1+ cell populations generated by a method of adding an antibody or fragment thereof as described herein to a starting population of immune cells containing Vδ1+ cells, extensive polyclonality is observed by RNAseq based methodologies designed to sequence through the CDR3 hypervariable regions of RNA extracted (see e.g. Example 10).

Accordingly in one aspect, there is provided a method of assessing an antibody or fragment thereof which binds to the Vδ1 chain of a γδ TCR comprising administering the antibody or fragment thereof to a cell population comprising Vδ1+ cells and determining the polyclonality of the expanded Vδ1+ cells. It is desirable for an antibody medicament to generate an expanded polyclonal population containing a plurality of Vδ1+ CDR3 sequences. Polyclonality can be determined using methods known in the art, such as by nucleic acid sequencing approaches capable of analysing the Vδ1 chain hypervariable CDR3 content of said Vδ1+ cells.

## Medicaments that expand polyclonal Vδ1+ cells for extended periods of time

An ideal multispecific antibody medicament may be able to enhance or promote or stimulate the proliferation of primary Vδ1+ cells without exhausting such cells *in vivo.* For example, and by way of comparison, anti-CD3 medicaments such as OKT3 (e.g. Muronomab), whilst capable of expanding CD3 positive T-cells may also exhaust or induce anergy. To assess the capacity of monospecific versions of the multispecific antibodies as described herein and which bind to the Vδ1 chain of a γδ TCR to drive continued cell division of viable Vδ1+ cells, longer term proliferation studies were undertaken using monospecific antibodies. Remarkably these studies revealed that monospecific versions of the multispecific antibodies as described herein and which bind to the Vδ1 chain of a γδ TCR are capable of driving cell division/proliferation of viable and still functionally cytotoxic Vδ1+ cells for over 40 days (see e.g. Example 10).

In one embodiment, there is provided a method of assessing an antibody or fragment thereof which binds to the Vδ1 chain of a γδ TCR comprising applying the antibody or fragment thereof to a cell population and monitoring the length of time Vδ1+ cell division occurs. Ideally, the antibody is capable of stimulating Vδ1+ cell division for a period of 5 to 60 days, such as at least 7 to 45 days, 7 to 21 days, or 7 to 18 days.

In a further embodiment, there provided a multispecific antibody or fragment thereof as described herein which binds to the Vδ1 chain of a γδ TCR and which when administered to a patient is capable of stimulating Vδ1+ cell division to increase the number by at least 2-fold in number, at least 5-fold in number, at least 10-fold in number, at least 25-fold in number, at least 50-fold in number, at least 60-fold in number, at least 70-fold in number, at least 80-fold in number, at least 90-fold in number, at least 100-fold in number, at least 200-fold in number, at least 300-fold in number, at least 400-fold in number, at least 500-fold in number, at 600-fold in number, at least 1 ,000-fold in number.

In a further aspect of the disclosure there is provided a method of selecting or characterizing or comparing antibodies or fragment thereof as described herein which bind to the Vδ1 chain of a γδ TCR by applying said antibodies to Vδ1+ cells or mixed cell population containing Vδ1+ cells and then measuring Vδ1+ cell numbers over time.

## Medicaments that modulate non-Vδ1+ immune cells through targeting Vδ1+ immune cells

A multispecific antibody or fragment thereof as described herein may also be assessed by measuring Vδ1+ cell mediated modulation of other immune cells. For example, a change observed in a non-γδ T cell 'fraction' can be measured following application of an antibody or fragment thereof as described herein to a model system comprising mixed population of immune cells such as one comprising human tissue αβ cells and γδ T cells. Further, the effect on non-γδ cell types in said models can be measured by flow cytometry. For example, by measuring the relative change in numbers of CD8+ αβ T cells upon addition of an antibody or fragment thereof as described herein to mixed cultures comprising γδ T cells and non-γδ T cells. Optionally, the observed change in number or phenotype of a non-γδ T-cell CD8+ lymphocyte population can then be compared to the change in number when an alternative comparator antibody is applied (e.g. OKT-3) to said mixed population.

Hence in another aspect of the disclosure, there is provided a method of assessing an antibody or fragment thereof which binds to the Vδ1 chain of a γδ TCR comprising administering the antibody or fragment thereof to a mixed population of immune cells or tissues comprising Vδ1+ cells and Vδ1-negative immune cells and measuring the effect on the Vδ1-negative immune cells. The effect can be determined/measured over a period of time and optionally compared to the effect observed in Vδ1-negative cells when said antibody is not applied for the same period of time. The effect may be measured as a change in the number of Vδ1-negative immune cells. For example, the antibody may increase the number Vδ1-negative immune cells by more than about 10%, more than about 20%, more than about 30%, more than about 40%, more than about 50%, more than about 70%, more than about 80%, more than about 90%, more than about 100%, more than about 500%, relative to the levels observed when said antibody is not applied.

In a further aspect of the invention the modulated Vδ1-negative cell is a CD45+ cell. In a further aspect of the invention the modulated cell is a αβ T-cell. In a further aspect of the invention the modulated αβ+ cell is CD8+ lymphocyte. In a further aspect of the invention the modulated αβ T-cell, or population thereof, exhibits evidence of enhanced cell division. In a further aspect of the disclosure there is provided a method of selecting or characterizing or comparing antibodies or fragment thereof as described herein which bind to the Vδ1 chain of a γδ TCR by administering said antibodies to a population of mixed immune cells comprising Vδ1+ cells and Vδ1-negative immune cells and then measuring an effect conferred on the Vδ1-negative cell population by Vδ1+ cells modulated by said antibodies or fragments thereof.

Optionally, and during "Vδ1+ cell mediated immune system modulation" as conferred by an antibody or fragment thereof as described herein, a concomitant increase in Vδ1+ cell number is also observed. And whilst not being bound by this theory, it is possible that said increase in Vδ1+ cell number may be causal in driving the concomitant expansion of co-present Vδ1-negative immune cells, such as αβ T-cells. An alternate hypothesis may be that antibody-induced cytokine secretions from the Vδ1+ T cells stimulate the expansion of Vδ1-negative immune cells.

In a further aspect of the invention the observed increase in αβ+ CD8+ lymphocyte population is compared to a comparator antibody such as OKT3 antibody or alternate anti-Vδ1 antibody. In a further aspect of the disclosure there is provided a method of selecting or characterizing or comparing antibodies or fragment thereof as described herein which bind to the Vδ1 chain of a γδ TCR by applying said antibodies to a population of mixed immune cells comprising Vδ1+ T-cells and αβ T-cells and then measuring the numbers of CD8+ αβ+ T-cells lymphocytes over time.

## Medicaments that modulate Tumour Infiltrating Lymphocytes (TILs)

A multispecific antibody or fragment thereof as described herein may also be assessed by measuring the effect conferred on tumour-infiltrating populations (TILs) in model systems. Surprisingly (see e.g. Example 18) during such assessments monospecific versions of the multispecific antibodies as described herein measurably modulated TIL populations in human tumours. For example, a change in either the number or phenotype of γδ+ lymphocyte TIL population or the non-γδ lymphocyte TIL population is measured following application of an antibody or fragment thereof as described herein to a human tumour such as a human renal cell carcinoma. Optionally, the observed change in number or phenotype of either the γδ+ lymphocyte TIL population or non-γδ lymphocyte TIL population can then be compared to the change observed when an alternative comparator antibody is applied (e.g. OKT-3) to said model system.

Hence in another aspect of the disclosure, there is provided a method of assessing an antibody or fragment thereof which binds to the Vδ1 chain of a γδ TCR comprising administering the antibody or fragment thereof to TILs located in, or derived from, a human tumour and determining the effect on the number of TILs. The effect can be determined/measured over a period of time and optionally compared to the TIL number observed when said antibody is not applied over the same period of time. The effect may be an increase in the number of TILs. For example, the antibody may increase the number of TILs more than about 10%, more than about 20%, more than about 30%, more than about 40%, more than about 50%, more than about 70%, more than about 80%, more than about 90%, more than about 100% relative to the number of TILs observed when said antibody is not applied. In a further aspect, the TILs in which the number observed are γδ+ lymphocyte TIL cells and/or non-γδ lymphocyte TIL cells.

In a further aspect of the disclosure there is provided a method of selecting or characterizing or comparing antibodies or fragment thereof as described herein which bind to the Vδ1 chain of a γδ TCR cells antibodies by applying said antibodies to TIL or TILs located in or derived from a human tumour and then measuring the change in number of TIL or TILs cells over a period of time.

## Medicaments that modulate human Vδ1+ cytotoxicity

A multispecific antibody or fragment thereof as described herein may also be assessed by measuring the conferred effect on Vδ1+ mediated cell cytotoxicity. Surprisingly, during such assessments of monospecific versions of the multispecific antibodies as described herein and some of the multispecific antibodies themselves (e.g. see e.g. Examples 19, 23, 24 and 27) measurably enhanced Vδ1+ mediated cell cytotoxicity was observed. For example, a reduction in the number of cancer cells or an increase in the number of killed cancer cells is observed following application of an antibody or fragment thereof to a model system comprising a mixed culture comprising Vδ1+ cells and said cancer cells. Optionally, the reduction in the number of cancer cells or the increase in the number of killed cancer cells can then be compared to the outcome when an alternative comparator antibody is applied (e.g. OKT-3) to said model systems.

Hence in another aspect of the disclosure, there is provided a method of assessing an antibody or fragment thereof which binds to the Vδ1 chain of a γδ TCR comprising applying the antibody or fragment thereof to a mixed population of cells comprising Vδ1+ cells and cancer cells and measuring the cytotoxicity of the Vδ1+ cells towards the cancer cells. The cytotoxicity may be measured by an increase in the number of dead cancer cells over a period of time, optionally compared to the number of dead cancer cells observed when said antibody is not applied to the mixed population of cells over the same period of time. For example, the observed increase in dead cells when said antibody is applied may be more than about 10%, by more than about 20%, by more than about 30%, by more than about 40%, by more than about 50%, more than about 70%, more than about 80%, more than about 90%, more than about 100%, more than about 200%, more than about 500%, relative to the number of dead cells observed when said antibody is not applied. In a further aspect of the disclosure there is provided a method of selecting or characterizing or comparing antibodies or fragment thereof as described herein which bind to the Vδ1 chain of a γδ TCR cells by adding said antibodies to said population of mixed immune cells comprising human Vδ1+ cells and cancer cells and then measuring an increase in dead cancer cells overtime.

## Medicaments that modulate Vδ1+ cell target-to-effector cell ratios (T:E Ratios)

A multispecific antibody or fragment thereof as described herein may also be assessed by measuring how said antibodies enhance Vδ1+ mediated cancer cell cytotoxicity by determining the target cell to effector cell ratio wherein the 50% of the target cells (EC50) are killed in a model system to assess said antibodies as potential medicaments. For example, mixed cultures comprising target cancer cells with human Vδ1+ effector cells. Surprisingly, during such assessments (e.g. see e.g. Example 19s and 23) multispecific antibodies as described herein (as well as monospecific versions of the multispecific antibodies described here) favourably modify the EC50 T:E ratio in model systems. Such modifications can be measured as numbers of Vδ1+ cells required to observe 50% killing of cancer cells over a set time. This can also be reported as change or as fold-improvements or as percent-improvements in cytotoxicity towards said cancer cells. Optionally, the T:E ratio conferred by antibodies of this invention can then be compared to the T:E ratios when an alternative comparator antibody is applied (e.g. OKT-3) to said model systems. In some scenarios, the multispecific antibodies of the invention present opportunities for improved cancer cell cytotoxicity even at lower E:T ratios, compared to monospecific antibodies.

Hence in another aspect of the disclosure, there is provided a method of assessing a multispecific antibody or fragment thereof which binds to the Vδ1 chain of a γδ TCR comprising applying the antibody or fragment thereof to a mixed population of cells comprising human Vδ1+ cells and cancer cells and measuring the number of Vδ1+ cells required to kill 50% of the cancer cells. This may be measured relative to the number Vδ1+ cells required to kill 50% of cancer cells without application of said antibody, optionally over the same period of time. For example, the reduction in the number of Vδ1+ cells required to kill 50% of the cancer cells when said antibody is applied may be greater than about 10%, greater than about 20%, greater than about 30%, greater than about 40%, greater than about 50%, greater than about 70%, greater than about 80%, greater than about 90%, greater than about 100%, greater about 200%, greater than about 500%, relative to the number of Vδ1+ cells required to kill 50% of the cancer cells when said antibody is not applied.

In a further aspect of the disclosure there is provided a method of selecting or characterizing or comparing antibodies or fragment thereof as described herein which bind to the Vδ1 chain by adding said antibodies to said population of cells comprising Vδ1+ cells plus cancer cells and then measuring the numbers of Vδ1 + cells required to kill 50% of the cancer cells.

## Medicaments which enhance Vδ1+ cell EC50 cytotoxicity

An alternate way to measure the observed enhanced cytotoxicity of human Vδ1+ cells or population thereof is to measure the number of cells required to kill 50% of the cancer cells over a set period of time in condition A (such as starting control) and compare this to the number of cells required to kill 50% of the cancer cells over a set period of time in condition B (such as upon application of antibody of the invention as described herein).

Whilst it is recognized that there are a variety of ways to measure such parameters, to aid in understanding, the following non-limiting hypothetical example will be outlined:

Hypothetically, effector cell cytotoxicity enhancement can be measured as follows:- In condition A (control treatment) it is observed that 1000 Vδ1+ cells are required to kill 50% of the cancer cells over a set period of time (e.g. 5 hours). In condition B (e.g. application of multispecific antibody of the invention described herein) it is observed that 500 Vδ1+ cells were required to kill 50% of the cancer cell over the same period of time. Hence in this example, the application of an antibody has enhanced the cytotoxicity of the Vδ1+ cell population by 200%:

$$(1000/500) \times 100 = 200\%$$

For example (see e.g. Example 19), surprisingly such percentage enhancements have been observed for antibodies of the invention as described herein.

In a further aspect of the disclosure there is provided a method of selecting or characterizing or comparing antibodies or fragment thereof as described herein which bind to the Vδ1 chain of a γδ TCR by adding said antibodies to said population of mixed immune cells comprising Vδ1+ cells and cancer cells and determining the relative or percent-change in cytotoxicity versus an equivalent or control experiment wherein there is no application of said antibody to said mixture of cells.

## Medicaments which enhance Vδ1+ cells diseased-cell specificity whilst sparing healthy cells

Another approach to assess multispecific antibodies thereof as described herein is to measure how said antibodies modulate diseased-cell specific cytotoxicity. Surprisingly during such studies, it was discovered monospecific and multispecific antibodies can specifically enhance the Vδ1+ cell specific killing of diseased-cells such as cancer cells (e.g. see e.g. Examples 19 and 23) whilst sparing healthy or non-diseased cells. Ideal antibody medicaments administered to a patient to ameliorate a symptom of cancer will confer enhanced cytotoxicity specifically towards diseased cells whilst sparing healthy cells. And medicaments which enhance effector cell cytotoxicity specifically towards diseased cells, such as cancer cells, can be said to exhibit an enhanced therapeutic index (TI) over medicaments which do not selectively enhance effector cell cytotoxicity specifically towards said diseased cells. The therapeutic index is also referred to as therapeutic ratio and is a quantitative measurement of the relative safety of a drug. It is a comparison of the amount of a therapeutic agent that causes the therapeutic effect to the amount that causes toxicity e.g. by conferring undesirable death in related or relevant healthy cell populations. A multispecific antibody or fragment thereof as described herein may be assessed by measuring its ability to change or to enhance or to fold-improve Vδ1+ cell capacity to selectivity kill diseased cells over and above healthy cells in model systems. For example, said model systems may comprise Vδ1+ effector cells, cancer cells, and control cells (such as healthy cells). Optionally, the fold-improvement in selective diseased-cell killing conferred by multispecific antibodies of the invention can then be compared to the fold-improvement observed when an alternative comparator antibody is applied (e.g. OKT-3) to said model systems.

The diseased-cell specificity and diseased-cell specificity-enhancement of Vδ1+ cells can be measured in cultures comprising Vδ1+ cells, diseased cells, and healthy cells. For example, Vδ1+ specificity towards diseased cells can be measured by observing the number of cancer cells killed by the Vδ1+ cells and then comparing the number of healthy cells killed by the Vδ1+ cells. Such comparisons can be controlled by including equivalent numbers of diseased cells and healthy cells in a model system also containing Vδ1+ cells e.g. "tricultures". Alternative comparison methodology can also be considered - for example when analytical or equipment limitations reduce the ability to distinguish and track all three cell types or more in parallel in a single assay (inclusive of Vδ1+ cells, diseased cells, and non-diseased cells). In said instances, comparing Vδ1+ cell cytotoxicity towards diseased cells in one experiment and then comparing Vδ1+ cell cytotoxicity towards healthy cells in a separate equivalent experiment offers an alternate approach to such studies.

In another aspect of the disclosure, there is provided a method of assessing a multispecific antibody or fragment thereof which binds to the Vδ1 chain of a γδ TCR comprising administering the multispecific antibody or fragment thereof to a cell population comprising Vδ1+ cells and target cells and measuring the cell cytotoxic specificity towards the target cells. In one embodiment, the cell cytotoxicity specificity to a first target cell type can be compared to the cytotoxicity observed towards a second target cell type, therefore the method may be repeated using different target cell types. In a further aspect of the invention the first target cell type is a diseased cell and the second target cell type is a control cell such as a healthy cell or a cell with a different disease to the first target cell type.

In a further aspect of the disclosure there is provided a method for selecting or characterizing or comparing multispecific antibodies or fragment thereof as described herein which bind to the Vδ1 chain of a γδ TCR wherein the effect conferred by said antibody on Vδ1+ cell cytotoxicity towards (i) a first cell type and (ii) a second cell type is measured and compared. In a further aspect of the invention an antibody is thereby selected which enhances the specific cytotoxicity towards the first cell type more so than towards the second cell type. In a further aspect of the invention the first cell type is a diseased-cell and the second cell type is a healthy cell.

As described herein, the multispecific antibodies or fragments thereof used in the assays may be presented on a surface, for example the surface of a cell, such as a cell comprising an Fc receptor. For example, the antibodies or fragments thereof may be presented on the surface of THP-1 cells, such as TIB-202™ cells (available from American Type Culture Collection (ATCC)). Alternatively, the multispecific antibodies or fragments thereof may be used directly in the assays.

In such functional assays, output may be measured by calculating the half maximal concentration, also referred to as "EC50" or "effective concentration at 50 percent". The term "IC50" refers to the inhibitory concentration. Both EC50 and IC50 may be measured using methods known in the art, such as flow cytometry methods. In some instances, EC50 and IC50 are the same value or can be considered equivalent. For example, the effective concentration (EC) of effector cells required to inhibit (e.g. kill) 50% of a certain cell type may also be considered the 50% inhibitory concentration (IC). For the avoidance of doubt, the values of EC50 in the present application are provided using IgG1 formatted antibody when referring to an antibody. Such values can be easily converted based on the molecular weight of the antibody format for equivalent values as follows:

$$(\mu g/ml) / (MW\ in\ kDa) = \mu M$$

The EC50 for downregulation of the γδ TCR upon antibody (or fragment) binding may be less than 0.50 μg/ml, such as less than 0.40 μg/ml, 0.30 μg/ml, 0.20 μg/ml, 0.15 μg/ml, 0.10 μg/ml, 0.06 μg/ml or 0.05 μg/ml. In a preferred embodiment, the EC50 for downregulation of the γδ TCR upon antibody (or fragment) binding is less than 0.10 μg/ml. In particular, the EC50 for downregulation of the γδ TCR upon antibody (or fragment) binding may be less than 0.06 μg/ml, such as less than 0.05 μg/ml, 0.04 μg/ml or 0.03 μg/ml. In particular, said EC50 values are when the antibody is measured in an IgG1 format. For example, the EC50 γδ TCR downregulation value can be measured using flow cytometry (e.g. as described in the assay of e.g. Examples 6 and 23).

The EC50 for γδ T cell degranulation upon antibody (or fragment) binding may be less than 0.050 μg/ml, such as less than 0.040 μg/ml, 0.030 μg/ml, 0.020 μg/ml, 0.015 μg/ml, 0.010 μg/ml or 0.008 μg/ml. In particular, the EC50 for γδ T cell degranulation upon antibody (or fragment) binding may be less than 0.005 μg/ml, such as less than 0.002 μg/ml. In a preferred embodiment, the EC50 for γδ T cell degranulation upon antibody (or fragment) binding is less than 0.007 μg/ml. In particular, said EC50 values are when the antibody is measured in an IgG1 format. For example, the γδ T cell degranulation EC50 value can be measured by detecting CD107a expression (i.e. a marker of cell degranulation) using flow cytometry (e.g. as described in the assay of Example 7). In one embodiment, CD107a expression is measured using an anti-CD107a antibody, such as anti-human CD107a BV421 (clone H4A3) (BD Biosciences).

The EC50 for γδ T cell killing upon the antibody (or fragment) binding may be less than 0.50 μg/ml, such as less than 0.40 μg/ml, 0.30 μg/ml, 0.20 μg/ml, 0.15 μg/ml, 0.10 μg/ml or 0.07 μg/ml. In a preferred embodiment, the EC50 for γδ T cell killing upon the antibody (or fragment) binding is less than 0.10 μg/ml. In particular, the EC50 for γδ T cell killing upon the antibody (or fragment) binding may be less than 0.060 μg/ml, such as less than 0.055 μg/ml, in particular less than 0.020 μg/ml. In particular, said EC50 values are when the antibody is measured in an IgG1 format. For example, the EC50 γδ T cell killing value can be measured by detecting proportion of dead cells (*i.e.* using a cell viability dye) using flow cytometry following incubation of the antibody, γδ T cell and target cells (*e.g.* as described in the assay of Example 8). In one embodiment, death of the target cell is measured using a cell viability dye is Viability Dye eFluor™ 520 (ThermoFisher).

In the assays described in these aspects, the antibody or fragment thereof may be presented on the surface of a cell, such as a THP-1 cell, for example TIB-202™ (ATCC). The THP-1 cells are optionally labelled with a dye, such as CellTracker™ Orange CMTMR (ThermoFisher).

**Medicaments that downregulate CD3 molecules associated with a Vδ1 TCR.**

Current multispecific TCE-formatted medicaments typically engage and activate a T-Cell via CD3 binding events. This can result in downregulation of CD3 molecule complexes from the surface of a T-cell. However, it also well understood that TCEs can also over-stimulate T-cells via such engagement and downregulation. CD3 molecule complexes are not specific to one class of T-cell and are therefore not a precise target to aim for. Stimulating all T-cells (mainly αβ subtype) via CD3 can in turn can result in overproduction of cytokines, leading to acute cytokine flares (so-called cytokine storms). Additionally, in non-targeted approaches which engage and activate all T-cells via CD3, paradoxically the T-cells can become overactivated which leads to chronic T-cell exhaustion and/or T-cell death. Indeed, this non-specific pan T cell activation leads to activation of both effector and regulatory T cells whereas the presently presented approach interacts specifically with an effector population. This 'sledge-hammer' approach is therefore far from ideal when one may wish to upregulate selective T-cells only.

By contrast, presented herein are multispecific antibodies, in particular T-cell engagers, which engage the T-cell/CD3 complex differently. Specifically, these TCEs can engage via the TRDV1 domain of Vδ1 TCRs expressed only on Vδ1+ cells. In doing so such TCE-based medicaments function differently. First, these TCEs are able to down-regulate a TCR via engaging a TRDV-1 epitope. In turn this engagement event can downregulate TCR-associated CD3 molecule complexes. Such CD3 downregulation can be synonymous with T-cell activation. However, by engaging the T-cell/CD3 complex via the TRDV1 domain in this way, only CDR3 molecules associated with the Vδ1 TCR are then downregulated. This approach of specifically targeting and activating Vδ1 cells allows many of the above issues (cytokine storms, T-cell exhaustion/depletion and ADCC, for example) to be avoided. This mechanism is clearly shown in Figure 30.

Stimulation of T-cells via the CD3 'sledge-hammer' approach can also contribute to depletion of T-cells via Fc gamma receptor driven mechanisms, such as ADCC. Therefore, the majority of the CD3-targeting bispecific antibodies currently in clinical practice have Fc domains with reduced binding activity to FcγR or are bispecific fragments intentionally without the Fc region. CD3-targeting therapies may also have reduced binding affinity of the T-cell receptor complex binding arms.

This reduction in affinity may result in reduced efficacy and less optionality in terms of TCE design and functionality. For example, affinity of the binding domains in such TCEs is known to drive distribution profile in vivo. Specifically, it is typically observed that TCE distribution is biased towards its highest affinity target. Hence, by reducing the affinity of a TCE binding domain to the T-cell complex, it is typical to then bias distribution away from T-cells; the very cells needed to drive efficacy of such TCEs. It is partly for such reasons that TCE therapeutic windows have been termed 'prohibitively narrow'.

The approach presented here specifically targets and activates vδ1 cells, optionally avoiding the need to ablate Fc function or reduce affinity.

Additionally, in the approach presented here, the antibodies engage on the TCR of vδ1 cells but full activation does not occur unless tumour cells are also present. Full engagement of the presently presented antibodies on the TCR leads to partial downregulation and the vδ1 cells bound by the presently presented antibodies only become fully activated and become cytotoxic in the tumour microenvironment. This represents another vital safety advantage for the present approach, since off target cytotoxicity is reduced and the full potency of the present antibodies to activate vδ1 cells is only unleashed in the presence of tumour cells.

One mechanism behind γδ T cells being able to detect stress signals on tumour cells is believed to be due to the NCRs (natural cytotoxicity receptors) they express. The NCRs are able to engage NCR ligands on tumour cells. A dual mechanism of activation is therefore employed, wherein the γδ T cells are activated via TCR stimulation and the NCRs sense the tumour cells to enable full activation and cytotoxicity.

This is in contrast to stimulation of αβ T cells via CD3, wherein all stimulation is via the TCR. Such cells are therefore almost indiscriminate between healthy or transformed cells because they don't have this antigen presentation independent sensing of tumour cells via NCRs. Therefore, if CD3 antibodies are Fc they will attract other immune cells which can trigger a cascade of unpredictable and desirable events such as cytokine storms, exhaustion and even overactivation of immune cells leading to, for example, NK cells killing T cells etc. In the present approach, stimulation of γδ T cells with the presently presented antibodies does not lead to such concerns because both γδ T cells (and other immune cells such as NK cells) are able to distinguish between healthy cells and tumour cells. For example, via their NCR sensing mechanism and therefore do not kill each other.

For example, in initial cynomolgus studies with the anti-vδ1 Fc-enabled multispecific antibody presented herein, with specificity to both human and cyno vδ1 antigen (SEQ ID NO: 1 and SEQ D NO: 172), was found to be safe and well tolerated, in dose-escalating, repeat dose *in-vivo* studies by all parameters measured. None of the side effects typically associated with T cell activation, such as cytokine release or weight loss were observed.

These findings also highlight another advantage of the approach described herein. Specifically, and unlike TCEs typified by CD3 engagers and the like, TCE bispecifics of the invention can optionally be designed as full-length antibody, for example comprising heavy chains with a VH-CH1-CH2-CH3 format with cognate light chains with a VL-CL format. Unlike smaller bispecific formats (e.g. less than 70 KDa), such full-length bispecific format can exhibit longer half-lives in-vivo and thereby require less frequent dosing regimens. The longer half-lives observed by such formats are for a variety of reasons inclusive of increased size (>70KDa) which means such formats are not filtered by kidneys (glomerulus pore size cutoff 60-70KDa). In one embodiment, the multispecific antibodies may be larger than about 70KDa, and may comprise human IGHC sequence (e.g. IGHA, IGHD, IGHD, IGHM, IGHG sequence) as listed by IMGT. Such IgG1 formats can also be re-cycled by FcRn mechanism. The clear downside of such full-length antibody formats for TCE bispecifics in particular is that such format exhibit unfavourable safety profiles due to the reduce clearance rate, increased and more chronic exposures.

Therefore, the present approach allows the possibility of Fc functionality without any concern of off-target effects, such as NK cells killing γδ T cells or vice versa. The present approach is therefore superior to CD3 directed approaches which are stunted by the necessity of workarounds such as reducing CD3 affinity, eliminating Fc function etc. to limit collateral damage outside the tumour environment. The multispecific antibodies, in particular T-cell engagers, presented here are able to bind to vδ1 cells without any damage potential and the full activation and cytotoxicity enhancement is only engaged when the vδ1 cells are in close contact with tumour cells.

Hence in one embodiment there is provided a method of downregulating a TRDV1-containing Vδ1 TCR and the associated CD3 molecule complex on the surface of a cell with a TCE multispecific antibody, and the use of such multispecific antibodies for this purpose.

## Polynucleotides and expression vectors

In one aspect of the disclosure there is provided a polynucleotide encoding the multispecific antibody or fragment of the invention. In one embodiment, the polynucleotide comprises or consists of a sequence having at least 70%, such as at least 80%, such as at least 90%, such as at least 95%, such as at least 99% sequence identity with any one of SEQ ID NOs: 99-110. In one embodiment, the expression vector comprises the VH region of any one of SEQ ID NOs: 99-110. In another embodiment, the expression vector comprises the VL region of any one of SEQ ID NOs: 99-110. In a further embodiment the polynucleotide comprises or consists of SEQ ID NO: 99-110. In a further aspect there is provided a cDNA comprising said polynucleotide. The polynucleotides may further comprise sequence encoding a second binding domain, wherein the second binding domain may confer specificity to the second antigen.

The polynucleotides and expression vectors of the disclosure may also be described in reference to the amino acid sequence encoded. Therefore, in one embodiment, the polynucleotide comprises or consists of a sequence encoding the amino acid sequence of any one of SEQ ID NOs: 62 to 85. In one embodiment, the expression vector comprises a sequence encoding the amino acid sequence of any one of SEQ ID NOs: 62 to 73. In another embodiment, the expression vector comprises a sequence encoding the amino acid sequence of any one of SEQ ID NOs: 74 to 85. The polynucleotides and expression vectors may further comprise sequences encoding a further binding domain, wherein the further binding domain may confer specificity to the second antigen.

To express the antibodies, or fragments thereof, polynucleotides encoding partial or full-length light and heavy chains, as described herein, are inserted into expression vectors such that the genes are operatively linked to transcriptional and translational control sequences. Therefore, in one aspect of the disclosure there is provided an expression vector comprising the polynucleotide sequence as defined herein.

It will be understood that the nucleotide sequences described herein may comprise additional sequences encoding amino acid residues to aid with translation, purification and detection, however alternative sequences may be used depending upon the expression system used. For example, the initial (5'-end) nine nucleotides of SEQ ID NOs: 99-110 and the final (3'-end) 36 nucleotides of SEQ ID NOs: 99-100, 102-103, 105-110, or the final (3'-end) 39 nucleotides of SEQ ID NOs: 101 and 104 are optional sequences. These optional sequences can be removed, modified or substituted if alternate design, translation, purification or detection strategies are adopted.

Mutations can be made to the DNA or cDNA that encode polypeptides which are silent as to the amino acid sequence of the polypeptide, but which provide preferred codons for translation in a particular host. The preferred codons for translation of a nucleic acid in, *e.g. E. coli* and *S. cerevisiae,* as well as mammalian, specifically human, are known.

Mutation of polypeptides can be achieved for example by substitutions, additions or deletions to a nucleic acid encoding the polypeptide. The substitutions, additions or deletions to a nucleic acid encoding the polypeptide can be introduced by many methods, including for example error-prone PCR, shuffling, oligonucleotide-directed mutagenesis, assembly PCR, PCR mutagenesis, *in vivo* mutagenesis, cassette mutagenesis, recursive ensemble mutagenesis, exponential ensemble mutagenesis, site-specific mutagenesis, gene reassembly, artificial gene synthesis, Gene Site Saturation Mutagenesis (GSSM), synthetic ligation reassembly (SLR) or a combination of these methods. The modifications, additions or deletions to a nucleic acid can also be introduced by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, ensemble mutagenesis, chimeric nucleic acid multimer creation, or a combination thereof.

In particular, artificial gene synthesis may be used. A gene encoding a polypeptide of the disclosure can be synthetically produced by, for example, solid-phase DNA synthesis. Entire genes may be synthesized *de novo,* without the need for precursor template DNA. To obtain the desired oligonucleotide, the building blocks are sequentially coupled to the growing oligonucleotide chain in the order required by the sequence of the product. Upon the completion of the chain assembly, the product is released from the solid phase to solution, deprotected, and collected. Products can be isolated by high-performance liquid chromatography (HPLC) to obtain the desired oligonucleotides in high purity.

Expression vectors include, for example, plasmids, retroviruses, cosmids, yeast artificial chromosomes (YACs) and Epstein-Barr virus (EBV) derived episomes. The polynucleotide is ligated into a vector such that transcriptional and translational control sequences within the vector serve their intended function of regulating the transcription and translation of the polynucleotide. Expression and/or control sequences can include promoters, enhancers, transcription terminators, a start codon (i.e. ATG) 5' to the coding sequence, splicing signals for introns and stop codons. The expression vector and expression control sequences are chosen to be compatible with the expression host cell used. SEQ ID NO: 99-110 comprise the nucleotide sequences encoding single chain variable fragments that may be comprised in multispecific antibodies of the invention, comprising a VH region and a VL region joined by a synthetic linker (encoding SEQ ID NO: 98). It will be understood that polynucleotides or expression vectors of the disclosure may comprise the VH region, the VL region or both (optionally including the linker). Therefore, polynucleotides encoding the VH and VL regions can be inserted into separate vectors, alternatively sequences encoding both regions are inserted into the same expression vector. The polynucleotide(s) are inserted into the expression vector by standard methods (e.g. ligation of complementary restriction sites on the polynucleotide and vector, or blunt end ligation if no restriction sites are present).

A convenient vector is one that encodes a functionally complete human CH or CL immunoglobulin sequence, with appropriate restriction sites engineered so that any VH or VL sequence can be easily inserted and expressed, as described herein. The expression vector can also encode a signal peptide that facilitates secretion of the antibody (or fragment thereof) from a host cell. The polynucleotide may be cloned into the vector such that the signal peptide is linked in-frame to the amino terminus of the antibody. The signal peptide can be an immunoglobulin signal peptide or a heterologous signal peptide (*i.e.* a signal peptide from a non-immunoglobulin protein).

In one aspect of the disclosure there is provided a cell (*e.g.* a host cell, such as a recombinant host cell) comprising the polynucleotide or expression vector as defined herein. It will be understood that the cell may comprise a first vector encoding the light chain of the antibody or fragment thereof, and a second vector encoding the heavy chain of the antibody or fragment thereof. Alternatively, the heavy and light chains both encoded on the same expression vector introduced into the cell.

In one embodiment, the polynucleotide or expression vector encodes a membrane anchor or transmembrane domain fused to the antibody or fragment thereof, wherein the antibody or fragment thereof is presented on an extracellular surface of the cell.

Transformation can be by any known method for introducing polynucleotides into a host cell. Methods for introduction of heterologous polynucleotides into mammalian cells are well known in the art and include dextran-mediated transfection, calcium phosphate precipitation, polybrene-mediated transfection, protoplast fusion, electroporation, encapsulation of the polynucleotide(s) in liposomes, biolistic injection and direct microinjection of the DNA into nuclei. In addition, nucleic acid molecules may be introduced into mammalian cells by viral vectors.

Mammalian cell lines available as hosts for expression are well known in the art and include many immortalized cell lines available from the American Type Culture Collection (ATCC). These include, inter alia, Chinese hamster ovary (CHO) cells, NSO, SP2 cells, HeLa cells, baby hamster kidney (BHK) cells, monkey kidney cells (COS), human hepatocellular carcinoma cells (*e.g.* Hep G2), A549 cells, 3T3 cells, and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. Antigen-binding fragments of antibodies such as the scFv and Fv fragments can be isolated and expressed in *E. coli* using methods known in the art.

The antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Antibodies (or fragments) of the invention can be obtained and manipulated using the techniques disclosed for example in Green and Sambrook, Molecular Cloning: A Laboratory Manual (2012) 4th Edition Cold Spring Harbour Laboratory Press.

Monoclonal antibodies can be produced using hybridoma technology, by fusing a specific antibody-producing B cell with a myeloma (B cell cancer) cell that is selected for its ability to grow in tissue culture and for an absence of antibody chain synthesis.

A monoclonal antibody directed against a determined antigen can, for example, be obtained by:

a) immortalizing lymphocytes obtained from the peripheral blood of an animal previously immunized with a determined antigen, with an immortal cell and preferably with myeloma cells, in order to form a hybridoma,
b) culturing the immortalized cells (hybridoma) formed and recovering the cells producing the antibodies having the desired specificity.

Alternatively, the use of a hybridoma cell is not required. Antibodies capable of binding to the target antigens as described herein may be isolated from a suitable antibody library via routine practice, for example, using the phage display, yeast display, ribosomal display, or mammalian display technology known in the art. Accordingly, monoclonal antibodies can be obtained, for example, by a process comprising the steps of:

a) cloning into vectors, especially into phages and more particularly filamentous bacteriophages, DNA or cDNA sequences obtained from lymphocytes especially peripheral blood lymphocytes of an animal (suitably previously immunized with determined antigens),
b) transforming prokaryotic cells with the above vectors in conditions allowing the production of the antibodies,
c) selecting the antibodies by subjecting them to antigen-affinity selection,
d) recovering the antibodies having the desired specificity.

Optionally, isolated polynucleotide encoding multispecific antibodies or fragment thereof as described herein can also be readily manufactured to make sufficient quantities to be employed as a medicaments to ameliorate the signs or symptoms of disease. When employed as a medicament in this manner, typically the polynucleotides of interest are first operatively linked to an expression vector or expression cassette designed to express said antibodies or fragment thereof in a subject or patient. Such expression cassettes and methods of delivery of polynucleotides or what are sometime termed 'nucleic-based' medicaments are well known in the art. For recent review see Hollevoet and Declerck (2017) J. Transl. Med. 15(1): 131.

Also provided is a method for the production of a multispecific antibody or antigen-binding fragment or variant thereof, comprising culturing a recombinant host cell of the disclosure in a cell culture medium under conditions to express the encoding nucleic acid sequence of the plasmid or vector inside the cell. If both binding domains are produced in the same recombinant cell, the method may further comprise obtaining the multispecific antibody or antigen-binding fragment or variant thereof from the cell culture supernatant. The obtained multispecific antibody or fragment thereof may then be further processed and/or formulated into a pharmaceutical composition. Alternatively if the binding domains of the multispecific antibody are generated in separate recombinant host cells then the multispecific antibody may be obtained by first collecting the separate cell supernatants from the respective recombinant host cells before combining said binding domains into a multispecific antibody which can then be further processed and/or formulated into a pharmaceutical composition, Further, there is provided a method of producing a recombinant host cell that expresses a multispecific antibody or fragment or variant thereof, comprising transfecting said cell with a plasmid or vector of the disclosure. Said cells can then be cultured for the production of the multispecific antibody or fragment or variant thereof.

**Pharmaceutical compositions**

According to a further aspect of the invention, there is provided a composition comprising the multispecific antibody or fragment thereof as defined herein. In such embodiments, the composition may comprise the multispecific antibody, optionally in combination with other excipients. Also included are compositions comprising one or more additional active agents (*e.g.* active agents suitable for treating the diseases mentioned herein).

According to a further aspect of the invention, there is provided a pharmaceutical composition comprising the multispecific antibody or fragment thereof as defined herein, together with a pharmaceutically acceptable diluent or carrier. The multispecific antibodies of the invention can be incorporated into pharmaceutical compositions suitable for administration to a subject. Typically, the pharmaceutical composition comprises an antibody of the invention and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, salts, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable substances such as wetting or minor amounts of auxiliary substances such as wetting or emulsifying agents, preservatives or buffers, which enhance the shelf life or effectiveness of the antibody or fragment thereof.

The compositions of this invention may be in a variety of forms. These include, for example, liquid, semisolid and solid dosage forms, such as liquid solutions (*e.g.* injectable and infusible solutions), dispersions or suspensions, tablets, pills, powders, liposomes and suppositories. The preferred form depends on the intended mode of administration and therapeutic application. Typical preferred compositions are in the form of injectable or infusible solutions.

The preferred mode of administration is parenteral (*e.g.* intravenous, subcutaneous, intraperitoneal, intramuscular, intrathecal). In a preferred embodiment, the antibody is administered by intravenous infusion or injection. In another preferred embodiment, the antibody is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration.

It is within the scope of the invention to use the pharmaceutical composition of the invention in therapeutic methods for the treatment of diseases as described herein as an adjunct to, or in conjunction with, other established therapies normally used in the treatment of such diseases.

In a further aspect of the invention, the antibody, composition or pharmaceutical composition is administered sequentially, simultaneously or separately with at least one active agent.

**Treatment methods**

According to a further aspect of the invention, there is provided an isolated multispecific antibody or fragment thereof as defined herein for use as a medicament. References herein to a multispecific antibody or fragment thereof "for use" as a medicament or in therapy are limited to administration of the antibody or fragment thereof to a subject. Such uses do not include administration of the multispecific antibody or fragment thereof to a cell culture (i.e. *in vitro* or ex *vivo)* wherein said cell culture or derived cell therapy product is used as a therapeutic.

In one embodiment, the multispecific antibody or fragment thereof is for use in the treatment of cancer, an infectious disease or an inflammatory disease. In one embodiment, the invention is for use in a method of treating a disease or disorder in a subject in need thereof, comprising the step of administering a multispecific antibody or fragment thereof to the subject. In various embodiments, the disease or disorder is cancer, an infectious disease or an inflammatory disease. In one embodiment, the multispecific antibody or fragment thereof is for use in the treatment of cancer, an infectious disease or an inflammatory disease, leads to the death of diseased cells while sparing healthy cells. In a further embodiment, the multispecific antibody or fragment thereof is for use in the treatment of cancer.

In one embodiment, the multispecific antibody or fragment thereof is for use in the treatment of cancer, an infectious disease or an inflammatory disease. In a further embodiment, the multispecific antibody or fragment thereof is for use in the treatment of cancer.

According to a further aspect of the invention, there is provided the pharmaceutical composition as defined herein for use as a medicament. In one embodiment, the pharmaceutical composition is for use in the treatment of cancer, an infectious disease or an inflammatory disease. In a further embodiment, the pharmaceutical composition is for use in the treatment of cancer.

According to a further aspect of the disclosure, there is provided a method of modulating an immune response in a subject in need thereof comprising administering a therapeutically effective amount of the isolated multispecific antibody or fragment thereof as defined herein. In various embodiments, modulating an immune response in a subject comprises binding or targeting $\gamma\delta$ T cells, activating $\gamma\delta$ T cells, causing or increasing proliferation of $\gamma\delta$ T cells, causing or increasing expansion of $\gamma\delta$ T cells, causing or increasing $\gamma\delta$ T cell degranulation, causing or increasing $\gamma\delta$ T cell mediated killing activity, causing or increasing $\gamma\delta$ T cell mediated killing activity while sparing healthy cells, causing or increasing $\gamma\delta$ T cytotoxicity, causing or increasing $\gamma\delta$ T cytotoxicity while sparing healthy cells, causing or increasing $\gamma\delta$ T cell mobilization, increasing survival of $\gamma\delta$ T cells, or increasing resistance to exhaustion of $\gamma\delta$ T cells. Modulating the immune response in a subject may further comprise binding or targeting the second antigen. For example, in some embodiments, binding of the second antigen, in particular when it is an immunomodulatory antigen, may stimulate immunomodulation via the second antigen in addition to immunomodulation via binding of the multispecific antibody to TRDV1. Hence the modulation of the immune response may comprise modulation via two different signalling pathways, a first signalling pathway modulated via TRDV1 antigen engagement and a second signalling pathway modulated via engagement of a second immunomodulatory antigen.

According to a further aspect of the disclosure, there is provided method of treating a cancer, an infectious disease or an inflammatory disease in a subject in need thereof, comprising administering a therapeutically effective amount of the isolated multispecific antibody or fragment thereof as defined herein. Alternatively, a therapeutically effective amount of the pharmaceutical composition is administered.

According to further aspects of the disclosure, there is provided the use of a multispecific antibody or fragment thereof as defined herein for the manufacture of a medicament, for example in the treatment of cancer, an infectious disease or an inflammatory disease.

In one embodiment, the multispecific antibody or fragment thereof is administered to a subject, wherein the subject has cancer, an infectious disease or an inflammatory disease.

According to a further aspect of the invention, there is provided the pharmaceutical composition as defined herein for use as a medicament. In one embodiment, the pharmaceutical composition is administered to a subject, wherein the subject has cancer, an infectious disease or an inflammatory disease.

According to a further aspect of the disclosure, there is provided a method of administering a therapeutically effective amount of the isolated multispecific antibody or fragment thereof as defined herein to a subject, wherein the subject has cancer, an infectious disease or an inflammatory disease. Alternatively, a therapeutically effective amount of the pharmaceutical composition is administered.

According to further aspects of the disclosure, there is provided the use of a multispecific antibody or fragment thereof as defined herein for the manufacture of a medicament, for example for the administration to a subject, wherein the subject has cancer, an infectious disease or an inflammatory disease.

In various embodiments, the cancer that can be treated by the disclosed methods and compositions include, but are not limited to acute lymphoblastic, acute myeloid leukemia, adrenocortical carcinoma, appendix cancer, basal cell carcinoma, bile duct cancer, bladder cancer, bone cancer, osteosarcoma and malignant fibrous histiocytoma, brain stem glioma, brain tumor, brain tumor, brain stem glioma, central nervous system atypical teratoid/rhabdoid tumor, central nervous system embryonal tumors, cerebellar astrocytoma, cerebral astrocytoma/malignant glioma, craniopharyngioma, ependymoblastoma, ependymoma, medulloblastoma, medulloepithelioma, pineal parenchymal tumors of intermediate differentiation, supratentorial primitive neuroectodermal tumors and pineoblastoma, visual pathway and hypothalamic glioma, brain and spinal cord tumors, breast cancer, bronchial tumors, Burkitt lymphoma, carcinoid tumor, gastrointestinal carcinoid tumor, central nervous system atypical teratoid/rhabdoid tumor, central nervous system embryonal tumors, central nervous system lymphoma, cerebellar astrocytoma cerebral astrocytoma/malignant glioma, cervical cancer, chordoma, chronic lymphocytic leukemia, chronic myelogenous leukemia, chronic myeloproliferative disorders, colon cancer, colorectal cancer, craniopharyngioma, cutaneous T-cell lymphoma, esophageal cancer, Ewing family of tumors, extragonadal germ cell tumor, extrahepatic bile duct cancer, intraocular melanoma, retinoblastoma, gallbladder cancer, gastric (stomach) cancer, gastrointestinal carcinoid tumor, gastrointestinal stromal tumor (gist), germ cell tumor, gestational trophoblastic tumor, glioma, glioma brain stem, glioma cerebral astrocytoma, glioma visual pathway and hypothalamic, hairy cell leukemia, head and neck cancer, hepatocellular (liver) cancer, Langerhans cell histiocytosis, Hodgkin lymphoma, hypopharyngeal cancer, hypothalamic and visual pathway glioma, intraocular melanoma, islet cell tumors, kidney (renal cell) cancer, Langerhans cell histiocytosis, laryngeal cancer, acute lymphoblastic leukemia, acute myeloid leukemia, chronic lymphocytic leukemia, chronic myelogenous leukemia, hairy cell leukemia, lip and oral cavity cancer, liver cancer, non-small cell lung cancer, small cell lung cancer, aids-related lymphoma, Burkitt lymphoma, cutaneous T-cell lymphoma, non-Hodgkin lymphoma, primary central nervous system lymphoma, Waldenstrom macroglobulinemia, malignant fibrous histiocvtoma of bone and osteosarcoma, medulloblastoma, melanoma, Merkel cell carcinoma, mesothelioma, metastatic squamous neck cancer with occult primary, mouth cancer, multiple endocrine neoplasia syndrome, multiple myeloma/plasma cell neoplasm, mycosis, fungoides, myelodysplastic syndromes, myelodysplastic/myeloproliferative diseases, myelogenous leukemia, myeloid leukemia, myeloid leukemia acute, multiple myeloma, myeloproliferative disorders, nasal cavity and paranasal sinus cancer, nasopharyngeal cancer, neuroblastoma, non-small cell lung cancer, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, pancreatic cancer, papillomatosis, parathyroid cancer, penile cancer, pharyngeal cancer, pheochromocytoma, pineal parenchymal tumors of intermediate differentiation, pineoblastoma and supratentorial primitive neuroectodermal tumors, pituitary tumor, plasma celt neoplasm/multiple myeloma, pleuropulmonary blastoma, primary central nervous system lymphoma, prostate cancer, rectal cancer, renal cell (kidney) cancer, renal pelvis and ureter, respiratory tract carcinoma involving the nut gene on chromosome 15, retinoblastoma, rhabdomyosarcoma, salivary gland cancer, sarcoma, Ewing family of tumors, Kaposi sarcoma, soft tissue sarcoma, uterine sarcoma, Sezary syndrome, skin cancer (nonmelanoma), skin cancer (melanoma), Merkel cell skin carcinoma, small cell lung cancer, small intestine cancer, soft tissue sarcoma, squamous cell carcinoma, squamous neck cancer, stomach (gastric) cancer, supratentorial primitive neuroectodermal tumors, T-cell lymphoma, testicular cancer, throat cancer, thymoma and thymic carcinoma, thyroid cancer, transitional cell cancer of the renal pelvis and ureter, gestational trophoblastic tumor, urethral cancer, uterine cancer, uterine sarcoma, vaginal cancer, vulvar cancer, Waldenstrom macroglobulinemia, and Wilms tumor. In various embodiments, the cancer that can be treated by the disclosed methods and compositions is treated while healthy cells are spared,

In various embodiments, the inflammatory diseases that can be treated by the disclosed methods and compositions include, but are not limited to Achalasia, Acute disseminated encephalomyelitis (ADEM), Acute motor axonal neuropathy, Acute respiratory distress syndrome (ARDS), Addison's disease, Adiposis dolorosa, Adult Still's disease, Adult-onset Still's disease, Agammaglobulinemia, Alopecia Areata, Amyloidosis, Amyotrophic lateral sclerosis, Ankylosing spondylitis, Anti-GBM/Anti-TBM nephritis, Anti-N-Methyl-D-Aspartate (Anti-NMDA) Receptor Encephalitis, Antiphospholipid syndrome, Antiphospholipid syndrome (APS, APLS), Antisynthetase syndrome, Anti-tubular basement membrane nephritis, Aplastic anemia, Atopic allergy, Atopic dermatitis, Autoimmune angioedema, Autoimmune comorbidities, Autoimmune dysautonomia, Autoimmune encephalomyelitis, Autoimmune enteropathy, Autoimmune hemolytic anemia, Autoimmune hepatitis, Autoimmune inner ear disease (AIED), Autoimmune lymphoproliferative syndrome, Autoimmune myocarditis, Autoimmune neutropenia, Autoimmune oophoritis, Autoimmune orchitis, Autoimmune pancreatitis (AIP), Autoimmune peripheral neuropathy, Autoimmune polyendocrine syndrome (APS) type 1, Autoimmune polyendocrine syndrome (APS) type 2, Autoimmune polyendocrine syndrome (APS) type 3, Autoimmune retinopathy, Autoimmune thrombocytopenic purpura, Autoimmune thyroiditis, Autoimmune urticaria, Autoimmune uveitis, Autoimmune vasculitis, Axonal & neuronal neuropathy (AMAN), Balo concentric sclerosis, Baló disease, Behcet's disease, Benign mucosal pemphigoid, Bickerstaffs encephalitis, Blau syndrome, Bullous pemphigoid, Castleman disease (CD), Celiac disease, Chagas disease, Chronic fatigue syndrome, Chronic inflammatory demyelinating polyneuropathy (CIDP), Chronic obstructive pulmonary disease, Chronic recurrent multifocal osteomyelitis (CRMO), Churg-Strauss Syndrome (CSS) or Eosinophilic Granulomatosis (EGPA), Cicatricial pemphigoid, Cogan syndrome, Cold agglutinin disease, Complement component 2 deficiency, Complex regional pain syndrome, Congenital heart block, Connective tissue, systemic, and multi-organ, Contact dermatitis, Coxsackie myocarditis, CREST syndrome, Crohn's disease, Cushing's syndrome, Cutaneous leukocytoclastic angiitis, Dego's disease, Dermatitis herpetiformis, Dermatomyositis, Devic'sDisease (neuromyelitis optica), Diabetes mellitus type 1, Digestive system, Discoid lupus, Dressler's syndrome, Drug-induced lupus, Eczema, Endometriosis, Enthesitis-related arthritis, Eosinophilic esophagitis (EoE), Eosinophilic fasciitis, Eosinophilic gastroenteritis, Eosinophilic granulomatosis with polyangiitis (EGPA), Eosinophilic pneumonia, Epidermolysis bullosa acquisita, Erythema nodosum, Erythroblastosis fetalis, Esophageal achalasia, Essential mixed cryoglobulinemia, Evans syndrome, Exocrine, Felty syndrome, Fibrodysplasia ossificans progressiva, Fibromyalgia, Fibrosing alveolitis, Gastritis, Gastrointestinal pemphigoid, Giant cell arteritis (temporal arteritis), Giant cell myocarditis, Glomerulonephritis, Goodpasture's syndrome, Granulomatosis with Polyangiitis, Graves' ophthalmopathy, Graves' disease, Guillain-Barre syndrome, Hashimoto's thyroiditis, Hashimoto's encephalopathy, Hemolytic anemia, Henoch-Schonlein purpura (HSP), Herpes gestationis or pemphigoid gestationis (PG), Hidradenitis Suppurativa (HS) (Acne Inversa), Hypogammaglobulinemia, Idiopathic giant-cell myocarditis, Idiopathic inflammatory demyelinating diseases, Idiopathic pulmonary fibrosis, IgA Nephropathy, IgA vasculitis (IgAV), IgG4-related disease, IgG4-related sclerosing disease, Immune thrombocytopenic purpura (ITP), Inclusion body myositis (IBM), Inflammatory Bowel Disease, Intermediate uveitis, Interstitial cystitis (IC), Interstitial lung disease, IPEX syndrome, Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lupus nephritis, Lupus vasculitis, Lyme disease chronic, Majeed syndrome, Meniere's disease, Microscopic polyangiitis (MPA), Mixed connective tissue disease (MCTD), Mooren's ulcer, Morphea, Mucha-Habermann disease, MultifocalMotor Neuropathy (MMN) orMMNCB, Multiple sclerosis, Myasthenia gravis, Myocarditis, Myositis, Narcolepsy, Neonatal Lupus, Nervous system, Neuromyelitis optica, Neuromyotonia, Neutropenia, Ocular cicatricial pemphigoid, Opsoclonus myoclonus syndrome, Optic neuritis, Ord's thyroiditis, Oshtoran syndrome, Palindromic rheumatism (PR), Paraneoplastic cerebellar degeneration (PCD), Paroxysmal nocturnal hemoglobinuria (PNH), Parry-Romberg syndrome, Parsonage-Turner syndrome, ParsPlanitis (peripheral uveitis), Pediatric Autoimmune Neuropsychiatric Disorder Associated with Streptococcus (PANDAS), Pelvic Inflammatory Disease (PID), Pemphigus, Pemphigus vulgaris, Peripheral neuropathy, Perivenous encephalomyelitis, Pernicious anemia (PA), Pityriasis lichenoides et varioliformis acuta, POEMS syndrome, Polyarteritis nodosa, Polyglandular syndromes type I, II, III, Polymyalgia rheumatica, Polymyositis, Postmyocardial infarction syndrome, Postpericardiotomy syndrome, Primary biliary cholangitis (PBC), Primary biliary cirrhosis, Primary immunodeficiency, Primary sclerosing cholangitis, Progesterone dermatitis, Progressive inflammatory neuropathy, Psoriasis, Psoriatic arthritis, Pure red cell aplasia (PRCA), Pyoderma gangrenosum, Rasmussen's encephalitis, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Rheumatoid vasculitis, Sarcoidosis, Schizophrenia, Schmidt syndrome, Schnitzler syndrome, Scleritis, Scleroderma, Serum sickness, Sjögren'sSyndrome, Sperm & testicular autoimmunity, Spondyloarthropathy, Stiff person syndrome (SPS), Subacute bacterial endocarditis (SBE), Susac'sSyndrome, Sweet's syndrome, Sydenham's chorea, Sympathetic ophthalmia (SO), Systemic lupus erythematosus (SLE), Takayasu's arteritis, Temporal arteritis/Giant cell arteritis, Thrombocytopenia, Thrombocytopenic purpura (TTP), Thyroid gland, Tolosa-Hunt syndrome (THS), Transverse myelitis, Type 1 diabetes, Ulcerativecolitis(UC), Undifferentiated connective tissue disease (UCTD), Undifferentiated spondyloarthropathy, Urticarial vasculitis, Uticaria, Uveitis, Vasculitis, Vitiligo, and Vogt-Koyanagi-HaradaDisease. In various embodiments, the inflammatory disease that can be treated by the disclosed methods and compositions is treated, while healthy cells are spared.

In various embodiments, the infectious disease that can be treated by the disclosed methods and compositions include, but are not limited to Acinetobacter infection, Actinomycosis, Acute Flaccid Myelitis (AFM), African sleeping sickness (African trypanosomiasis), AIDS (acquired immunodeficiency syndrome), Ameba infection, Amebiasis, Anaplasma phagocytophilum infection, Anaplasmosis, Angiostrongyliasis, Anisakiasis, Anthrax, Arboviral diseases, neuroinvasive and non-neuroinvasive, Arcanobacterium haemolyticum infection, Argentine hemorrhagic fever, Ascariasis, Aspergillosis, Astrovirus infection, Avian Influenza, Babesiosis, Bacillus cereus infection, Bacterial infection, Bacterial meningitis, Bacterial pneumonia, Bacterial vaginosis, Bacteroides infection, Balantidiasis, Bartonellosis, Baylisascaris infection, BK virus infection, Black piedra, Blastocystosis, Bolivian hemorrhagic fever, Botulism, Botulism (foodborne), Botulism (infant), Botulism (other), Botulism (wound), Brazilian hemorrhagic fever, Brucellosis, Bubonic plague, Burkholderia infection, Buruli ulcer, Calicivirus infection (Norovirus and Sapovirus), California serogroup virus diseases, Campylobacter, Campylobacteriosis, Candida auris, clinical, Candidiasis (Moniliasis; Thrush), Capillariasis, Carbapenemase Producing Carbapenem-Resistant Enterobacteriaceae (CP-CRE), Carbapenem-resistant Infection (CRE/CRPA), Carrion’s disease, Cat-scratch disease, Cellulitis, Chagas disease (trypanosomiasis), Chancroid, Chickenpox, Chikungunya Virus Infection (Chikungunya), Chlamydia, Chlamydia trachomatis, Chlamydophila pneumoniae infection, Cholera, Chromoblastomycosis, Chytridiomycosis, Ciguatera, Clonorchiasis, Clostridium difficile colitis, Clostridium Difficile Infection, Clostridium perfringens, Coccidioidomycosis fungal infection (Valley fever), Colorado tick fever (CTF), Common cold (Acute viral rhinopharyngitis; Acute coryza), Congenital syphilis, Conjunctivitis, COVID-19 (Coronavirus Disease 2019), CP-CRE, Enterobacter spp., CP-CRE, Escherichia coli (E. coli), CP-CRE, Klebsiella spp., Creutzfeldt-Jacob Disease, transmissible spongiform encephalopathy (CJD), Creutzfeldt-Jakob disease (CJD), Crimean-Congo hemorrhagic fever (CCHF), Crusted Scabies, Cryptococcosis, Cryptosporidiosis (Crypto), Cutaneous larva migrans (CLM), Cyclospora, Cyclosporiasis, Cysticercosis, Cytomegalovirus infection, Dengue virus infections, Dengue, 1,2,3,4 (Dengue Fever), Dengue-like illness, Desmodesmus infection, Diarrheal Illness, Dientamoebiasis, Diphtheria, Diphyllobothriasis, Dracunculiasis, E. coli, E. coli infection, Shiga toxin-producing (STEC), Eastern equine encephalitis virus disease, Ebola Hemorrhagic Fever (Ebola), Echinococcosis, Ehrlichia chaffeensis infection, Ehrlichia ewingii infection, Ehrlichiosis, Anaplasmosis, Encephalitis, Arboviral or parainfectious, Enterobiasis (Pinworm infection), Enterococcus infection" Enterovirus Infection, D68 (EV-D68), Enterovirus Infection, Non-Polio (Non-Polio Enterovirus), Epidemic typhus, Epstein-Barr virus infectious mononucleosis (Mono), Erythema infectiosum (Fifth disease), Exanthem subitum (Sixth disease), Fasciolasis, Fasciolopsiasis, Fatal familial insomnia (FFI), Fifth Disease, Filariasis, Flu (Seasonal), Food Poisoning, Food poisoning by Clostridium perfringens, Free-living amebic infection, Fungal infection, Fusobacterium infection, Gas gangrene (Clostridial myonecrosis), Genital Herpes, Genital Warts, Geotrichosis, German Measles, Gerstmann-Sträussler-Scheinker syndrome (GSS), Giardiasis, Glanders, Gnathostomiasis, Gonorrhea, Granuloma inguinale, Granuloma inguinale (Donovanosis), Group A streptococcal infection, Group A Streptococcus, Group B streptococcal infection, Guanarito virus, Haemophilus Influenza disease, Type B (Hib or H-flu), Haemophilus influenzae infection, Hand, foot and mouth disease (HFMD), Hansen’s Disease, Hantavirus infection, Hantavirus Pulmonary Syndrome (HPS), Heartland virus disease, Helicobacter pylori infection, Hemolytic Uremic Syndrome (HUS), Hemorrhagic fever with renal syndrome (HFRS), Hendra virus infection, Hepatitis A (Hep A), Hepatitis B (Hep B), Hepatitis C (Hep C), Hepatitis D (Hep D), Hepatitis E (Hep E), Herpes, Herpes B Virus, Herpes simplex, Herpes Zoster, zoster VZV (Shingles), Hib Disease, Histoplasmosis infection (Histoplasmosis), Hookworm infection, HPV (Human Papillomavirus), Human bocavirus infection, Human ewingii ehrlichiosis, Human granulocytic anaplasmosis (HGA), Human Immunodeficiency Virus/AIDS (HIV/AIDS), Human metapneumovirus infection, Human monocytic ehrlichiosis, Human papillomavirus (HPV) infection, Human parainfluenza virus infection, Hymenolepiasis, Impetigo, Influenza (flu), Influenza (Seasonal), Invasive pneumococcal disease, Isosporiasis, Junin virus,, Kawasaki Syndrome, Keratitis, Kingella kingae infection, Kuru, Lassa fever, Lassa virus, Legionellosis (Legionnaires’ disease), Leishmaniasis, Leprosy (Hansens Disease), Leptospirosis, Listeriosis (Listeria), Lujo virus, Lyme disease, Lymphatic filariasis (Elephantiasis), Lymphocytic Choriomeningitis (LCMV), Lymphogranuloma venereum infection (LGV), Machupo virus, Malaria, Marburg virus infection, Measles, Melioidosis (Whitmore’s disease), Meningitis, Meningitis-Bacterial, Meningitis - Viral, Meningococcal disease, Metagonimiasis, Microsporidiosis, Middle East respiratory syndrome (MERS), Molluscum contagiosum (MC), Monkeypox, Mononucleosis, Mosquito-borne Illness, MRSA, Mumps,, Murine typhus (Endemic typhus), Mycetoma, Mycoplasma genitalium infection, Mycoplasma pneumonia, Myiasis, Neisseria meningitidis, Neonatal conjunctivitis (Ophthalmia neonatorum), Nipah virus infection, Nocardiosis, Norovirus, Onchocerciasis (River blindness), Opisthorchiasis, Orf Virus (Sore Mouth), Paracoccidioidomycosis (South American blastomycosis), Paragonimiasis, Paralytic Shellfish Poisoning (Paralytic Shellfish Poisoning, Ciguatera), Pasteurellosis, PEP, Parasitic infection, Pertussis (whooping cough), Pink Eye, Pneumococcal Disease, Pneumococcal infection, Pneumocystis pneumonia (PCP), Pneumonia, Pneumonic Plague, Poliomyelitis (Polio), Poliomyelitis, paralytic, Poliovirus infection, Pontiac fever, Powassan virus disease, Prevotella infection, Primary amoebic meningoencephalitis (PAM), Progressive multifocal leukoencephalopathy, Protozoan infection, Psittacosis (Parrot Fever), Pustular Rash diseases (Small pox, monkeypox, cowpox), Rabies, Raccoon Roundworm, Rat Bite Fever, Recreational Water Illnesses, Relapsing fever, Respiratory syncytial virus infection, Reye’s Syndrome, Rhinosporidiosis, Rhinovirus infection, Rickettsial infection, Rickettsiosis (Rocky Mountain Spotted Fever), Rift Valley fever (RVF), Ringworm, Rotavirus infection, Rubella, Sabia virus, Salmonella, Salmonella Paratyphi infection, Salmonella Typhi infection, Salmonellosis, SARS (severe acute respiratory syndrome), Scabies, Scarlet fever, Schistosomiasis, Scombroid, Sepsis, Septic Shock, Septicemic Plague, Severe Acute Respiratory Syndrome (SARS), Shiga toxin-producing Escherichia coli, Shigella, Shigellosis, Shingles, Shingles (Herpes zoster), Smallpox, Sore Mouth (Orf Virus), Sporotrichosis, Spotted fever rickettsiosis, St. Louis encephalitis virus disease, Staphyloccal Infection, Staphyloccal Infection (Methicillin-resistant (MRSA)), Staphylococcal food poisoning, Staphylococcal Infection (Vancomycin Intermediate (VISA)), Strep Throat, Streptococcal Disease, Group A (invasive) (Strep A (invasive)), Streptococcal Disease, Group B (Strep-B), Streptococcal toxic shock syndrome, Strongyloidiasis, Subacute sclerosing panencephalitis, Syphilis, Taeniasis, Tetanus Infection, Tickborne Diseases, Tinea barbae, Tinea capitis, Tinea corporis, Tinea cruris, Tinea manum, Tinea nigra, Tinea pedis, Tinea unguium, Tinea versicolor, Toxic shock syndrome, Toxocariasis (ocular larva migrans (OLM)), Toxocariasis (visceral larva migrans (VLM)), Toxoplasmosis, Trachoma, Trichinellosis, Trichomoniasis, Trichonosis Infection (Trichinosis), Trichuriasis (whipworm infection), Tuberculosis (TB), Tularemia (Rabbit fever), Typhoid fever, Typhoid Fever, Group D, Typhus, Typhus fever, Ureaplasma urealyticum infection, Vaginosis, Valley fever, Variant Creutzfeldt-Jakob disease (vCJD, nvCJD), Varicella (Chickenpox), Venezuelan equine encephalitis, Venezuelan hemorrhagic fever, Vibrio cholerae (Cholera), Vibrio parahaemolyticus enteritis, Vibrio vulnificus infection, Vibriosis, Viral infection, Viral hemorrhagic fever, Viral Hemorrhagic Fever (Ebola, Lassa, Marburg), Viral Hemorrhagic Fevers (VHF), Viral pneumonia, West Nile virus disease, Western equine encephalitis virus disease, White piedra (tinea blanca), Whooping Cough, Yellow Fever, Yersenia (Yersinia), Yersinia pseudotuberculosis infection, Yersiniosis, Zeaspora, Zika fever, Zika Virus, Zika virus disease, congenital, Zika virus disease, non-congenital, Zika Virus Infection (Zika), Zika virus infection, congenital, Zika virus infection, non-congenital, and Zygomycosis. In various embodiments, the infectious disease that can be treated by the disclosed methods and compositions is treated, while healthy cells are spared.

In one embodiment, the disclosure is a method of activating at least one γδ T cell in a subject, comprising the step of administering a multispecific antibody or fragment thereof as defined herein.

In one embodiment, the disclosure is a method of causing or increasing proliferation of γδ T cells in a subject, comprising the step of administering to the subject a multispecific antibody or fragment thereof as defined herein.

In one embodiment, the disclosure is a method of causing or increasing γδ T cell degranulation in a subject, comprising the step of administering to the subject a multispecific antibody or fragment thereof as defined herein.

In one embodiment, the disclosure is a method of causing or increasing γδ T cell killing activity (e.g. T cell mediated killing activity) in a subject, comprising the step of administering to the subject a multispecific antibody or fragment thereof as defined herein. In one embodiment, the invention is a method of causing or increasing γδ T cell killing activity (e.g. T cell mediated killing activity) in a subject, while sparing healthy cells, comprising the step of administering to the subject a multispecific antibody or fragment thereof as defined herein.

In one embodiment, the disclosure is a method of causing or increasing γδ T cytotoxicity in a subject, comprising the step of administering to the subject a multispecific antibody or fragment thereof as defined herein. In one embodiment, the invention is a method of causing or increasing γδ T cytotoxicity in a subject, while sparing healthy cells, comprising the step of administering to the subject a multispecific antibody or fragment thereof as defined herein.

In one embodiment, the disclosure is a method of causing or increasing γδ T cell mobilization in a subject, comprising the step of administering to the subject a multispecific antibody or fragment thereof as defined herein.

In one embodiment, the disclosure is a method of increasing survival of γδ T cells in a subject, comprising the step of administering to the subject a multispecific antibody or fragment thereof as defined herein.

In one embodiment, the disclosure is a method of or increasing resistance to exhaustion of γδ T cells in a subject, comprising the step of administering to the subject a multispecific antibody or fragment thereof as defined herein.

According to a further aspect of the disclosure, there is provided a method of stimulating an immune response in a subject, the method comprising administration to the subject a multispecific antibody or fragment thereof in an amount effective at stimulating an immune response.

## Uses of antibodies or fragments thereof

According to a further aspect of the disclosure, there is provided the use of a multispecific antibody or fragment thereof as described herein to study antigen recognition, activation, signal transduction or function of γδ T cells (in particular Vδ1 T cells). As described herein, the antibodies have been shown to be active in assays which can be used to investigate γδ T cell function. Such multispecific antibodies may also be useful for inducing the proliferation of γδ T cells, therefore may be used in methods of expanding γδ T cells (such as Vδ1 T cells).

Multispecific antibodies which bind to the Vδ1 chain can be used to detect γδ T cells. For example, the antibody may be labelled with a detectable label or reporter molecule or used as a capture ligand to selectively detect and/or isolate Vδ1 T cells in a sample. Labelled antibodies find use in many methods known in the art, for example immunohistochemistry and ELISA.

The detectable label or reporter molecule can be a radioisotope, such as $^{3}H$, $^{14}C$, $^{32}P$, $^{35}S$, or $^{125}I$; a fluorescent or chemiluminescent moiety such as fluorescein isothiocyanate, or rhodamine; or an enzyme such as alkaline phosphatase, β-galactosidase, horseradish peroxidase, or luciferase. Fluorescent labels applied to antibodies of the invention may then be used in fluorescence-activated cell sorting (FACS) methods.

Thus in various embodiments, the disclosure includes *in vivo* methods of modulating γδ T cells, methods of binding γδ T cells, methods of targeting γδ T cells, methods of activating γδ T cells, methods of proliferating γδ T cells, methods of expanding γδ T cells, methods of detecting γδ T cells, methods of causing γδ T cell degranulation, methods of causing γδ T cell killing activity, methods of selecting antibodies or fragments thereof, the methods comprising the step of administering a multispecific antibody or fragment thereof to a subject as described herein.

Other features and advantages of the present invention will be apparent from the description provided herein. It should be understood, however, that the description and the specific examples while indicating preferred embodiments of the invention are given by way of illustration only, since various changes and modifications will become apparent to those skilled in the art. The invention will now be described using the following, non-limiting examples:

## EXAMPLES

### EXAMPLE 1. Materials and Methods

**Human Antibody Discovery**

Human phage display was employed to generate the human anti-human variable Vδ1+ domain antibodies as described herein. The library was constructed as described in Schofield et al (Genome biology 2007, 8(11): R254) and comprised a single chain fragment variable (scFv) displaying library of ~40 billion human clones. This library was screened using antigens, methods, selections, deselection, screening, and characterization strategies as described herein.

**Antigen preparation**

The design of the soluble yδ TCR heterodimers comprising the TCRα and TCR β constant regions used in the below Examples were generated according to Xu et al. (2011) PNAS 108: 2414-2419. Vγ or Vδ domains were fused in-frame to a TCRα or TCRβ constant region lacking the transmembrane domain, followed by a leucine zipper sequence or an Fc sequence, and a histidine tag/linker.

The expression construct was transiently transfected in mammalian EXPI HEK293 suspension cells (either as single or co-transfections for heterodimer). Secreted recombinant proteins were recovered and purified from culture supernatant by affinity chromatography. To ensure good recovery of monomer antigen, samples were further purified using preparative size exclusion chromatography (SEC). Purified antigens were analysed for purity by SDS-PAGE and aggregation state by analytical SEC.

Antigen functional validation

The specificity of the antigens containing delta variable 1 (Vδ1) chain was confirmed in DELFIA immunoassay (Perkin Elmer) and in flow-based assay in competition with γδ T cells using REA173-Miltenyi Biotec anti-Vδ1 antibody.

Dissociation-enhanced lanthanide fluorescence immunoassay (DELFIA)

For the confirmation of antigen's specificity, DELFIA immunoassay was performed with the antigen directly coated to the plate (3 μg/mL of antigen in 50 μL PBS at 4 °C overnight (Nunc #437111) and serial dilution of primary antibodies starting at 300nM. For detection DELFIA Eu-N1 Anti-Human IgG (Perkin Elmer # 1244-330) was used as secondary antibody at 1/500 dilution in 50 μL of 3 % of MPBS (PBS + 3 % (w/V) skimmed milk powder). Development was with 50 μL of DELFIA enhancement solution (Perkin Elmer #4001-0010).

Affinity ranking of antibody of interest were performed using DELFIA immunoassay in which antibodies were captured via protein G coated on the plate and soluble biotinylated L1 (DV1-GV4) antigen was added at 5nM in 50 μL (3MPBS). For detection 50 μL of streptavidin-Eu (1:500 in assay buffer, Perkin Elmer) was used and signal was developed with DELFIA enhancement solution. D1.3 hIgG1 (described in England et al. (1999) J. Immunol. 162: 2129-2136) was used as a negative control.

Phage display selection outputs were subcloned into the scFv expression vector pSANG10 (Martin et al. (2006) BMC Biotechnol. 6: 46). Soluble scFv were expressed and screened for binding in DELFIA on directly immobilised targets. Hits were defined as a DELFIA signal above 3000 fluorescence units.

**Antibody preparation**

Selected scFvs were subcloned into IgG1 frameworks using commercially available plasmids. expi293F suspension cells were transfected with said plasmids for antibody expression. For convenience, unless otherwise noted, the antibodies characterised in these Examples refer to IgG1 formatted antibodies selected from phage display as scFv. However, the antibodies of the invention may be in any antibody format as previously discussed.

## Antibody purification

IgG antibodies were batch purified from supernatants using protein A chromatography. Concentrated protein A eluates were then purified using Size Exclusion Chromatography (SEC). Quality of purified IgG was analysed using ELISA, SDS-PAGE and SEC-HPLC.

## γδ T cell preparation

Populations of enriched γδ T cells were prepared according to the methods described in WO2016/198480 (i.e. blood-derived γδ T cells) or WO2020/095059 (i.e. skin-derived γδ T cells). Briefly, for blood-derived γδ T cells PBMCs were obtained from blood and subjected to magnetic depletion of αβ T cells. The αβ-depleted PBMCs were then cultured in CTS OpTmiser media (ThermoFisher) in the presence of OKT-3 (or respective anti-Vδ1 antibody), IL-4, IFN-y, IL-21 and IL-1β for 7 days. At day 7 of culture, the media was supplemented with OKT-3 (or respective anti-Vδ1 antibody), IL-21 and IL-15 for a further 4 days. At day 11 of culture, the media was supplemented with OKT-3 (or respective anti-Vδ1 antibody) and IL-15 for a further 3 days. At day 14 of culture, half of the media was replaced with fresh complete OpTmiser and supplemented with OKT-3 (or respective anti-Vδ1 antibody), IL-15 and IFN-γ. From day 17 of culture onwards, the culture was supplemented with OKT-3 (or respective anti-Vδ1 antibody) and IL-15 every 3 to 4 days; half of the media was replaced with fresh media every 7 days.

For skin-derived γδ T cells, skin samples are prepared by removing subcutaneous fat and a 3mm biopsy punch is used to make multiple punches. Punches are placed on carbon matrix grids and placed in the well of a G-REX6 (Wilson Wolf). Each well is filled with complete isolation medium containing AIM-V media (Gibco, Life Technologies), CTS Immune Serum Replacement (Life Technologies), IL-2 and IL-15. For the first 7 days of culture, complete isolation medium containing Amphotericin B (Life Technologies) was used ("+AMP"). Media was changed every 7 days by gently aspirating the upper media and replacing with 2X complete isolation medium (without AMP), trying not to disturb the cells at the bottom of the plate or bioreactor. Beyond three weeks in culture, the resulting egressed cells are then passaged into fresh tissue culture vessels and fresh media (e.g. AIM-V media or TexMAX media (Miltenyi)) plus recombinant IL-2, IL-4, IL-15 and IL-21 before harvest. Optionally, αβ T cells also present within the culture are then removed with aid of αβ T cell depletion kits and associated protocols, such as those provided by Miltenyi. For further reference see WO2020/095059.

## γδ T cell binding assay

The binding of antibodies to γδ T cells was tested by incubating a fixed concentration of purified antibodies with 250000 γδ T cells. This incubation was performed under blocking conditions to prevent unspecific binding of antibodies via the Fc receptor. Detection was performed by addition of a secondary, fluorescent dye-conjugated antibody against human IgG1. For negative controls, cells were prepared with a) an isotype antibody only (recombinant human IgG), b) the fluorescent dye-conjugated anti-human IgG antibody only and c) a combination of a) and b). A control well of completely unstained cells was also prepared and analysed. As positive controls, a purified murine monoclonal IgG2 anti-human CD3 antibody and a purified murine monoclonal IgG1 anti-human TCR Vδ1 antibody were used in two different concentrations and stained with a fluorescent dye-conjugated goat anti-mouse secondary antibody. The assay was accepted if the lower concentration positive controls' mean fluorescence intensity in the FITC channel was at least tenfold as high as the highest negative control.

## SPR Analysis

A MASS-2 instrument with an amine high capacity chip (both from Sierra Sensors, Germany) was used to perform SPR analysis. 15 nM IgG were captured via protein G to an amine high capacity chip (100 nM for TS8.2). L1 (DV1-GV4) antigen was flown over the cell at a 1:2 dilution series from 2000 nM to 15.625 nM with the following parameters: 180 s association, 600 s dissociation, flowrate 30 μL/min, running buffer PBS + 0.02 % Tween 20. All experiments were performed at room temperature on MASS-2 instrument. Steady state fitting was determined according to Langmuir 1:1 binding using software Sierra Analyzer 3.2.

## Comparator antibodies

Antibodies of the invention were compared to commercially available antibodies in test assays as described.

| Antibody | Source | Catalogue No. |
|---|---|---|
| Ultra-LEAF™ Purified anti-human CD3 Antibody (OKT3), functional | Biolegend | 317326 |
| Ultra-LEAF™ Purified anti-human IgG2a Antibody (isotype control for OKT3) | Biolegend | 400264 |
| Human TCR Vδ1 purified mAb (functional TS8.2) | ThermoFisher | TCR1730 |
| Ultra-LEAF™ Purified Mouse IgG1, κ Isotype Ctrl Antibody for TS8.2 | Biolegend | 400166 |
| Anti-human CD107a BV421 (clone H4A3) | BD Biosciences | 562623 |
| BV421 Mouse IgG1, k Isotype Control Clone X40 (RUO) | BD Biosciences | 562438 |
| Anti-TCR Vδ1-PE-Vio770, human (flow, discontinued) | Miltenyi | 130-100-540 |
| Cetuximab | In-house | - |
| D1.3 HEL (anti-chicken lysozyme; mouse VH-VL + Human IgG1 Fc). | In-house | - |
| Anti-RSV (aka Motavizumab) | In-house | - |

## γδ TCR downregulation and degranulation assay

THP-1 (TIB-202™, ATCC) target cells loaded or not with test antibodies were labelled with CellTracker™ Orange CMTMR (ThermoFisher, C2927) and incubated with γδ T cells at 2:1 ratio in the presence of CD107a antibody (Anti-human CD107a BV421 (clone H4A3) BD Biosciences 562623). After 2 hours of incubation, the surface expression of γδ TCR (to measure TCR downregulation) and expression of CD107a (to measure degranulation) on γδ T cells was evaluated using flow cytometry.

## Killing assay (e.g. for Figure 6)

Gamma delta T cell killing activity and effect of test antibodies on the killing activity of γδ T cells was accessed by flow cytometry. After 4 hours of *in vitro* co-culture at 20:1 ratio of γδ T cells and CellTracker™ Orange CMTMR (ThermoFisher, C2927) labelled THP-1 cells (loaded or not with the antibody) were stained with Viability Dye eFluor™ 520 (ThermoFisher, 520 65-0867-14) to distinguish between live and dead target THP-1 cells. During sample acquisition, target cells were gated on the CellTracker™ Orange CMTMR positivity and examined for cell death based on the uptake of Viability Dye. CMTMR and eFluor™ 520 double positive cells were recognized as the dead target cells. The killing activity **of** γδ T cells was presented as a % of the dead target cells.

## Epitope mapping

All protein samples (antigen L1 (DV1-GV4) and antibodies 1245_P01_E07, 1245_P02_G04, 1252_P01_C08, 1251_P02_C05 and 1141_P01_E01) used for epitope mapping were analyzed for protein integrity and aggregation level using a high-mass MALDI.

In order to determine the epitope of L1 (DV1-GV4)/1245_P01_E07, L1 (DV1-GV4)/1245_P02_G04, L1 (DV1-GV4)/1252_P01_C08, L1(DV1-GV4)/1251_P02_C05, and L1(DV1-GV4)/1141_P01_E01 complexes with high resolution, the protein complexes were incubated with deuterated cross-linkers and subjected to multi-enzymatic proteolysis using trypsin, chymotrypsin, Asp-N, elastase and thermolysin. After enrichment of the cross-linked peptides, the samples were analyzed by high resolution mass spectrometry (nLC-LTQ-Orbitrap MS) and the data generated were analyzed using XQuest and Stavrox software.

## SYTOX-flow killing assay

The SYTOX assay allows the quantification of T cell mediated cytolysis of target cells using flow cytometry. Dead/dying cells are detected by a dead cell stain (SYTOX® AADvanced™, Life Technologies, S10274) which only penetrates into cells with compromised plasma membranes but cannot not cross intact cell membranes of healthy cells. NALM-6 target cells were labelled with CTV dye (Cell Trace Violet™, Life Technologies, C34557) and were thus distinguishable from the unlabelled effector T cells. Dead/dying target cells are identified through double staining of the dead cell dye and the cell trace dye.

After 16 hours in vitro co-culture of effector and CTV labelled target cells at indicated Effector-to-Target ratios (E:T, 1:1 or 10:1) the cells were stained with SYTOX® AADvanced™ and acquired on a FACSLyric™ (BD). The killing results are presented as % target cell reduction which is calculated by taking into account the number of live target cells (sample counts) in the test samples over the live target cells in the control wells without added effector cells (maximum counts):

$$\% \text{ target reduction} = 100 - ((\text{sample counts} / \text{maximum counts}) \times 100)$$

**EXAMPLE 2. Antigen design**

Gamma delta (γδ) T cells are polyclonal with CDR3 polyclonality. In order to avoid a situation where generated antibodies would be selected against the CDR3 sequence (as the CDR3 sequence will differ from TCR clone to TCR clone), the antigen design involved maintaining a consistent CDR3 in different formats. This design aimed to generate antibodies recognising a sequence within the variable domain, which is germline encoded and therefore the same in all clones, thus providing antibodies which recognise a wider subset of γδ T cells.

Another important aspect of the antigen preparation process was to design antigens which are suitable for expression as a protein. The γδ TCR is a complex protein involving a heterodimer with inter-chain and intra-chain disulphide bonds. A leucine zipper (LZ) format and Fc format were used to generate soluble TCR antigens to be used in the phage display selections. Both the LZ and Fc formats expressed well and successfully displayed the TCR (particularly heterodimeric TCRs, e.g. Vδ1 Vy4).

It was found that the CDR3 sequence from a public database entry for the γδ TCR expressed well as proteins (RCSB Protein Data Bank entries: 3OMZ). This was therefore selected for antigen preparation.

Antigens containing the delta variable 1 chain were expressed in LZ formats as a heterodimer (i.e. in combination with different gamma variable chains - "L1 ", "L2", "L3") and in Fc format either as a heterodimer ("F1", "F2", "F3") or as a homodimer (i.e. in combination with another delta variable 1 chain - "Fc1/1"). All delta variable 1 chains of the antigens contained the 3OMZ CDR3. Another series of γδ TCR antigens using similar formats were designed containing different delta variable chains (such as delta variable 2 and delta variable 3) and used to deselect antibodies with non-specific or off target binding ("L4", "F9", "Fc4/4", "Fc8/8"). These antigens were also designed to include the 3OMZ CDR3 to ensure that antibodies binding in the CDR3 region were also deselected.

Antigen functional validation was performed to confirm that the designed antigens would be suitable to generate anti-TRDV1 (TCR delta variable 1) antibodies and multispecific antibodies. Detection was seen only with antigens containing the δ1 domain **(Figure 1).**

**EXAMPLE 3. Phage Display**

Phage display selections were performed against libraries of human scFvs using either heterodimeric LZ TCR format in round 1 and 2, with deselections on heterodimeric LZ TCR in both rounds. Or round 1 was performed using homodimeric Fc fusion TCR with deselection on human IgG1 Fc followed by round 2 on heterodimeric LZ TCR with deselection on heterodimeric LZ TCR (see **Table 2).**

**Table** 2. **Overview phage display selections**

| Target | Round 1 selection | Round 1 deselection | Round 2 selection | Round 2 deselection |
|---|---|---|---|---|
| DV1 | bt-L1 (DV1-GV4) | L4 (DV2-GV4) | bt-L3 (DV1-GV8) | L4 (DV2-GV4) |
| DV1 | bt-Fc1/1 (DV1-DV1) | Fc | bt-L1 (DV1-GV4) | L4 (DV2-GV4) |
| bt = biotin. | | | | |

Selections were performed in solution phase using 100 nM biotinylated proteins. Deselections were performed using 1 μM non-biotinylated proteins.

Success of the phage display selections was analysed by polyclonal phage ELISA (DELFIA). All DV1 selection outputs showed the desired binding to the targets Fc 1/1, L1, L2, L3, F1 and F3. Varying degrees of binding to non-targets L4, F9, Fc 4/4, Fc 8/8 and Fc were detected (see **Figure 2A** and **B)**.

## EXAMPLE 4. Antibody and multispecific antibody selection

Hits obtained in Example 3 were sequenced (using standard methods known in the art). 130 unique clones were identified, which showed a unique combination of VH and VL CDR3. Of these 130 unique clones, 125 showed a unique VH CDR3 and 109 showed a unique VL CDR3.

Unique clones were re-arrayed and specificity was analysed by ELISA (DELFIA). A panel of 94 unique human scFv binders which bind TRDV1 (L1, L2, L3, F1, F2, F3) but not TRDV2 (L4), were identified from the selections.

Affinity ranking of the selected binders was included to aid the choice of clones going forward. A large number of binders showed affinities in the nanomolar range, reacting with 25 to 100 nM biotinylated antigen. A handful of binders showed a strong reaction with 5 nM antigen, indicating possible single digit nanomolar affinities. Some binders showed no reaction with 100 nM antigen, indicating affinities in the micromolar range.

For the selection of clones to proceed with to IgG conversion, the aim was to include as many germline lineages and as many different CDR3s as possible. Further, sequence liabilities like glycosylation, integrin binding sites, CD11c/CD18 binding sites, unpaired cysteines were avoided. In addition, a variety of affinities was included.

Selected clones were screened for binding to natural, cell-surface expressed $\gamma\delta$TCR using skin derived $\gamma\delta$ T cells obtained from different donors. The clones chosen to be converted to IgG are shown in **Table 3.** Additionally said anti-TRDV1 binders were then also formatted into multispecific antibodies by standard recombination, expression, processing and isolation methodology. Thereafter binding to both first target antigen (TRDV1) and second antigen were confirmed in said multispecific format.

**Table 3. DV1 binders for IgG conversion**

| Clone ID | Heavy CDR1 | SEQ ID NO. | Heavy CDR2 | SEQ ID NO. | Heavy CDR3 | SEQ ID NO. | Light CDR1 | SEQ ID NO. | Light CDR2 | SEQ | Light CDR3 | SEQ ID NO. | 100 nM L1 |
|---|---|---|---|---|---|---|---|---|---|---|---|---|---|
| 1245_P01_E07 | GFTFSDYY | 38 | ISSSGSTI | 26 | VDYADAFDI | 2 | QSIGTY | 50 | VAS | 160 | QQSYSTLLT | 14 | 162591 |
| 1252_P01_C08 | GFTVSSNY | 39 | IYSGGST | 27 | PIELGAFDI | 3 | NIGSQS | 51 | YDS | 161 | QVWDSSSDHVV | 15 | 1977 |
| 1245_P02_G04 | GDSVSSKSAA | 40 | TYYRSKWST | 28 | TWSGYVDV | 4 | QDINDW | 52 | DAS | 162 | QQSYSTPQVT | 16 | 5896 |
| 1245_P01_B07 | GFTFSDYY | 41 | ISSSGSTI | 29 | ENYLNAFDI | 5 | QSLSNY | 53 | AAS | 163 | QQSYSTPLT | 17 | 64271 |
| 1251_P02_C05 | GFTFSSYA | 42 | ISGGGGTT | 30 | DSGVAFDI | 6 | QNIRTW | 54 | DAS | 164 | QQFKRYPPT | 18 | 65269 |
| 1141_P01_E01 | GYSFTSYW | 43 | IYPGDSDT | 31 | HQVDTRTADY | 7 | RSDVGGYNY | 55 | EVS | 165 | SSYTSTSTLV | 19 | 136780 |
| 1139_P01_E04 | GDSVSSNSAA | 44 | TYYRSKWYN | 32 | SWNDAFDI | 8 | QSISTW | 56 | DAS | 166 | QQSYSTPLT | 20 | 23786 |
| 1245_P02_F07 | GDSVSSNSAA | 45 | TYYRSKWYN | 33 | DYYYSMDV | 9 | QSISSW | 57 | DAS | 167 | QQSHSHPPT | 21 | 10450 |
| 1245_P01_G06 | GFTFSDYY | 46 | ISSSGSTI | 34 | HSWNDAFDV | 10 | QSISSY | 58 | AAS | 168 | QQSYSTPDT | 22 | 22474 |
| 1245_P01_G09 | GDSVSSNSAA | 47 | TYYGSKWYN | 35 | DYYYSMDV | 11 | QSISTW | 59 | DAS | 169 | QQSYSTPVT | 23 | 18430 |
| 1138_P01_B09 | GFTFSDYY | 48 | ISSSGSTI | 36 | HSWSDAFDI | 12 | QDISNY | 60 | DAS | 170 | QQSYSTPLT | 24 | 29193 |
| 1251_P02_G10 | GFTFSDYY | 49 | ISSSGSTI | 37 | HSWNDAFDI | 13 | QSISSH | 61 | AAS | 171 | QQSYSTLLT | 25 | 17053 |

**EXAMPLE 5: Antibody SPR analysis**

Prepared IgG antibodies where passed through a γδ cell binding assay, and 5 were selected for further functional and biophysical characterization. SPR analysis was performed to determine the equilibrium dissociation constants ($K_D$). Sensorgrams of the interaction of the tested antibody with the analyte, along with steady state fits (if available), are presented in **Figure 3.** No binding was detected for TS8.2 with 80 RU of IgG captured on the chip. Results are summarised in **Table 4.**

**Table 4. Results of IgG capture**

| Analyte | Clone ID | $K_D$ (nM) | $K_D$ (M) |
|---|---|---|---|
| L1 (DV1-GV4) | 1245_P01_E07 | 12.4 | 1.24e-08 |
| L1 (DV1-GV4) | 1252_P01_C08 | 100 | 1.00e-07 |
| L1 (DV1-GV4) | 1245_P02_G04 | 126 | 1.26e-07 |
| L1 (DV1-GV4) | 1245_P01_B07 | 341 | 3.41e-07 |
| L1 (DV1-GV4) | 1251_P02_C05 | 1967* | 1.97e-06 |
| L1 (DV1-GV4) | 1139_P01_E04 | 251 | 2.51e-07 |
| L1 (DV1-GV4) | 1245_P02_F07 | 193 | 1.93e-07 |
| L1 (DV1-GV4) | 1245_P01_G06 | 264 | 2.64e-07 |
| L1 (DV1-GV4) | 1245_P01_G09 | 208 | 2.08e-07 |
| L1 (DV1-GV4) | 1138_P01_B09 | 290 | 2.90e-07 |
| L1 (DV1-GV4) | 1251_P02_G10 | 829 | 8.29e-07 |
| L1 (DV1-GV4) | **TS8.2** (commercial anti-Vδ1 antibody) | 44 | 4.40e-08 |
| *Binding of 1252_P02_C05 did not reach saturation, therefore data was extrapolated. | | | |

**EXAMPLE 6: TCR engagement assay**

The inventors designed several assays to be used for functional characterization of the selected antibodies. The first assay assessed γδ TCR engagement by measuring downregulation of the γδ TCR upon antibody binding. Selected antibodies were tested against commercial anti-CD3 and anti-Vδ1 antibodies which were used as positive controls or against 1252_P01_C08 as a positive control (for 1139_P01_E04, 1245_P02_F07, 1245_P01_G06 and 1245_P01_G09). Commercial anti-panγδ was used as a negative control because it is a panγδ antibody, recognising all γδ T cells irrespective of variable chain, and therefore is likely to have a different mode of action.

The assay was performed using skin-derived γδ T cells obtained from three different donor samples (samples with 94%, 80% and 57% purity). Results are shown in **Figure 4.** EC50 values are summarised in **Table 4,** below.

**EXAMPLE 7: T cell degranulation assay**

A second assay assessed the degranulation of γδ T cells. It is thought γδ T cells may mediate target cell killing by perforin-granzyme-mediated activation of apoptosis. Lytic granules within the cytoplasm of the γδ T cell may be released toward the target cell upon T cell activation. Therefore, labelling target cells with antibodies to CD107a and measuring the expression by flow cytometry can be used to identify degranulating γδ T cells.

As for **Example 6,** selected antibodies were tested against commercial anti-CD3 and anti-Vδ1 antibodies as positive controls or against 1252_P01_C08 as a positive control (for 1139_P01_E04, 1245_P02_F07, 1245_P01_G06 and 1245_P01_G09). IgG2a, IgG1 and D1.3 antibodies were used as negative controls. The assay was performed using skin-derived γδ T cells obtained from three different donor samples (samples with 94%, 80% and 57% purity). Results are shown in **Figure 5.** EC50 values are summarised in **Table 5,** below.

**EXAMPLE 8: Killing assay**

A third assay assessed the ability of γδ T cells activated with the selected antibodies to kill target cells.

As for **Example 6,** selected antibodies were tested against commercial anti-CD3 and anti-Vδ1 antibodies as positive controls or against 1252_P01_C08 as a positive control (for 1139_P01_E04, 1245_P02_F07, 1245_P01_G06 and 1245_P01_G09) and anti-panγδ as a negative control. IgG2a, IgG1 and D1.3 antibodies were also used as isotype controls. The assay was performed using skin-derived γδ T cells obtained from two donors (94% and 80% purity) and the results are shown in **Figure 6.**

Results from the three functional assays tested in **Examples 6-8** are summarised in **Table 5.**

**Table 5. Summary of results obtained from functional assays**

| Clone ID | TCR downregulation (EC50 μg/ml - 3 donors) | T cell degranulation (EC50 μg/ml - 3 donors) | Killing assay (EC50 μg/ml - 2 or 3 donors) |
|---|---|---|---|
| 1245_P01_E07 | 0.04-0.11 | 0.007-0.004 | 0.06 |
| 1252_P01_C08 | 0.02-0.03 | 0.001-0.0006 | 0.02 |
| 1245_P02_G04 | 0.01-0.05 | 0.002 | 0.10 |
| 1245_P01_B07 | Positive; 0.35 (1 donor only) | Positive; 0.1 (1 donor only) | 0.13 |
| 1251_P02_C05 | Positive; N/D | Positive; N/D | N/D* |
| 1139_P01_E04 | 0.027-0.057 | 0.005 | 0.005-0.019 |
| 1245_P02_F07 | 0.032-0.043 | 0.001-0.002 | 0.006-0.018 |
| 1245_P01_G06 | 0.042-0.055 | 0.001 | 0.007-0.051 |
| 1245_P01_G09 | 0.029-0.040 | 0.001 | 0.003-0.008 |
| 1138_P01_B09 | 0.078-0.130 | N/D | 0.055-0.199 |
| 1251_P02_G10 | 0.849; N/D | N/D | N/D** |
| **OKT3** (anti-CD3 antibody) | 0.03-0.04 | 0.001-0.008 | 0.05 |
| **TS8.2** (anti-Vδ1 antibody) | 0.48-0.8 | 0.07-0.16 | N/D* |
| N/D: could not be determined; N/D*: could not be determined, titration curve did not reach plateau; N/D**: Reduced killing profile, EC50 not established | | | |

**EXAMPLE 9: Epitope mapping**

In order to determine the epitope of antigen/antibody complexes with high resolution, the protein complexes were incubated with deuterated cross-linkers and subjected to multi-enzymatic cleavage. After enrichment of the cross-linked peptides, the samples were analysed by high resolution mass spectrometry (nLC-LTQ-Orbitrap MS) and the data generated were analysed using XQuest (version 2.0) and Stavrox (version 3.6) software.

After trypsin, chymotrypsin, Asp-N, elastase and thermolysin proteolysis of the protein complex L1(DV1-GV4)/1245_P01_E07 with deuterated d0d12, the nLC-orbitrap MS/MS analysis detected 13 cross-linked peptides between L1(DV1-GV4) and the antibody 1245_P01_E07. Results are presented in **Figure 7.**

After trypsin, chymotrypsin, Asp-N, elastase and thermolysin proteolysis of the protein complex L1(DV1-GV4)/1252_P01_C08 with deuterated d0d12, the nLC-orbitrap MS/MS analysis detected 5 cross-linked peptides between L1(DV1-GV4) and the antibody 1252_P01_C08. Results are presented in **Figure 8.**

After trypsin, chymotrypsin, Asp-N, elastase and thermolysin proteolysis of the protein complex L1(DV1-GV4)/1245_P02_G04 with deuterated d0d12, the nLC-orbitrap MS/MS analysis detected 20 cross-linked peptides between L1(DV1-GV4) and the antibody 1245_P02_G04. Results are presented in **Figure 9.**

After trypsin, chymotrypsin, Asp-N, elastase and thermolysin proteolysis of the protein complex L1(DV1-GV4)/1251_P02_C05 with deuterated d0d12, the nLC-orbitrap MS/MS analysis detected 5 cross-linked peptides between L1(DV1-GV4) and the antibody 1251_P02_C05. Results are presented in **Figure 10.**

Epitope binding with another antibody, Clone ID 1141_P01_E01, was also tested. After trypsin, chymotrypsin, Asp-N, elastase and thermolysin proteolysis of the protein complex L1(DV1-GV4)/1141_P01_E01 with deuterated d0d12, the nLC-orbitrap MS/MS analysis detected 20 cross-linked peptides between L1(DV1-GV4) and the antibody 1141_P01_E01. Results are presented in **Figure 11.**

A summary of the epitope mapping results is presented in **Table 6.**

**Table 6. Results of epitope mapping for antigen/antibody complexes**

| Clone ID | Epitope mapping, amino acid numbering of SEQ ID NO: 1 |
|---|---|
| 1245_P01_E07 | 5, 9, 16, 20, 62, 64, 72, 77 |
| 1252_P01_C08 | 50, 53, 59, 62, 64 |
| 1245_P02_G04 | 37, 42, 50, 53, 59, 64, 68, 69, 72, 73, 77 |
| 1251_P02_C05 | 59, 60, 68, 72 |
| 1141_P01_E01 | 3, 5, 9, 10, 12, 16, 17, 62, 64, 68, 69 |

**EXAMPLE 10: Expansion of Vδ1 T cells**

Expansion of isolated γδ T cells was investigated in the presence of selected antibodies and comparator antibodies. Comparator antibodies were selected from: OKT3 anti-CD3 antibody as a positive control, no antibody as a negative control or IgG1 antibody as an isotype control. Commercially available anti-Vδ1 antibodies, TS-1 and TS8.2 were also tested for comparison.

*Experiment 1:*

An initial investigation was conducted by seeding 70,000 cells/well with Complete Optimizer and cytokines as described in the "γδ T cell preparation" for blood-derived γδ T cells of Example 1. Selected and comparator antibodies were tested at various concentrations ranging from 4.2 ng/ml to 420 ng/ml. This experiment was conducted using tissue culture plates which allow the binding/immobilisation of the antibodies to the plastic.

Cells were harvested on days 7, 14 and 18 and the total cell count was determined using a cell counter (NC250, ChemoMetec). The results are shown in **Figure 7.** Cell viability of Vδ1 T cells was also measured on each harvest and all antibodies were shown to maintain cell viability throughout the experiments (data not shown). On day 18, the percentage, cell count and fold change of Vδ1 T cells was also analysed. The results are shown in **Figure 8.**

As can be seen in **Figure 7,** the total number of cells produced in cultures with antibodies increased steadily throughout the culture and were comparable or better than the commercial anti-Vδ1 antibodies. At day 18, the proportion of Vδ1 positive cells in the presence of 1245_P02_G04 ("G04"), 1245_P01_E07 ("E07"), 1245_P01_B07 ("B07") and 1252_P01_C08 ("C08") antibodies at most concentrations tested was greater than in cultures where OKT3, TS-1 or TS8.2 control antibodies were present (see **Figure 8A).**

*Experiment 2:*

A subsequent experiment was performed on isolated cells in a culture vessel with cytokines as described in the "γδ T cell preparation" of Example 1. Compared to Experiment 1, a different culture vessel was used whose surface does not facilitate antibody binding/immobilisation. Selected and comparator antibodies were tested at various concentrations ranging from 42 pg/ml to 42 ng/ml. During Experiment 2, results were obtained from experiments run in triplicates.

Cells were harvested on days 7, 11, 14 and 17 and the total cell count was determined using a cell counter as before. The results are shown in **Figure 9.** On day 17, the percentage, cell count and fold change of Vδ1 T cells was also analysed. The results are shown in **Figure 10.**

The cell composition, including non-Vδ1 cells, were also measured during Experiment 2. Day 17 cells were harvested and analysed by flow cytometry for surface expression of Vδ1, Vδ2 and αβTCR. The proportions of each cell type in each culture are shown graphically in **Figure 11** and the percentage values are provided in **Table 6.**

**Table 1. Cell composition at day 17 - Percentage of live cells of each subset**

| | αβ- γδ- | Vδ1 | Vδ2 | non Vδ1/Nδ2 | αβ |
|---|---|---|---|---|---|
| no AB | 63.00 | 18.17 | 0.86 | 7.10 | 0.37 |
| OKT-3 | 25.63 | 50.43 | 0.25 | 20.13 | 1.13 |
| IgG1 | 65.77 | 15.59 | 1.11 | 6.91 | 0.42 |
| TS8.2 42ng/ml | 30.60 | 53.57 | 3.59 | 7.46 | 0.14 |
| TS-1 42ng/ml | 18.77 | 65.90 | 0.91 | 9.51 | 0.12 |

(continued)

| | αβ- γδ- | **Vδ1** | **Vδ2** | **non Vδ1/Nδ2** | αβ |
|---|---|---|---|---|---|
| C08 42ng/ml | 0.79 | 96.43 | 0.08 | 2.51 | 0.05 |
| C08 4.2ng/ml | 1.91 | 94.67 | 0.18 | 2.63 | 0.05 |
| C08 420pg/ml | 8.47 | 80.57 | 0.28 | 8.42 | 0.04 |
| C08 42pg/ml | 35.97 | 25.93 | 3.04 | 19.50 | 0.31 |
| B07 42ng/ml | 0.94 | 95.57 | 0.46 | 2.73 | 0.05 |
| B07 4.2ng/ml | 1.79 | 94.10 | 0.40 | 3.28 | 0.01 |
| B07 420pg/ml | 3.08 | 91.80 | 0.29 | 3.94 | 0.02 |
| B07 42pg/ml | 17.93 | 62.90 | 0.85 | 9.16 | 0.07 |
| E07 42ng/ml | 2.29 | 85.13 | 0.19 | 11.65 | 0.04 |
| E07 4.2ng/ml | 2.15 | 91.23 | 0.13 | 5.77 | 0.04 |
| E07 420pg/ml | 9.25 | 73.90 | 0.42 | 13.05 | 0.02 |
| E07 42pg/ml | 49.23 | 18.67 | 2.17 | 7.70 | 0.43 |
| G04 42ng/ml | 1.90 | 88.53 | 0.47 | 8.09 | 0.05 |
| G04 4.2ng/ml | 4.25 | 89.67 | 0.93 | 3.98 | 0.02 |
| G04 420pg/ml | 25.97 | 50.60 | 1.45 | 12.72 | 0.11 |
| G04 42pg/ml | 44.00 | 13.77 | 2.33 | 26.30 | 0.32 |
| C05 42ng/ml | 25.00 | 42.03 | 3.75 | 13.67 | 1.32 |
| C05 4.2ng/ml | 46.87 | 22.03 | 2.58 | 16.46 | 0.38 |
| C05 420pg/ml | 33.53 | 44.60 | 2.23 | 11.13 | 0.22 |
| C05 42pg/ml | 36.83 | 25.23 | 6.16 | 18.00 | 0.30 |

As can be seen from these results, the proportion of Vδ1 positive cells is greater in cultures with B07, C08, E07 and G04 present compared to OKT3, TS-1 or TS8.2 controls. Therefore, the tested antibodies produce and expand Vδ1 positive cells more efficiently than commercially available antibodies, even when present at low concentrations in culture.

Cells from day 17 of Experiment 2 were also analysed for additional cell markers, including CD3-CD56+ to identify the presence of Natural Killer (NK) cells and Vδ1 T cells which express CD27 (i.e. CD27+). The results are summarised in Table 7.

**Table 8. Cell composition at day 17 - Percentage of NK and CD27+ cells**

| | **% CD56+CD3-** | | **%CD27+ of Vδ1** | |
|---|---|---|---|---|
| | Mean | **SEM** | **Mean** | **SEM** |
| no AB | 66.33 | 8.49 | 92.43 | 1.58 |
| OKT-3 | 7.90 | 1.04 | 99.03 | 0.14 |
| IgG1 | 70.67 | 6.41 | 87.87 | 0.81 |
| TS8.2 42ng/ml | 31.63 | 1.99 | 66.73 | 5.55 |
| TS-1 42ng/ml | 22.97 | 1.75 | 94.40 | 1.14 |
| C08 42ng/ml | 1.00 | 0.15 | 98.17 | 0.31 |
| C08 4.2ng/ml | 2.06 | 0.07 | 95.07 | 1.23 |
| C08 420pg/ml | 8.63 | 1.64 | 88.43 | 3.65 |
| C08 42pg/ml | 45.10 | 3.44 | 91.50 | 2.50 |

(continued)

| | % CD56+CD3- | | %CD27+ of Vδ1 | |
|---|---|---|---|---|
| | Mean | SEM | Mean | SEM |
| B07 42ng/ml | 1.40 | 0.39 | 95.47 | 1.37 |
| B07 4.2ng/ml | 1.70 | 0.16 | 96.70 | 0.43 |
| B07 420pg/ml | 3.47 | 0.38 | 95.17 | 0.86 |
| B07 42pg/ml | 22.03 | 4.66 | 88.03 | 3.00 |
| E07 42ng/ml | 2.59 | 0.93 | 92.27 | 2.10 |
| E07 4.2ng/ml | 1.98 | 0.09 | 95.77 | 0.52 |
| E07 420pg/ml | 8.72 | 1.33 | 92.43 | 0.14 |
| E07 42pg/ml | 67.73 | 1.23 | 93.60 | 1.16 |
| G04 42ng/ml | 2.20 | 0.32 | 93.80 | 0.36 |
| G04 4.2ng/ml | 3.53 | 0.51 | 91.63 | 1.80 |
| G04 420pg/ml | 30.53 | 5.00 | 81.37 | 3.11 |
| G04 42pg/ml | 51.13 | 8.90 | 94.20 | 0.93 |
| C05 42ng/ml | 37.17 | 6.53 | 93.80 | 0.87 |
| C05 4.2ng/ml | 52.27 | 8.16 | 85.40 | 4.46 |
| C05 420pg/ml | 37.93 | 1.57 | 90.83 | 2.01 |
| C05 42pg/ml | 43.40 | 8.64 | 92.17 | 2.02 |
| SEM: Standard error of the mean | | | | |

**EXAMPLE 11: Functionality of Vδ1 T cells**

Vδ1 T cells expanded in the presence of the selected antibodies retained a polyclonal repertoire of CDR3 regions and were also tested for functionality using the SYTOX-flow killing assay. The results are presented for cells obtained during Experiment 1 at day 14 using cells in a 10:1 Effector-to-Target (E:T) ratio (**Figure 12A)** and for cells obtained during Experiment 2 at day 17 (post freeze-thaw) using cells at a 1:1 and 10:1 E:T ratio **(Figure 12B).**

As can be seen in **Figure 12,** Vδ1 positive cells expanded in the presence of all antibodies effectively lysed target cells, indicating that they are functional even after freezing and thawing the cells.

**EXAMPLE 12: Functionality of cells after storage**

The functionality of cells after a storage step of freezing and then thawing was also investigated. A portion of cells was removed from culture at day 17 of Experiment 2 and frozen. Cells were then thawed and further expanded in culture with IL-15. **Figure 13** shows the total cell counts after 7 days of culturing cells post freeze-thaw for cultures contacted with B07, C08, E07, G04 or OKT-3 antibodies prior to freezing. All cultures showed the ability to proliferate after storage. Culturing was continued until day 42 and total cell counts were monitored during this period (results shown in **Figure 14).** Total cell numbers were maintained or increased in the cultures previously exposed to selected antibodies.

**EXAMPLE 13: Binding equivalence studies on modified anti-Vδ1 antibodies**

An ELISA-based antigen titration binding study was undertaken to compare the 1245_P02_G04 antibody manufactured in HEK to sequence and glycosylation variants thereof manufactured in CHO. Specifically, modifications to framework, to allotype, to hinge-mediated effector functionality, to Asn 297 glycosylation, and/or method of manufacture were undertaken and then included in this study. The assay ELISA set-up was as follows: Antigen comprised Antigen L1 (TRDV1/TRGV4); blocking buffer - 2% Marvel/PBS; mAbs diluted in a 1/2 dilution series starting at 5μg/ml; Antigen-antibody incubation in ELISA plates - 1 hour; Wash to remove non-specific binding - 3 × PBS-Tween, then 3 × PBS; Secondary antibody employed - DELFIA Eu labelled anti-human IgG (PerkinElmer; Cat #: 1244-330; 50 μg/ml) at 1/500 dilution; thereafter 1hr incubation prior to addition of DELFIA Enhancement Solution (PerkinElmer, used as per instruction); measurement by time-resolved fluorometry (TRF). For the antibodies made in CHO, standard expression vectors containing heavy and light chain cassettes were prepared under low-endotoxin conditions based on anion exchange chromatography. DNA concentration was determined by measuring the absorption at a wavelength of 260 nm. Sequences were verified with Sanger sequencing (with up to two sequencing reactions per plasmid depending on the size of the cDNA.) Suspension-adapted CHO K1 cells (originally from ATCC and adapted to serum-free growth in suspension culture) were employed for manufacture. The seed cells were grown in a chemically defined, animal-component free, serum-free medium. Cells were then transfected with vectors and transfection reagent, and cells were grown further. Supernatant was harvested by centrifugation and subsequent filtration (0.2 μm filter) and the antibody was purified using MabSelect™ SuRe™ prior to formulation. To generate the example defucosylated antibody, protocols first described by von Horsten HH et al. (2010) Glycobiology 20(12):1607-18 were introduced into the CHO expression platform described above and ahead of expression and purification. Once manufactured and purified, mAb defucosylation was confirmed by MS-based analysis.

The results of this study are summarised in **Figure 15.** Therein the y-axis indicates ELISA signal and the x-axis indicates Vδ1 antigen concentrations (ug/ml) employed. Antibodies included in this titration study are outlined and for further detail, RSV = anti-RSV control mAb control (made in CHO). G04 = 1245_P02_G04 (made in HEK, SEQ ID NO:112). AD3 = variant G04 (made in CHO; SEQ ID NO:129 and comprising a variable domain sequence SEQ ID NO:131 and SEQ ID NO:132 and comprising a constant domain SEQ ID NO:133 and SEQ ID NO:134). AD4 = hinge modified AD3 (made in CHO; SEQ ID NO:130 and comprising a constant domain SEQ ID NO:133 and SEQ ID NO:135). AD3gly = defucosylated AD3 made in engineered CHO. Under all titrations, equivalent antigen binding was observed for all variants.

**EXAMPLE 14: Anti-Vδ1 antibody binding equivalence studies on human germline Vδ1 antigen and a polymorphic variant thereof.**

An ELISA-based binding study comparison was undertaken to study anti-Vδ1 antibody binding to human germline Vδ1 antigen as per IMGT database (see SEQ ID NO:1) versus binding to polymorphic human germline Vδ1 antigen (SED ID NO:128). Specifically, a comparison of antibody binding and cross-reactivity with Antigen L1 (containing canonical TRDV1/TRGV4 germline sequence) and L1AV (variant TRDV1/TRGV4 comprising said TRDV1 germline polymorphism) was performed. The results are presented in **Figure 16.** Antibodies indicated as follows: G04 = 1245_P02_G04 (SEQ ID NO:112); G04 LAGA = G04 with Hinge Fc modification (L235A, G237AEU numbering; SEQ ID NO: 136). E07 LAGA =1245_P01_E07 with L235A, G237A, SEQ ID NO: 137. C08 LAGA =1252_P01_C08 with L235A, G237A, SEQ ID NO:138, D1.3 = control. A serial dilution of each antibody to said antigens was performed and at all dilutions, for each antibody variant, equivalent binding to both antigens was observed. Shown is the equivalent binding observed for one example dilution (1nM antibody) in said series.

**EXAMPLE 15: Anti-Vδ1 antibody binding conferred increase in Vδ1+ cell cytokine secretion**

In brief, all antibodies were diluted to 10μg/ml and incubated overnight to bind the antibodies to the plate, before washing. Skin-derived γδ T cells from two different skin donations were prepared as outlined elsewhere herein (see Example 1; specifically, the section on skin-derived γδ T cell preparation). These skin cells were then added to tissue culture plates (100,000 cells per well) containing the bound antibodies as indicated. Cells were then left for one day prior to harvest of the supernatant and storage at -80°C. For cytokine analysis of the supernatants, an MSD U-PLEX Human Assay: K151TTK-1, K151UCK-1 was employed (Mesoscale Diagnostics, Maryland). Antibodies employed in this study included IgG1 (non- Vδ1-binding control), B07 (1245_P01_B07), E07 (1245_P01_E07), G04 (1245_P02_G04; 1245), and C08 (1252_P01_C08). The results of this study are presented in **Figure 17.** Specifically, **Figure 17 (A)** and **(B)** respectively outline the quantities of TNF-alpha and IFN-gamma detected in the supernatant when skin-derived γδ T cells and the higher levels observed when anti-Vδ1 antibodies as indicated are applied

**EXAMPLE 16: Anti-Vδ1 antibody conferred increase in Vδ1+ cell Granzyme B levels/activity**

Skin-derived γδ T cells were prepared as outlined elsewhere herein (see Example 1; the section on skin-derived γδ T cell preparation). THP-1 cells were first loaded with GranToxiLux probe (a cell permeable, fluorogenic substrate is designed to detect Granzyme B activity in the target cells) and in accordance with manufacturer's instructions (OncoImmunin, Inc. Gaithersburg, US). The THP-1 cells were then pulsed with the antibodies as indicated in **Figure 18** at 10μg/ml prior to mixing with the skin-derived γδ T at a target/effector ratio of 1:20. The co-cultures were then briefly centrifuged to ensure rapid conjugate formation prior to co-culture for 1 hour and subsequent flow analysis in accordance with GranToxiLux protocols. Antibodies employed in this study included IgG1 (non- Vδ1-binding control), B07 (1245_P01_B07), E07 (1245_P01_E07), G04 (1245_P02_G04; 1245), and C08 (1252_P01_C08). The results are presented in **Figure 18** and highlight higher levels of Granzyme B in the target cancer cells observed when anti-Vδ1 antibodies as indicated are applied to this Vδ1+/THP-1 co-culture model system.

**EXAMPLE 17: Anti-Vδ1 antibody conferred modulation and proliferation of immune cells in human tissue.**

Human skin punch-biopsies (from five different donors) were incubated for 21-days in culture with the antibodies as indicated. Skin samples were prepared by removing subcutaneous fat etc. as described elsewhere herein (see Example 1; the section on skin-derived γδ T cell preparation). Replicate punches from each donation were then placed on carbon matrix grids which were then placed in the well of a G-REX6 (Wilson Wolf). Each well was filled with complete medium as also described elsewhere herein. To investigate and compare the effect of differing antibodies, these were added on day 0, 7, 14 to a working concentration of 100ng/ml. After 21 days in culture cells were harvested and analysed by flow cytometry. The results of said study are presented in **Figure 19** and specifically highlight marked differences in modulatory effect of the differing antibodies: Order from left to right: Vd1 TS8.2 = TS8.2 (Thermo Fisher); OKT-3 (Biolegend); C08 IgG1 =1252_P01_C08; E07 =1245_P01_E07; G04 = 1245_P02_G04. **Figure 19 (A)** highlights the mean quantity of viable pan-γδ TCR positive cells observed at end of culture; results presented as a gated fraction (percent mean + Std Dev) of the total live cell population as analysed by flow cytometry. For pan-γδ content analysis flow gating strategy as follows: Singlets > Live cells> Pan-γδ antibody (Miltenyi, 130-113-508). **Figure 19 (B)** highlights the mean quantity of viable Vδ1+ TCR positive cells observed at end of culture; results presented as a gated percent fraction (percent mean +Std Dev) of the total live cell population as analysed by flow cytometry. For Vδ1+ cell content analysis, flow gating strategy as follows: Singlets > Live cells> Panγδ (Miltenyi, 130-113-508) > Vδ1 (Miltenyi, 130-100-553). **Figure 19 (C)** highlights the number of viable double positive Vδ1+ CD25+ cells observed at end of culture; results presented as a gated fraction (percent mean +Std Dev) of the total live cell population. For CD25+ Vδ1+ cell content analysis, Flow gating strategy as follows: Singlets > Live cells> Panγδ (Miltenyi, 130-113-508) > Vδ1 (Miltenyi, 130-100-553) > CD25 (Miltenyi, 130-113-286. The combined results of this study summarise the differential effect of the antibodies of the invention as described herein relative to comparator antibodies TS8.2 and OKT3. And whilst not being bound by this theory, one possibility for the less favourable effects conferred on Vδ1 + cells by TS8.2 or OKT3 may be due to the deleterious effect conferred by these comparator molecules on immune cell function over time in this model system.

**EXAMPLE 18: Anti-Vδ1 antibody conferred modulation and proliferation of immune cells in TILs.**

Studies were undertaken to explore anti-Vδ1 antibody conferred modulation and proliferation of human tumour infiltrating lymphocytes (TILs). For these studies, human renal cell carcinoma (RCC) tumour biopsies were shipped fresh and processed upon receipt. Specifically, the tissue was chopped into ~2mm$^2$. Up to 1g of tissue was placed into each Miltenyi C tube along with 4.7mL RPMI and enzymes from Miltenyi's Tumour Dissociation Kit at concentrations recommended by the manufacturer aside from Enzyme R which was used at 0.2 × concentration to prevent cleavage of pertinent cell surface molecules. C-Tubes were placed on the gentleMACS™ Octo Dissociator with Heaters. Program 37C_h_TDK_1 for the dissociation of soft tumours was selected. The digest was then filtered through a 70mM filter to generate a single cell suspension. RPMI containing 10% FBS was added to the digest to quench enzymatic activity. The cells are washed 2 × with RPMI/10%FBS and resuspended for counting. Derived cells were then seeded in TC wells (24-well G-REX, Wilson Wolf) at 2.5x10e6 per well. Cells were then incubated without or without cytokines and with or without antibodies for 18 days. Antibodies included in the study are outlined in Figure 20. These include OKT3 (to 50ng/ml) and 1252_P01_C08 aka "C08" herein (to 500ng/ml). When included, bolus additions of these antibodies were added on day 0, 7, 11 and 14. During said incubation, media was replaced with fresh media on days 11 and day 14. Flow cytometry analysis was performed on day 0 and day 18 to determine the lymphocyte phenotype as well as fold change in cell number. Cells were first gated on live CD45+ cells and then as indicated. In arms where recombinant cytokines were included these were added as follows. Day 0: IL-4, IFN-y, IL-21, IL-1β. Additional IL-15 was included on day 7, 11, 14. Additional IL-21 and IFN-y were included on day 7 and day 14 respectively. **Figure 20 (A)** shows the fold-increase in TIL Vδ1+ cells following 18 days culture in the presence of C08 or OKT3 with and without cytokine support (CK) where indicated.. These results show substantial fold increases in TIL Vδ1+ cells with the application of either the C08 or comparator OKT3 antibody in the presence of cytokines, as compared to antibody or cytokines alone. **Figure 20 (B)** shows increases in total Vδ1 cell number at harvest following . These results show substantial increases in TIL Vδ1+ cell number following culture with C08 or comparator OKT3 antibody in the presence of cytokines, as compared to antibody or cytokines alone. **Figure 20 (C)** presents an example gating strategy used in the flow cytometric analysis of the cells. From the live CD45+ cell population cells were gated on lymphocytes based on their forward and side scatter properties (not shown), γδ T cells were then separated from αβ T cells by staining for the T cell receptors. Finally, the proportion of Vδ1 cells within the total γδ T cell population was determined. Example data for day 18 is shown for 2 conditions as indicated (+/-1252_P01_C08): 64.3% cells were CD45+, of those CD45% cells, 53.1% were γδ+, and of the γδ cells, 89.7% were Vδ1+. **Figure 20 (D)** presents a cell-surface phenotypic profile of TIL Vδ1+ cells at harvest. Higher levels of CD69 were observed following culture with the C08 antibody. **Figure 20 (E)** presents analysis of the TIL γδ-negative, CD8-positive lymphocyte fraction within the live CD45-positive gate at harvest. In summary, the combined results highlight the modulatory effects conferred by anti-Vδ1 antibody of the invention described herein on TIL populations.

**EXAMPLE 19: Anti-Vδ1 antibody conferred enhancement of Vδ1+ cell mediated cytotoxicity and diseased-cell-specific cytotoxicity.**

Cytotoxicity/potency-assays and studies were undertaken in model systems comprising a triculture of Vδ1+ effector cells, THP-1 monocytic cancer cells, and healthy primary monocytes +/- anti-Vδ1 antibodies (1245_P02_G04; 1245_P01_E07; 1252_P01_C08) as described herein and inclusive of controls (no mAb or D1.3) as indicated in **Figure 21.** In brief, all antibodies were diluted to 10μg/ml in PBS and incubated overnight at 4oC in 384-well ultra imaging assay plates (Perkin Elmer) to bind antibodies to the plate, before washing with PBS. Healthy control monocytes were isolated from peripheral blood mononuclear cells (PBMCs; Lonza) by negative selection using magnetic activated cell sorting (MACS; Miltenyi Biotec). Monocytes and cultured THP-1 cells (ATCC) were stained with [0.5μM] CellTrace Violet and CellTrace CFSE live cell dyes respectively for 20 minutes before mixing in a 1:1 ratio. Expanded skin derived Vδ1 γδ T-cells were detached from tissue culture flasks and serial diluted to generate a range of effector to target ratios (E:T) before adding to THP1:monocyte cell suspensions. Cell suspensions were seeding into 384-well assay plates to give a final cell seeding density of 1,000 THP-1 cells per well, 1,000 Monocytes per well, and a range of γδ T-cells (top E:T ratio of 60:1). To determine the numbers of live THP-1 and healthy control monocytes after 24 hours, confocal images were acquired using an Opera Phenix high content platform capturing nine fields of view at 10x magnification. Live cell counts were quantified based on size, morphology, texture and intensity of live cell stains. Results are presented in **Figure 21. Figure 21 (A)** presents THP-1 and monocyte cell numbers after 24 hours in triple co-culture with γδ T-cells in the presence of plate-bound mAbs or controls as indicated. Cell numbers were calculated using high content confocal microscopy using live cell imaging. **Figure 21 (B)** presents a bar chart representation designed to highlight the window between diseased-cell specific killing and non-diseased healthy cell sparing at the top E:T ratio (60:1) after 24 hours co-culture: Left-hand bar chart; fold-increase in killing of diseased-cells (THP-1) versus killing of non-diseased cells (primary human monocytes). Right-hand bar chart; same data but represented as percent enhanced killing versus control. **Figure 21 (C)** presents tabulated results summarizing the percent improvement in potency of Vδ1 γδ T-cells killing THP-1 target cells in the presence of Vδ1 mAbs compared to no mAb control as calculated from figure (A). **Figure 21 (D)** presents tabulated results of EC50 values as calculated from Figure (A) represented as γδ T-cell numbers required to confer 50% THP-1 cell killing. The combined results and discoveries as outlined in **Figure 21** highlight the ability of antibodies described herein to enhance cytotoxicity and diseased-cell specificity of Vδ1+ cells.

**EXAMPLE 20: Multi-specific antibody conferred enhancement of Vδ1+ effector cell mediated cytotoxicity; targeting a tissue-centric disease associated antigen.**

Cytotoxicity/potency-assay studies were undertaken to explore the effect of multi-specific antibodies on co-cultures of Vδ1+ effector cells and A-431 cancer cells. A-431 ($EGFR^{++}$; ATCC) target cells were seeded in a 384-well imaging plate (Perkin Elmer) at 1,000 cells/well and incubated at 37°C overnight in DMEM (10% FCS). Antibodies and multi-specific antibodies as indicated were diluted to 10μg/ml and added assay plate (2μg/ml final assay concentration). Expanded skin-derived Vδ1 γδ T-cells were detached from tissue culture flasks and serial diluted to give a range of E:T ratios (top E:T ratio of 60:1) before adding to assay plate. A-431 cells were incubated with Vδ1 γδ T-cells in the presence of antibodies or controls at 30°C, 5% $CO_2$. After 24 hours incubation, Hoechst 33342 (ThermoFisher) was added to stain cells (2μM final). To determine the numbers of live A-431 cells, confocal images were acquired using an Opera Phenix high content platform capturing nine fields of view at 10x magnification. Live cell counts were quantified base on size, morphology, texture, and intensity of live cell stains. Effector/Target (E:T) time course studies to determine the ET ratio wherein 50% of target cells are killed in model systems +/- the controls, comparators, antibodies and multi-specific antibodies as indicated. Results are presented in **Figure 22.**

First, **Figure 22 (A-D)** present example co-culture results wherein Vb1 + / A-431 co-cultures were studies +/- multi-specific antibodies comprising anti-Vδ1 × anti-TAA (EGFR) bispecific binding moieties wherein the anti-Vδ1 VL+VH binding domain (to the first target) is combined with the CH1-CH2-CH3 domain of an anti-EGFR binding moiety (to the second target). Controls and comparators employed as indicated; from left to right: No mAb = no antibody added; D1.3 = D1.3 control; D1.3 IgG LAGA = D1.3 + L235A,G237A; D1.3 FS1-67 = D1.3 variable domain with EGFR binding constant domain plus L235A, G237A (SEQ ID NO: 139); Cetuximab (in-house generated). More specifically, **Figure 22 (A)** presents the results for five-hour co-cultures with aforementioned controls, comparators, and the following test articles:

C08-LAGA = 1252_P01_C08 with L235A,G237A (SEQ ID NO:138); C08 FS1-67 =1252_P01_C08 combined with EGFR binding domain containing a L235A,G237A (SEQ ID NO:140). **Figure 22 (B)** presents equivalent data of five-hour co-cultures with aforementioned controls, comparators, and the following test articles: G04-LAGA = 1245_P02_G04 with L235A,G237A; G04 FS1-67 = 1245_P02_G04 combined with EGFR binding domain containing L235A,G237A (SEQ ID NO: 141). **Figure 22 (C)** presents equivalent data of five-hour co-cultures with controls, comparators, and the following test articles: E07-LAGA = 1245_P01_E07 with L235A,G237A; E07 FS1-67 = 1245_P01_E07 combined with EGFR binding domain containing L235A,G237A (SEQ ID NO:142). **Figure 22 (D)** presents a Table summarizing the percent improvement in cytotoxicity of Vδ1 γδ T-cells in the presence of controls, comparators, and test articles over 5, 12 and 24 hours. A greater than 450% enhancement can be observed when antibodies or fragment thereof as described herein are presented in a multi-specific format.

Second, **Figure 22 (E-H)** present example results wherein Vδ1+ / A-431 co-cultures were studied +/- multi-specific antibodies comprising anti-Vδ1 × anti-TAA (EGFR) bispecific binding moieties wherein the anti-Vδ1 binding domain (to the first target) comprises a full-length antibody (VH-CH1-CH2-CH3/VL-CL) then combined with an anti-EGFR scFv binding moiety (to the second target). Controls and comparators employed as indicated; from left to right: No mAb = no antibody added; D1.3 = Control; D1.3 IgG LAGA = D1.3 + L235A,G237A; D1.3 LAGA Cetuximab = D1.3 with L235A, G237A plus a C-term Cetuximab-derived scFv (SEQ ID NO:143); Cetuximab (in-house generated). More specifically, **Figure 22 (E)** presents five-hour co-cultures with aforementioned controls, comparators, and the following test articles: C08-LAGA = 1252_P01_C08 with L235A,G237A; C08 LAGA Cetuximab =1252_P01_C08 with L235A,G237A and with C-term Cetuximab-derived scFv (SEQ ID NO: 144). **Figure 22 (F)** presents five-hour culture with aforementioned controls, comparators, and the following test articles: G04-LAGA = 1245_P02_G04 with L235A,G237A; G04 LAGA Cetuximab = 1245_P02_G04 with L235A,G237A and with C-term Cetuximab-derived scFv (SEQ ID NO: 145). **Figure 22 (G)** presents five-hour culture with controls, comparators, and the following test articles: E07-LAGA = 1245_P01_E07 with L235A,G237A; E07 LAGA Cetuximab = 1245_P01_E07 with L235A,G237A and with C-term Cetuximab-derived scFv (SEQ ID NO: 146). **Figure 22 (H)** presents a Table summarizing the percent improvement in potency of Vδ1 γδ T-cells in the presence of controls, comparators, and test articles over 5, 12 and 24 hours. A greater than 300% enhancement can be observed when antibodies or fragment thereof as described herein are presented in a multi-specific format.

Third, **Figure 22 (I and J)** outline an example alternative approach to representing the data. Specifically shown is percentage improvement conferred by multi-specific antibody E07 FS1-67 **(I)** or C08 FS1-67 **(J)** upon Vδ1+ effector cell cytotoxicity towards EGFR+ cells relative at the 24-hour time point relative to all component parts and comparators as indicated.

**EXAMPLE 21: Multi-specific antibody conferred enhancement of Vδ1+ mediated cytotoxicity and diseased-cell-specific cytotoxicity; targeting a hemopoietic-centric disease associated antigen.**

Cytotoxicity/potency-assays and studies were undertaken in model systems comprising a triculture Vδ1+ effector cells, and Raji cancer cells, and healthy primary monocytes +/- multi-specific antibodies comprising anti-Vδ1 × anti-TAA (CD19) multi-specific antibody in orderto determine whether tumour associated antigen (TAA) linked Vδ1 monoclonal antibodies in a bispecific format can enhance Vδ1 γδ T-cell killing of specific target cells. Specifically, Raji cells (CD19++; ATCC) were incubated with Vδ1 γδ T-cells in the presence of Vδ1 × CD19 multi-specific antibodies. All antibodies were diluted to 4μg/ml (final assay concentration 1μg/ml) and added to a 384-well imaging plate (Perkin Elmer). Expanded skin derived Vδ1 γδ T-cells were detached from tissue culture flasks and serial diluted to give a range of effector to target ratios (E:T). Raji cells were stained with [0.5μM] CellTrace Far Red before mixing in a 1:1 ratio with titrated Vδ1 γδ T-cells. Cell suspensions were seeding into 384-well assay plates to give a final cell seeding density of 1,000 Raji cells per well, and a range of γδ T-cells (top E:T ratio of 30:1). To determine the numbers of live Raji after 24 hours, confocal images were acquired using an Opera Phenix high content platform capturing nine fields of view at 10x magnification. Live cell counts were quantified base on size, morphology, texture and intensity of live cell stains. Results are captured in **Figure 23.** Antibodies and comparators employed therein are as indicated. Specifically, RSV IgG = Motavizumab non-binding control, G04 = 1245_P02_G04; E07= 1245_P01_E07; D1.3 VHVL = D1.3 HEL with heavy-chain C-terminal anti-CD19 scFv (see SEQ ID NO: 157 for scFv binding module employed); G04 VHVL = 1245_P02_G04 LAGA with heavy chain C-terminal anti-CD19 scFv (SEQ ID NO: 158); E07 VHVL = 1245_P01_E07 LAGA with heavy chain C-terminal anti-CD19 scFv (SEQ ID NO: 159). **Figure 23 (A)** Table summarizing (i) the calculated EC50s represented as γδ T-cell numbers or E:T ratios required to induce 50% Raji cell killing, and (ii) percentage improvement in EC50s compared to no mAb control. **Figure 23 (B)** Bar chart representing the percentage improvement in ability of γδ T-cells to lyse 50% of Raji target cells in the presence of Vδ1-CD19 multi-specific antibodies.

**EXAMPLE 24: Vδ1-CD19 bispecific antibody binding affinity to human and cyno Vδ1**

The binding affinity of the antibodies to target (i.e. the Vδ1 chain of a γδ TCR, both human and cyno antigens) is established by SPR analysis using a Reichert 4SPR instrument (Reichert Technologies). Antibody is coated onto a Planar Protein A Sensor Chip (Reichert Technologies) to give an increase on baseline of approximately 500 uRIU. Recombinant human Vδ1 heterodimer or cyno Vδ1 heterodimer was flown over the cell. All experiments were performed at room temperature. These data demonstrate the bispecific antibody binds both human and cyno Vδ1.

**EXAMPLE 25A: Vδ1-CD19 bispecific antibodies in Vδ1 γδT-cells activation and cytotoxicity assays.**

Healthy B-cells were isolated from PBMCs (Lonza), using negative magnetic activated cell sorting (MACS; MiltenyiBiotec). Expression of CD19 on cancerous NALM-6 cells (ATCC), Raji cells (ATCC) and healthy isolated B-cells was determined. Briefly, 5×10^4 cells were incubated with CD19 antibodies (Biolegend) for 15 minutes at 4°C before washing and fixing. Expression of CD19 was determined by flow cytometry (MACSQuant10, MiltenyiBiotec). The results are shown in **Figure 25A.**

The effect of CD19-Vδ1 bispecific antibodies on $CD19^+$ target cell cytotoxicity and $CD19^+$ healthy cell sparing was determined using high content confocal imaging in the Opera Phenix (Perkin Elmer). NALM-6, Raji and B-cells were stained with [0.5μM] CellTrace CFSE live cell dye for 20 minutes. Bispecific antibodies and controls were serially diluted into PBS before adding to 384-well imaging assay plates (Perkin Elmer). Expanded skin derived Vδ1 γδ T-cells were detached from tissue culture flasks and re-suspended in basal growth media before mixing 1:1 with either NALM-6, Raji or B-cells in suspension. Cell suspensions were seeding into 384-well assay plates to give a final cell seeding density of 2,000 NALM-6, Raji or B-cells per well and 2,000 Vδ1 γδ T-cells per well. Final assay antibody concentrations ranged between 6.6nM to 66pM. Cells were co-cultured for 24 hours before staining with DRAQ7 (1:300 final, Abcam). To determine the numbers of live target cells, confocal images were acquired using an Opera Phenix high content platform capturing nine fields of view at 10x magnification. Live cell counts were quantified base on size, morphology, texture and intensity of CFSE staining and the absence of DRAQ7 staining. The results are shown in **Figure 25, B to E.**

To determine the effect of CD19-Vδ1 bispecific antibodies on Vδ1 γδ T-cell activation and degranulation, Vδ1 TCR downregulation and CD107a upregulation was quantified in the presence of $CD19^+$ target cells and $CD19^+$ healthy cells. Briefly, antibodies were serially diluted in PBS before adding to U-bottomed 96-well plates. NALM-6 and isolated B-cells were stained with [0.5μM] CellTrace CFSE live cell dye for 20 minutes. Skin derived Vδ1 γδ T-cells were detached from culture flasks and cell suspensions were mixed 0.5:1 with NALM-6 or B-cells. Cell suspensions were seeded into assay plates at 2.5×10^4 Vδ1 γδ T-cells per well to 5×10^4 NALM-6 or B-cells per well. Final assay antibody concentrations range from 60nM to 3pM. The cells were incubated for 4 hours at 37°C, 5% $CO_2$. Cells were washed and stained for dead cells (eFlour 520, Invitrogen), Vδ1 TCR (MiltenyiBiotec) and aCD107a (Miltenyi) surface expression for 30 minutes at 4°C. The cells were washed in FACS buffer and resuspended in Cell Fix (BD sciences) before incubating overnight at 4oC in the dark. The VD1 TCR expression level was measured by flow cytometry the following day using the MACS Quant Analyzer 16. The results are shown in **Figure 25, F to K.**

**Conclusion:** Vδ1-CD19 bispecific antibodies enhance γδT-cell mediated cytotoxicity of $CD19^+$ target cells while sparing healthy $CD19^+$ cells.

**Example 38: Vδ1-CD19 bispecific antibodies selectively enhance the cytotoxic effect of Vδ1 γδT-cells on cancer cells while sparing healthy cells**

The effect of CD19-Vδ1 bispecific antibodies on CD19+ target cell cytotoxicity and CD19+ healthy cell sparing was determined using high content confocal imaging in the Opera Phenix (Perkin Elmer). Healthy B-cells were isolated from PBMCs (Lonza), using negative magnetic activated cell sorting (MACS; MiltenyiBiotec). Raji cells and healthy primary B-cells were stained with CellTrace dyes. Expanded skin derived Vδ1 γδ T-cells and donor matched αβT-cells were removed from culture, washed and resuspended in culture media before mixing 1:1 with Raji cells and B-cells. Bispecific antibodies and controls were serially diluted in PBS before adding to 384-well imaging assay plates (Perkin Elmer). Cell suspensions of either γδ T-cells, Raji cells, and B-cells, or αβT-cells, Raji cells, and B-cells, or Vδ1 γδ T-cells, αβT-cells Raji cells, and B-cells were added to 384-well imaging assay plate to achieve 2,000 of each cell type per well and final antibody assay concentrations ranging between 6.6nM to 66pM. To determine the numbers of live target cells, confocal images were acquired using an Opera Phenix high content platform capturing nine fields of view at 10x magnification. Live cell counts were quantified base on size, morphology, texture and intensity of CFSE staining at 24 hours. The results are shown in **Figure 38, A to F.**

To determine whether activation of Vδ1 γδ T-cells or αβT-cells resulted in elevated production of the pro-tumorigenic cytokine IL-17, supernatants were collected from imaging plates after images were acquired at 24 hours. Supernatants were run on the U-Plex 10-plex Meso Scale Discovery (MSD) to quantify IL-17A levels. Results are shown in **Figure 38, G to I.**

IL-17A (Interleukin-17A) is a pro-tumorigenic cytokine which is produced by activated T-cells. IL-17A can enhance tumour growth and dampen the anti-cancer immune response. As shown in **Figure 38, G to I,** anti-vδ1 antibodies do not induce secretion of IL-17A, whereas anti-CD3 antibodies do.

**Conclusion:** Vδ1-CD19 bispecific antibodies enhance γδT-cell mediated cytotoxicity of CD19+ target cells while sparing healthy CD19+ cells. In contrast, anti-CD3xCD19 bispecifics enhanced both γδT-cell and αβT-cell mediated lysis of CD19+ target cells, however activation of αβT-cells also enhanced the lysis of healthy primary CD19+ B-cells, as well as the secretion of the pro-tumorigenic cytokine IL-17A.

**EXAMPLE 26: Vδ1-Her2 bispecific antibodies in binding and high content cytotoxicity assays.**

Her2 and Vδ1 expression was determined on SK-BR-3 cells (Caltag-Medsystems Ltd), BT-474 cells (ATCC), MDA-MB-231-Luc cells (Creative Biogene Biotechnology) and Vδ1 γδT-cells. Briefly, 5×10^4 cells were incubated with Her2 and Vδ1 antibodies (MiltenyiBiotec) for 15 minutes at 4°C before washing and fixing. Expression of Her2 and Vδ1 was determined by flow cytometry (MACSQuant10, MiltenyiBiotec). The results are shown in **Figure 26A and 26B.** Binding of Her2-Vδ1 bispecific antibodies was determined by incubating target cells with a range of concentrations of anti-Vδ1 antibody or Vδ1 bispecific antibodies, or controls (IgG control or Trastuzumab) for 15 minutes. After washing, cells were incubated for a further 15 minutes with anti-human IgG secondary antibodies before washing and fixing. The amount of antibody bound to each cell type was determined by flow cytometry. The results are shown in **Figure 26, C to F.**

The effect of Her2-Vδ1 bispecific antibodies on $Her2^+$/$Her2^-$ target cell cytotoxicity was determined using high content confocal imaging in the Opera Phenix (Perkin Elmer). Briefly, SK-BR-3, BT-474 and MDA-MB-231 cells were seeded into 384-well imaging plates (Perkin Elmer) to give a final seeding density of 2,000 target cells per well before incubating overnight at 37°C, 5% $CO_2$. Antibodies were diluted into PBS and serially diluted 1:10 before adding to the assay plates to give a final assay concentrations of 333nM to 0.33pM. Expanded skin derived Vδ1 γδ T-cells were detached from tissue culture flasks and re-suspended in basal growth media before adding to the assay plate at 2,000 cells per well at a 1:1 Effector:Target ratio. Cells were co-cultured for 24 hours before staining with Hoechst (1:1000 final, Invitrogen) and DRAQ7 (1:300 final, Abcam). To determine the numbers of live target cells, confocal images were acquired using an Opera Phenix high content platform capturing nine fields of view at 10x magnification. Live cell counts were quantified base on size, morphology, texture and intensity of live cell stains and the absence of DRAQ7 staining. The results are shown in **Figures 26, G to J.**

Conclusion: Vδ1-Her2 bispecific antibodies enhance γδT-cell mediated cytotoxicity of Her2+ target cells while sparing Her2- cells.

**EXAMPLE 27: Vδ1-EGFR bispecific antibody binding affinity to human Vδ1 and human EGFR antigen**

The binding affinity of the antibodies to target (i.e. the Vδ1 chain of a γδ TCR and EGFR) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human EGFR was flown over the cell at a concentration of 100 nM with the following parameters: 180 s association, 480 s dissociation, flowrate 25 μL/min, running buffer PBS + 0.05 % Tween 20. All experiments were performed at room temperature. The results are shown in **Figures 27A and 27B.**

Conclusions: This data demonstrates that the Vδ1/EGFR bispecific antibodies demonstrate binding to human Vδ1 that is comparable to their parent monospecific antibody in addition to the introduction of human EGFR binding capability.

**EXAMPLE 28: Target cell binding of Vδ1-EGFR bispecific antibodies**

EGFR-positive A431 and Vδ1-positive primary γδT-cells were assayed to determine the specificity and affinity of EGFR/Vb1 bispecific antibody binding. Target cells were detached from tissue culture flask, resuspended in PBS and seeded at a final density of 100,000 cells per well in v-bottom 96-well plates. Cells underwent centrifugation and the cell pellets were resuspended in FcR blocking reagent according to the manufacturer's instructions, and incubated for 20 minutes at 4C prior to a further wash. Antibodies were diluted to 500 nM in PBS and serially diluted 1:10 in PBS to 50 pM, and added to the cells, followed by a 20-minute incubation at 4C. To determine the quantity of mAb bound to the cell surface, the cells were then stained with a murine anti-human IgG secondary antibody, conjugated to APC (product code.., dilution: 1:100) in addition to a viability dye. Following 20-minute incubation at 4C, the cells were washed twice, and fluorescence measured using the MACSQuant. The results are shown in **Figure 28, A to F.** Conclusions: This data demonstrates that the Vδ1/EGFR bispecific antibodies demonstrate binding to Vδ1 γδT-cells that is comparable to their parent monospecific mAb, in addition to the introduction of binding to the EGFR-positive A431 cell line.

**EXAMPLE 29: Assessing γδ T cell activation and target cell cytotoxicity in vitro**

Expanded skin-derived Vδ1 γδ T-cells and A431 cells were detached from tissue culture flasks and re-suspended in basal growth media and seeded in 96-well plates at the relevant cell dilutions dependent on the desired effector:target ratio. mAbs were diluted and added to each well at the specified concentration. The cultures were then incubated at 37°C, 5% $CO_2$ for4 **(D)** or 24 hours **(A-C, E).** To determine the numbers of live cells, the cells were harvested and stained with viability dye at a 1:1000 dilution for 20 minutes. To determine CD25 status, cells were surface stained with an anti-CD25 antibody following cell harvest. To measure degranulation, a fluorophore-conjugated anti-CD107α antibody was added directly into the cell-antibody mix at the start of the co-culture. Following, two washes and cell fixation, fluorescence was measured using the MACSQuant and live cell counts and median fluorescence intensity determined. The results are shown in **Figure 29, A to E.**

**Conclusions:** This data demonstrates that the Vδ1/EGFR bispecific antibodies induce activation and degranulation of primary Vδ1-positive γδ T-cells leading to increased cell-mediated lysis of EGFR-positive A431 cell line.

**EXAMPLE 30: Anti-vδ1 antibody causes CD3 down-regulation on vδ1 cells.**

Studies were undertaken to explore the effect of stimulating/activating vδ1 cells with anti-vδ1 antibody with respect to down-regulation of CD3 on vδ1 cells. This was tested by incubating the anti-vδ1 clone ADT1-4-2 with PBMC and then analysing the TCR by phenotyping.

Cryopreserved human peripheral blood mononuclear cells (PBMC) were commercially sourced and seeded into round bottom 96-well tissue culture plates at 250,000 cells/well in 250ul of complete media (RPMI supplemented with 10% FCS, pen/strep, non-essential amino acids, sodium pyruvate and HEPES) with 10ng/ml IL15. A titration vδ1 antibody ADT1-4-2 was added to a final concentration of 1ug/ml (6.67nM), 0.01ug/ml (0.067nM) or 0.0001ug/ml (0.00067nM). RSV IgG antibody was included as a control at matched concentration. Cultures were incubated for 14 days, with media and antibody replenished every 3 days. Flow cytometry analysis was performed at the end-point to phenotype the vδ1 cells and TCR expression in each condition. Cells were gated firstly on live singlets, followed by panγδ (Miltenyi REA592; 130-113-508), which was the parent gate for vδ1 (Miltenyi REA173; 130-100-553), which was itself the parent gate for CD3 (Miltenyi REA613; 130-113-142). Cell populations were identified through positive staining, and then the relative level of expression of each marker between samples through the MFI.

**Figure 30A** shows the vδ1 TCR MFI upon antibody stimulation as an indication of mAb target engagement. **Figure 30B** shows the MFI of CD3 expression on positively gated vδ1 cells. Stimulation with the vδ1 antibody clone ADT1-4-2 engaged vδ1 cells and resulted in down-regulation of both vδ1 and CD3 on vδ1 cells.

**EXAMPLE 31: Vδ1-FAPα bispecific antibodies enhance Vδ1 γδT-cell activation and lysis of FAPα+ fibroblasts.**

The binding kinetics of the binding of anti-Vδ1, anti-FAPa and anti-Vδ1xFAPα antibodies to their targets (i.e. the Vδ1 chain of a γδ TCR and FAPα) are established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human FAPα was flown over the cell at a concentration of 100 nM with the following parameters: 180 s association, 480 s dissociation, flowrate 25 μL/min, running buffer PBS + 0.05 % Tween 20. All experiments were performed at room temperature. The results are shown in **Figure 31, A.**

Binding of Vδ1-FAPα bispecific antibodies was determined by incubating $FAP\alpha^+$ target cells or $V\delta1^+$ effector cells with a range of concentrations of anti-Vδ1 antibody or Vδ1 bispecific antibodies, or controls (IgG control or anti-FAPα) for 15 minutes. After washing, cells were incubated for a further 15 minutes with anti-human Fc secondary antibodies before washing and fixing. The amount of antibody bound to each cell type was determined by flow cytometry. The results are shown in **Figure 31, B to C.**

To determine the effect of Vδ1-FAPα bispecific antibodies on Vδ1 γδ T-cell activation and degranulation, Vδ1 TCR downregulation and CD107a upregulation was quantified in the presence of $FAP\alpha^+$ target cells. Briefly, anti-Vδ1, anti-FAPa and anti-Vδ1xFAPα bispecific antibodies were serially diluted in PBS before adding to U-bottomed 96-well plates. $FAP\alpha^+$ target cells (BJ fibroblasts or human dermal fibroblasts) were stained with [0.5μM] CellTrace CFSE live cell dye for 20 minutes. Skin derived Vδ1 γδ T-cells were detached from culture flasks and cell suspensions were mixed 1:1 with $FAP\alpha^+$ target cells or diluted 1:1 with media. Cell suspensions were seeded into assay plates at 2.5×10^4 Vδ1 γδ T-cells per well in the presence or absence of 2.5×10^4 $FAP\alpha^+$ target cells per well. Final assay antibody concentrations range from 200nM to 2pM. The cells were incubated for 4 hours at 37°C, 5% $CO_2$ before washing and staining for dead cells (eFlour 520, Invitrogen), Vδ1 TCR (MiltenyiBiotec) and aCD107a (Miltenyi) surface expression for 30 minutes at 4°C. The cells were washed in FACS buffer and resuspended in Cell Fix (BD sciences) before incubating overnight at 4°C in the dark. The VD1 TCR and CD107a expression level, determined by median fluorescence intensity (MFI), was measured by flow cytometry the following day using the MACS Quant Analyzer 16. The results are shown in **Figure 31 D-G.**

Moderate Vδ1 TCR downregulation is observed in the absence of FAPα$^+$ fibroblasts **(Figure 31, D).** However, in the presence of FAPα$^+$ fibroblasts, anti-FAPα-Vδ1 bispecific antibodies greatly enhance TCR downregulation **(Figure 31, E).** This shows the effect of anti-FAPα-Vδ1 bispecific antibodies on Vδ1 TCR downregulation on Vδ1 γδT-cells is enhanced by via binding to a tumour specific antigen such as FAPα, on adjacent cells.

In the presence of anti-Vδ1xFAPα bispecific antibodies and FAPα$^+$ fibroblasts, CD107a (a marker of degranulation) is upregulated on Vδ1 γδT-cells compared to monoclonal controls **(Figure 31, G),** When the FAPα$^+$ fibroblasts are not present, CD107a is not upregulated on Vδ1 γδT-cells compared to monoclonal controls **(Figure 31, F),** This shows the effect of anti-FAPα-Vb1 bispecific antibodies on CD107a upregulation on Vδ1 γδ T-cells is specific and the anti- FAPα-Vδ1 bispecific antibodies enhance the degranulation and activation of Vδ1 γδT-cells in the presence of FAPα$^+$ cells.

The effect of FAPα-Vδ1 bispecific antibodies on FAPα$^+$ target cell cytotoxicity was determined using high content confocal imaging in the Opera Phenix (Perkin Elmer). FAPα$^+$ target cells (BJ fibroblasts, human dermal fibroblasts) were seeded into 384-well imaging plates (Perkin Elmer) and incubated for 24 hours. Bispecific antibodies and controls were serially diluted into PBS before adding to assay plates. Expanded skin derived Vδ1 γδ T-cells were detached from tissue culture flasks and re-suspended in basal growth media before adding to assay plates for an Effector:Target ratio of 1:1. Final assay antibody concentrations ranged between 6.6nM to 66fM. Cells were co-cultured for 24 hours before staining with DRAQ7 (1:300 final, Abcam) and Hoechst (1:1,000, Invitrogen). To determine the numbers of live target cells, confocal images were acquired using an Opera Phenix high content platform capturing nine fields of view at 10x magnification. Live cell counts were quantified base on size, morphology, texture and intensity of Hoechst staining and the absence of DRAQ7 staining. The results are shown in **Figure 31, H.** In the presence of anti-FAPα-Vδ1 bispecific antibodies, a marked increase in fibroblast cytotoxicity was observed compared to anti-Vδ1 and anti-FAPa controls **(Figure 31, H).** This demonstrates that directly bridging Vδ1 γδ T-cells with target cells can specifically enhance the activation and cytotoxic effect of Vδ1 γδ T-cells.

**Conclusions:** Anti-Vδ1-FAPα bispecific antibodies bind specifically to Vb1$^+$ γδ T-cells and FAPα+ target cells resulting in enhanced activation of Vδ1 γδ T-cells in the presence of FAPα$^+$ cells, indicated by elevated Vδ1 TCR downregulation, CD107a upregulation, and lysis of FAPα$^+$ fibroblasts.

**EXAMPLE 32: Vδ1-MSLN bispecific antibodies enhance Vδ1 γδT-cell activation and lysis of MSLN$^+$ target cells.**

The binding kinetics of the binding of anti-Vδ1, anti-MSLN (Mesothelin) and anti-Vδ1xMSLN antibodies to their targets (i.e. the Vδ1 chain of a γδ TCR and MSLN) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human MSLN was flown over the cell at a top concentration of 100 nM with the following parameters: 180 s association, 480 s dissociation, flowrate 25 μL/min, running buffer PBS + 0.05 % Tween 20. All experiments were performed at room temperature. The results are shown in **Figure 32, A.**

Binding of MSLN-Vδ1 bispecific antibodies was determined by incubating MSLN$^+$ target cells or Vδ1$^+$ effector cells with a range of concentrations of anti-Vδ1 antibody or Vδ1 bispecific antibodies, or controls (IgG control or anti-MSLN) for 15 minutes. After washing, cells were incubated for a further 15 minutes with anti-human IgG secondary antibodies before washing and fixing. The amount of antibody bound to each cell type was determined by flow cytometry. The results are shown in **Figure 32, B to C.**

To determine the effect of MSLN-Vδ1 bispecific antibodies on Vδ1 γδ T-cell activation and degranulation, Vδ1 TCR downregulation and CD107a upregulation was quantified in the presence of MSLN$^+$ target cells. Briefly, anti-Vδ1, anti-MSLN and anti-Vδ1xMSLN bispecific antibodies were serially diluted in PBS before adding to U-bottomed 96-well plates. MSLN$^+$ target cells (OVCAR-3 or HeLa) were stained with [0.5μM] CellTrace CFSE live cell dye for 20 minutes. Skin derived Vδ1 γδ T-cells were detached from culture flasks and cell suspensions were mixed 1:1 with MSLN$^+$ target cells or diluted 1:1 with media. Cell suspensions were seeded into assay plates at 2.5×10^4 Vδ1 γδ T-cells per well in the presence or absence of 2.5×10^4 MSLN$^+$ target cells per well. Final assay antibody concentrations range from 200nM to 2pM. The cells were incubated for 4 hours at 37°C, 5% $CO_2$ before washing and stained for dead cells (eFlour 520, Invitrogen), Vδ1 TCR (MiltenyiBiotec) and aCD107a (Miltenyi) surface expression for 30 minutes at 4°C. The cells were washed in FACS buffer and resuspended in Cell Fix (BD sciences) before incubating overnight at 4°C in the dark. The VD1 TCR and CD107a expression level, determined by median fluorescence intensity (MFI), was measured by flow cytometry the following day using the MACS Quant Analyzer 16. The results are shown in **Figure 32 D-G.**

Moderate Vδ1 TCR downregulation is observed in the absence of MSLN$^+$ target cells **(Figure 32, D).** However in the presence of MSLN$^+$ OVCAR-3 cells, anti-MSLN-Vδ1 bispecific antibodies greatly enhance TCR downregulation **(Figure 32, E).** This shows the effect of anti-MSLN-Vb1 bispecific antibodies on Vδ1 TCR downregulation on Vδ1 γδ T-cells is enhanced by via binding to a tumour specific antigen such as MSLN, on adjacent cells.

In the presence of anti-Vδ1xMSLN bispecific antibodies and $MSLN^+$ OVCAR-3 cells, CD107a (a marker of degranulation) is upregulated on Vδ1 γδT-cells compared to monoclonal controls **(Figure 32, G),** When the $MSLN^+$ OVCAR-3 cells are not present, CD107a is not upregulated on Vδ1 γδT-cells compared to monoclonal controls **(Figure 32, F),** This shows the effect of anti-MSLN-Vδ1 bispecific antibodies on CD107a upregulation on Vδ1 γδ T-cells is specific and the anti- MSLN-Vδ1 bispecific antibodies enhance the degranulation and activation of Vδ1 γδT-cells in the presence of $MSLN^+$ cells.

The effect of MSLN-Vδ1 bispecific antibodies on $MSLN^+$ target cell cytotoxicity was determined using high content confocal imaging in the Opera Phenix (Perkin Elmer). $MSLN^+$ target cells (HeLa or OVCAR-2) were seeded into 384-well imaging plates (Perkin Elmer) and incubated for 24 hours. Bispecific antibodies and controls were serially diluted into PBS before adding to assay plates. Expanded skin derived Vδ1 γδ T-cells were detached from tissue culture flasks and re-suspended in basal growth media before adding to assay plates for an Effector:Target ratio of 1:1. Final assay antibody concentrations ranged between 6.6nM to 66fM. Cells were co-cultured for 24 hours before staining with DRAQ7 (1:300 final, Abcam) and Hoechst (1:1,000, Invitrogen). To determine the numbers of live target cells, confocal images were acquired using an Opera Phenix high content platform capturing nine fields of view at 10x magnification. Live cell counts were quantified base on size, morphology, texture and intensity of Hoechst staining and the absence of DRAQ7 staining. The results are shown in **Figure 32, H.** In the presence of anti-MSLN-Vδ1 bispecific antibodies, a marked increase in target cell cytotoxicity was observed compared to anti-Vδ1 and anti-MSLN controls **(Figure 32, H).** This demonstrates that directly bridging Vδ1 γδ T-cells with target cells can specifically enhance the activation and cytotoxic effect of Vδ1 γδ T-cells.

**Conclusions:** Anti-Vδ1xMSLN bispecific antibodies bind specifically to $Vb1^+$ γδ T-cells and $MSLN^+$ target cells resulting in enhanced activation of Vδ1 γδT-cells in the presence of $MSLN^+$ cells, indicated by elevated Vδ1 TCR down-regulation, CD107a upregulation, and lysis of $MSLN^+$ target cells.

**EXAMPLE 33: Vδ1-PD-1 bispecific antibodies enhance Vδ1 γδT-cell activation and block PD-1/PD-L1 checkpoint inhibition**

The binding kinetics of the binding of anti-Vδ1, anti-PD-1 and anti-Vδ1xPD-1 antibodies to bind (i.e. the Vδ1 chain of a γδ TCR and PD-1) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human PD-1 was flown over the cell at a top concentration of 100 nM. The results are shown in Figure 33 A.

To assess the dual binding of bispecific antibodies to both target ligands, recombinant PD-1 was first immobilised on a Carboxymethyl Dextran Sensor Chip (Reichert Technologies) at 10ug/ml before flowing over the bispecific antibodies at 100nM. The ability to subsequently bind to the Vδ1 γδ TCR was then assessed by flowing over recombinant human Vδ1 heterodimer at 100nM. All experiments were performed at room temperature. The results are shown in **Figure 33 B.**

Binding of anti-Vδ1xPD-1 bispecific antibodies to Vδ1 γδ T-cells and $PD\text{-}1^+$ immune cells was assessed by flow cytometry. Initially CD4 and CD8 T-cells were negatively selected by magnetic sorting from PBMC buffy coats extracted from whole blood. Following activation by anti-CD3/ anti-CD28 antibodies conjugated to Dynabeads (Invitrogen), cell surface expression of PD-1 was detected on CD4 and CD8 T-cells. Activated T-cells and Vδ1 γδ T-cells were incubated with a range of concentrations of anti-Vδ1xPD-1 bispecific antibodies or controls (IgG control or anti-PD-1) for 15 minutes. After washing, cells were incubated for a further 15 minutes with anti-human IgG secondary antibodies before washing and fixing. The amount of antibody bound to each cell type was determined by flow cytometry. The results are shown in **Figure 33 C, D.**

To determine the effect of anti-Vδ1xPD-1 bispecific antibodies on Vδ1 γδ T-cell activation, Vδ1 TCR downregulation was quantified in the presence of $PD\text{-}1^+$ T-cells. Briefly, anti-Vδ1, anti-PD-1 and anti-Vδ1xPD-1 bispecific antibodies were serially diluted in PBS before adding to assay plates. $PD\text{-}1^+$ T-cells were stained with CellTrace CFSE live cell dye and mixed 1:1 with skin derived Vδ1 γδ T-cells or diluted 1:1 with media. Cell suspensions were seeded into assay plates at 2.5×10^4 Vδ1 γδ T-cells per well in the presence or absence of 2.5×10^4 $PD\text{-}1^+$ T-cells per well. Final assay antibody concentrations range from 200nM to 2pM. Cells were incubated for 4 hours at 37°C, 5% $CO_2$ before washing and stained for dead cells (eFlour 520, Invitrogen) and Vδ1 TCR (MiltenyiBiotec). Cells were washed and resuspended in Cell Fix (BD sciences). VD1 TCR expression level was determined by median fluorescence intensity (MFI), measured by flow cytometry using the MACS Quant Analyzer 16. The results are shown in **Figure 33 E, F.**

To assess the effect of anti-Vδ1xPD-1 bispecific antibodies on the activation of $PD\text{-}1^+$ T-cells, $PD\text{-}1^+$ NFAT Jurkat cells (Promega, JA2191) were incubated with anti-Vδ1xPD-1 bispecific antibodies or controls (PD-1 monoclonal antibody or anti-RSVIgGxanti-PD-1) for 5 hours at 37°C, 5% $CO_2$. The assay was performed in the presence or absence of recombinant Vδ1 protein pre-coated at 1μg/well in opaque, white 96-well plates. After 5 hours, Bio-Glo Luciferase reagent (Promega) was added to cells in a 1:1 ratio. After incubating for 5 minutes at room temperature the luminescence signal was detected on a BioTek Synergy plate reader. Raw luminescence signal was converted to fold relative luminsence units (RLU). The results are shown in **Figure 33 G.**

**Conclusions:** anti-Vδ1xPD-1 bispecific antibodies enhance the activation of Vδ1 γδ T-cells, for example by crosslinking via PD-1$^+$ CD4 or CD8 T-cells, as well as blocking the PD-1/PD-L1 immune checkpoint inhibition in CD4 or CD8 T-cells.

**EXAMPLE 34: Vδ1-4-1BB bispecific antibodies enhance Vδ1 γδT-cell and CD8 T-cell activation**

The binding kinetics of the binding of anti-Vδ1, anti-4-1BB and anti-Vδ1x4-1BB antibodies to their targets (i.e. the Vδ1 chain of a γδ TCR and 4-1BB) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human 4-1BB was flown over the cell at a top concentration of 100 nM. The results are shown in Figure **34 A.**

To assess the dual binding of bispecific antibodies to both target ligands, recombinant 4-1BB was first immobilised on a Carboxymethyl Dextran Sensor Chip (Reichert Technologies) at 10ug/ml before flowing over the bispecific antibodies at 100nM. The ability to subsequently bind to the Vδ1 γδ TCR was then assessed by flowing over recombinant human Vδ1 heterodimer at 100nM. All experiments were performed at room temperature. The results are shown in **Figure 34 B.**

Binding of anti-Vδ1x4-1BB bispecific antibodies to Vδ1 γδ T-cells and 4-1BB$^+$ immune cells was assessed by flow cytometry. Initially CD8$^+$ T-cells were negatively selected by magnetic sorting from PBMC buffy coats extracted from whole blood. Following activation by anti-CD3/ anti-CD28 antibodies conjugated to Dynabeads (Invitrogen), cell surface expression of 4-1 BB was elevated on CD8 T-cells. Activated 4-1BB$^+$ CD8 T-cells and Vδ1 γδ T-cells were incubated with a range of concentrations of anti-Vδ1x4-1BB bispecific antibodies or controls (IgG control or anti-4-1BB) for 15 minutes. After washing, cells were incubated for a further 15 minutes with anti-human IgG secondary antibodies before washing and fixing. The amount of antibody bound to each cell type was determined by flow cytometry. The results are shown in **Figure 34 C, D.**

To determine the effect of anti-Vδ1x4-1BB bispecific antibodies on Vδ1 γδ T-cell activation, Vδ1 TCR downregulation was quantified in the presence of 4-1BB$^+$ T-cells. Briefly, anti-Vδ1, anti-4-1BB and anti-Vδ1x4-1BB bispecific antibodies were serially diluted in PBS before adding to assay plates. 4-1BB$^+$ CD8 T-cells were stained with CellTrace CFSE live cell dye and mixed 1:1 with skin derived Vδ1 γδ T-cells or diluted 1:1 with media. Cell suspensions were seeded into assay plates at 2.5×10^4 Vδ1 γδ T-cells per well in the presence or absence of 2.5×10^4 4-1BB$^+$ CD8 T-cells per well. Final assay antibody concentrations range from 200nM to 2pM. Cells were incubated for 4 hours at 37°C, 5% $CO_2$ before washing and stained for dead cells (eFlour 520, Invitrogen) and Vδ1 TCR (MiltenyiBiotec). Cells were washed and resuspended in Cell Fix (BD sciences). VD1 TCR expression level was determined by median fluorescence intensity (MFI), measured by flow cytometry using the MACS Quant Analyzer 16. The results are shown in **Figure 34, E, F.**

To assess the effect of anti-Vδ1x4-1BB bispecific antibodies on the activation of 4-1BB$^+$ T-cells, 4-1BB$^+$ NFAT Jurkat cells (Promega, JA2191) were incubated with anti-Vδ1x4-1BB bispecific antibodies or controls (anti-4-1BB monoclonal antibodies or anti-RSVIgGxanti-4-1BB) for 5 hours at 37°C, 5% $CO_2$. Assay was performed in the presence or absence of recombinant Vδ1 protein pre-coated at 1μg/well in opaque, white 96-well plates. After 5 hours, Bio-Glo Luciferase reagent (Promega) was added to cells in a 1:1 ratio. After incubating for 5 minutes at room temperature the luminescence signal was detected on a BioTek Synergy plate reader. Raw luminescence signal was converted to fold relative luminsence units (RLU). The results are shown in **Figure 34, G.**

**Conclusions:** anti-Vδ1x4-1BB bispecific antibodies enhance the activation of Vδ1 γδ T-cells, for example by crosslinking to 4-1BB$^+$ CD8 T-cells, as well as activating 4-1BB$^+$ T-cells.

**EXAMPLE 35: Vδ1-OX40 bispecific antibodies enhance Vδ1 γδT-cell and CD4 T-cell activation**

The binding kinetics of the binding of anti-Vδ1, anti-OX40 and anti-Vδ1xOX40 antibodies to their targets (i.e. the Vδ1 chain of a γδ TCR and OX40) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human OX40 was flown over the cell at a top concentration of 100 nM. The results are shown in **Figure 35, A.**

To assess the dual binding of bispecific antibodies to both target ligands, recombinant OX40 was first immobilised on a Carboxymethyl Dextran Sensor Chip (Reichert Technologies) at 10ug/ml before flowing over the bispecific antibodies at 100nM. The ability to subsequently bind to the Vδ1 γδ TCR was then assessed by flowing over recombinant human Vδ1 heterodimer at 100nM. All experiments were performed at room temperature. The results are shown in **Figure 35, B.**

Binding of anti-Vδ1xOX40 bispecific antibodies to Vδ1 γδ T-cells and OX40$^+$ immune cells was assessed by flow cytometry. Initially CD4$^+$ T-cells were negatively selected by magnetic sorting from PBMC buffy coats extracted from whole blood. Following activation by anti-CD3/ anti-CD28 antibodies conjugated to Dynabeads (Invitrogen), cell surface expression of OX40 was elevated on CD4 T-cells. Activated OX40⁺ CD4 T-cells and Vδ1 γδ T-cells were incubated with a range of concentrations of anti-Vδ1xOX40 bispecific antibodies or controls (IgG control or anti-OX40) for 15 minutes. After washing, cells were incubated for a further 15 minutes with anti-human IgG secondary antibodies before washing and fixing. The amount of antibody bound to each cell type was determined by flow cytometry. The results are shown in **Figure 35, C, D.**

To determine the effect of anti-Vδ1xOX40 bispecific antibodies on Vδ1 γδ T-cell activation, Vδ1 TCR downregulation was quantified in the presence of $OX40^+$ T-cells. Briefly, anti-Vδ1, anti-OX40 and anti-Vδ1xOX40 bispecific antibodies were serially diluted in PBS before adding to assay plates. $OX40^+$ CD4 T-cells were stained with CellTrace CFSE live cell dye and mixed 1:1 with skin derived Vδ1 γδ T-cells or diluted 1:1 with media. Cell suspensions were seeded into assay plates at 2.5×10^4 Vδ1 γδ T-cells per well in the presence or absence of 2.5×10^4 $OX40^+$ CD4 T-cells per well. Final assay antibody concentrations range from 200nM to 2pM. Cells were incubated for 4 hours at 37°C, 5% $CO_2$ before washing and stained for dead cells (eFlour 520, Invitrogen) and Vδ1 TCR (MiltenyiBiotec). Cells were washed and resuspended in Cell Fix (BD sciences). VD1 TCR expression level was determined by median fluorescence intensity (MFI), measured by flow cytometry using the MACS Quant Analyzer 16. The results are shown in **Figure 35, E, F.**

To assess the effect of anti-Vδ1xOX40 bispecific antibodies on the activation of $OX40^+$T-cells, $OX40^+$ NFAT Jurkat cells (Promega, JA2191) were incubated with anti-Vδ1xOX40 bispecific antibodies or controls (OX40L (OX40 Ligand), anti-OX40 or anti-RSVIgGxanti-OX40) for 5 hours at 37°C, 5% $CO_2$. Assay was performed in the presence or absence of recombinant Vδ1 protein pre-coated at 1μg/well in opaque, white 96-well plates. After 5 hours, Bio-Glo Luciferase reagent (Promega) was added to cells in a 1:1 ratio. After incubating for 5 minutes at room temperature the luminescence signal was detected on a BioTek Synergy plate reader. Raw luminescence signal was converted to fold relative luminsence units (RLU). The results are shown in **Figure 35, G.**

**Conclusions:** anti-Vδ1xOX40 bispecific antibodies enhance the activation of Vδ1 γδ T-cells, for example by crosslinking to $OX40^+$ CD4 T-cells, as well as activating $OX40^+$ T-cells.

**EXAMPLE 36: Vδ1-TIGIT bispecific antibodies enhance Vδ1 γδT-cell activation and block TIGIT/PVR(CD155) checkpoint inhibition**

The binding kinetics of the binding of anti-Vδ1, anti-TIGIT and anti-Vδ1xTIGIT antibodies to their targets (i.e. the Vδ1 chain of a γδ TCR and TIGIT) is established by SPR analysis using a Reichert 4SPR instrument (Reichert Technologies). Antibody (1.5 ug/mL) is coated onto a Planar Protein A Sensor Chip (Reichert Technologies) to give an increase on baseline of approximately 500 uRIU. Recombinant human Vδ1 heterodimer or human TIGIT was flown over the cell at a top concentration of 100 nM. The results are shown in **Figure 36, A.**

To assess the dual binding of bispecific antibodies to both target ligands, recombinant TIGIT was first immobilised on a Carboxymethyl Dextran Sensor Chip (Reichert Technologies) at 10ug/ml before flowing over the bispecific antibodies at 100nM. The ability to subsequently bind to the Vδ1 γδ TCR was then assessed by flowing over recombinant human Vδ1 heterodimer at 100nM. All experiments were performed at room temperature. The results are shown in **Figure 36, B.**

Binding of anti-Vδ1xTIGIT bispecific antibodies to Vδ1 γδ T-cells and $TIGIT^+$ immune cells was assessed by flow cytometry. Initially CD4 and CD8 T-cells were negatively selected by magnetic sorting from PBMC buffy coats extracted from whole blood. Following activation by anti-CD3/ anti-CD28 antibodies conjugated to Dynabeads (Invitrogen), cell surface expression of 4-1BB was detected on CD4 and CD8 T-cells. Activated T-cells and Vδ1 γδ T-cells were incubated with a range of concentrations of anti-Vδ1x4-1BB bispecific antibodies or controls (IgG control or anti-4-1BB) for 15 minutes. After washing, cells were incubated for a further 15 minutes with anti-human IgG secondary antibodies before washing and fixing. The amount of antibody bound to each cell type was determined by flow cytometry. The results are shown in **Figure 36, C, D.**

To determine the effect of anti-Vδ1xTIGIT bispecific antibodies on Vδ1 γδ T-cell activation, Vδ1 TCR downregulation was quantified in the presence of $TIGIT^+$ T-cells. Briefly, anti-Vδ1, anti-TIGIT and anti-Vδ1xTIGIT bispecific antibodies were serially diluted in PBS before adding to assay plates. $TIGIT^+$ CD8 T-cells were stained with CellTrace CFSE live cell dye and mixed 1:1 with skin derived Vδ1 γδ T-cells or diluted 1:1 with media. Cell suspensions were seeded into assay plates at 2.5×10^4 Vδ1 γδ T-cells per well in the presence or absence of 2.5×10^4 $TIGIT^+$ CD8 T-cells per well. Final assay antibody concentrations range from 200nM to 2pM. Cells were incubated for 4 hours at 37°C, 5% $CO_2$ before washing and stained for dead cells (eFlour 520, Invitrogen) and Vδ1 TCR (MiltenyiBiotec). Cells were washed and resuspended in Cell Fix (BD sciences). VD1 TCR expression level was determined by median fluorescence intensity (MFI), measured by flow cytometry using the MACS Quant Analyzer 16. The results are shown in **Figure 36, E, F.**

To assess the effect of anti-Vδ1xTIGIT bispecific antibodies on the activation of $TIGIT^+$ T-cells, $TIGIT^+$ NFAT Jurkat cells (Promega, JA2191) were incubated with anti-Vδ1xTIGIT bispecific antibodies or controls (anti-TIGIT monoclonal antibody or anti-RSVIgGxanti-TIGIT) for 5 hours at 37°C, 5% $CO_2$. Assay was performed in the presence or absence of recombinant Vδ1 protein pre-coated at 1μg/well in opaque, white 96-well plates. After 5 hours, Bio-Glo Luciferase reagent (Promega) was added to cells in a 1:1 ratio. After incubating for 5 minutes at room temperature the luminescence signal was detected on a BioTek Synergy plate reader. Raw luminescence signal was converted to fold relative luminsence units (RLU). The results are shown in **Figure 36, G.**

**Conclusions:** anti-Vδ1xTIGIT bispecific antibodies enhance the activation of Vδ1 γδ T-cells by crosslinking via TIGIT⁺ CD8 T-cells, as well as blocking the TIGIT/CD155 immune checkpoint inhibition in CD8 T-cells.

**EXAMPLE 37: Anti-vδ1 antibodies do not induce ADCC.**

An ADCC Reporter Bioassay (Promega) was used to assess the level of ADCC (antibody dependent cell-mediated cytotoxicity) induced by anti-vδ1 antibodies compared to control antibodies.

ADCC refers to the biological phenomenon whereby effector cells kill target cells that are tagged by antibodies. The effector cells bind to the antibodies through their Fcy receptors and subsequently kill the target cell. The ADCC reporter bioassay presented here uncovers potential ADCC mechanisms of action of antibodies that are tested within the assay, by detecting the early initiation of ADCC via activation of gene transcription through the NFAT (nuclear factor of activated T-cells) pathway. The reporter assay is an engineered system that utilizes effector cells (Jurkats) that express high affinity FcγRIIIa receptor linked to the NFAT pathway which is further engineered in order to, upon its activation, induce further activation of the firefly luciferase enzyme. Luciferase activity is quantified with a luminescence readout which can correlate to levels of ADCC taking place.

This assay was utilized to understand whether anti-vδ1 antibodies, or suitably the anti-vδ1 arm of multispecific antibodies, would drive an ADCC reaction. The target cells utilized were γδ cells which bind to the anti-vδ1 antibody through the vδ1 γδ TCR. If an ADCC mechanism of action exists, the anti-vδ1 antibody would bind the Fcy receptors on the assay effector cells and generate a luminescence signal; if no signal is generated, this would suggest that ADCC is not taking place.

The ADCC Reporter Bioassay Kit (Promega) was utilised for this assay. One bottle of Bio-Glo Luciferase Assay Buffer was thawed and transferred to the substrate bottle. The mixture was kept at room temperature for 4-6 hours. A dilution plate was prepared with antibody concentrations (at 3X concentration) ranging from 10nM to 0.01 nM (final concentration) for the following antibodies: anti-vδ1 antibody of this invention, same anti-vδ1 antibody but Fc disabled (L235A, G237A), Rituximab, RSV and OKT3. The target cells (γδ cells) were seeded into the 2 assay plates at 25μl per well. Then 25μl of the appropriate antibody solution from the antibody dilution plate were transferred to the appropriate well. The effector cells (engineered Jurkat cells) were thawed into warm assay buffer, resuspended into 4ml of the assay buffer, and 25μl of the effector cell solution was pipetted into each well. The plates were then incubated at 37°C for 4.5 hours. Following the incubation period, the plates were allowed to equilibrate to room temperature, after which 75μl Bio-Glo Luciferase Assay reagent was added to each well and the plates were incubated for 10 mins at room temperature. The plates were then read using a Biotek H4 plate reader which collected the luminescence signals (as relative light units RLU) from the plates. Fold induction was calculated using the following equation: Fold of Induction = RLU (induced-background) /RLU (no antibody control-background).

As a positive control, OKT3 (anti-CD3 antibody) was used. As an additional positive control, Raji cells were seeded instead of γδ cells in the control wells. Raji cells are a commonly accepted cell line used to demonstrate a strong ADCC reaction when utilised with the anti-CD20 antibody, Rituximab. As an internal control and in order to understand whether anti-vδ1 antibodies drive ADCC in the absence of vδ1 binding on γδ cells, anti-vδ1 antibodies of this invention and the same anti-vδ1 antibodies but Fc-disabled, L235A, G237A) were also added with the effector cells alone.

The results are shown in **Figure** 37.

**Conclusions:** A strong ADCC reaction was shown in the positive control using Rituximab with Raji cells and an even stronger ADCC reaction was demonstrated with OKT3 against γδ cells. Contrastingly, no ADCC reaction was detected in either conditions using the anti-vδ1 antibodies of this invention, the same anti-vδ1 antibodies but also Fc disabled L235A, G237A, or RSV negative control. This demonstrates that in this system, antibodies of this invention that bind to vδ1 (such as anti-vδ1 mAbs or anti-vδ1 multispecific antibodies) do not show evidence of an ADCC mechanism of action. Remarkably, even Fc-enabled anti-Vδ1 antibodies do not deplete γδ T cells, which provides the option of maintaining Fc function in the anti-Vδ1 antibodies presented herein, adding functionality, for example in high Fcy tumour environments. This further highlights the suitability of such anti-Vδ1 antibodies for inclusion in bispecific antibody formats as described herein.

Suitably, the functional properties of the antibodies when provided in a monospecific format are shared by the multispecific antibodies of the invention that additionally specifically bind to a second antigen.

## Claims

1. A multispecific antibody or fragment thereof that specifically binds:

a. a first target epitope, wherein the first target epitope is an epitope of the variable delta 1 (Vδ1) chain of a γδ T cell receptor (TCR); and
b. a second target epitope, wherein the second epitope is OX40.

**2.** The multispecific antibody or fragment thereof of any preceding claim, wherein the first target epitope is an epitope comprising one or more amino acid residues within amino acid regions:

(i) 3-20 of SEQ ID NO: 1; and/or
(ii) 37-77 of SEQ ID NO: 1.

**3.** The multispecific antibody or fragment thereof of any preceding claim, wherein the first target epitope is an activating epitope of a γδ T cell.

**4.** The multispecific antibody or antigen-binding fragment thereof of any preceding claim, wherein binding of the activating epitope: (i) downregulates the γδ TCR; (ii) activates degranulation of the γδ T cell; and/or (iii) promotes γδ T cell mediated killing.

**5.** The multispecific antibody or fragment thereof of any preceding claim, wherein the first target epitope is an epitope that upregulates expression of CD107a, CD25, CD69 and/or Ki67.

**6.** The multispecific antibody or fragment thereof of any preceding claim, wherein the multispecific antibody or fragment thereof binds the first epitope with a binding affinity (KD) as measured by surface plasmon resonance of less than $1.5 \times 10^{-7}$ M, less than $1.3 \times 10^{-7}$ M, less than $1.0 \times 10^{-7}$ M, or less than $5.0 \times 10^{-8}$ M.

**7.** The multispecific antibody or fragment thereof of any preceding claim, wherein the antibody is a bispecific antibody.

**8.** The multispecific antibody or fragment thereof of any preceding claim, wherein the multispecific antibody or fragment thereof is a human antibody or antigen-binding fragment thereof.

**9.** The multispecific antibody or fragment thereof of any preceding claim, wherein the multispecific antibody or antigen-binding fragment thereof is an IgG multispecific antibody, optionally wherein the multispecific antibody or antigen-binding fragment thereof is an IgG1 multispecific antibody.

**10.** A pharmaceutical composition comprising the multispecific antibody or fragment thereof as defined in any one of claims 1 to 9 and a pharmaceutically acceptable diluent or carrier.

**11.** A multispecific antibody or fragment thereof of any one of claims 1 to 9, or the pharmaceutical composition of claim 10 for use in a method of treating a disease or disorder in a subject.

**12.** The multispecific antibody or fragment thereof for use according to claim 11, wherein the disease or disorder is cancer, an infectious disease or an inflammatory disease.

## Patentansprüche

**1.** Multispezifischer Antikörper oder Fragment davon, der bzw. das Folgendes spezifisch bindet:

a. ein erstes Zielepitop, wobei es sich bei dem ersten Zielepitop um ein Epitop der variablen Delta 1(Vδ1)-Kette eines γδ-T-Zell-Rezeptors (TCR) handelt; und
b. ein zweites Zielepitop, wobei es sich bei dem zweiten Epitop um OX40.

**2.** Multispezifischer Antikörper oder Fragment davon nach einem vorhergehenden Anspruch, wobei es sich bei dem ersten Zielepitop um ein Epitop handelt, das einen oder mehrere innerhalb der folgenden Aminosäureregionen liegende Aminosäurereste umfasst:

(i) 3-20 von SEQ ID NO: 1; und/oder
(ii) 37-77 von SEQ ID NO: 1.

3. Multispezifischer Antikörper oder Fragment davon nach einem vorhergehenden Anspruch, wobei es sich bei dem ersten Zielepitop um ein aktivierendes Epitop einer $\gamma\delta$-T-Zelle handelt.

4. Multispezifischer Antikörper oder antigenbindendes Fragment davon nach einem vorhergehenden Anspruch, wobei durch Bindung des aktivierenden Epitops (i) der $\gamma\delta$-TCR herunterreguliert, (ii) Degranulation der $\gamma\delta$-T-Zelle aktiviert und/oder (iii) durch $\gamma\delta$-T-Zellen vermitteltes Abtöten gefördert wird.

5. Multispezifischer Antikörper oder Fragment davon nach einem vorhergehenden Anspruch, wobei es sich bei dem ersten Zielepitop um ein Epitop handelt, durch das die Expression von CD107a, CD25, CD69 und/oder Ki67 heraufreguliert wird.

6. Multispezifischer Antikörper oder Fragment davon nach einem vorhergehenden Anspruch, wobei der multispezifische Antikörper bzw. das Fragment davon das erste Epitop mit einer Bindungsaffinität (KD) von kleiner $1,5 \times 10^{-7}$ M, kleiner $1,3 \times 10^{-7}$ M, kleiner $1,0 \times 10^{-7}$ M oder kleiner $5,0 \times 10^{-8}$ M, wie mit Oberflächenplasmonenresonanz gemessen, bindet.

7. Multispezifischer Antikörper oder Fragment davon nach einem vorhergehenden Anspruch, wobei es sich bei dem Antikörper um einen bispezifischen Antikörper handelt.

8. Multispezifischer Antikörper oder Fragment davon nach einem vorhergehenden Anspruch, wobei es sich bei dem multispezifischen Antikörper bzw. dem Fragment davon um einen menschlichen Antikörper bzw. ein antigenbindendes Fragment davon handelt.

9. Multispezifischer Antikörper oder Fragment davon nach einem vorhergehenden Anspruch, wobei es sich bei dem multispezifischen Antikörper bzw. dem antigenbindenden Fragment davon um einen multispezifischen IgG-Antikörper handelt, gegebenenfalls wobei es sich bei dem multispezifischen Antikörper bzw. dem antigenbindenden Fragment davon um einen multispezifischen IgG1-Antikörper handelt.

10. Pharmazeutische Zusammensetzung, umfassend den multispezifischen Antikörper bzw. das Fragment davon gemäß einem der Ansprüche 1 bis 9 und ein pharmazeutisch unbedenkliches Verdünnungs- oder Trägermittel.

11. Multispezifischer Antikörper oder Fragment davon nach einem der Ansprüche 1 bis 9 oder pharmazeutische Zusammensetzung nach Anspruch 10 zur Verwendung bei einem Verfahren zur Behandlung einer Krankheit oder Störung bei einem Individuum.

12. Multispezifischer Antikörper oder Fragment davon zur Verwendung gemäß Anspruch 11, wobei es sich bei der Krankheit bzw. Störung um Krebs, eine Infektionskrankheit oder eine Entzündungskrankheit handelt.

## Revendications

1. Anticorps multispécifique ou fragment correspondant qui se lie spécifiquement à :

   a. un premier épitope cible, le premier épitope cible étant un épitope de la chaîne delta 1 variable (V$\delta$1) d'un récepteur de cellules T (TCR) $\gamma\delta$ ; et
   b. un deuxième épitope cible, le deuxième épitope étant OX40.

2. Anticorps multispécifique ou fragment correspondant selon une quelconque revendication précédente, le premier épitope cible étant un épitope comprenant un ou plusieurs résidus d'acides aminés à l'intérieur des régions d'acides aminés :

   (i) 3 à 20 de la SEQ ID NO: 1 ; et/ou
   (ii) 37 à 77 de la SEQ ID NO: 1.

3. Anticorps multispécifique ou fragment correspondant selon une quelconque revendication précédente, le premier épitope cible étant un épitope d'activation d'une cellule T $\gamma\delta$.

4. Anticorps multispécifique ou fragment de liaison à un antigène correspondant selon une quelconque revendication précédente, la liaison de l'épitope d'activation : (i) régulant à la baisse le TCR $\gamma\delta$ ; (ii) activant la dégranulation du TCR $\gamma\delta$ ; et/ou (iii) promouvant la destruction médiée par les cellules T $\gamma\delta$.

**5.** Anticorps multispécifique ou fragment correspondant selon une quelconque revendication précédente, le premier épitope cible étant un épitope qui régule à la hausse l'expression de CD107a, CD25, CD69 et/ou Ki67.

**6.** Anticorps multispécifique ou fragment correspondant selon une quelconque revendication précédente, l'anticorps multispécifique ou le fragment correspondant se liant au premier épitope avec une affinité de liaison (KD) telle que mesurée par résonance de plasmon de surface de moins de $1{,}5 \times 10^{-7}$ M, de moins de $1{,}3 \times 10^{-7}$ M, de moins de $1{,}0 \times 10^{-7}$ M, de moins de $5{,}0 \times 10^{-8}$ M.

**7.** Anticorps multispécifique ou fragment correspondant selon une quelconque revendication précédente, l'anticorps étant un anticorps bispécifique.

**8.** Anticorps multispécifique ou fragment correspondant selon une quelconque revendication précédente, l'anticorps multispécifique ou le fragment correspondant étant un anticorps humain ou un fragment de liaison à un antigène correspondant.

**9.** Anticorps multispécifique ou fragment correspondant selon une quelconque revendication précédente, l'anticorps multispécifique ou le fragment de liaison à un antigène correspondant étant un anticorps multispécifique IgG, éventuellement l'anticorps multispécifique ou le fragment de liaison à un antigène correspondant étant un anticorps multispécifique IgG1.

**10.** Composition pharmaceutique comprenant l'anticorps multispécifique ou le fragment correspondant tel que défini dans l'une quelconque des revendications 1 à 9 et un diluant ou support pharmaceutiquement acceptable.

**11.** Anticorps multispécifique ou fragment correspondant selon l'une quelconque des revendications 1 à 9, ou composition pharmaceutique selon la revendication 10 pour une utilisation dans un procédé de traitement d'une maladie ou d'un trouble chez un sujet.

**12.** Anticorps multispécifique ou fragment correspondant pour une utilisation selon la revendication 11, la maladie ou le trouble étant un cancer, une maladie infectieuse ou une maladie inflammatoire.

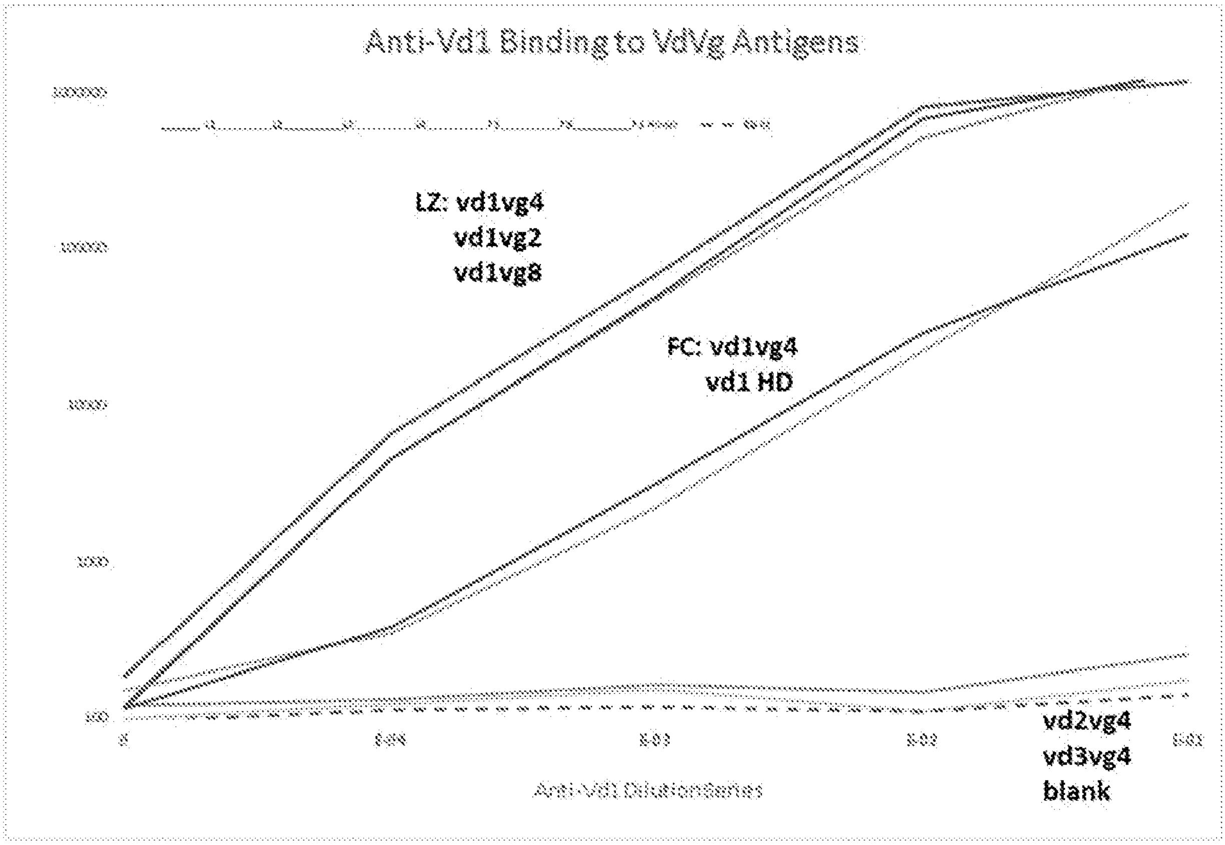
Anti-Vd1 Binding to VdVg Antigens
LZ: vd1vg4
vd1vg2
vd1vg8
FC: vd1vg4
vd1 HD
vd2vg4
vd3vg4
blank
Anti-Vd1 Dilution Series

FIGURE 1

A

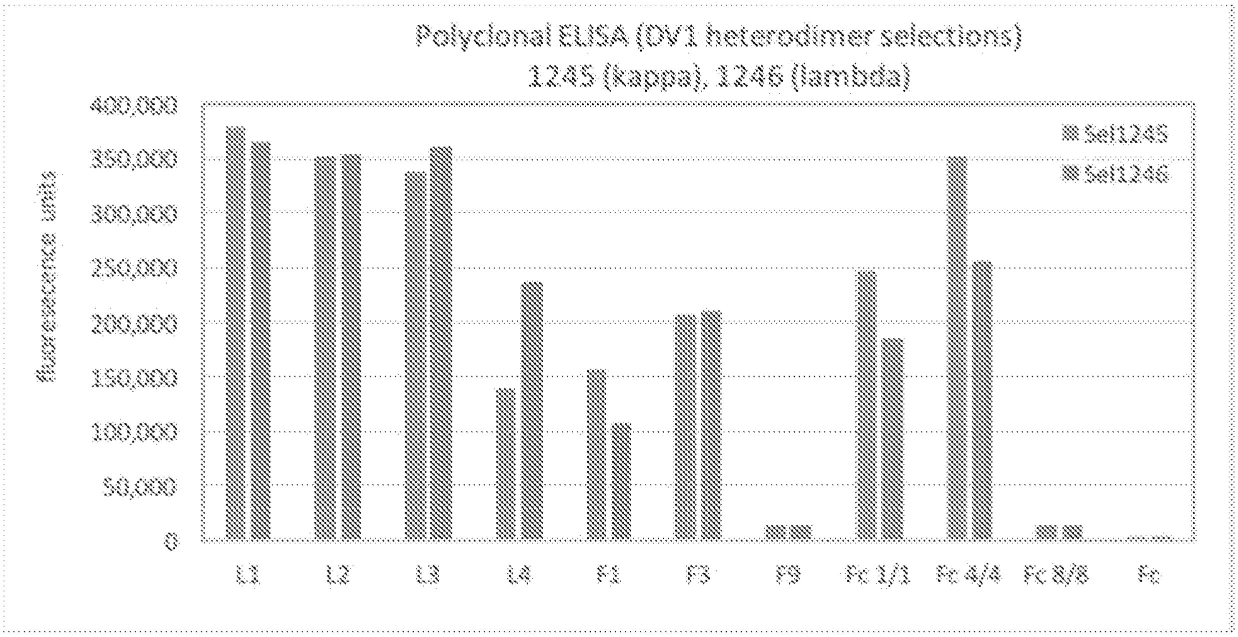
Polyclonal ELISA (DV1 heterodimer selections)
1245 (kappa), 1246 (lambda)
fluorescence units
400,000
350,000
300,000
250,000
200,000
150,000
100,000
50,000
0
Sel1245
Sel1246
L1
L2
L3
L4
F1
F3
F9
Fc 1/1
Fc 4/4
Fc 8/8
Fc

B

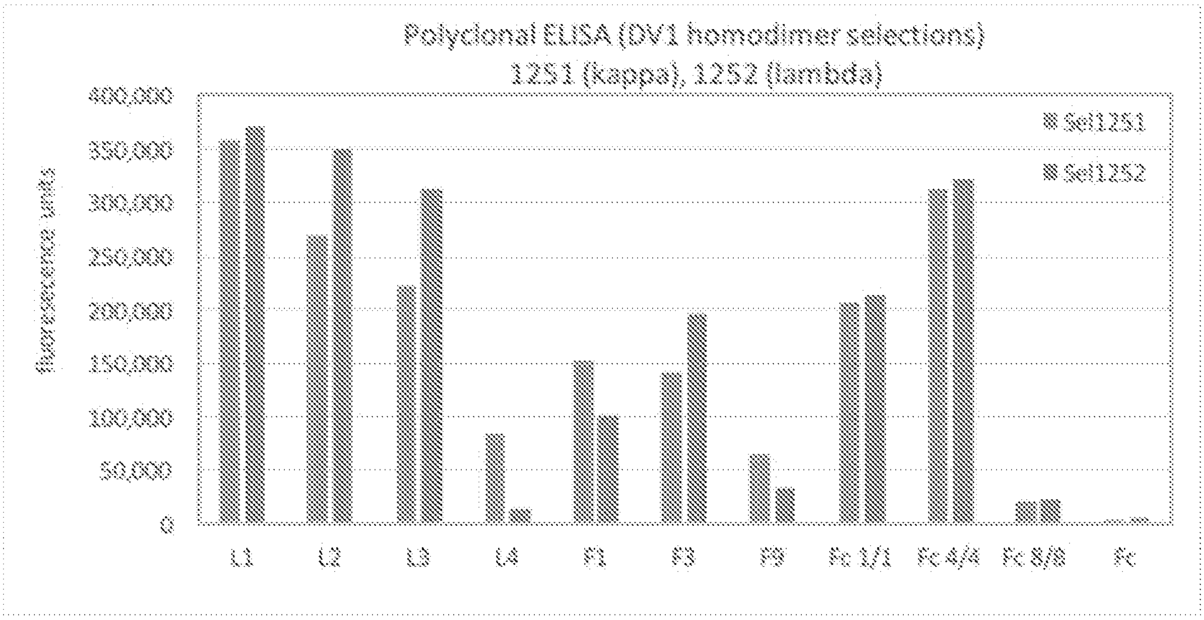
Polyclonal ELISA (DV1 homodimer selections)
1251 (kappa), 1252 (lambda)
fluorescence units
400,000
350,000
300,000
250,000
200,000
150,000
100,000
50,000
0
Sel1251
Sel1252
L1
L2
L3
L4
F1
F3
F9
Fc 1/1
Fc 4/4
Fc 8/8
Fc

FIGURE 2

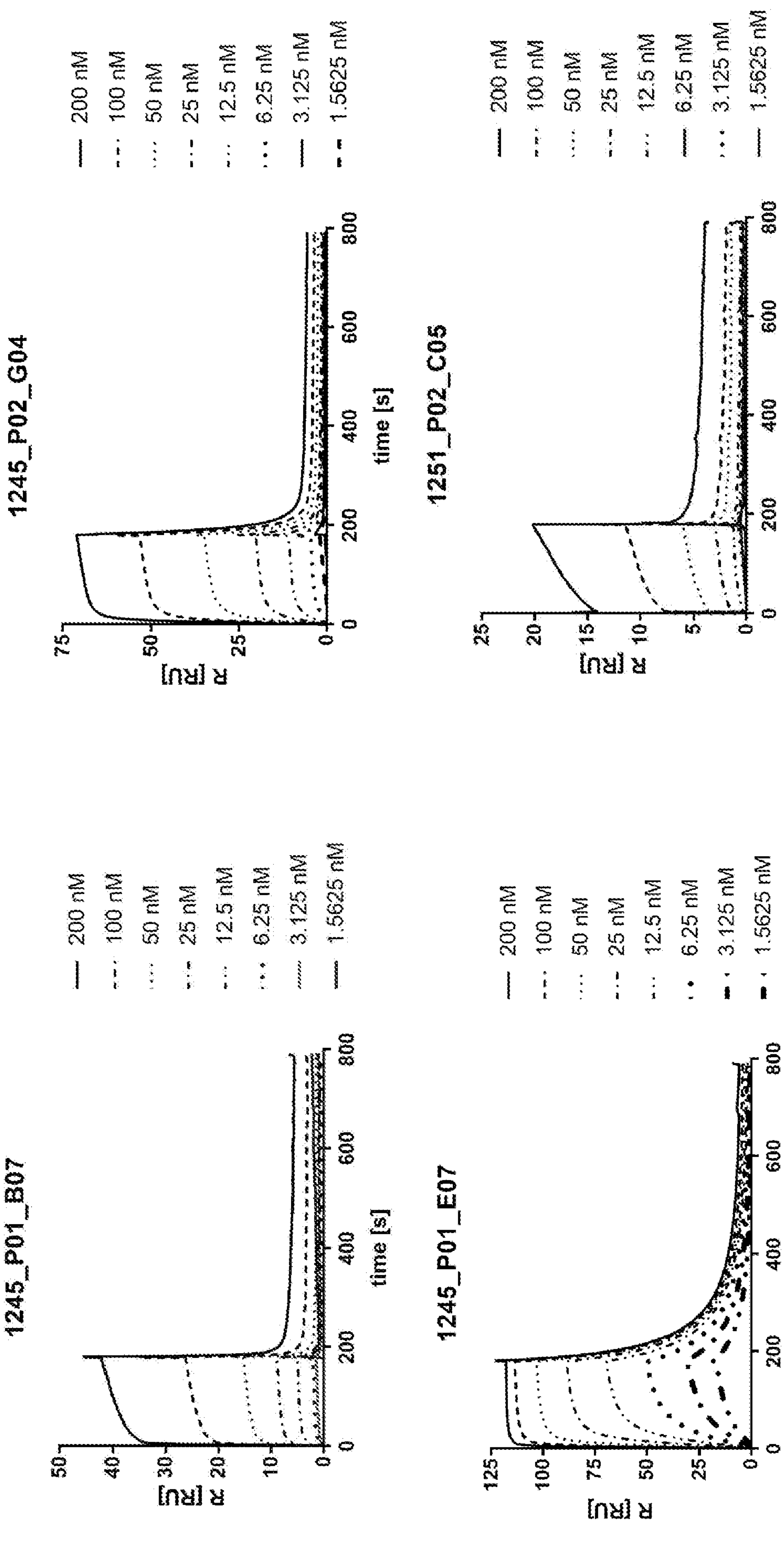

1245_P01_B07
R [RU]
time [s]
1245_P01_E07
1245_P02_G04
1251_P02_C05
200 nM
100 nM
50 nM
25 nM
12.5 nM
6.25 nM
3.125 nM
1.5625 nM


FIGURE 3

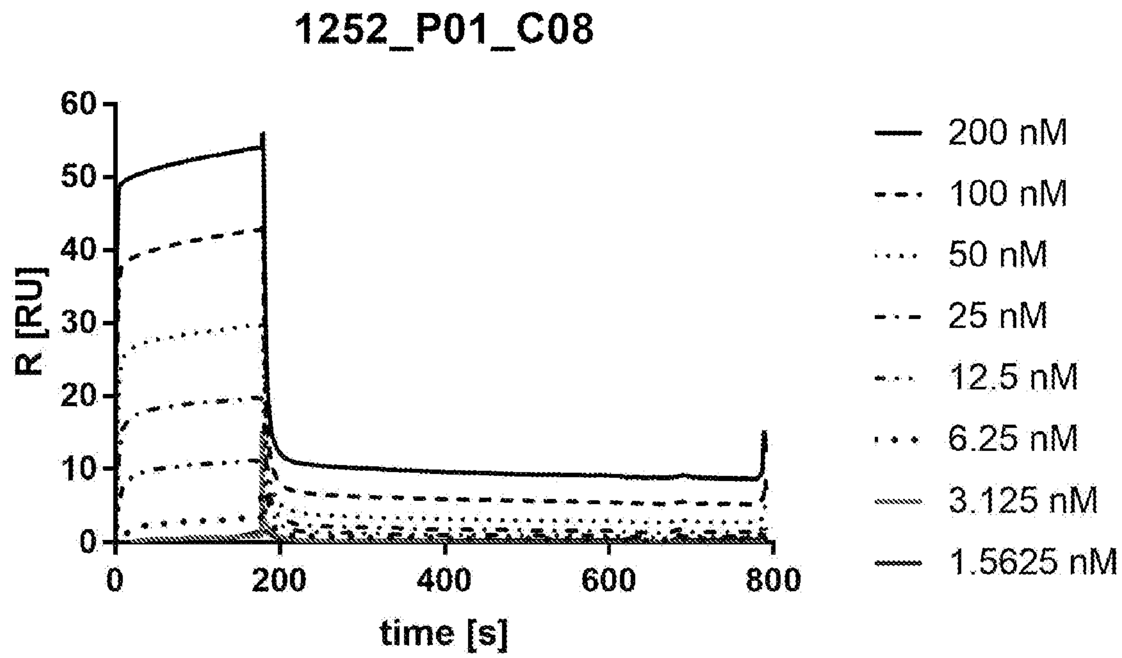


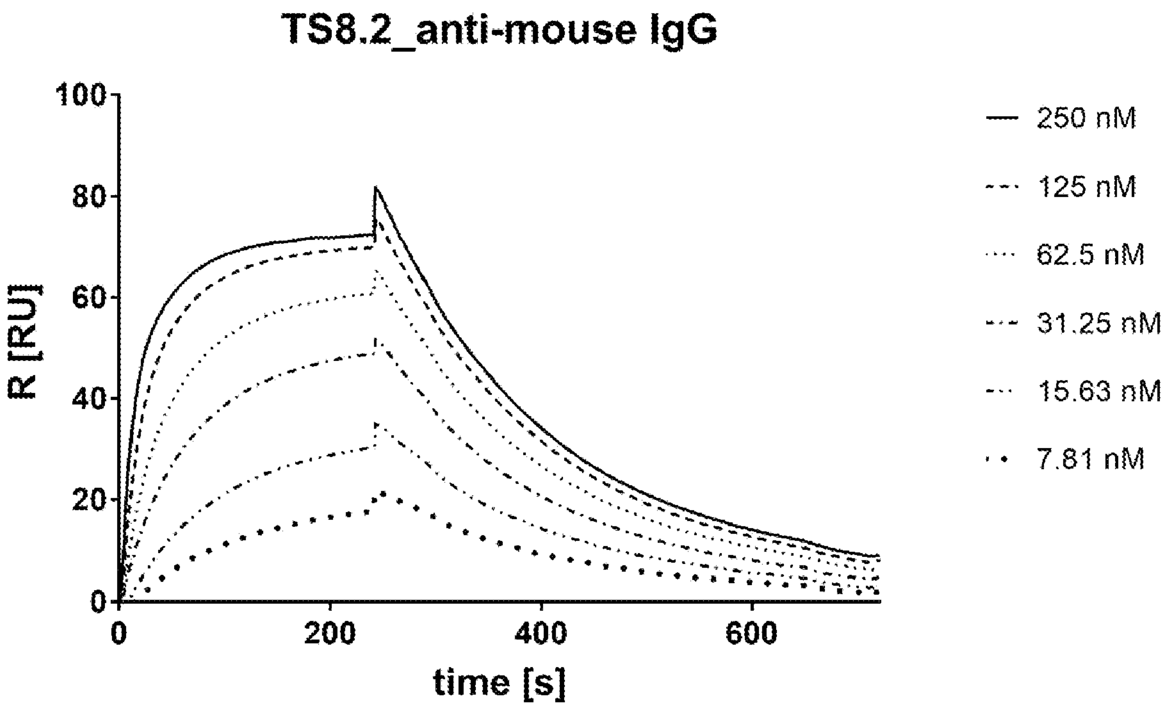


FIGURE 3 (contd.)

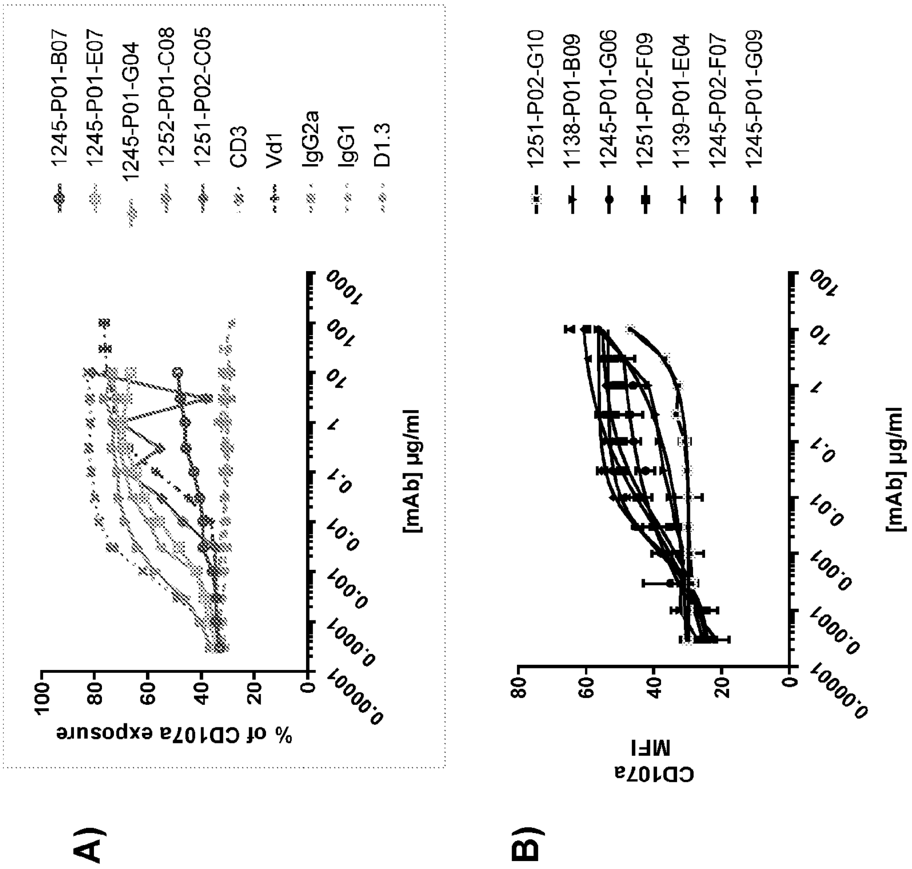


FIGURE 5

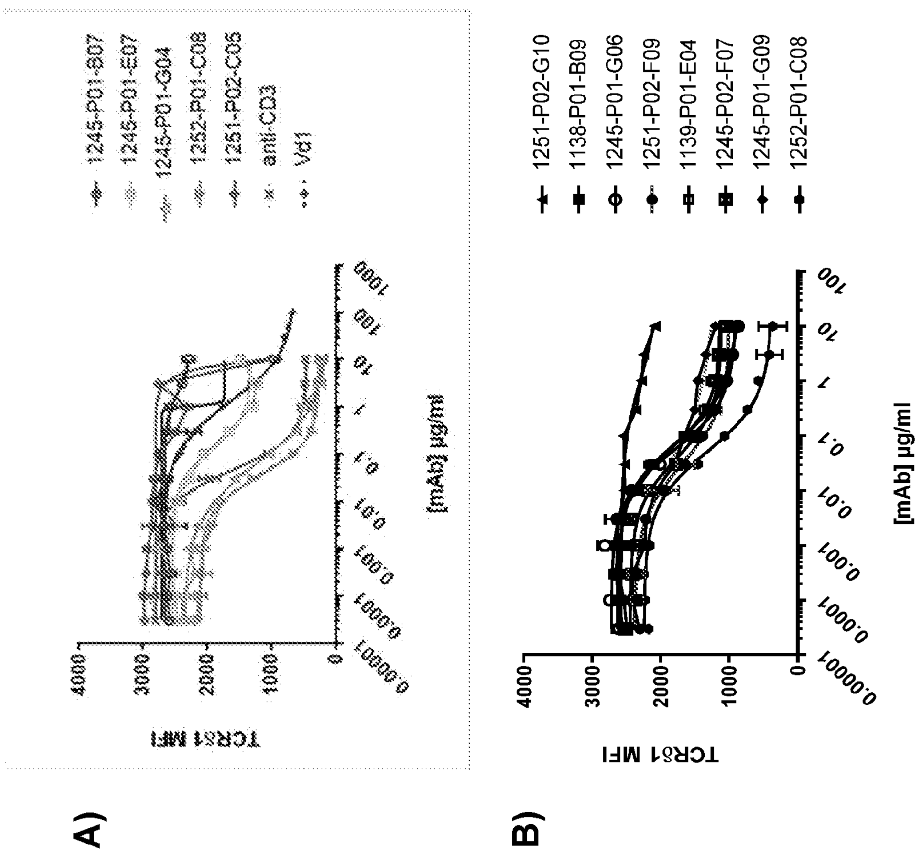


FIGURE 4

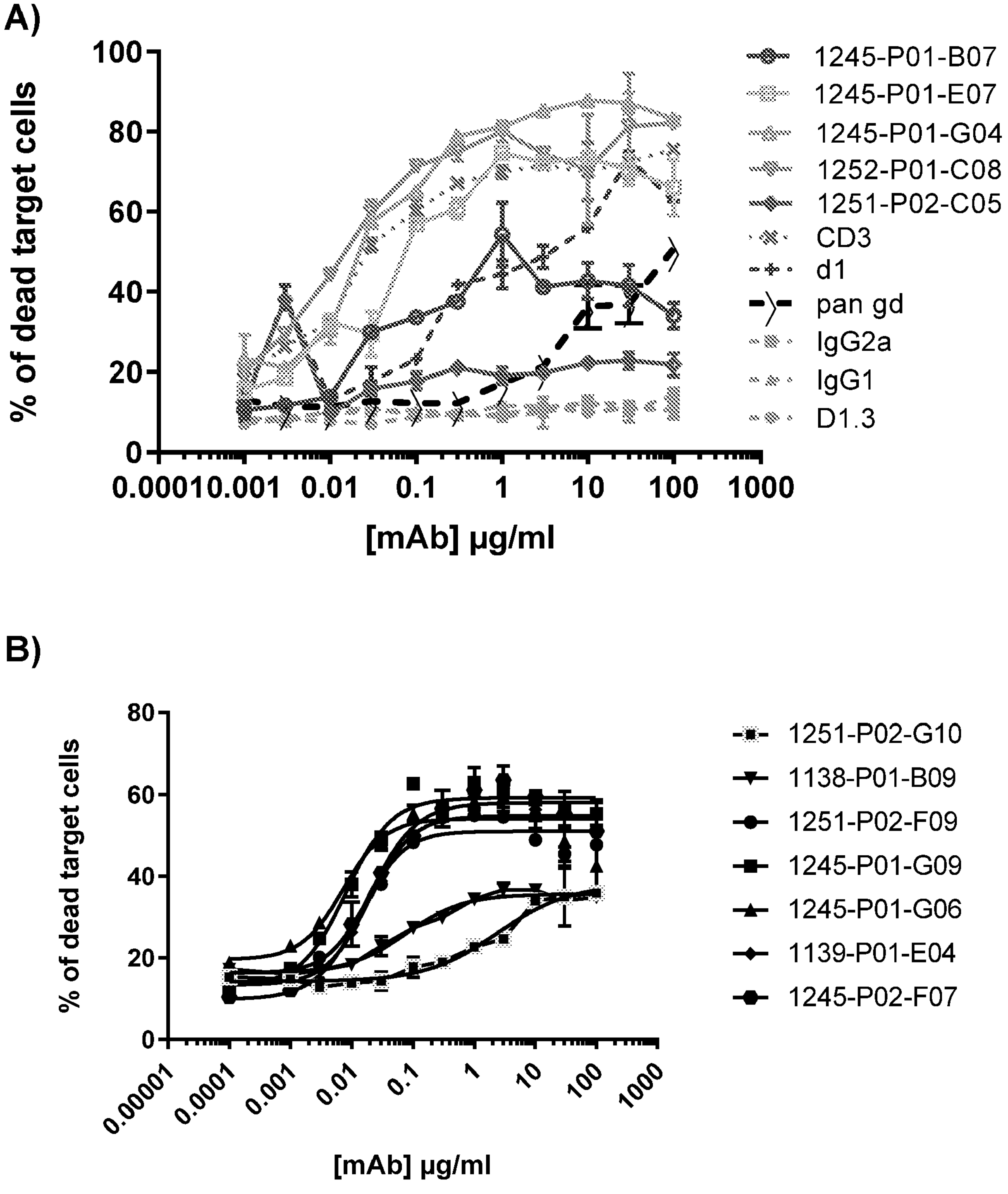
A)
100
80
60
40
20
0
% of dead target cells
0.00010.001
0.01
0.1
1
10
100
1000
[mAb] µg/ml
1245-P01-B07
1245-P01-E07
1245-P01-G04
1252-P01-C08
1251-P02-C05
CD3
d1
pan gd
IgG2a
IgG1
D1.3
B)
80
60
40
20
0
% of dead target cells
0.00001
0.0001
0.001
0.01
0.1
1
10
100
1000
[mAb] µg/ml
1251-P02-G10
1138-P01-B09
1251-P02-F09
1245-P01-G09
1245-P01-G06
1139-P01-E04
1245-P02-F07

FIGURE 6

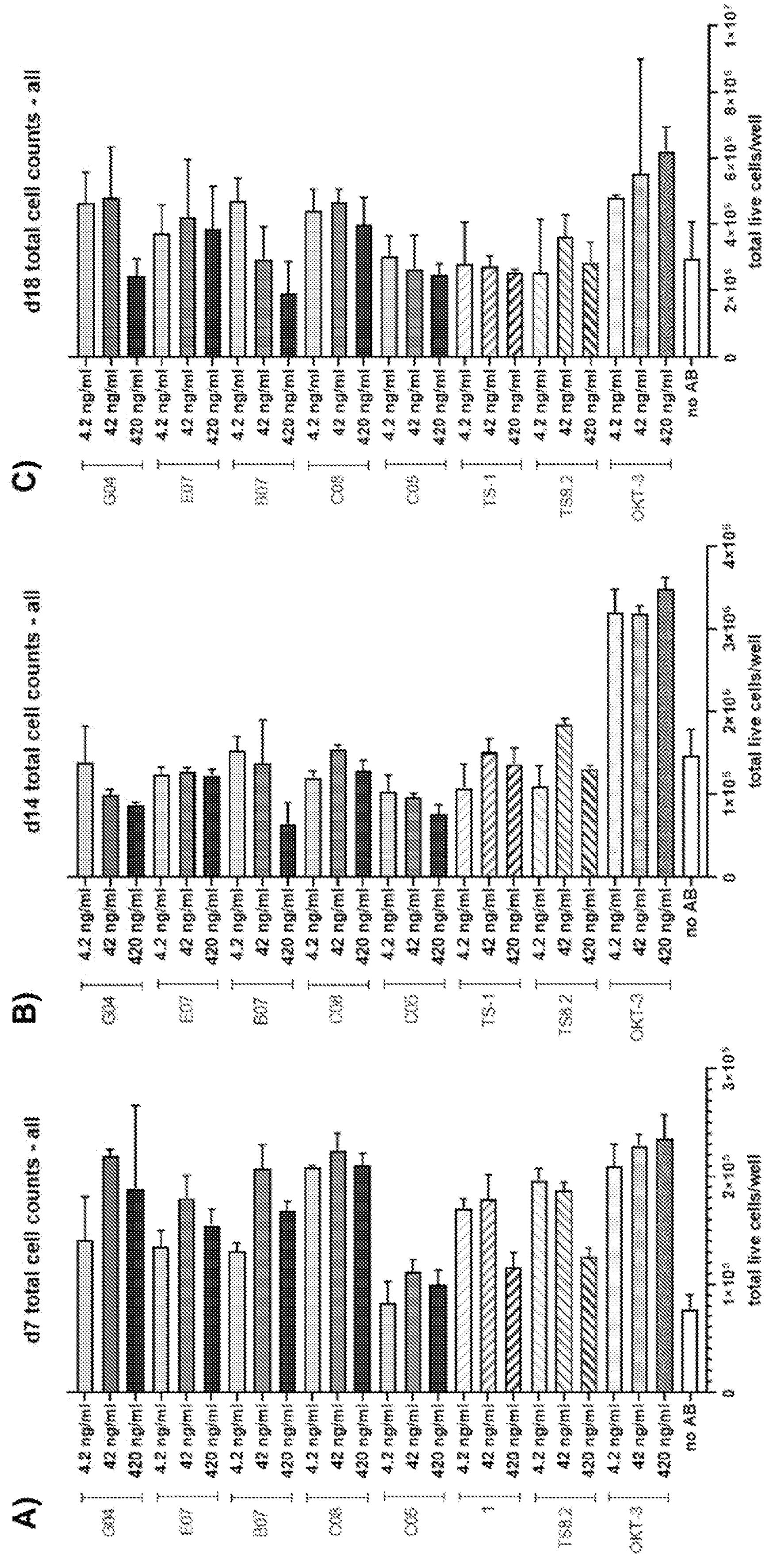
A)
d7 total cell counts - all
total live cells/well
B)
d14 total cell counts - all
total live cells/well
C)
d18 total cell counts - all
total live cells/well
4.2 ng/ml
42 ng/ml
420 ng/ml
no AB
G04
E07
B07
C08
C05
TS-1
TS8.2
OKT-3

FIGURE 7

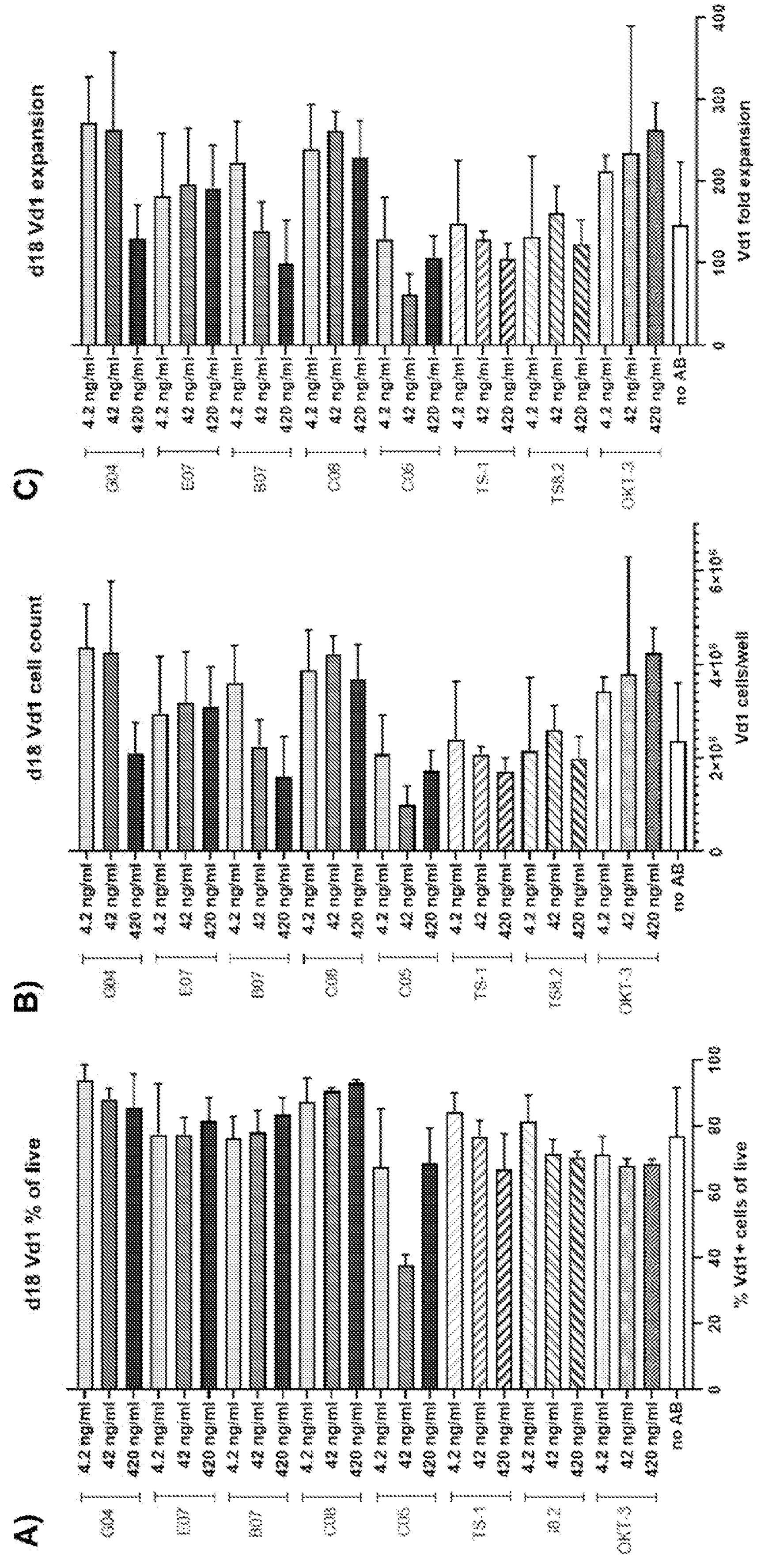
A)
d18 Vd1 % of live
% Vd1+ cells of live
0
20
40
60
80
100
B)
d18 Vd1 cell count
Vd1 cells/well
0
2×10⁶
4×10⁶
6×10⁶
C)
d18 Vd1 expansion
Vd1 fold expansion
0
100
200
300
400
G04
E07
B07
C08
C05
TS-1
TS8.2
OKT-3
4.2 ng/ml
42 ng/ml
420 ng/ml
no AB

FIGURE 8

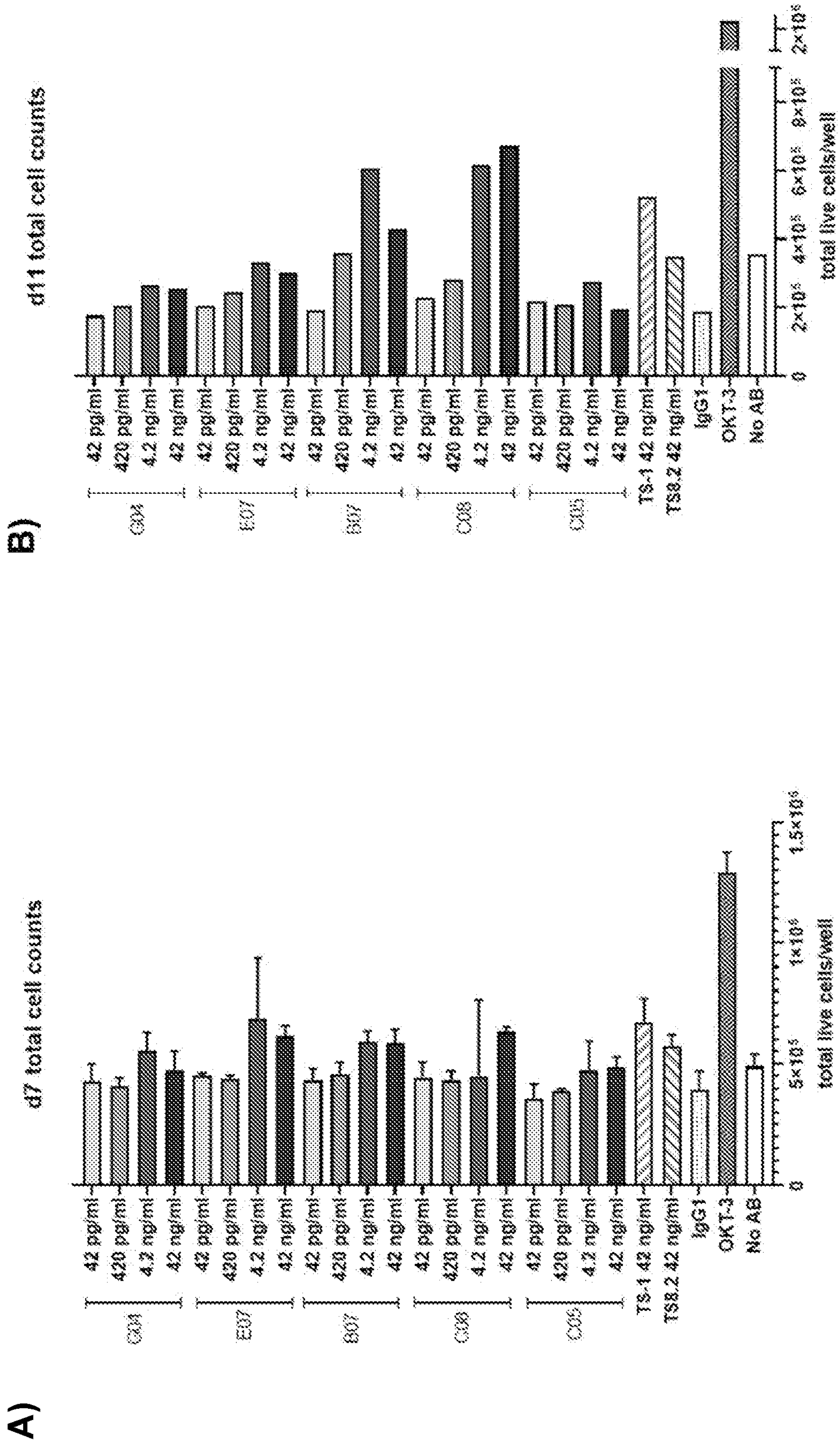
A)
d7 total cell counts
G04
E07
B07
C08
C05
42 pg/ml
420 pg/ml
4.2 ng/ml
42 ng/ml
TS-1 42 ng/ml
TS8.2 42 ng/ml
IgG1
OKT-3
No AB
0
5×10⁵
1×10⁶
1.5×10⁶
total live cells/well
B)
d11 total cell counts
0
2×10⁵
4×10⁵
6×10⁵
8×10⁵
2×10⁶
total live cells/well

FIGURE 9

D)

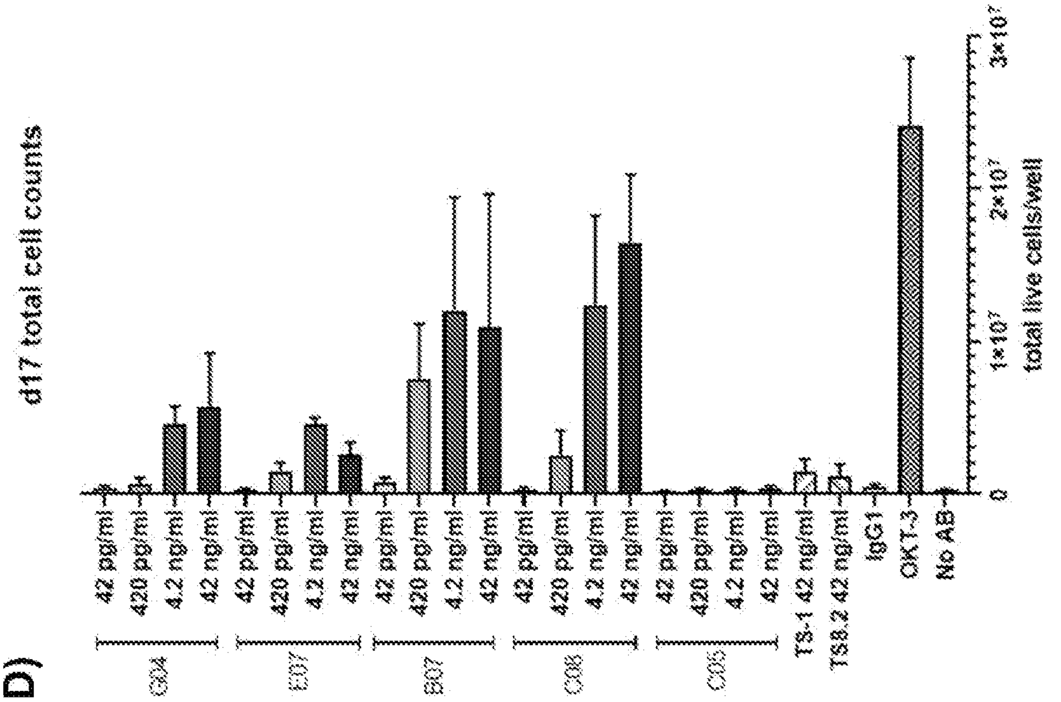


C)

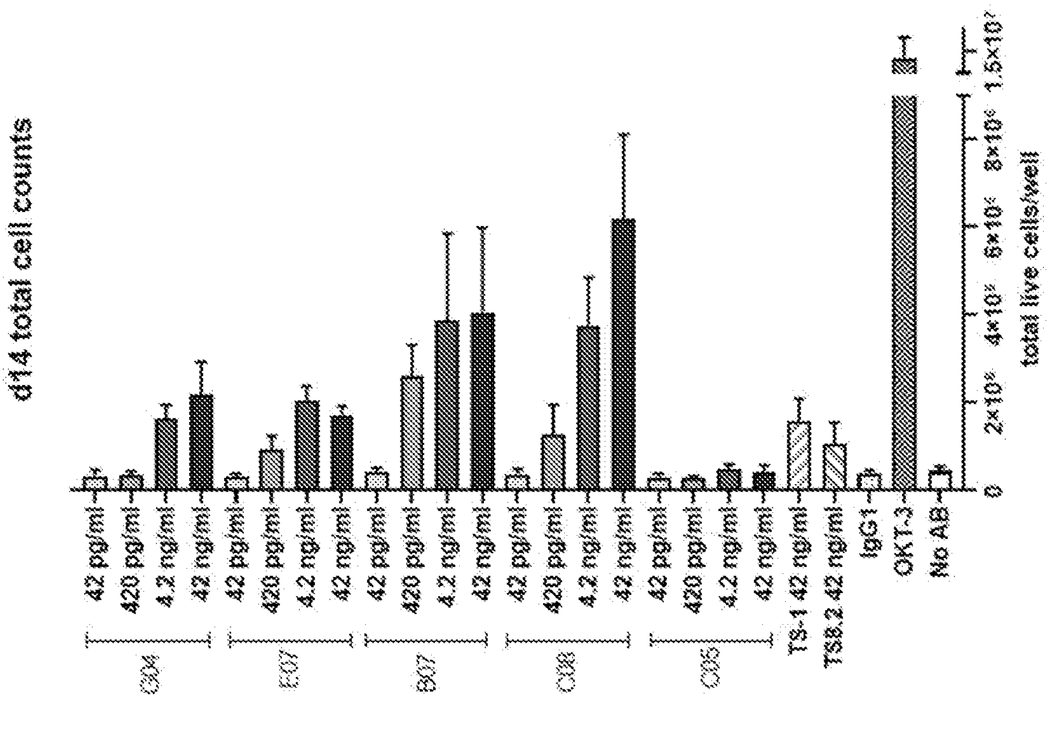


FIGURE 9 (contd.)

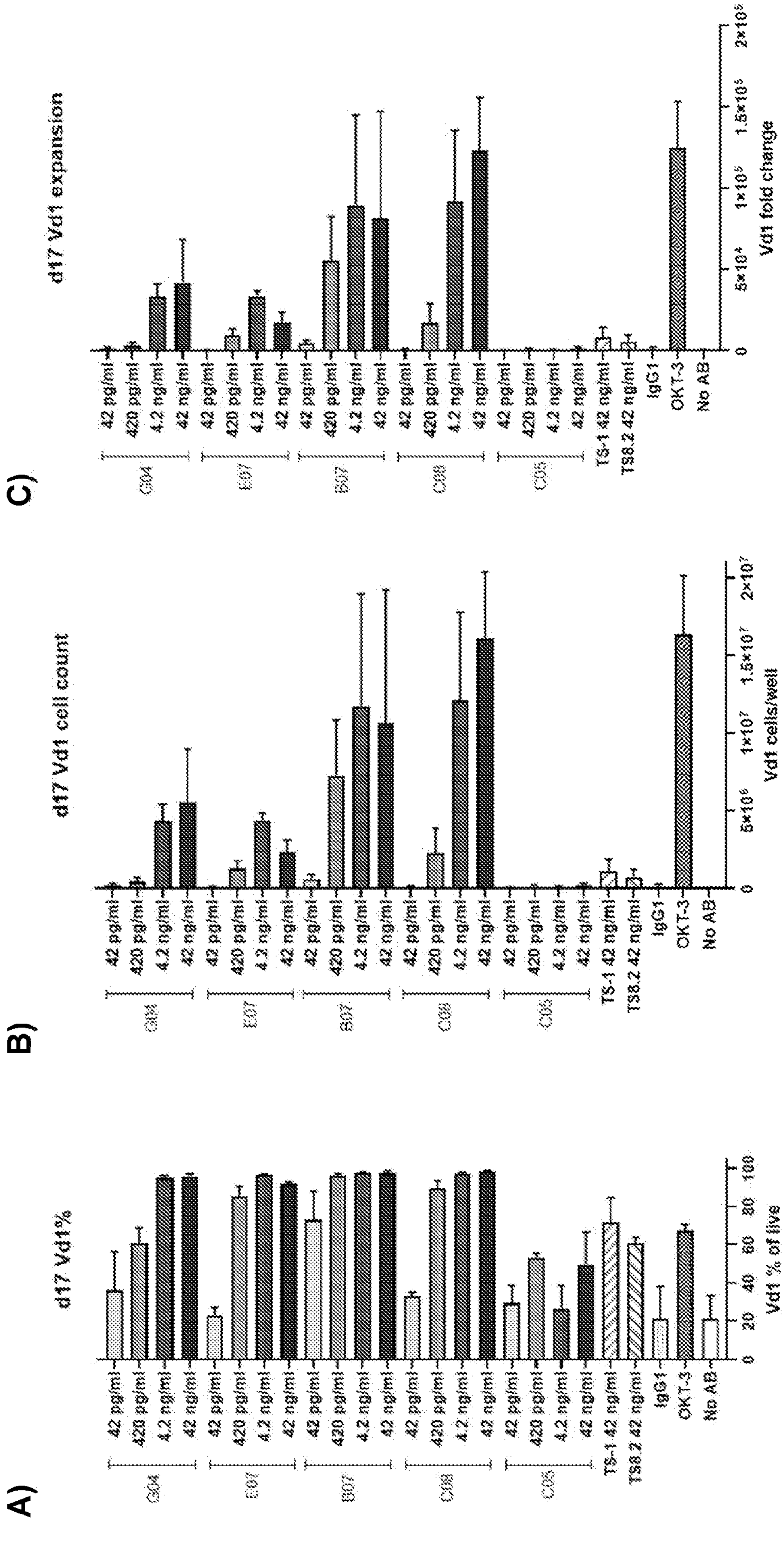
A)
d17 Vd1%
G04
E07
B07
C08
C05
42 pg/ml
420 pg/ml
4.2 ng/ml
42 ng/ml
TS-1 42 ng/ml
TS8.2 42 ng/ml
IgG1
OKT-3
No AB
0
20
40
60
80
100
Vd1 % of live
B)
d17 Vd1 cell count
0
5×10⁶
1×10⁷
1.5×10⁷
2×10⁷
Vd1 cells/well
C)
d17 Vd1 expansion
0
5×10⁴
1×10⁵
1.5×10⁵
2×10⁵
Vd1 fold change

FIGURE 10

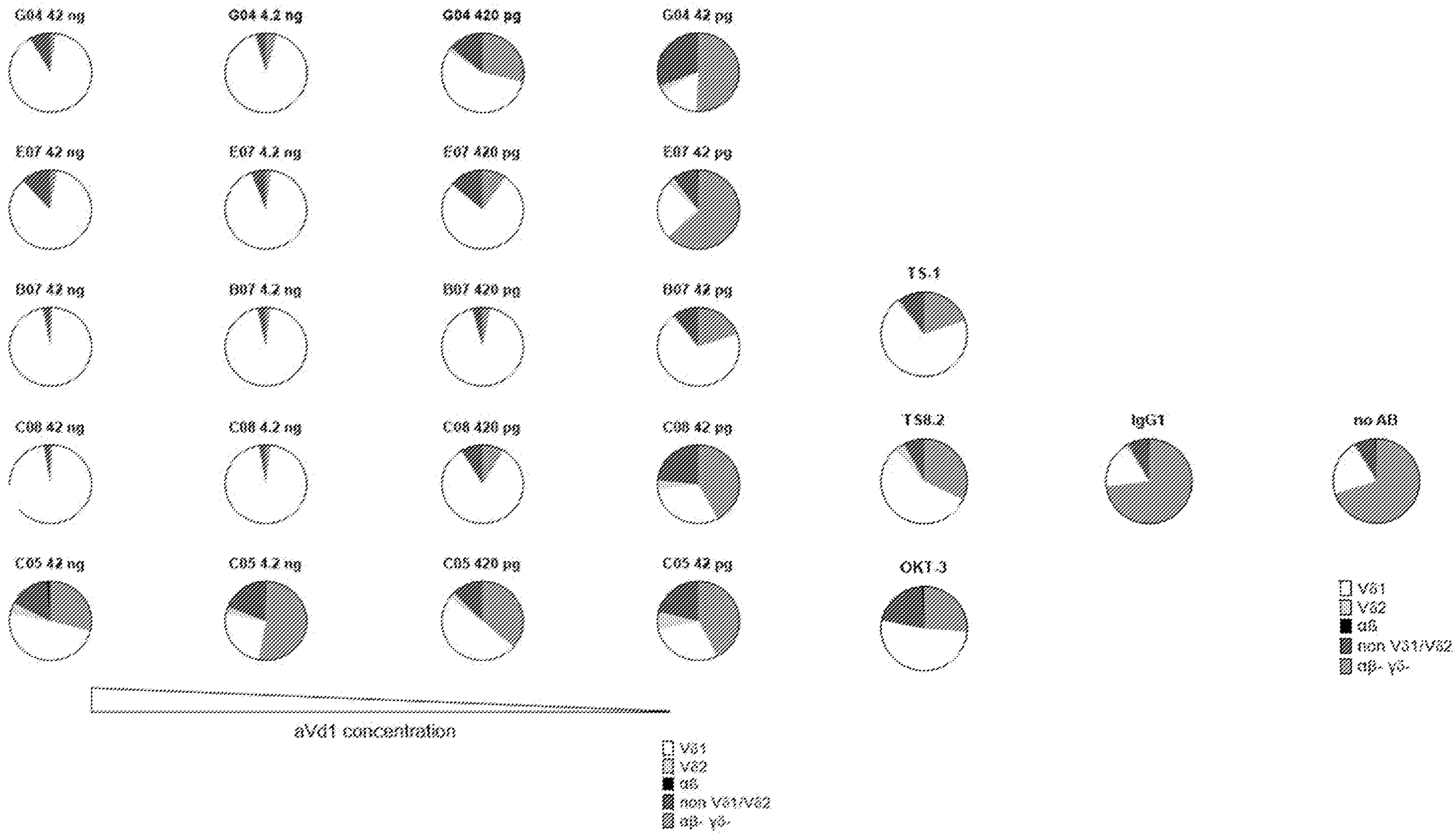
G04 42 ng
G04 4.2 ng
G04 420 pg
G04 42 pg
E07 42 ng
E07 4.2 ng
E07 420 pg
E07 42 pg
B07 42 ng
B07 4.2 ng
B07 420 pg
B07 42 pg
C08 42 ng
C08 4.2 ng
C08 420 pg
C08 42 pg
C05 42 ng
C05 4.2 ng
C05 420 pg
C05 42 pg
aVd1 concentration
Vδ1
Vδ2
αβ
non Vδ1/Vδ2
αβ- γδ-
TS.1
TS8.2
OKT.3
IgG1
no AB
Vδ1
Vδ2
αβ
non Vδ1/Vδ2
αβ- γδ-

FIGURE 11

A)

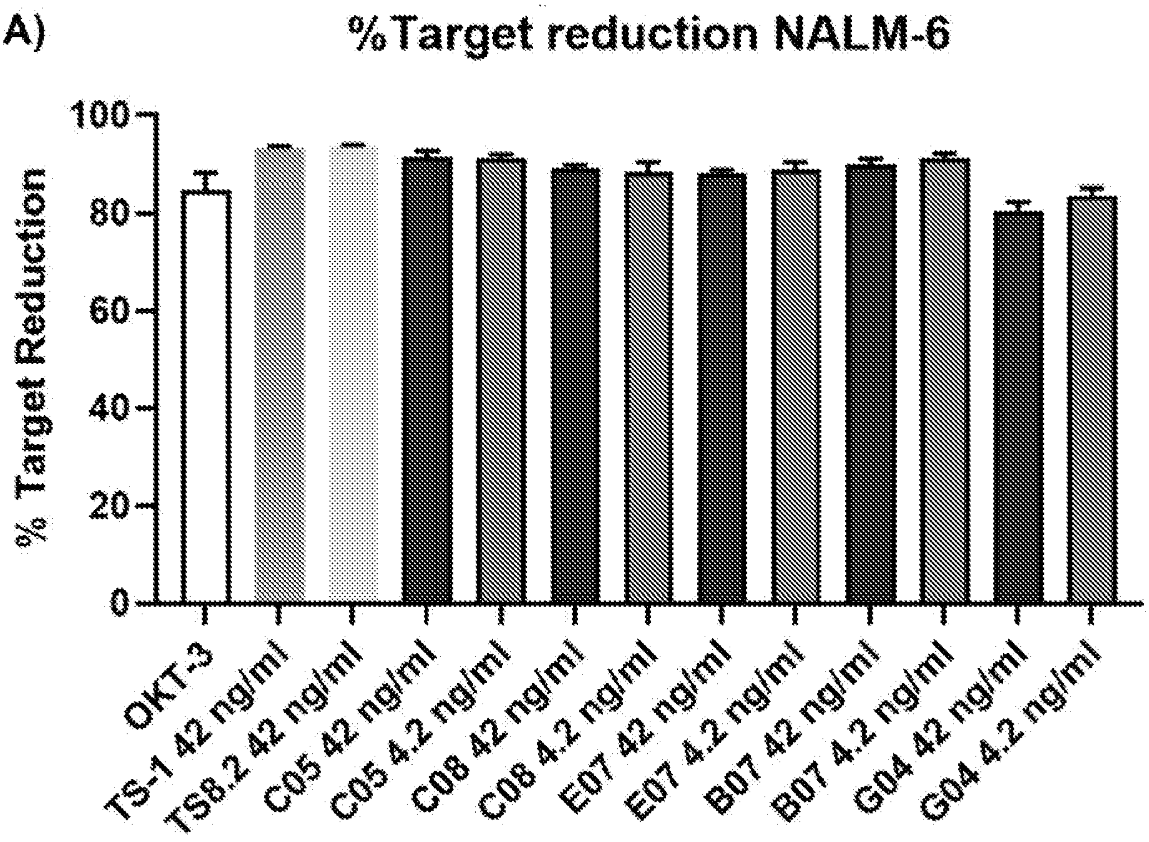
%Target reduction NALM-6
% Target Reduction
100
80
60
40
20
0
OKT-3
TS-1 42 ng/ml
TS8.2 42 ng/ml
C05 42 ng/ml
C05 4.2 ng/ml
C08 42 ng/ml
C08 4.2 ng/ml
E07 42 ng/ml
E07 4.2 ng/ml
B07 42 ng/ml
B07 4.2 ng/ml
G04 42 ng/ml
G04 4.2 ng/ml

B)

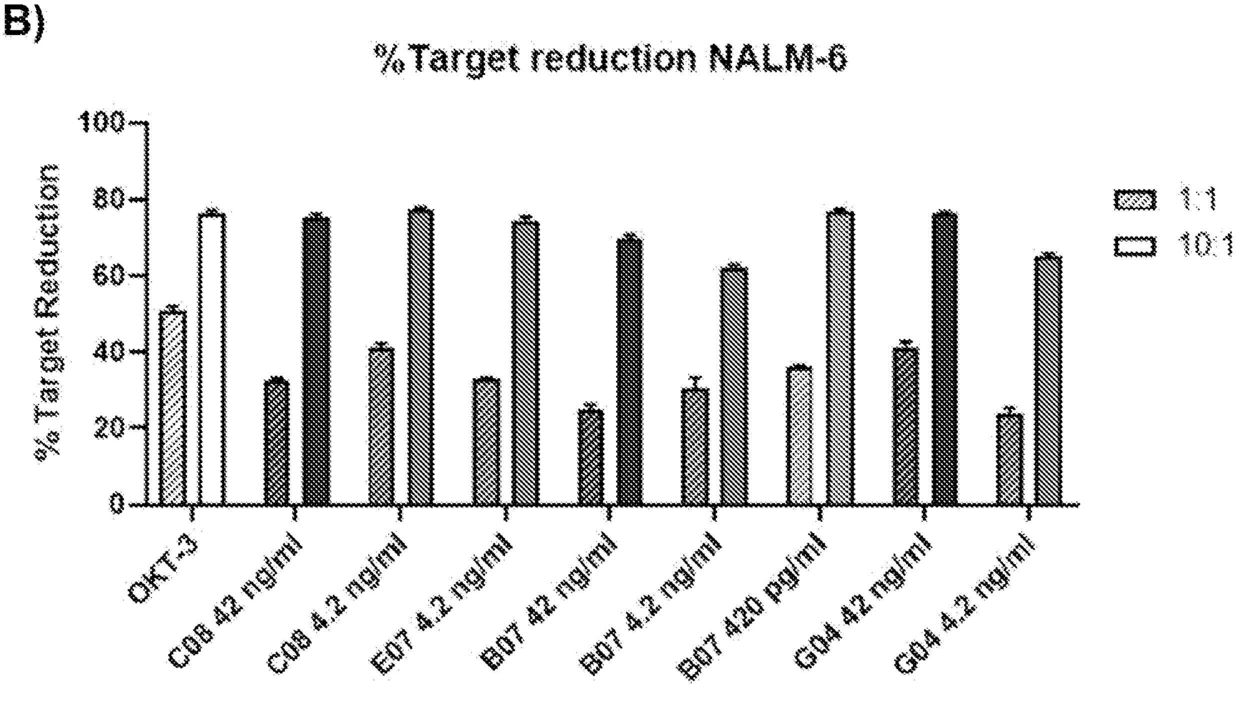
%Target reduction NALM-6
% Target Reduction
100
80
60
40
20
0
1:1
10:1
OKT-3
C08 42 ng/ml
C08 4.2 ng/ml
E07 4.2 ng/ml
B07 42 ng/ml
B07 4.2 ng/ml
B07 420 pg/ml
G04 42 ng/ml
G04 4.2 ng/ml

FIGURE 12

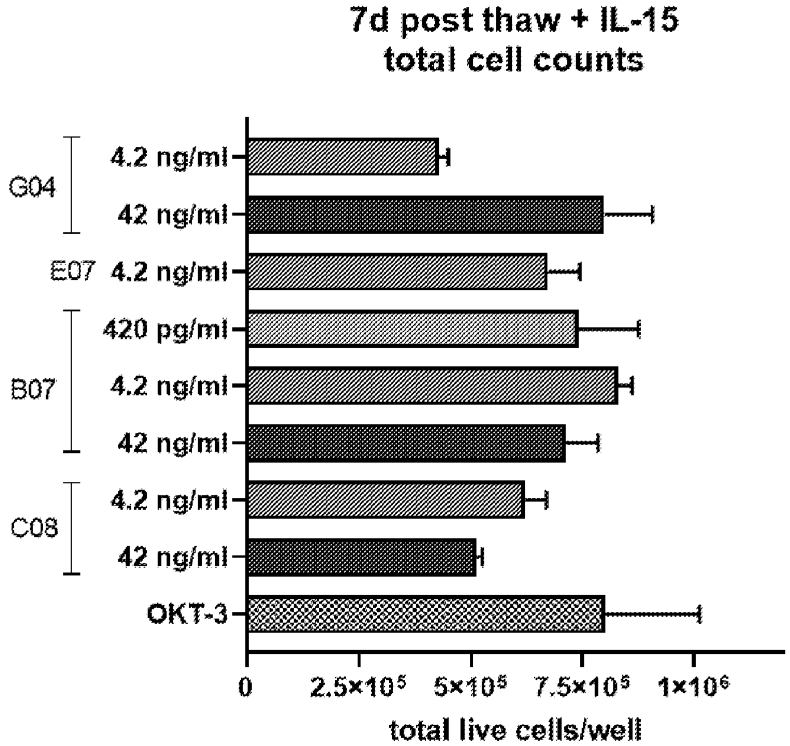


FIGURE 13

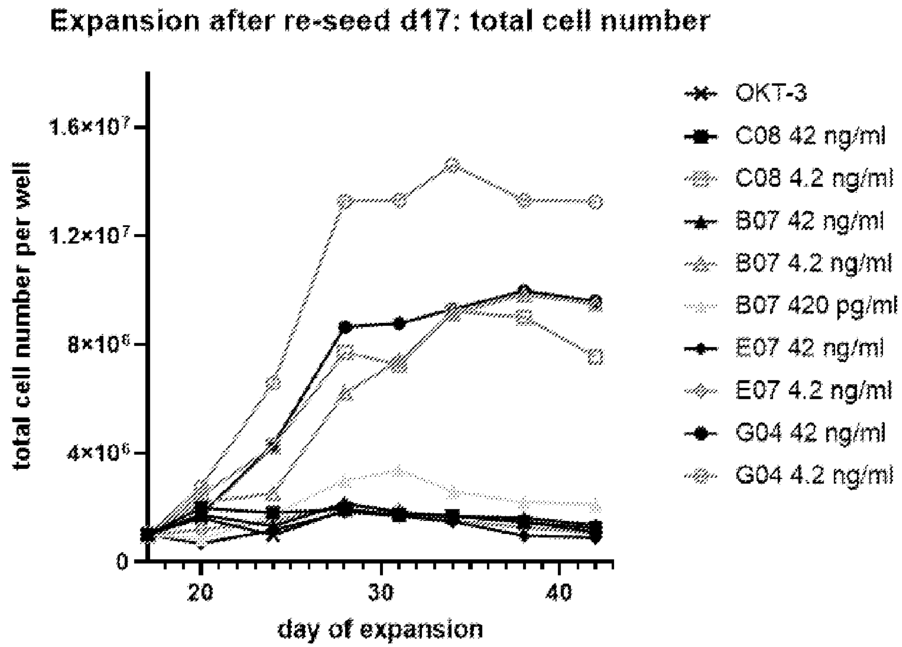


FIGURE 14

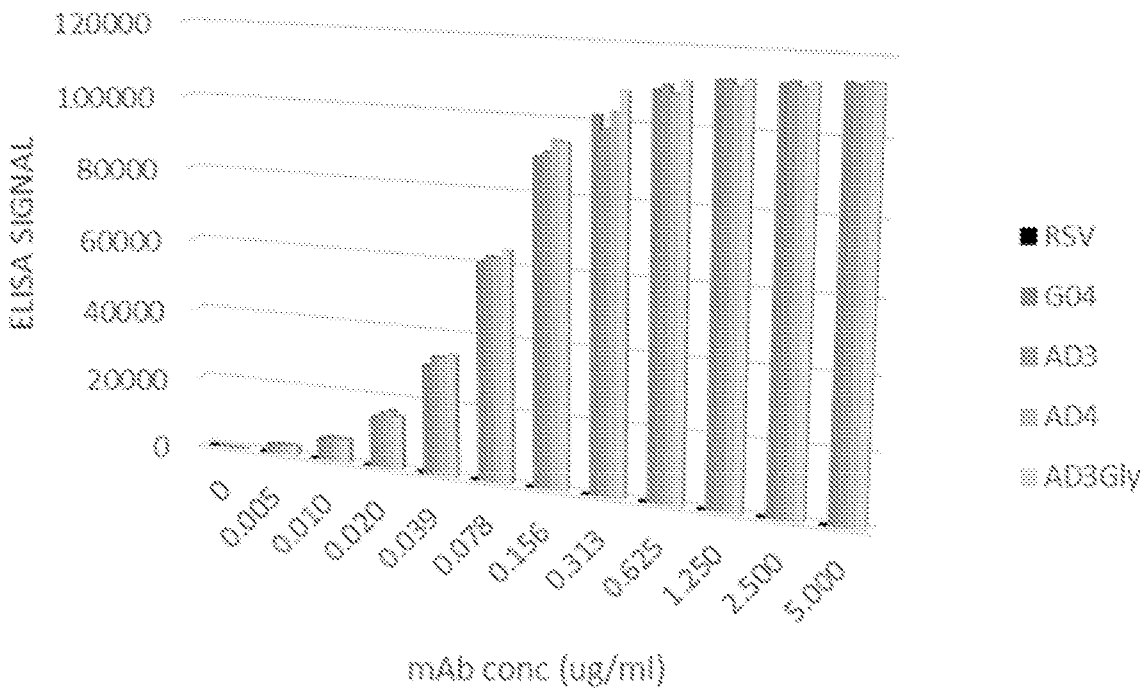


FIGURE 15

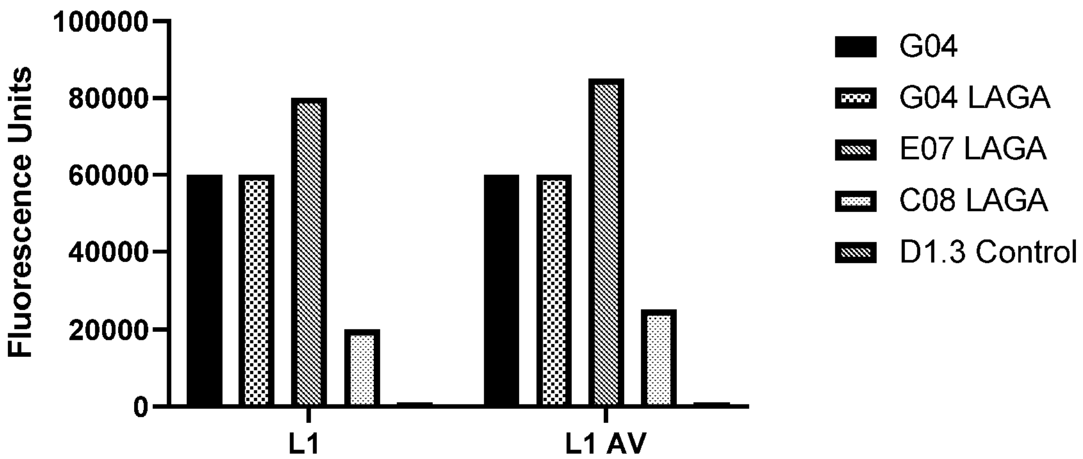


FIGURE 16

A)

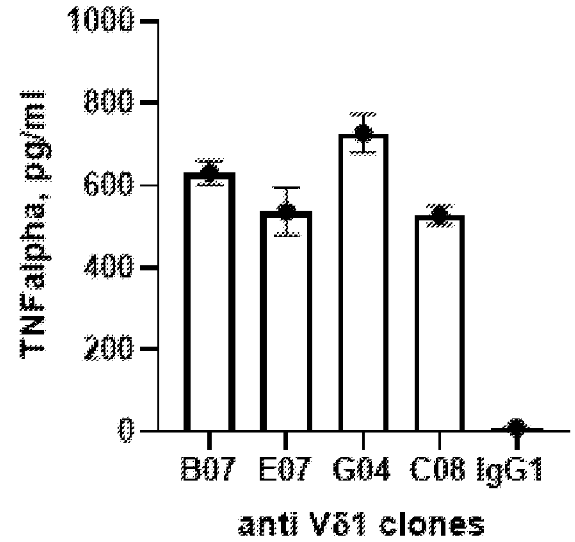


| TNF alpha levels in supernatant (pg/ml) | | | | |
|---|---|---|---|---|
| clone | Donor1 | Donor2 | Average | STDEV |
| B07 | 612.369 | 652.9294 | 632.6492 | 28.68055 |
| E07 | 579.6175 | 497.477 | 538.5472 | 58.08211 |
| G04 | 760.3178 | 695.8256 | 728.0717 | 45.60288 |
| C08 | 512.5485 | 545.4042 | 528.9763 | 23.23252 |
| IgG1 (control) | <LOQ | <LOQ | <LOQ | <LOQ |

B)

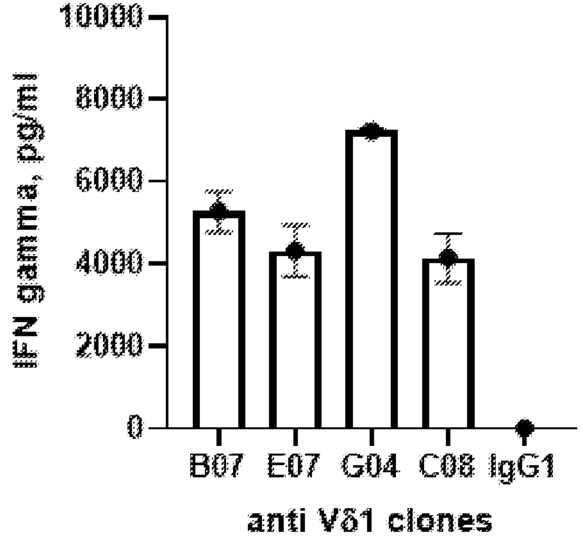


| IFN gamma levels in supernatant (pg/ml) | | | | |
|---|---|---|---|---|
| clone | Donor1 | Donor2 | Average | STDEV |
| B07 | 4916.846 | 5616.726 | 5266.786 | 494.89 |
| E07 | 4753.625 | 3875.864 | 4314.744 | 620.671 |
| G04 | 7279.766 | 7189.028 | 7234.397 | 64.16168 |
| C08 | 3713.296 | 4561.834 | 4137.565 | 600.0066 |
| IgG1 (control) | 469.6334 | 549.9306 | 509.782 | 56.77863 |

FIGURE 17

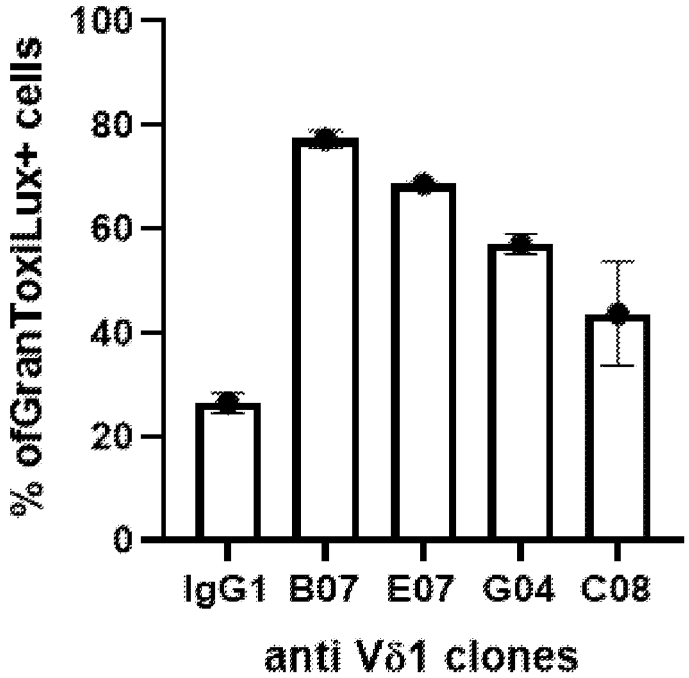


| % of GranToxiLux+ cells | | | | |
|---|---|---|---|---|
| | | | Average | STDVE |
| IgG1 | 25.1 | 27.8 | 26.45 | 1.909188 |
| B07 | 78.4 | 76.2 | 77.3 | 1.555635 |
| E07 | 68.6 | 68.7 | 68.65 | 0.070711 |
| G04 | 55.6 | 58.4 | 57 | 1.979899 |
| C08 | 50.7 | 36.6 | 43.65 | 9.970206 |

FIGURE 18

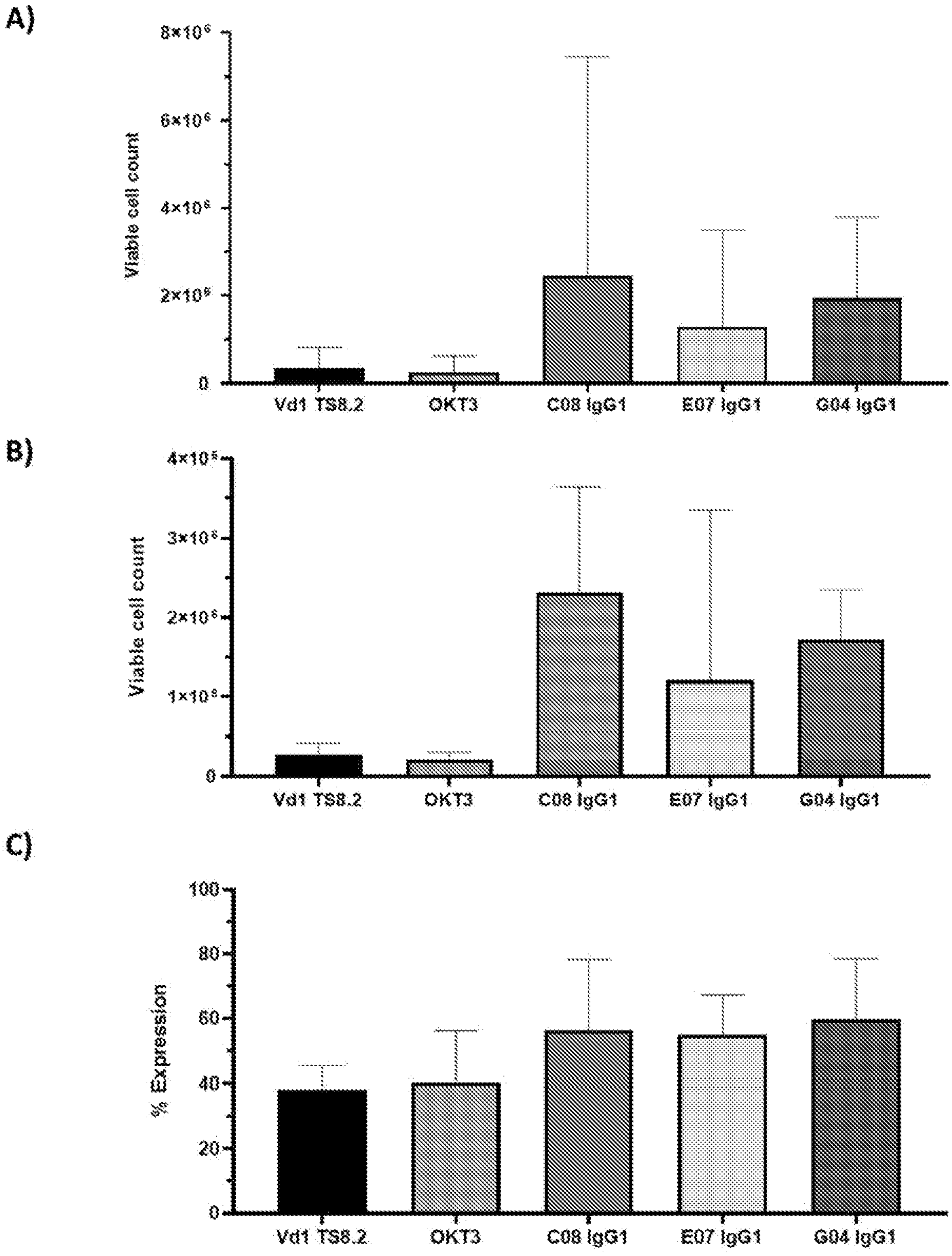
A)
8×10⁶
6×10⁶
4×10⁶
2×10⁶
0
Viable cell count
Vd1 TS8.2
OKT3
C08 IgG1
E07 IgG1
G04 IgG1
B)
4×10⁶
3×10⁶
2×10⁶
1×10⁶
0
Viable cell count
Vd1 TS8.2
OKT3
C08 IgG1
E07 IgG1
G04 IgG1
C)
100
80
60
40
20
0
% Expression
Vd1 TS8.2
OKT3
C08 IgG1
E07 IgG1
G04 IgG1

FIGURE 19

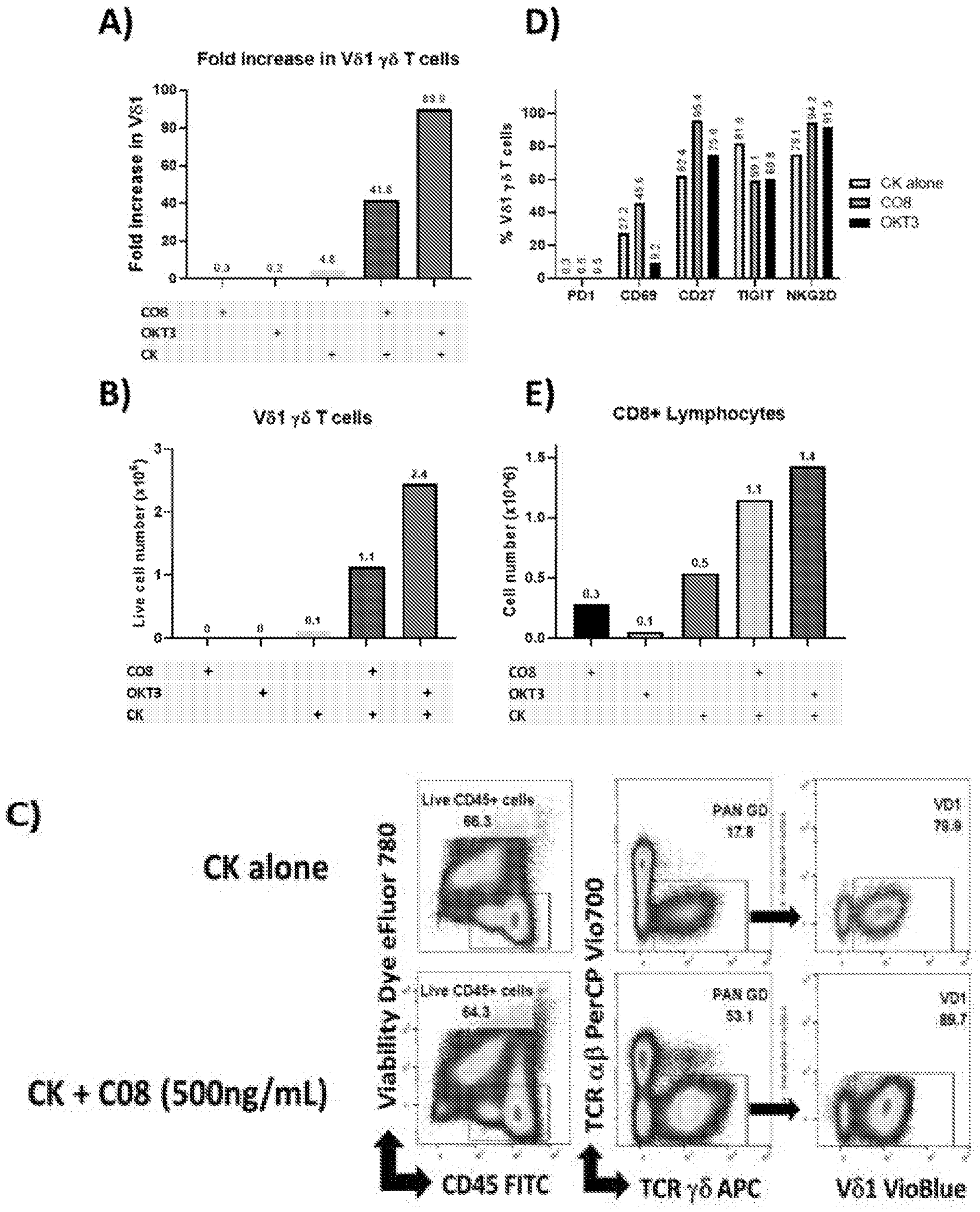
A)
Fold increase in Vδ1 γδ T cells
Fold increase in Vδ1
0.3
0.3
4.8
41.8
89.9
C08
OKT3
CK
D)
% Vδ1 γδ T cells
PD1
CD69
CD27
TIGIT
NKG2D
CK alone
C08
OKT3
B)
Vδ1 γδ T cells
Live cell number (x10^6)
0
0
0.1
1.1
2.4
C08
OKT3
CK
E)
CD8+ Lymphocytes
Cell number (x10^6)
0.3
0.1
0.5
1.1
1.4
C08
OKT3
CK
C)
CK alone
CK + C08 (500ng/mL)
Live CD45+ cells
86.3
Live CD45+ cells
84.3
PAN GD
17.8
PAN GD
53.1
VD1
79.9
VD1
89.7
Viability Dye eFluor 780
CD45 FITC
TCR αβ PerCP Vio700
TCR γδ APC
Vδ1 VioBlue

FIGURE 20

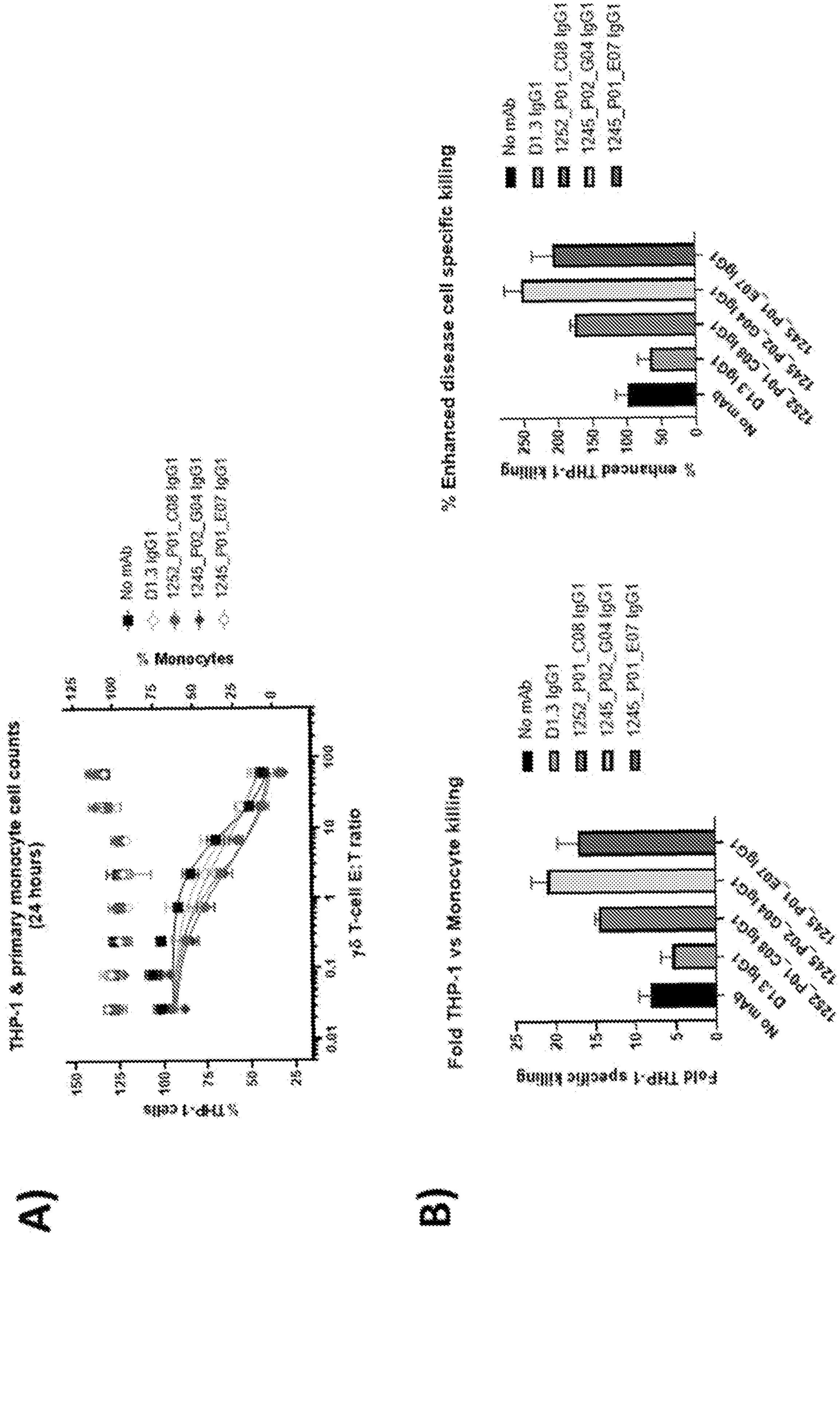
A)
THP-1 & primary monocyte cell counts
(24 hours)
% THP-1 cells
% Monocytes
γδ T-cell E:T ratio
No mAb
D1.3 IgG1
1252_P01_C08 IgG1
1245_P02_G04 IgG1
1245_P01_E07 IgG1
B)
Fold THP-1 vs Monocyte killing
Fold THP-1 specific killing
% Enhanced disease cell specific killing
% enhanced THP-1 killing
No mAb
D1.3 IgG1
1252_P01_C08 IgG1
1245_P02_G04 IgG1
1245_P01_E07 IgG1

FIGURE 21

C)

| | % improvement in EC50 |
|---|---|
| No mAb | 100% |
| D1.3 IgG1 | 109% |
| 1252_P01_C08 IgG1 | 337% |
| 1245_P02_G04 IgG | 383% |
| 1245_P01_E07 IgG | 159% |

D)

| | EC50s | |
|---|---|---|
| | γδT-cell | T:E ratio |
| No mAb | 8729 | 0.11 |
| D1.3 IgG1 | 8019 | 0.12 |
| 1252_P01_C08 IgG1 | 2594 | 0.39 |
| 1245_P02_G04 IgG | 2278 | 0.44 |
| 1245_P01_E07 IgG | 5485 | 0.18 |

FIGURE 21 (contd.)

;9

A B C

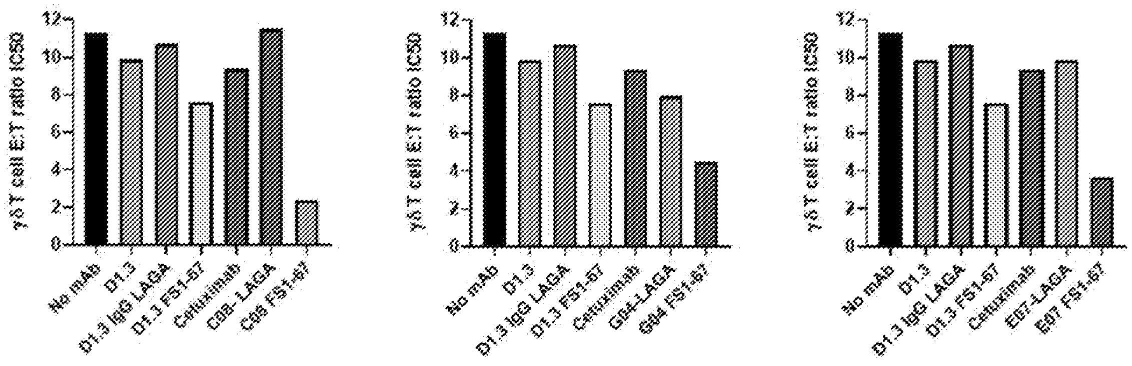


D

| | 5 hrs | 12 hrs | 24 hrs |
|---|---|---|---|
| No mAb | 100% | 100% | 100% |
| D1.3 | 114% | 99% | 112% |
| D1.3 IgG LAGA | 106% | 96% | 113% |
| D1.3 FS1-67 | 149% | 143% | 147% |
| Cetuximab | 121% | 110% | 126% |
| C08- LAGA | 99% | 84% | 101% |
| E07-LAGA | 115% | 117% | 127% |
| G04-LAGA | 141% | 128% | 163% |
| C08 FS1-67 | 467% | 424% | 371% |
| E07 FS1-67 | 303% | 292% | 259% |
| G04 FS1-67 | 254% | 232% | 212% |

E F G

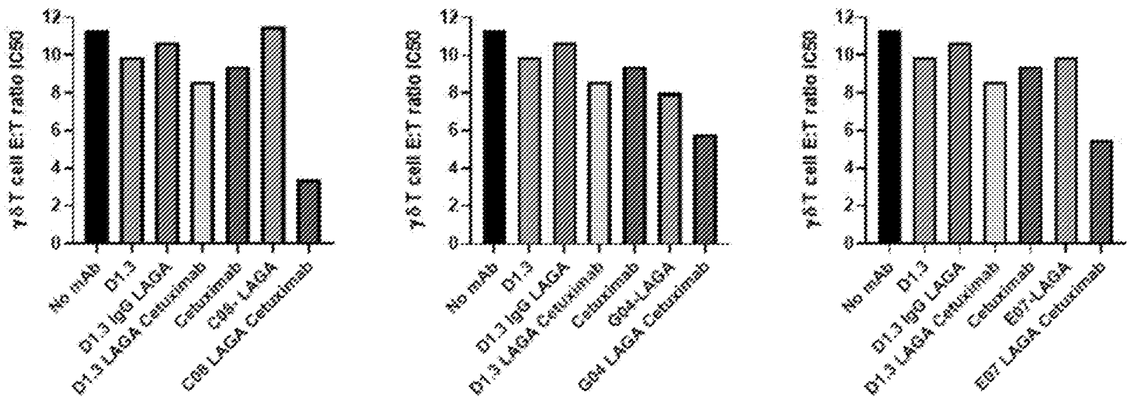


H

| | 5 hrs | 12 hrs | 24 hrs |
|---|---|---|---|
| No mAb | 100% | 100% | 100% |
| D1.3 | 114% | 99% | 112% |
| D1.3 IgG LAGA | 106% | 96% | 113% |
| D1.3 LAGA Cetuximab | 132% | 123% | 118% |
| Cetuximab | 121% | 110% | 126% |
| C08- LAGA | 99% | 84% | 101% |
| E07-LAGA | 115% | 117% | 127% |
| G04-LAGA | 141% | 128% | 163% |
| C08 LAGA Cetuximab | 334% | 313% | 245% |
| E07 LAGA Cetuximab | 208% | 216% | 187% |
| G04 LAGA Cetuximab | 197% | 210% | 158% |

FIGURE 22

I

| | EC50s | | |
|---|---|---|---|
| | γδT-cell | T:E ratio | % improvement |
| No mAb | 4102 | 0.24 | 100% |
| D1.3 IgG1 | 3651 | 0.27 | 112% |
| D1.3 IgG LAGA | 3640 | 0.27 | 113% |
| E07-LAGA | 3229 | 0.31 | 127% |
| D1.3 FS1-67 | 2794 | 0.36 | 147% |
| Cetuximab | 3246 | 0.31 | 126% |
| E07 FS1-67 | 1581 | 0.63 | 259% |

J

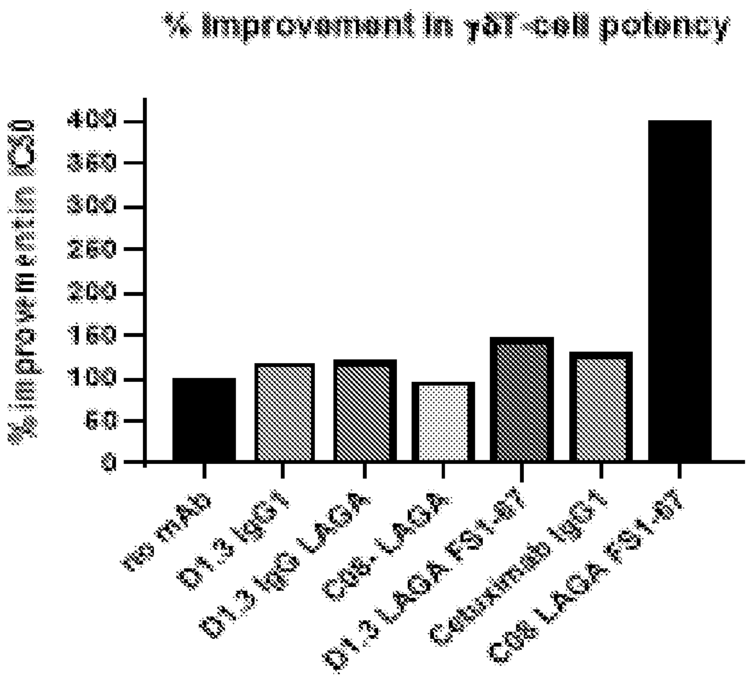


FIGURE 22 (contd.)

A)

| | EC50s | | |
|---|---|---|---|
| | γδT-cell | T:E ratio | % improvement |
| RSV IgG | 1988 | 1.99 | 100% |
| G04 | 2128 | 2.13 | 93% |
| E07 | 1775 | 1.78 | 112% |
| D1.3 VHVL | 1143 | 1.14 | 174% |
| G04 VHVL | 470 | 0.47 | 423% |
| E07 VHVL | 828 | 0.83 | 240% |

B)

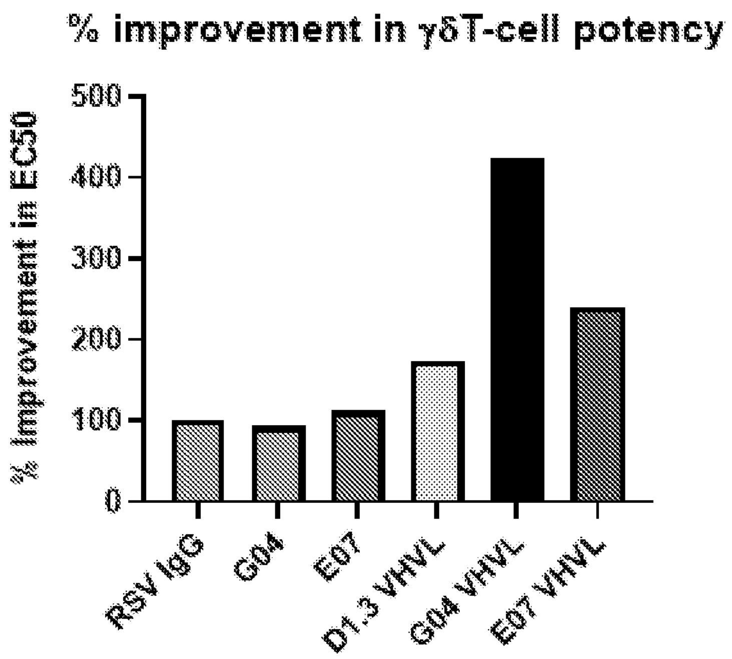


FIGURE 23

FIG 24

Anti-V δ1 mAb

Anti-V δ1/ CD19 BiTE

Human Vδ1

Cyno Vδ1

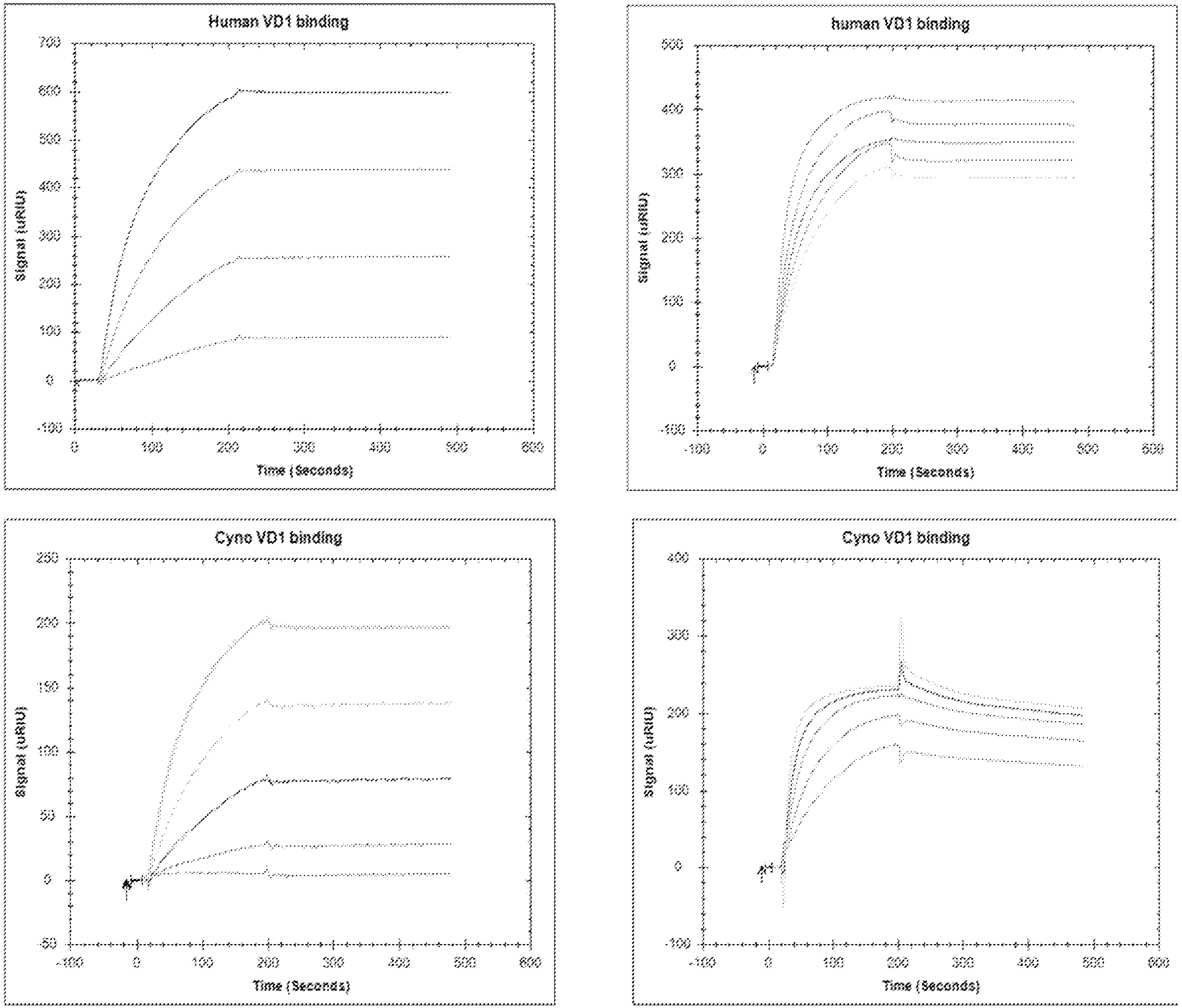

FIG 25A

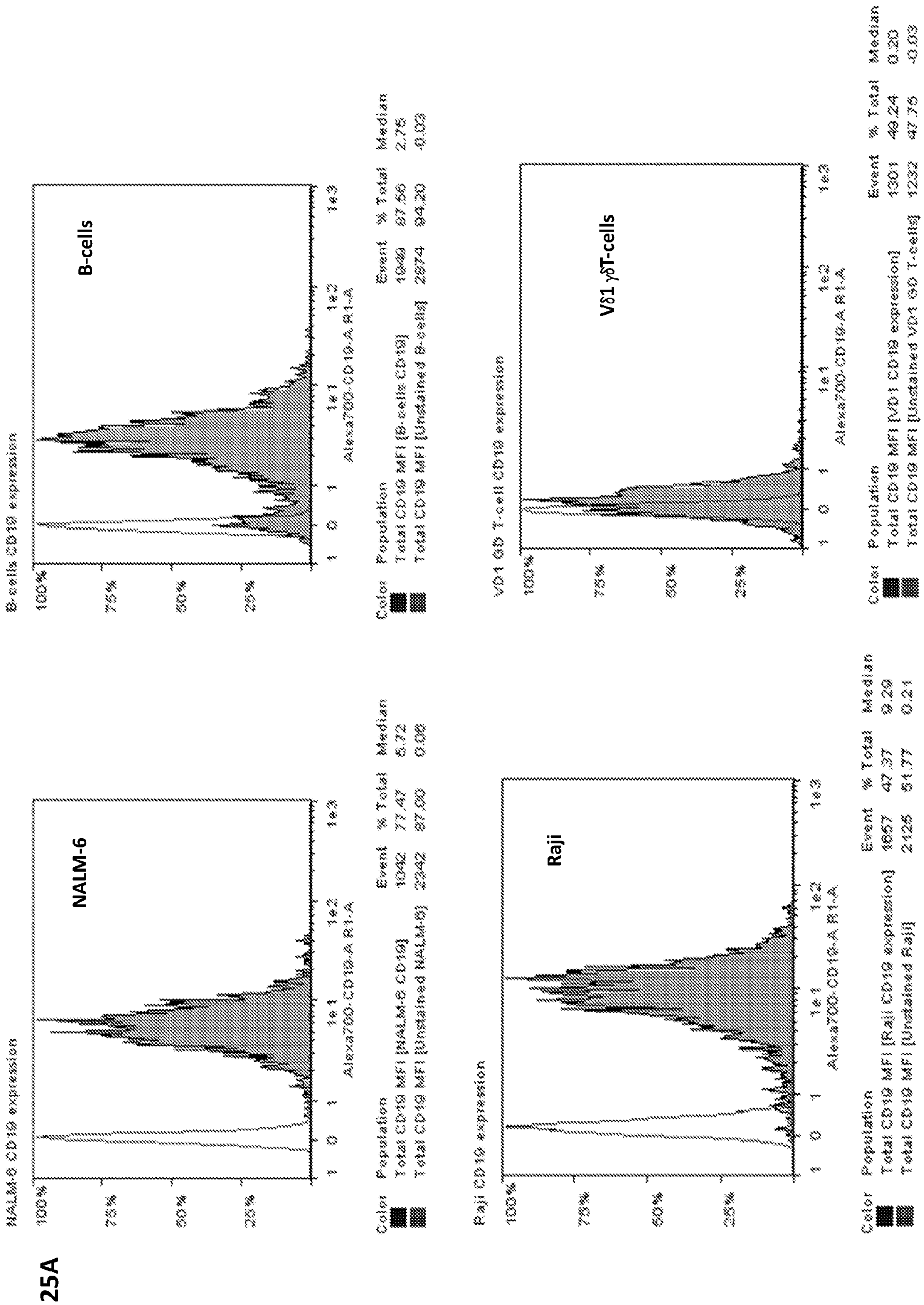
NALM-6 CD19 expression
NALM-6
Alexa700-CD19-A R1-A
Color Population Event % Total Median
Total CD19 MFI [NALM-6 CD19] 1042 77.47 5.72
Total CD19 MFI [Unstained NALM-6] 2342 87.00 0.06
B-cells CD19 expression
B-cells
Alexa700-CD19-A R1-A
Color Population Event % Total Median
Total CD19 MFI [B-cells CD19] 1949 67.56 2.75
Total CD19 MFI [Unstained B-cells] 2874 94.20 -0.03
Raji CD19 expression
Raji
Alexa700-CD19-A R1-A
Color Population Event % Total Median
Total CD19 MFI [Raji CD19 expression] 1657 47.37 9.29
Total CD19 MFI [Unstained Raji] 2125 51.77 0.21
VD1 GD T-cell CD19 expression
Vδ1 γδT-cells
Alexa700-CD19-A R1-A
Color Population Event % Total Median
Total CD19 MFI [VD1 CD19 expression] 1301 49.24 0.20
Total CD19 MFI [Unstained VD1 GD T-cells] 1232 47.75 -0.03
100%
75%
50%
25%
1 0 1 1e1 1e2 1e3

FIG 25 cont.

B

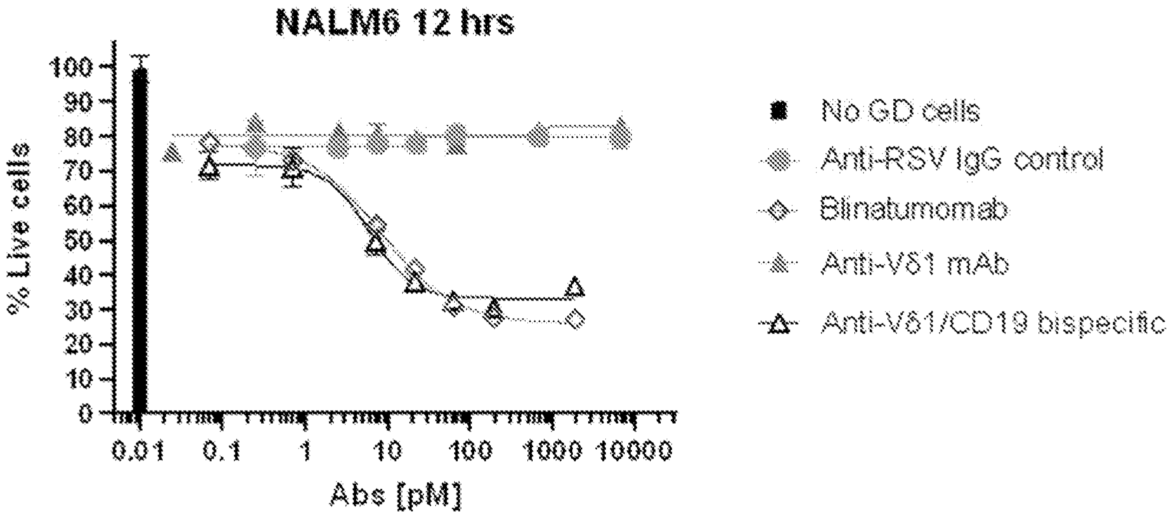


C

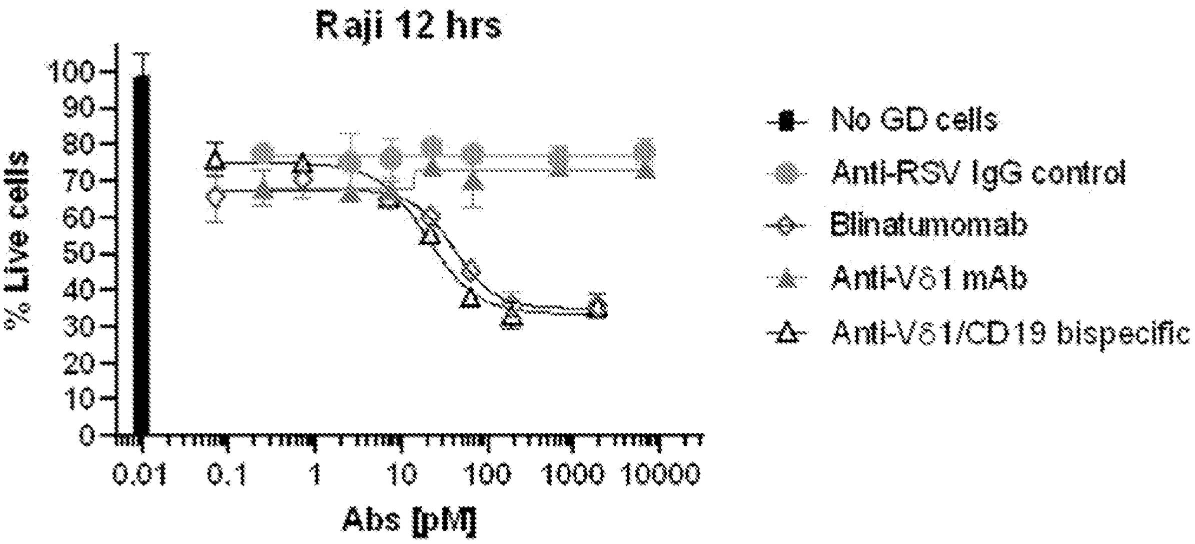


D

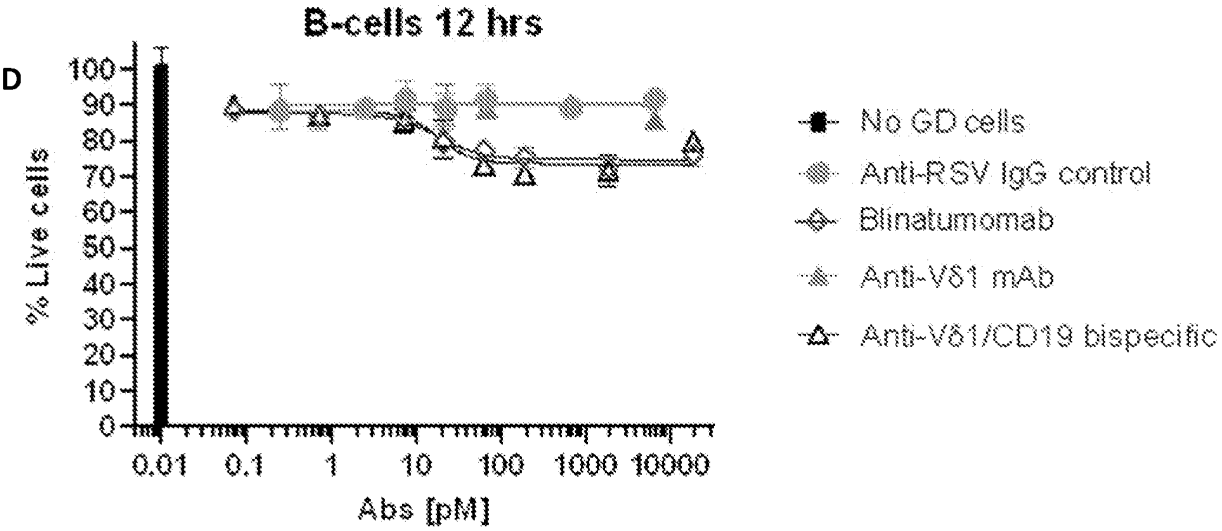

FIG 25 cont.

E

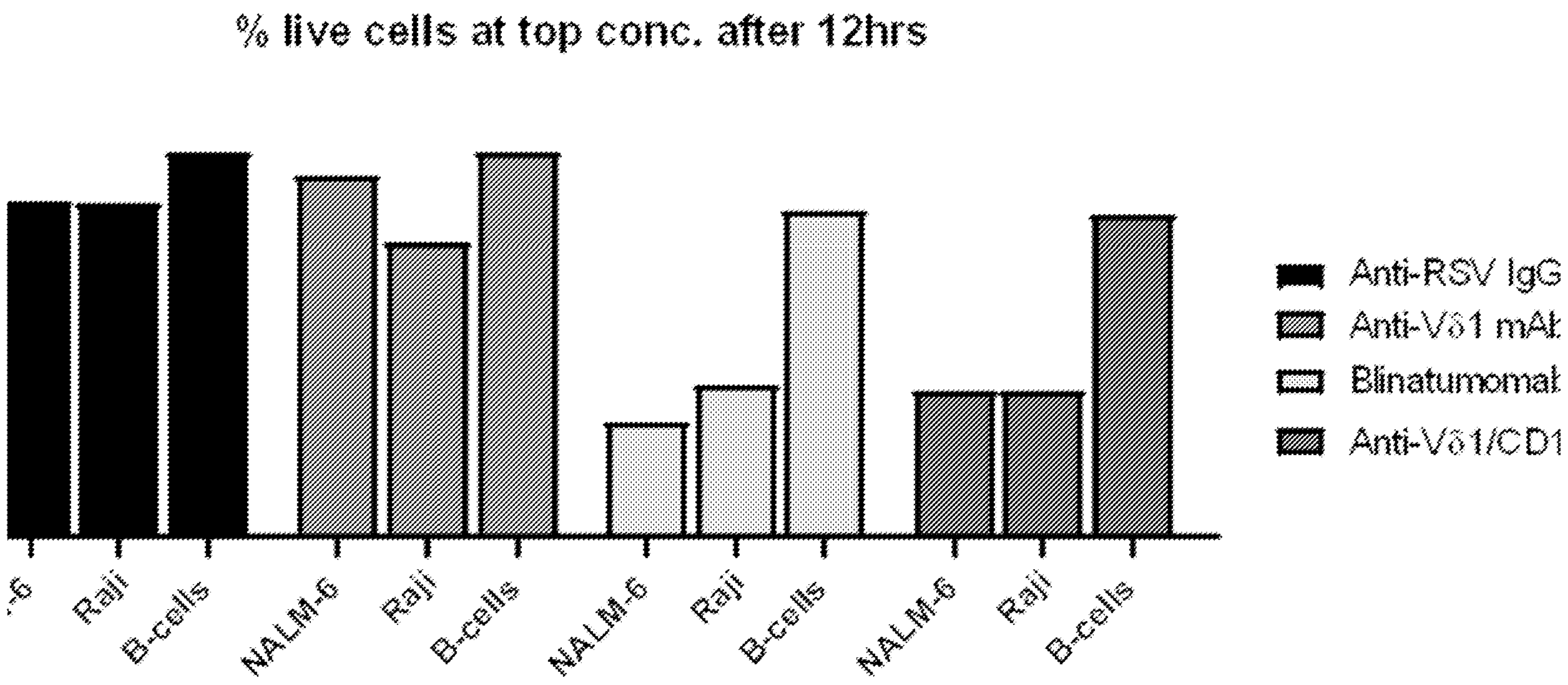
% live cells at top conc. after 12hrs
-6
Raji
B-cells
NALM-6
Raji
B-cells
NALM-6
Raji
B-cells
NALM-6
Raji
B-cells
Anti-RSV IgG
Anti-Vδ1 mAb
Blinatumomab
Anti-Vδ1/CD1

FIG 25 cont.

F

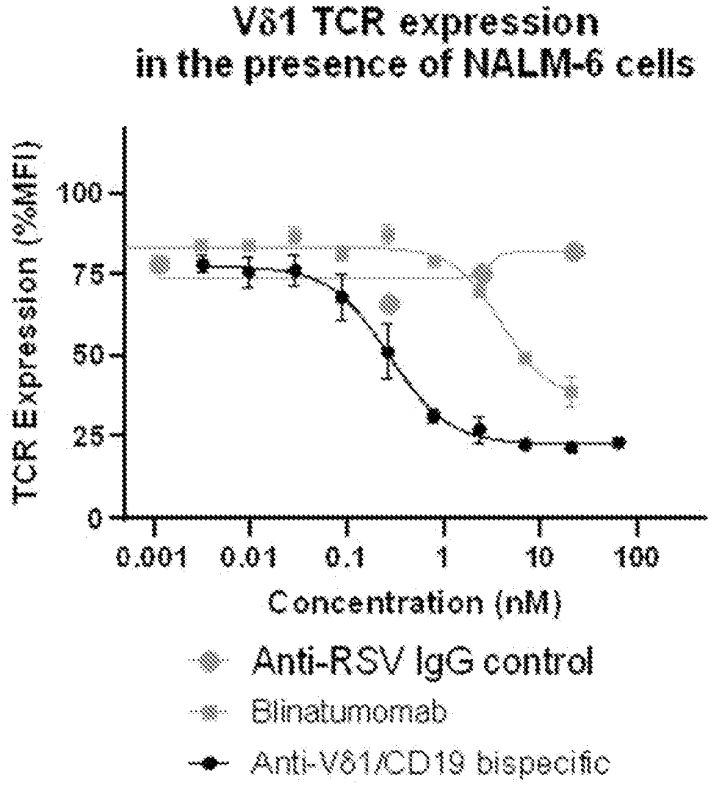


G

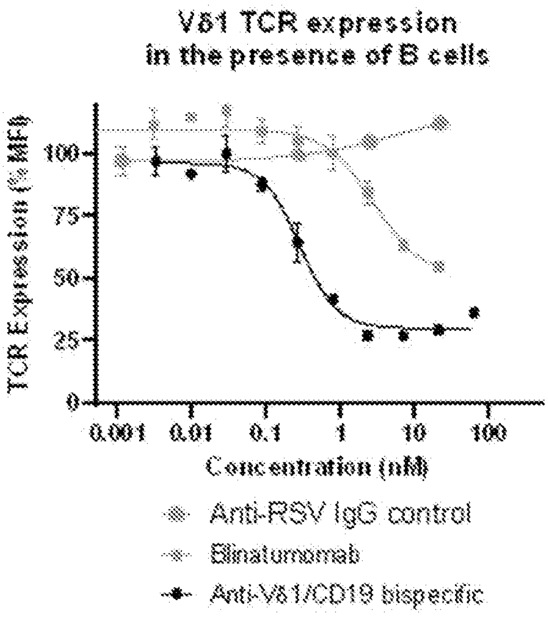

FIG 25 cont.

H

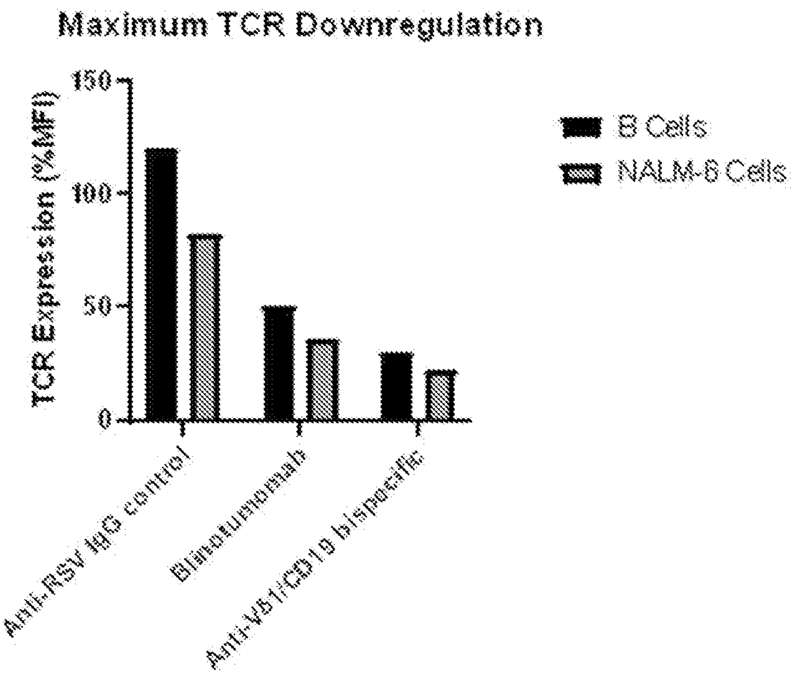


I

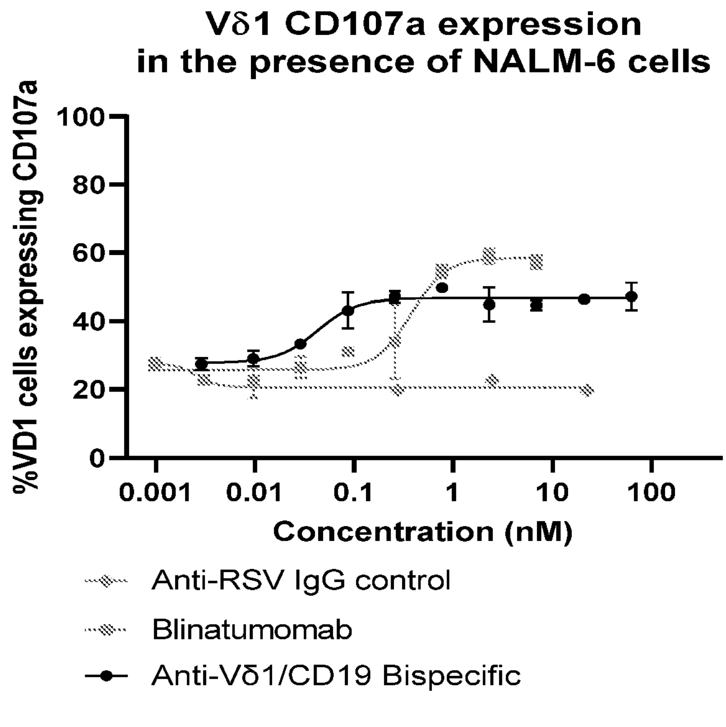

# FIG 25 cont.

J

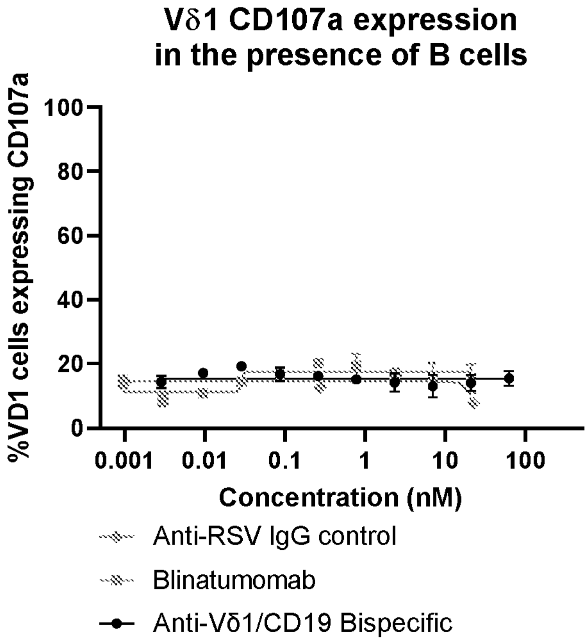


K

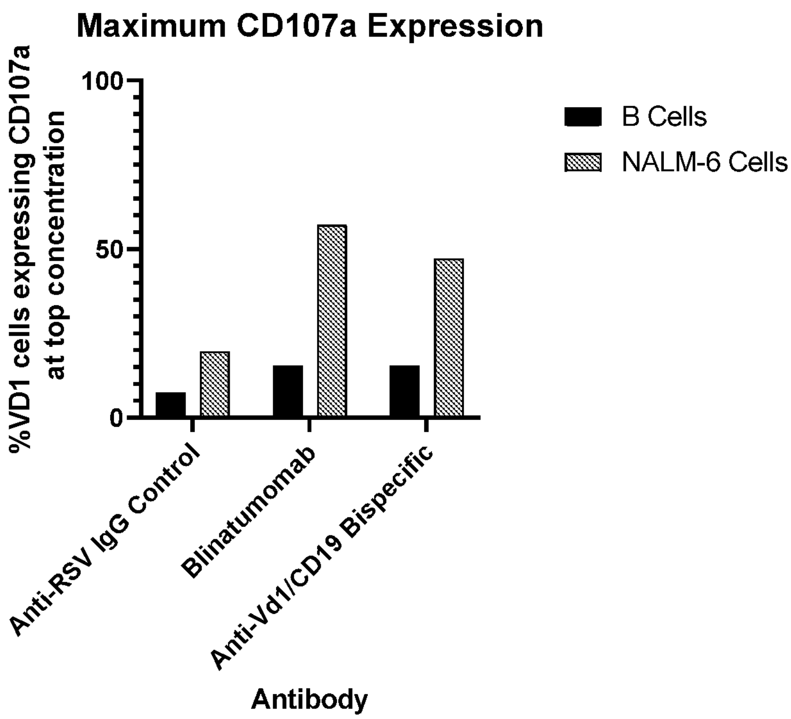

FIG 26

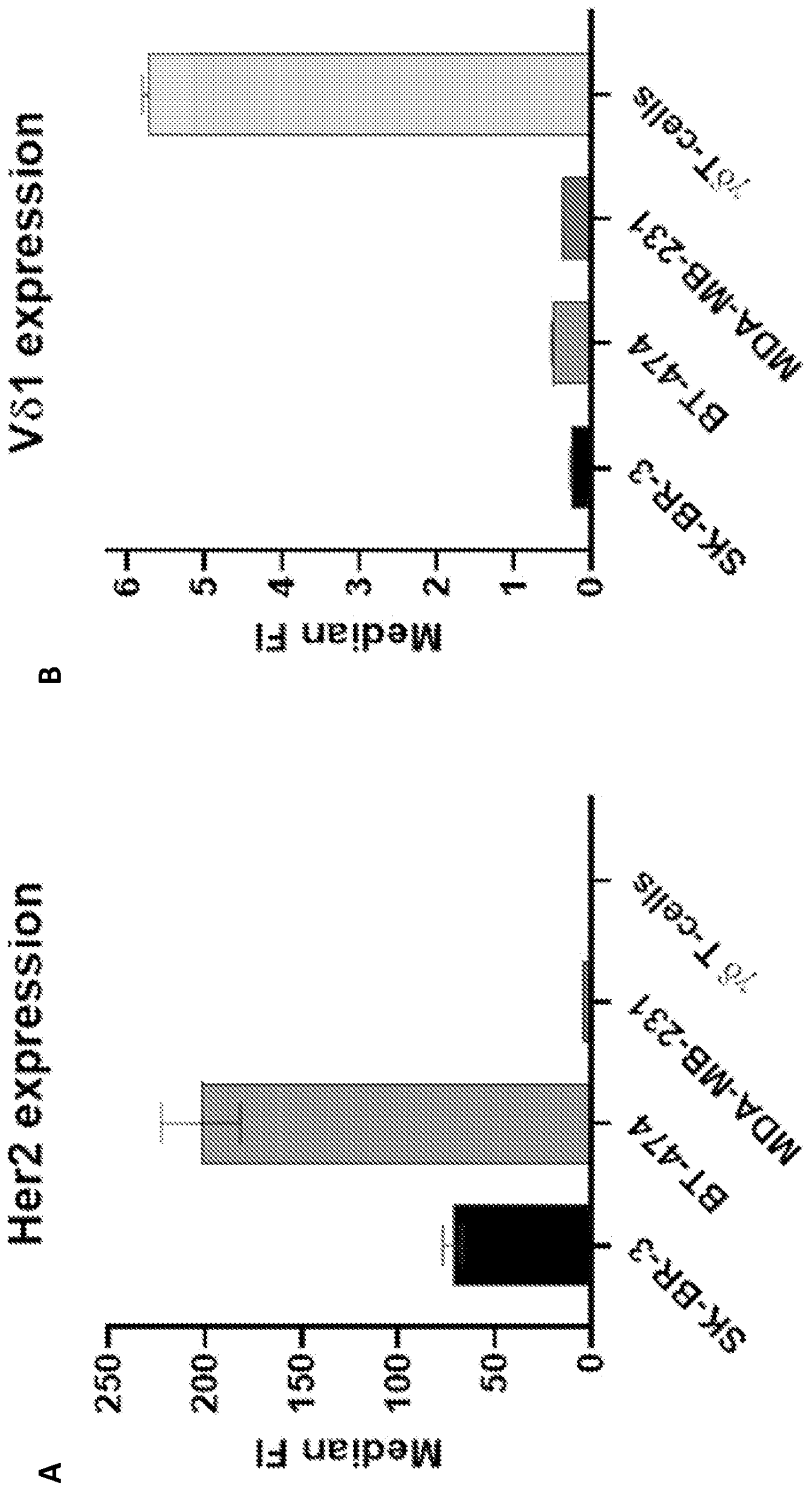
A
Her2 expression
Median FI
250
200
150
100
50
0
SK-BR-3
BT-474
MDA-MB-231
γδ T-cells
B
Vδ1 expression
Median FI
6
5
4
3
2
1
0
SK-BR-3
BT-474
MDA-MB-231
γδT-cells

FIG 26 cont.

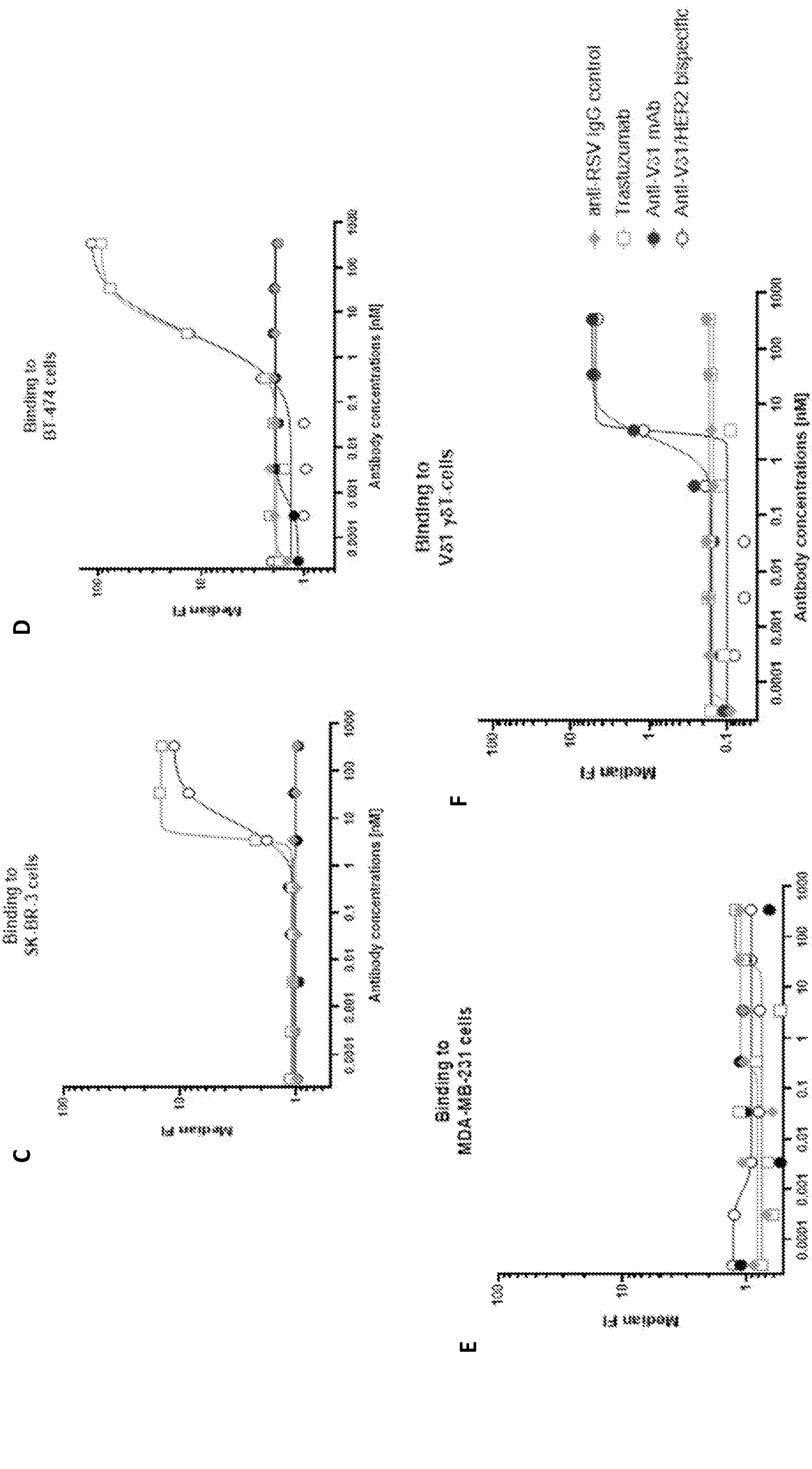
C
Binding to
SK BR 3 cells
Median FI
Antibody concentrations [nM]
D
Binding to
BT-474 cells
Median FI
Antibody concentrations [nM]
E
Binding to
MDA-MB-231 cells
Median FI
Antibody concentrations [nM]
F
Binding to
Vδ1 γδT-cells
Median FI
Antibody concentrations [nM]
anti-RSV IgG control
Trastuzumab
Anti-Vδ1 mAb
Anti-Vδ1/HER2 bispecific

**FIG 26 cont.**

G

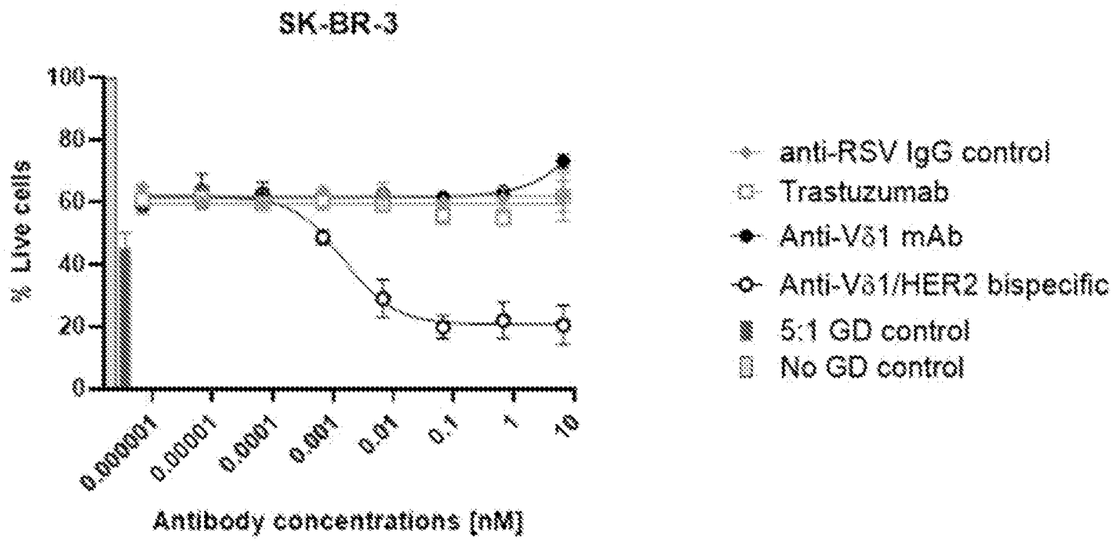


H

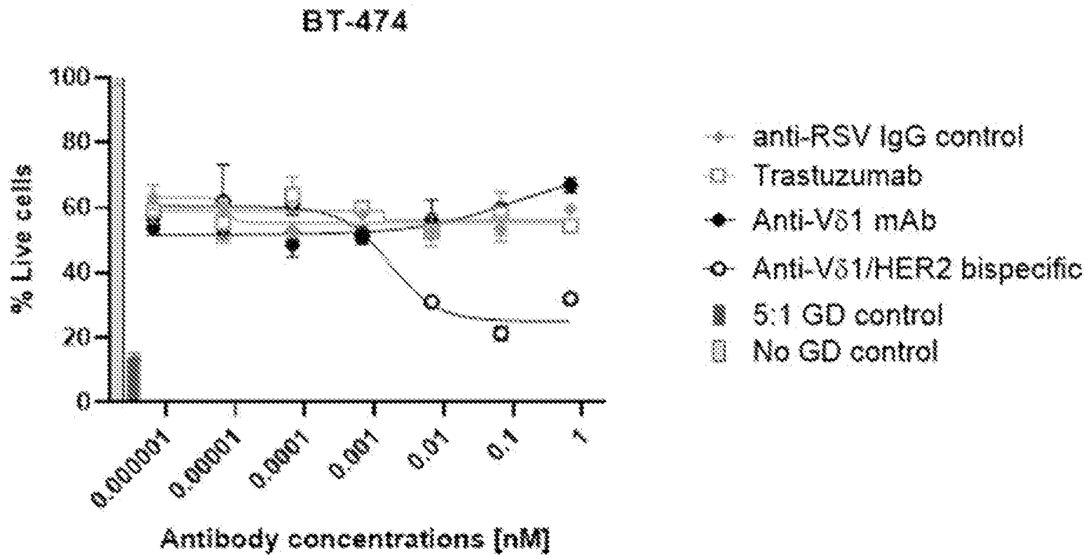


I

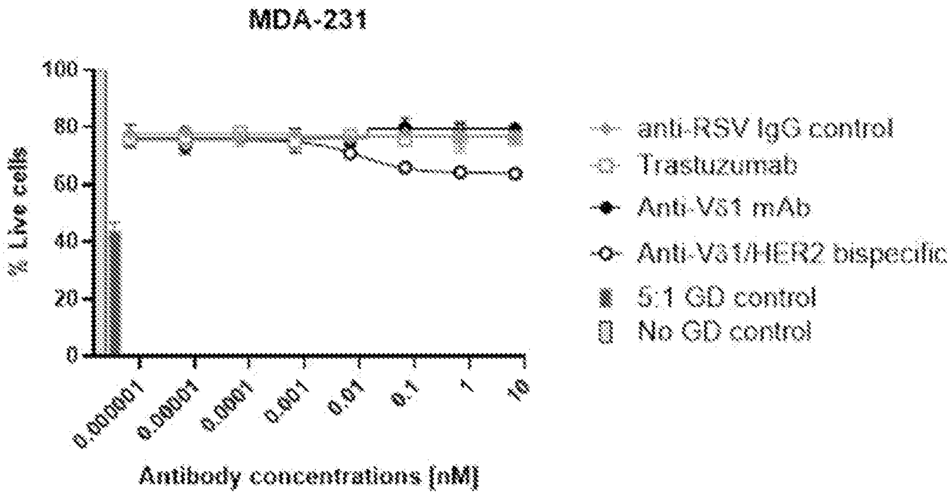

FIG 26 cont.

J

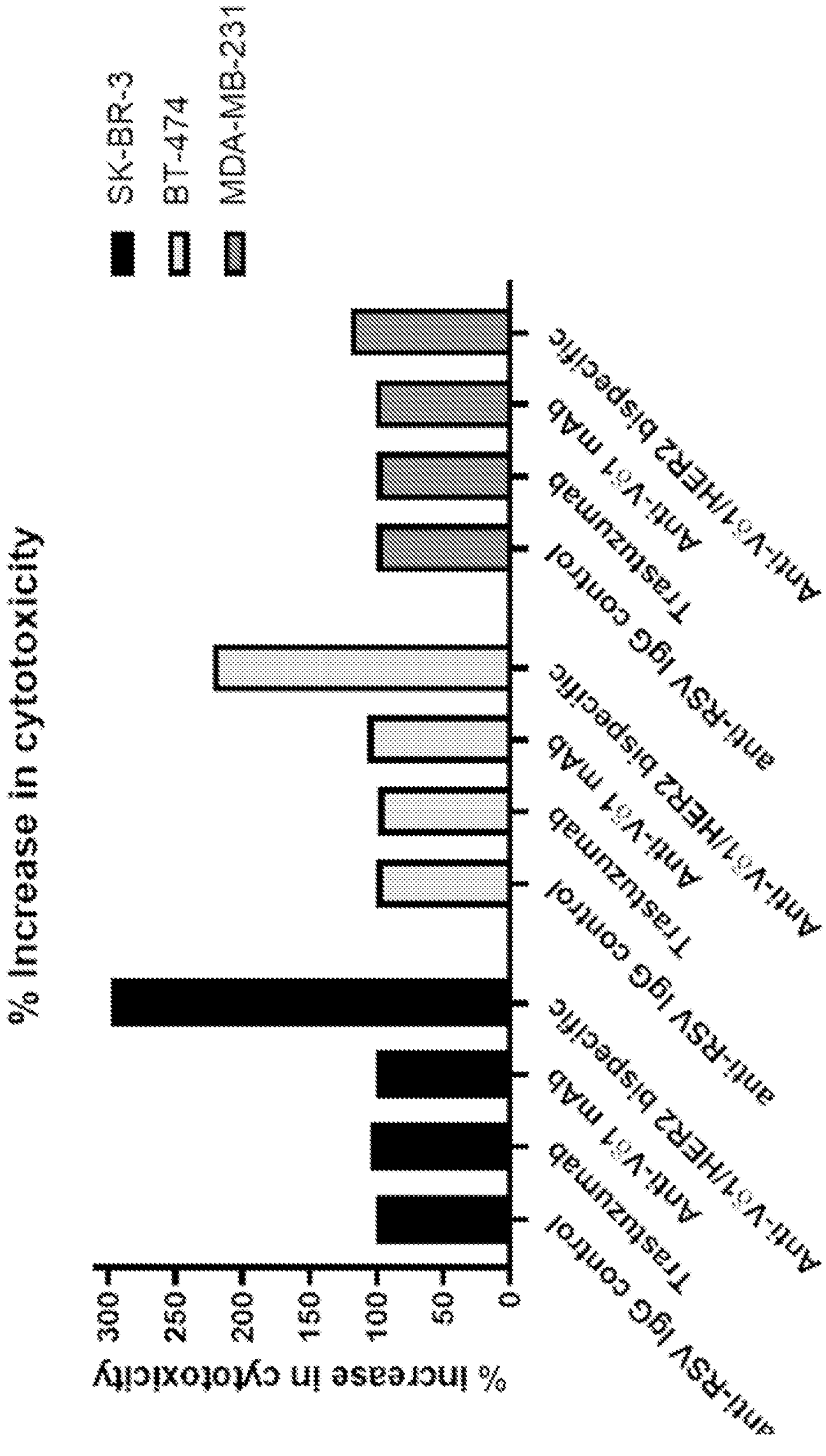
% Increase in cytotoxicity
SK-BR-3
BT-474
MDA-MB-231
% Increase in cytotoxicity
300
250
200
150
100
50
0
Anti-RSV IgG control
Trastuzumab
Anti-Vδ1 mAb
Anti-Vδ1/HER2 bispecific

# FIG 27A

**Binding to human Vd1**

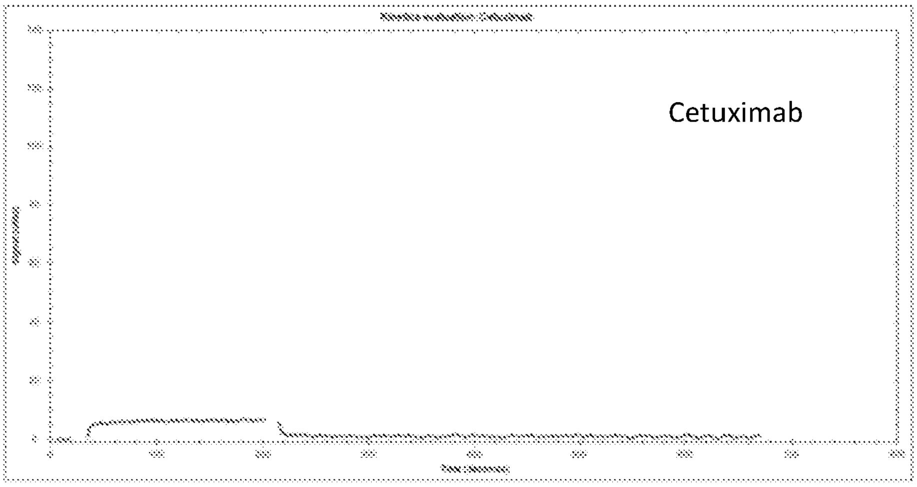


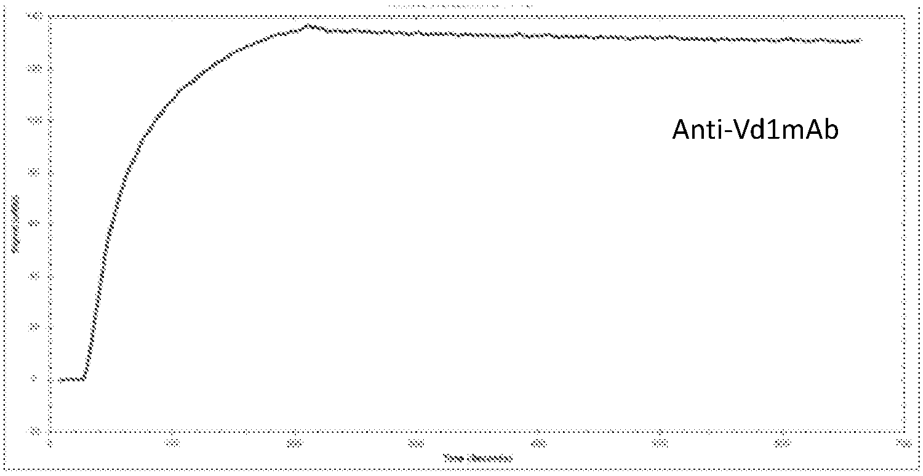


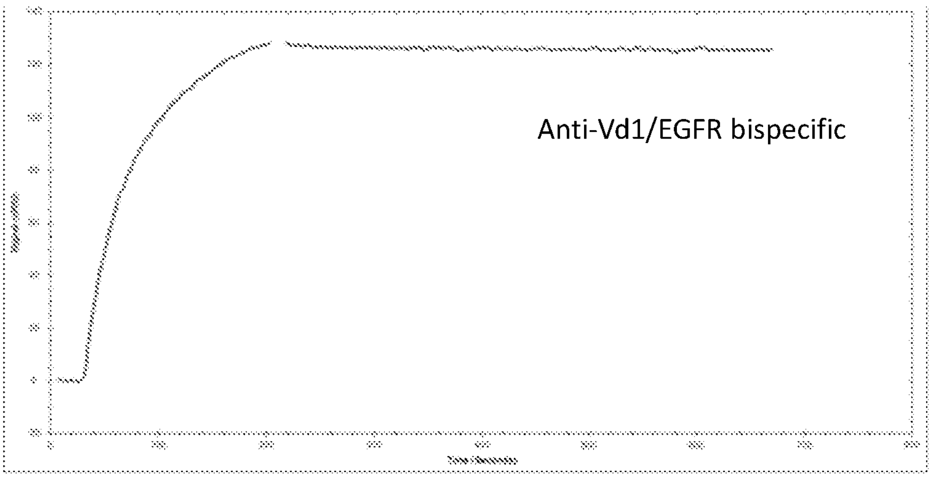

# FIG 27B

**Binding to human EGFR**

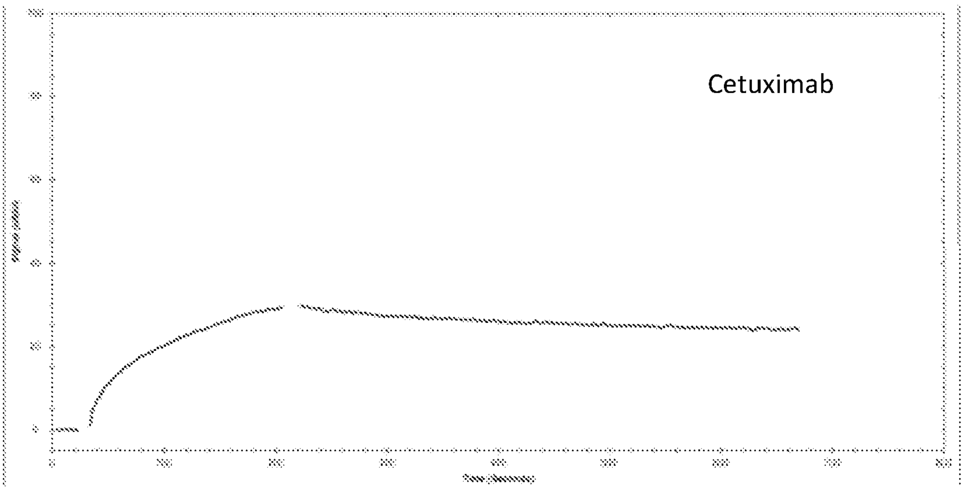


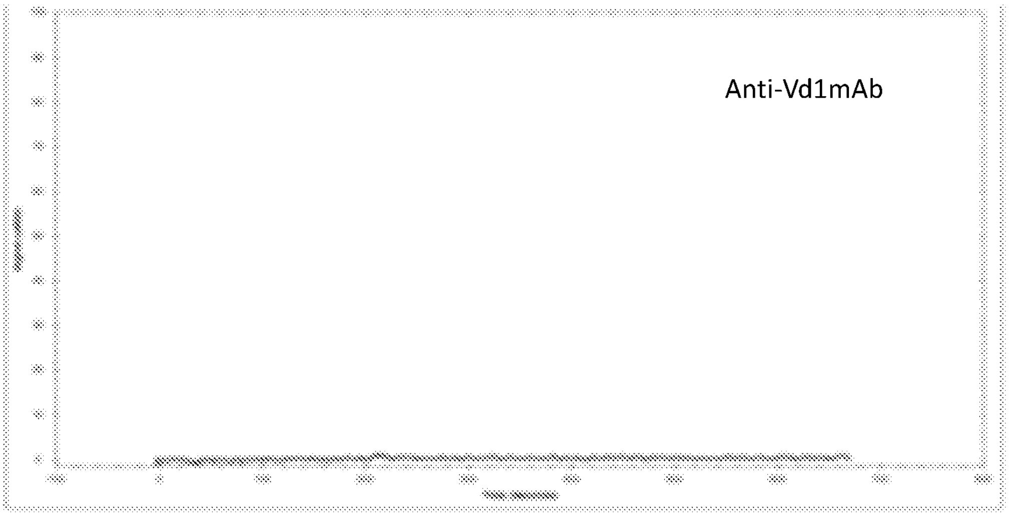


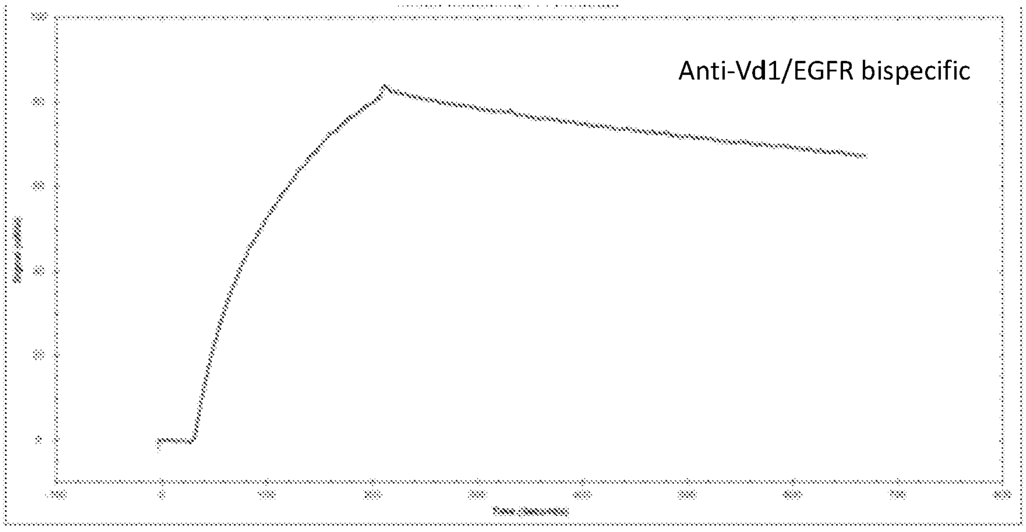

FIG 28

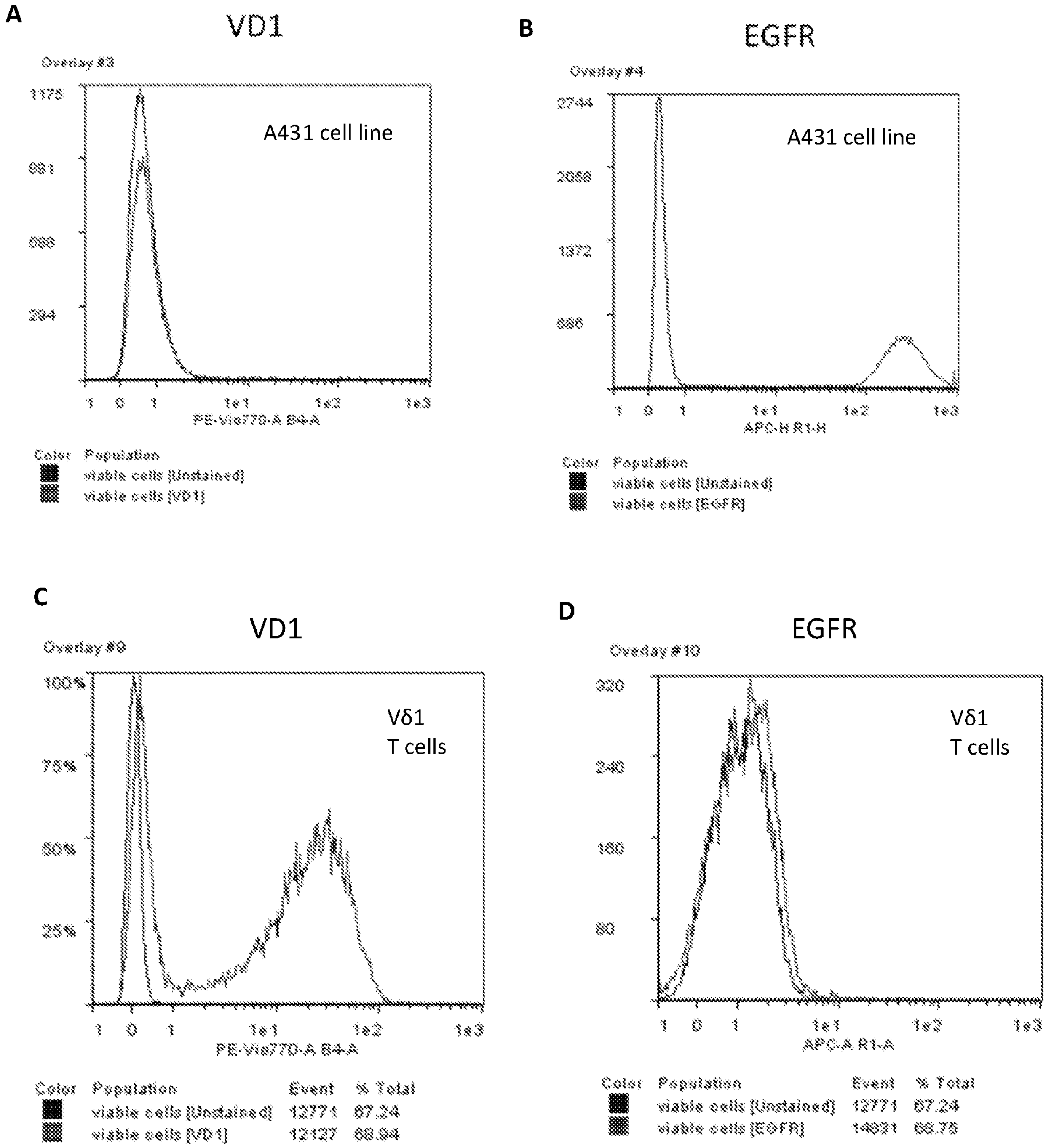
A
VD1
Overlay #3
A431 cell line
PE-Vio770-A B4-A
Color Population
viable cells [Unstained]
viable cells [VD1]
B
EGFR
Overlay #4
A431 cell line
APC-H R1-H
Color Population
viable cells [Unstained]
viable cells [EGFR]
C
VD1
Overlay #9
Vδ1
T cells
PE-Vio770-A B4-A
Color Population Event % Total
viable cells [Unstained] 12771 67.24
viable cells [VD1] 12127 68.94
D
EGFR
Overlay #10
Vδ1
T cells
APC-A R1-A
Color Population Event % Total
viable cells [Unstained] 12771 67.24
viable cells [EGFR] 14031 66.75

**FIG 28 cont.**

E

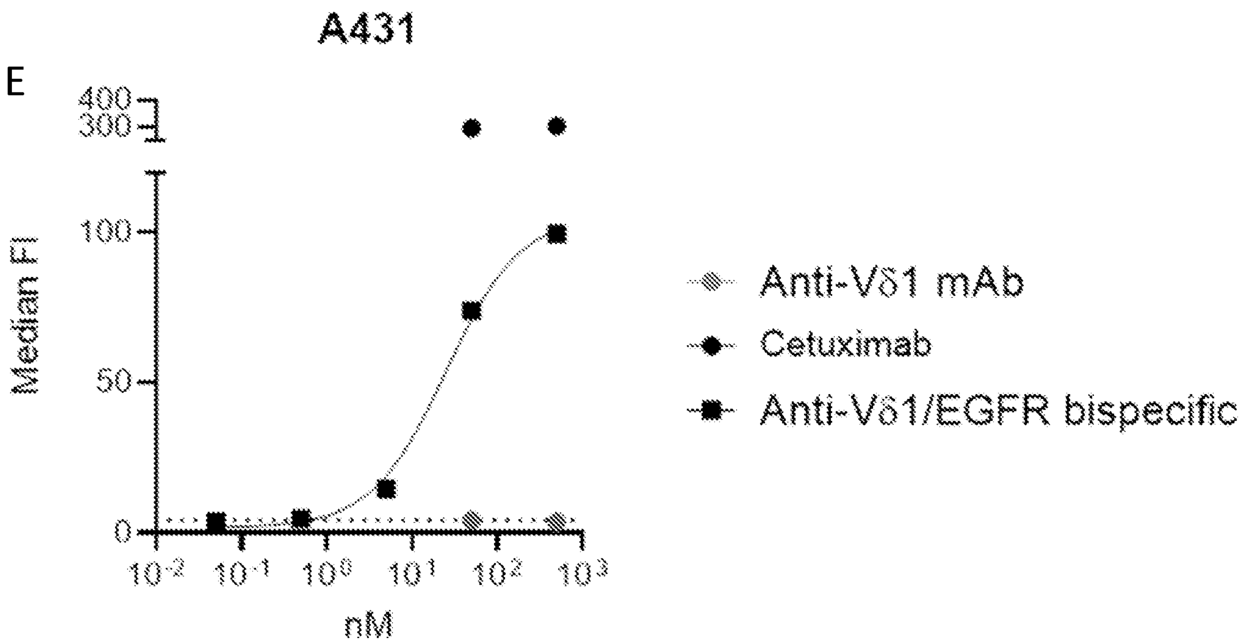


F

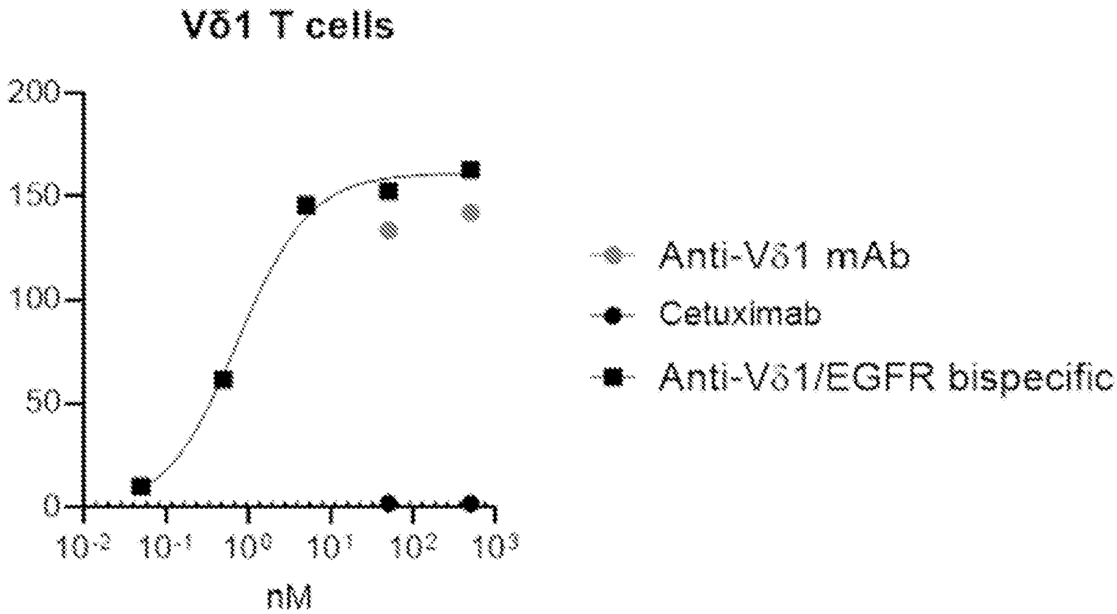

FIG 29

A

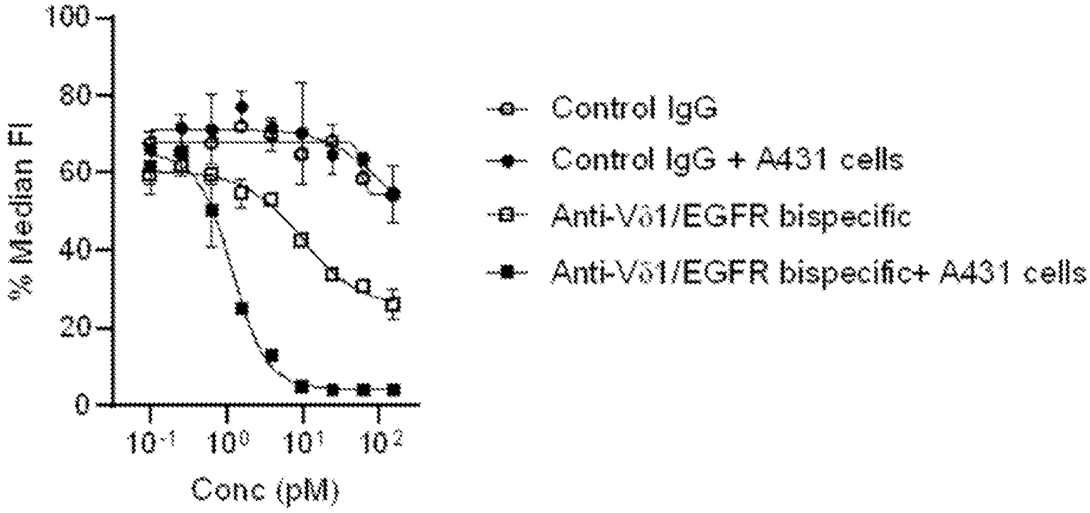


B

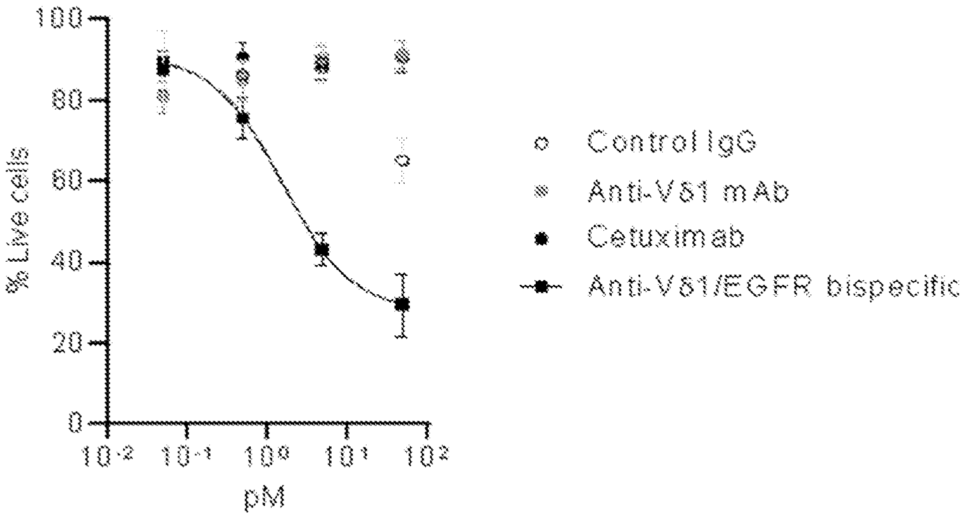


C

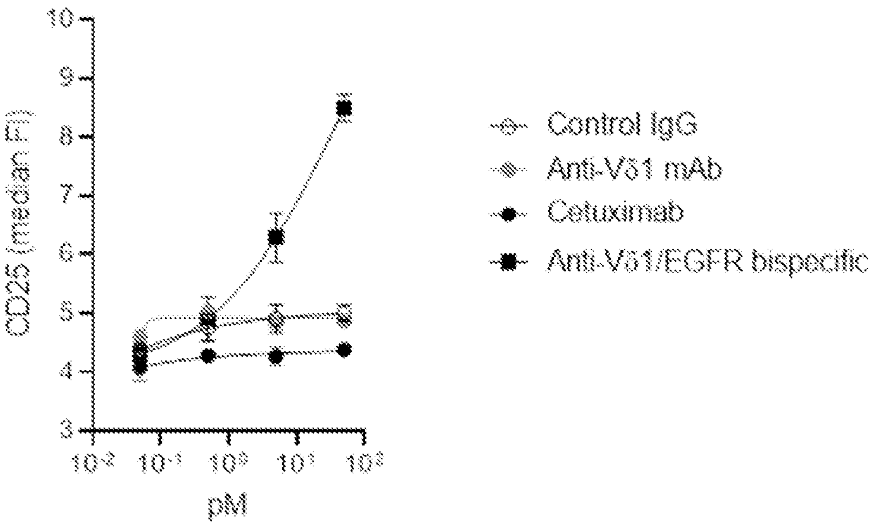


FIG 29 cont.

D

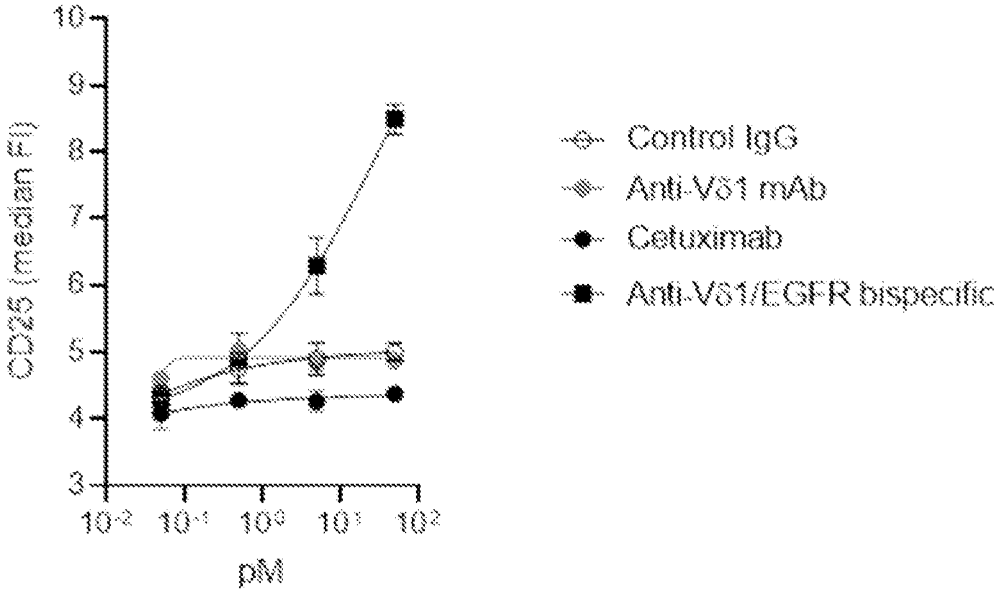
CD25 (median FI)
10
9
8
7
6
5
4
3
10⁻² 10⁻¹ 10⁰ 10¹ 10²
pM
Control IgG
Anti-Vδ1 mAb
Cetuximab
Anti-Vδ1/EGFR bispecific

E

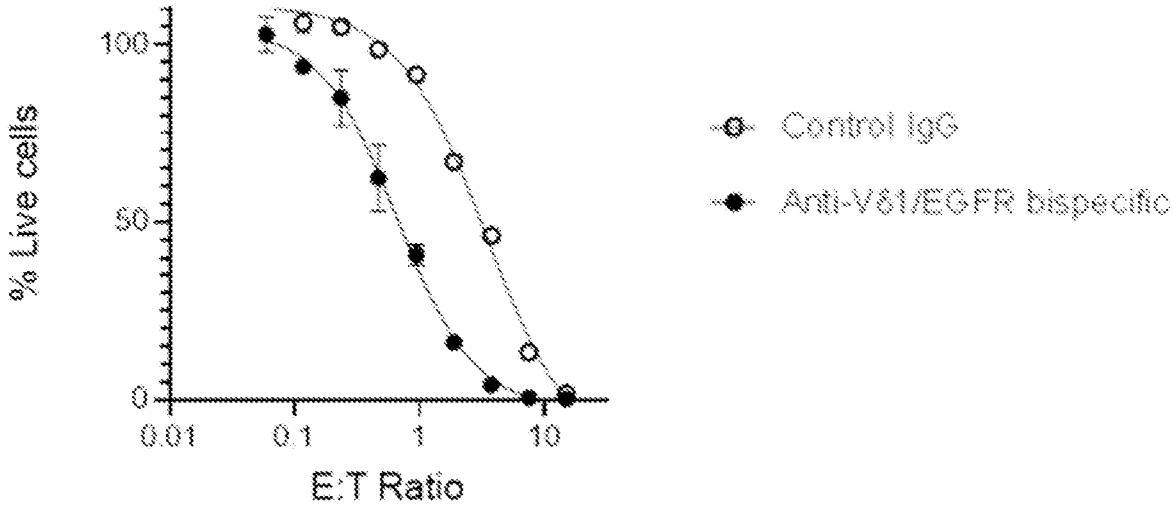
% Live cells
100
50
0
0.01 0.1 1 10
E:T Ratio
Control IgG
Anti-Vδ1/EGFR bispecific

FIG 30

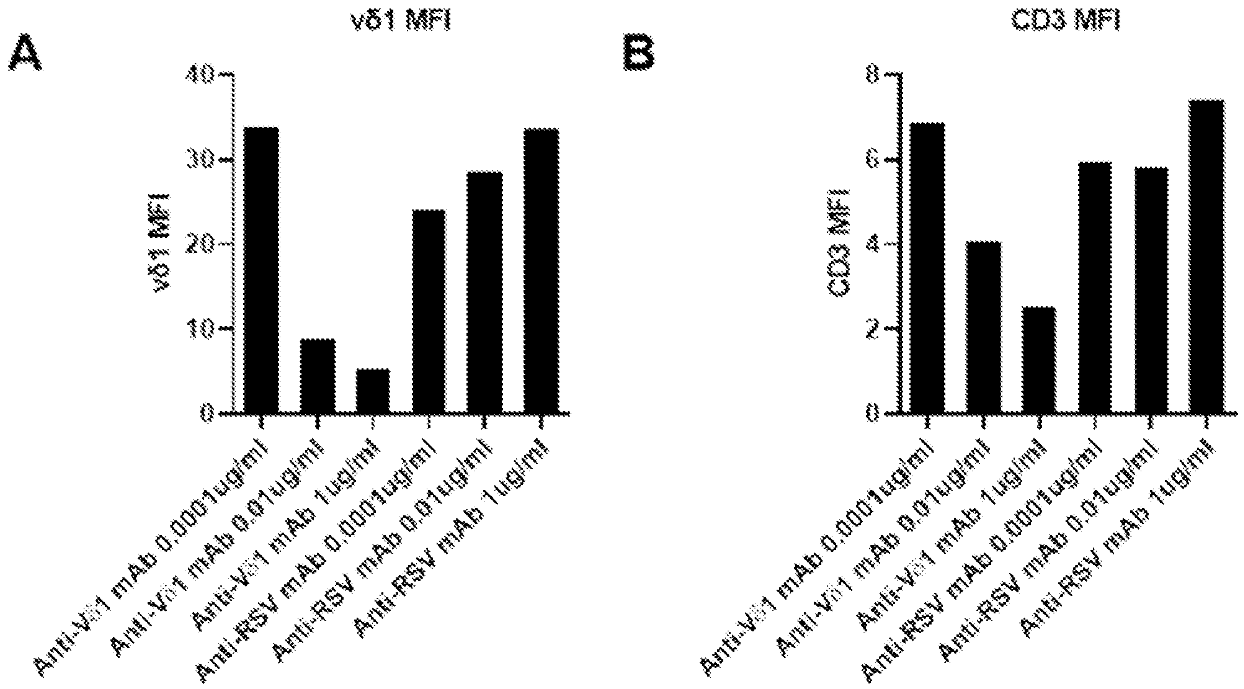
A
vδ1 MFI
vδ1 MFI
40
30
20
10
0
Anti-Vδ1 mAb 0.0001ug/ml
Anti-Vδ1 mAb 0.01ug/ml
Anti-Vδ1 mAb 1ug/ml
Anti-RSV mAb 0.0001ug/ml
Anti-RSV mAb 0.01ug/ml
Anti-RSV mAb 1ug/ml
B
CD3 MFI
CD3 MFI
8
6
4
2
0
Anti-Vδ1 mAb 0.0001ug/ml
Anti-Vδ1 mAb 0.01ug/ml
Anti-Vδ1 mAb 1ug/ml
Anti-RSV mAb 0.0001ug/ml
Anti-RSV mAb 0.01ug/ml
Anti-RSV mAb 1ug/ml

FIG 31

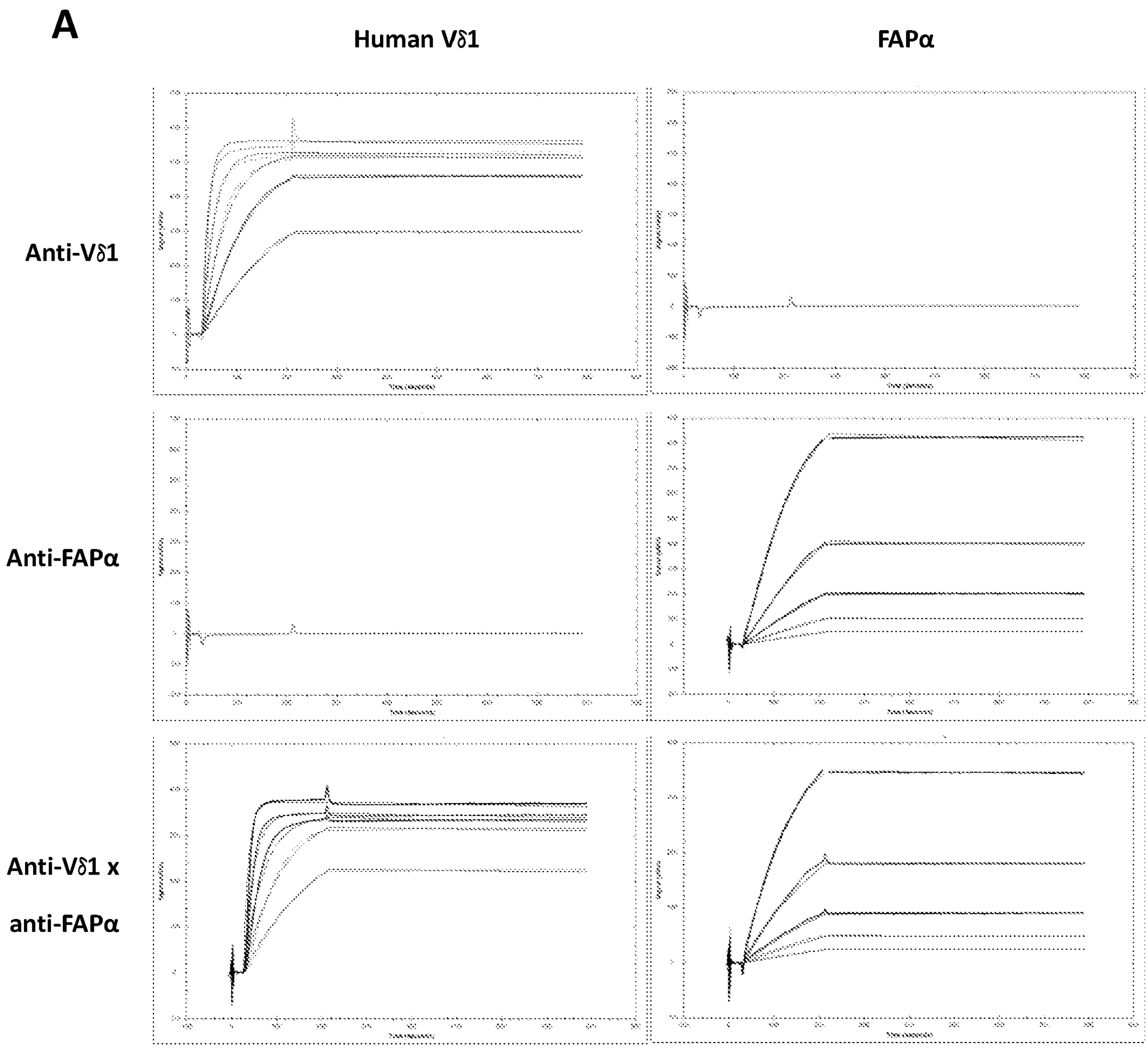

# FIG 31 cont.

**B**

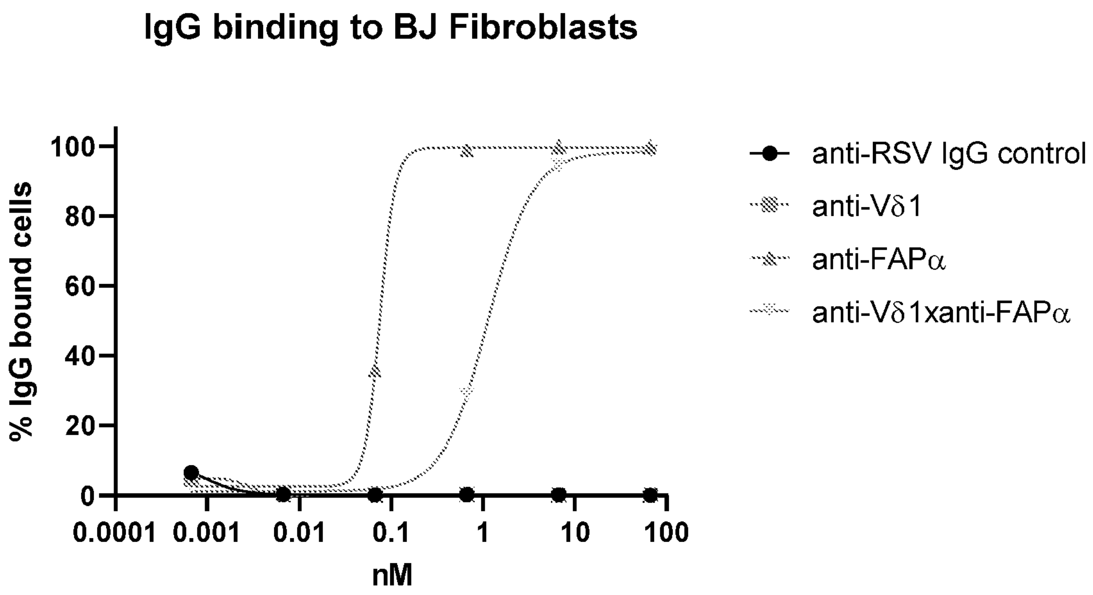


**C**

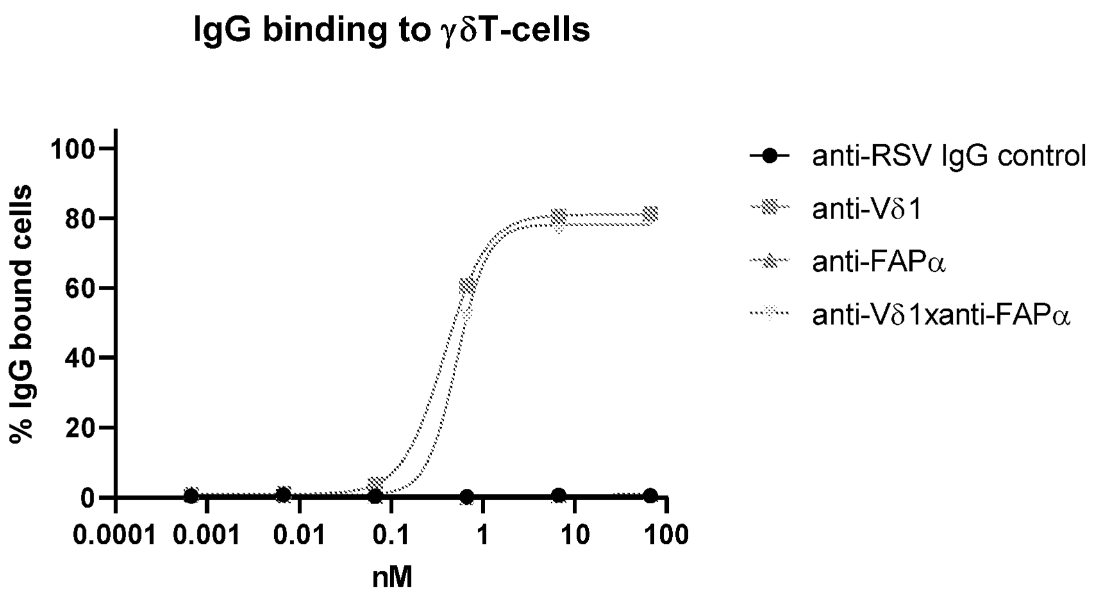

FIG 31 cont.

D

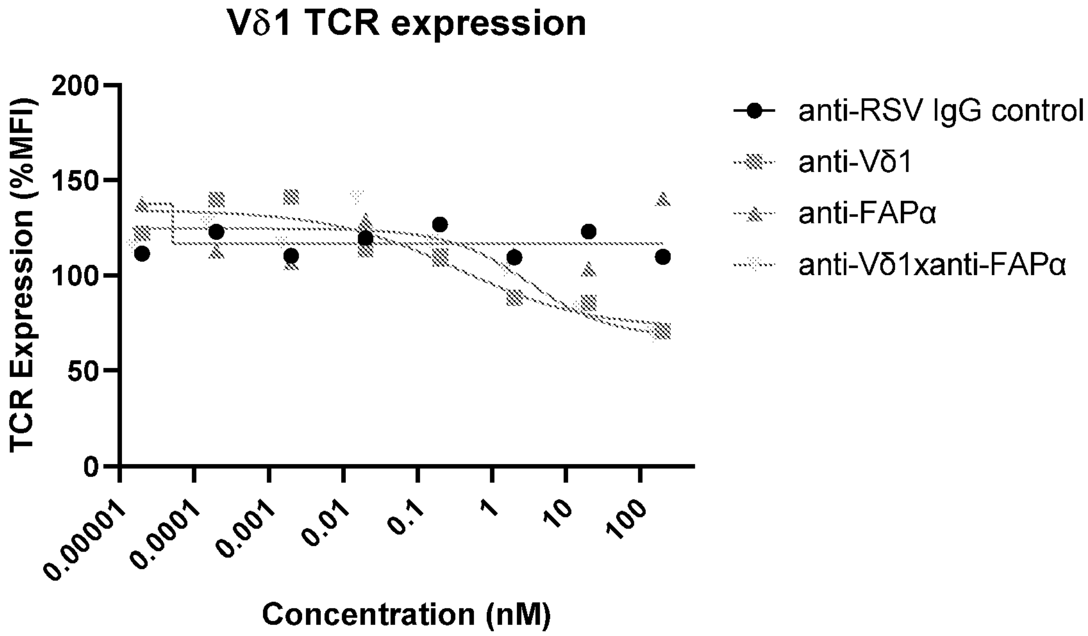


E

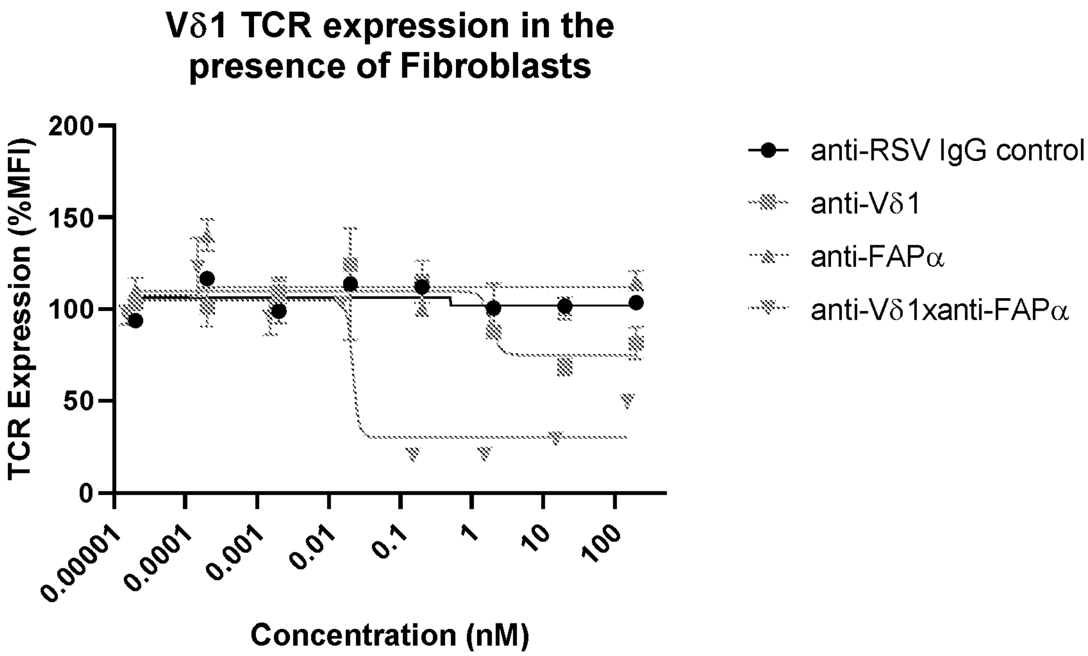

FIG 31 cont.

F

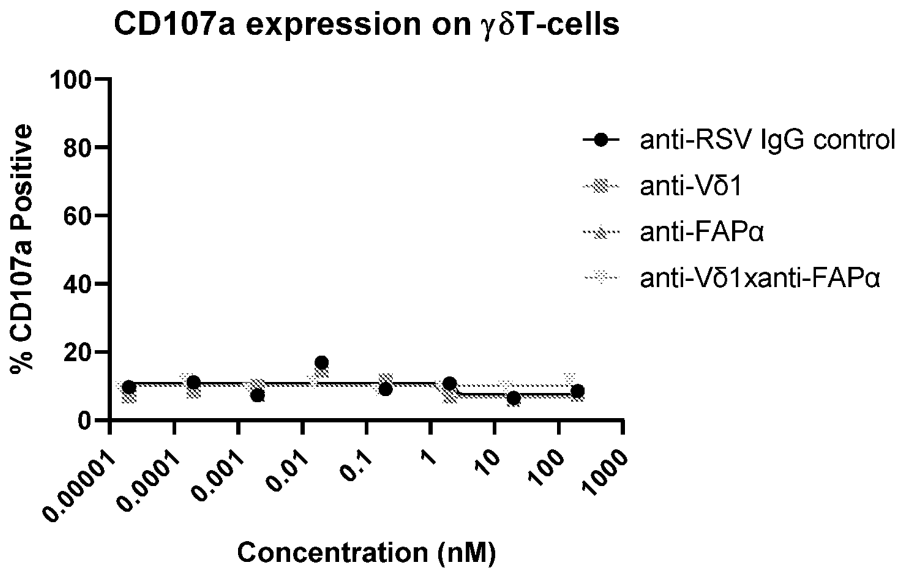


G

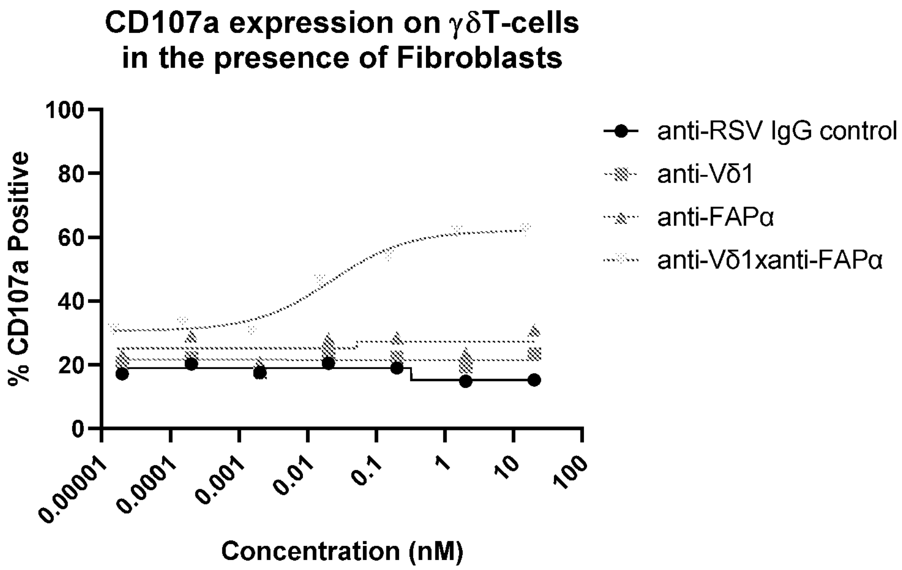


H

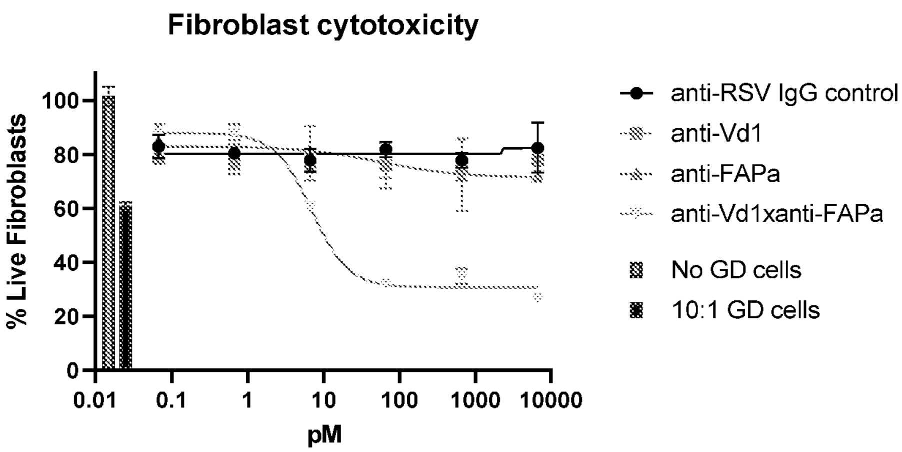

FIG 32

A

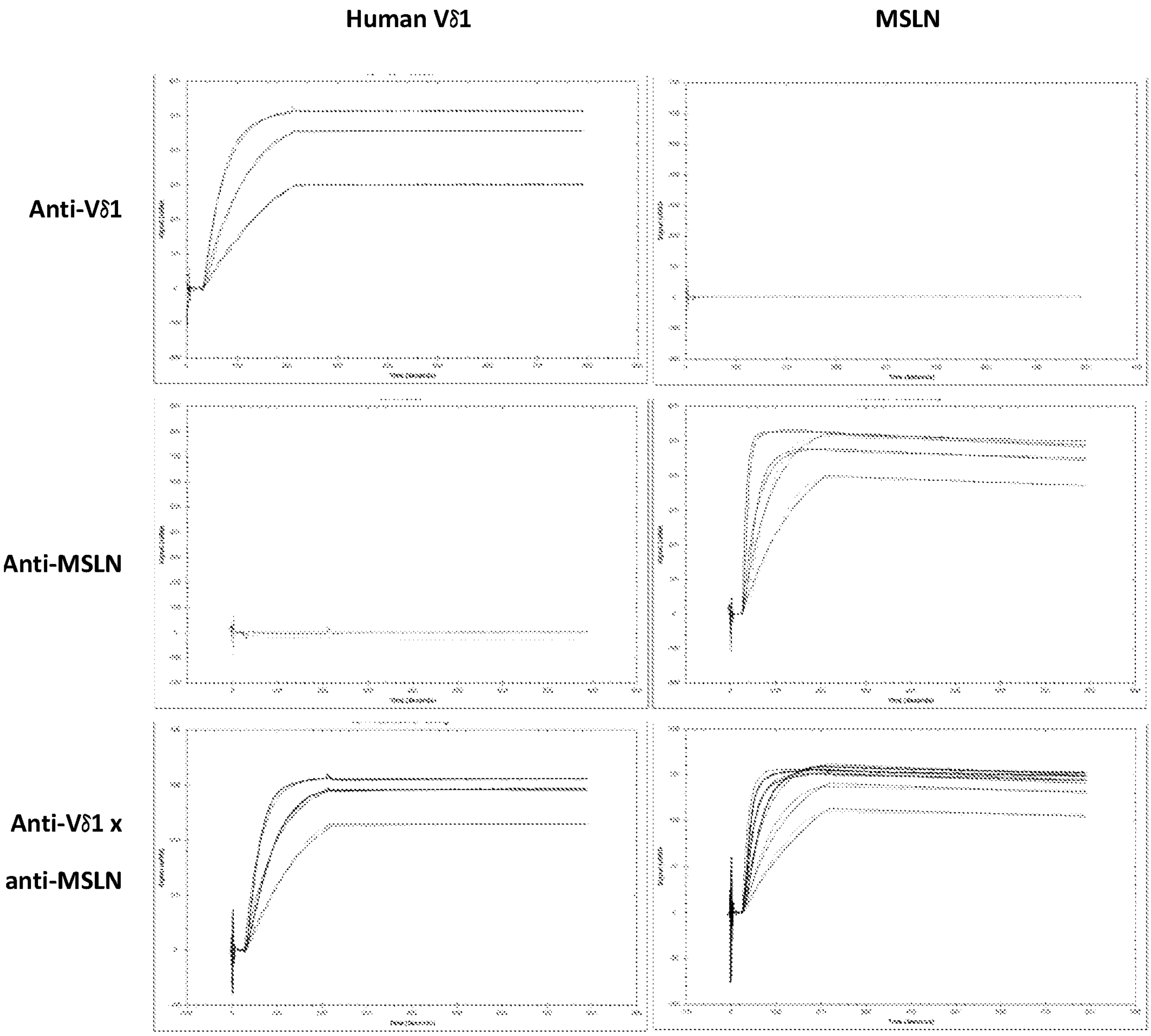

**FIG 32 cont.**

**B**

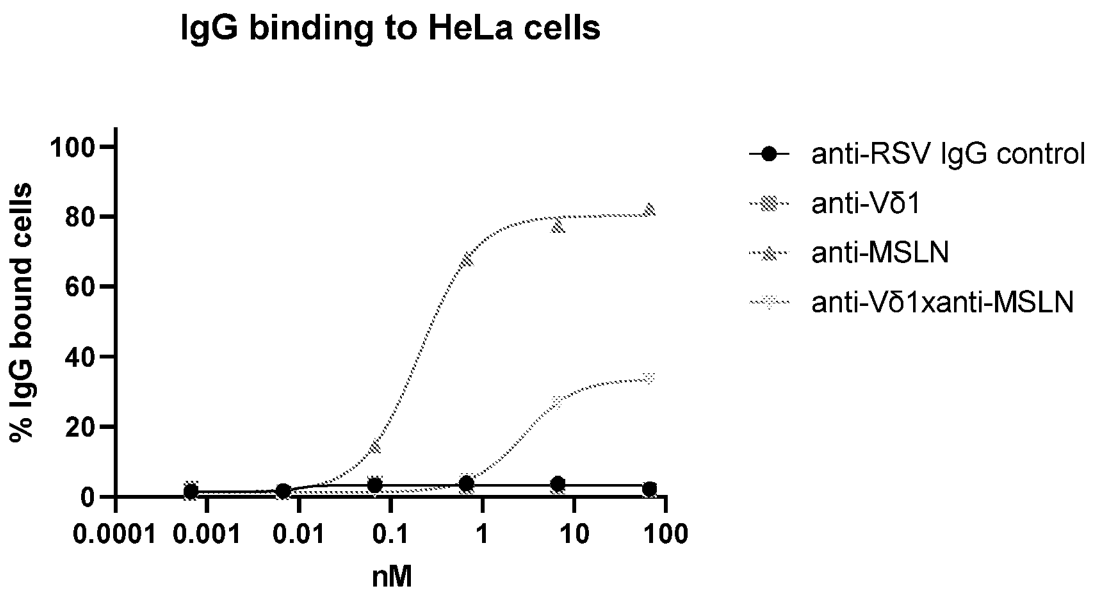


**C**

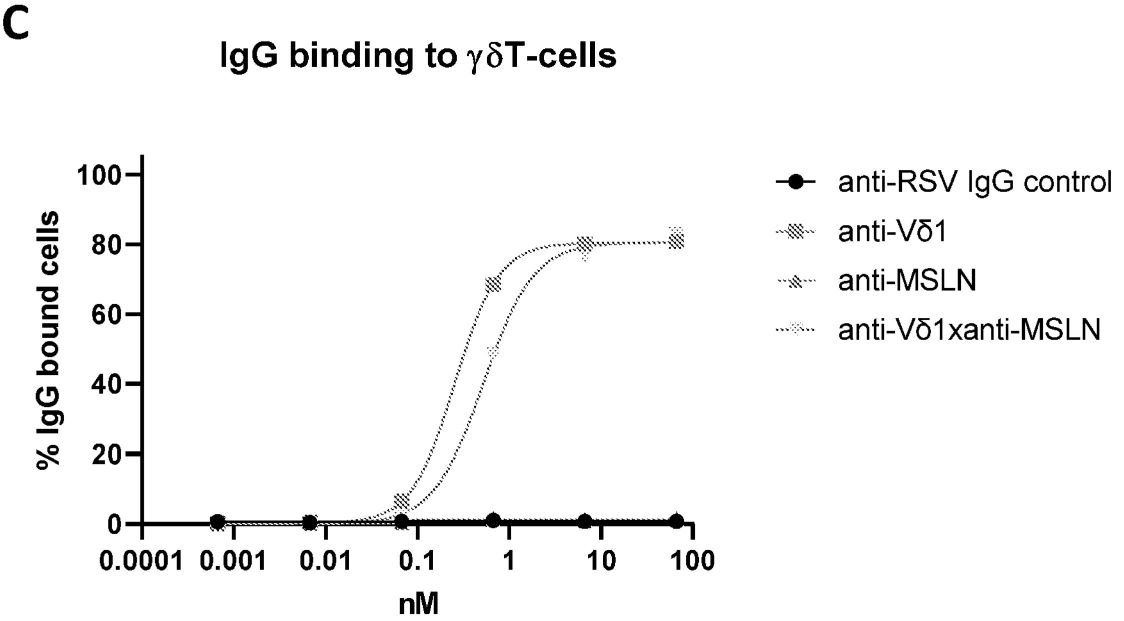

FIG 32 cont.

D

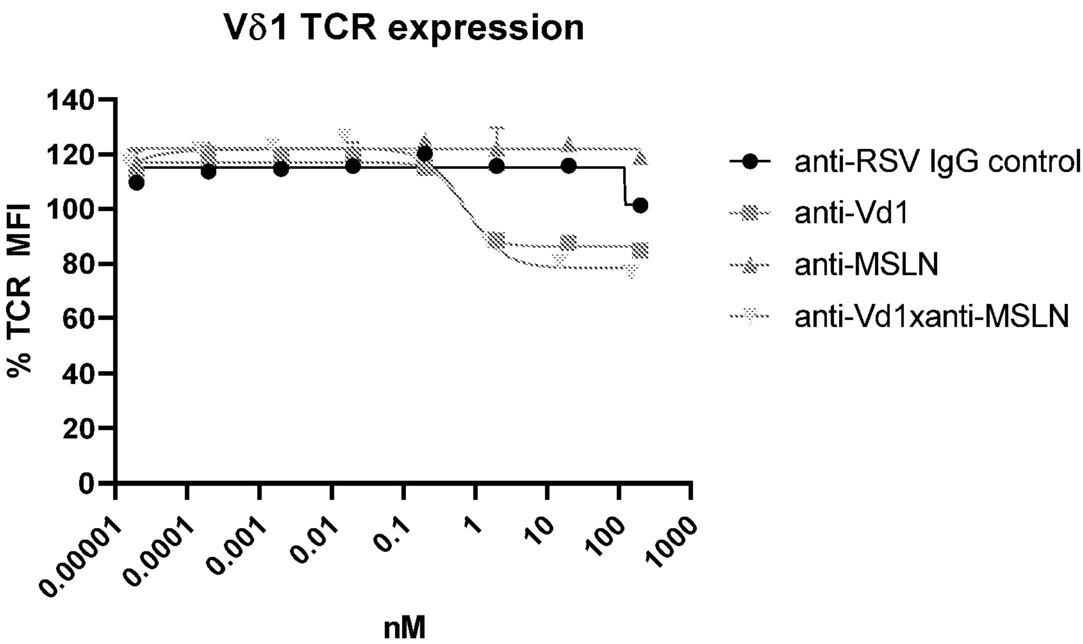


E

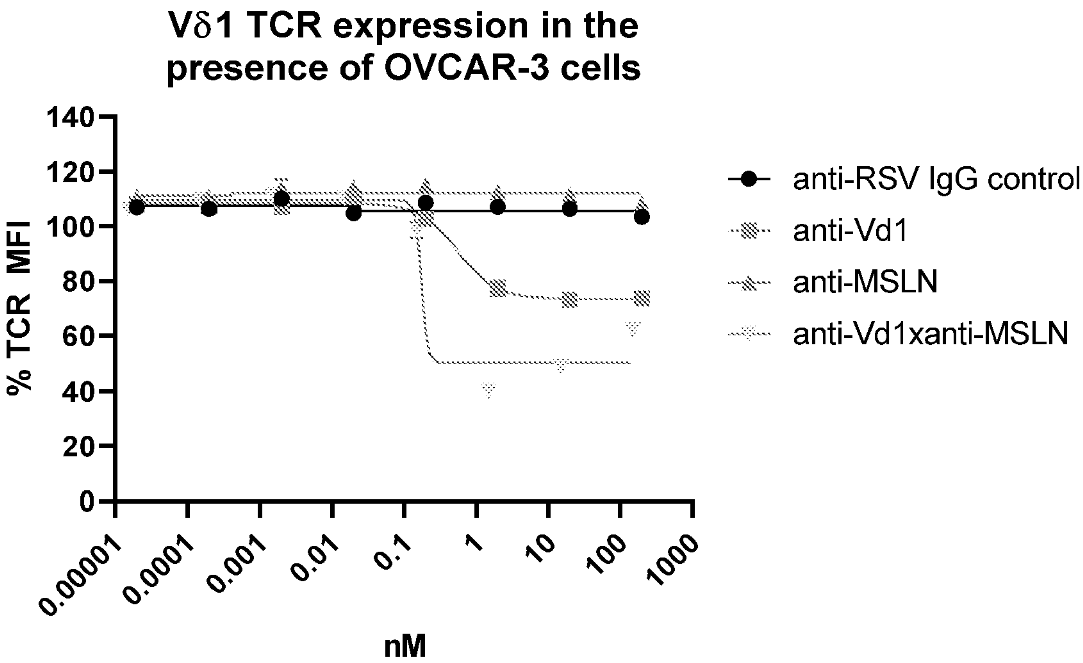

FIG 32 cont.

F

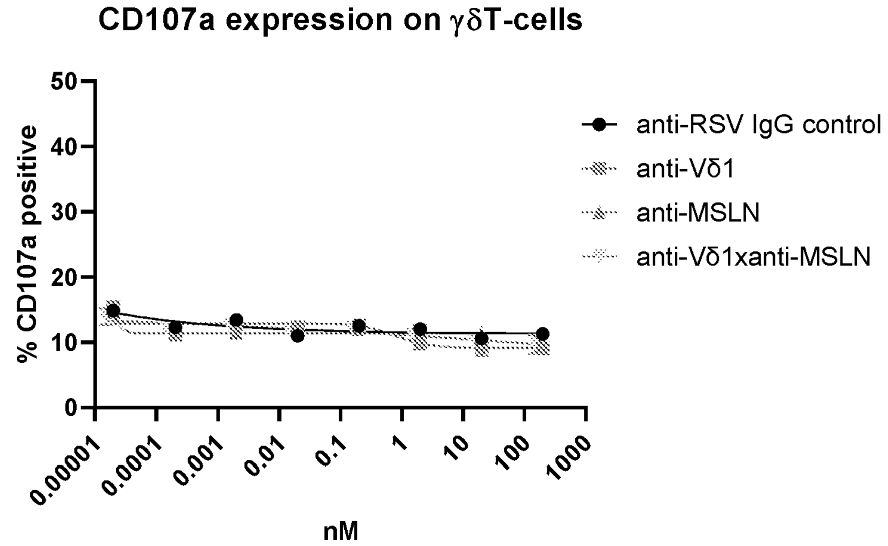


G

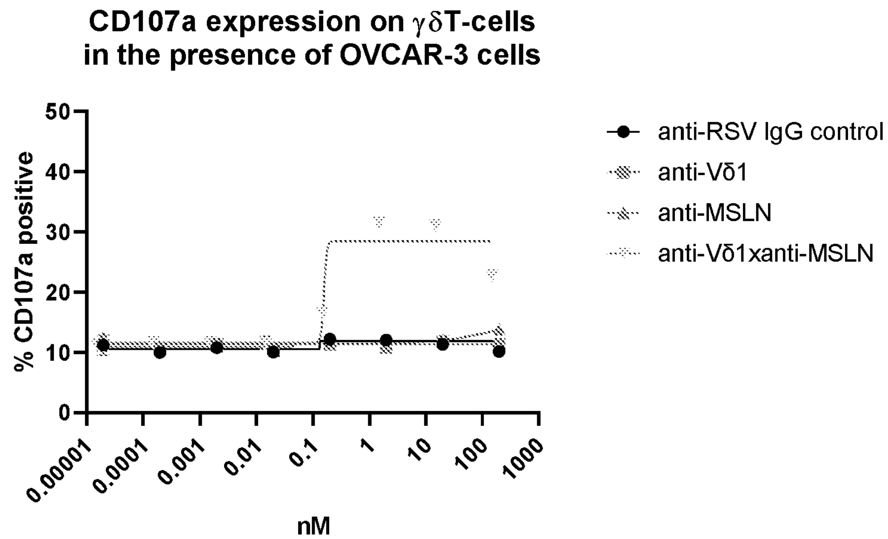


H

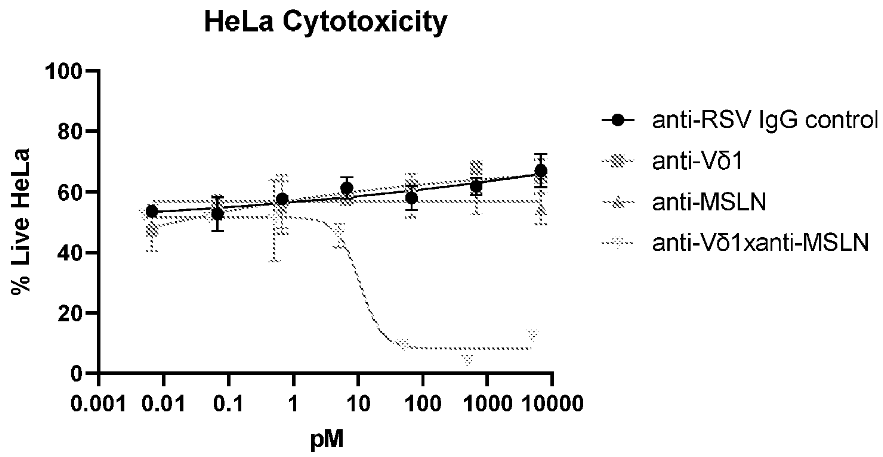

FIG 33

A

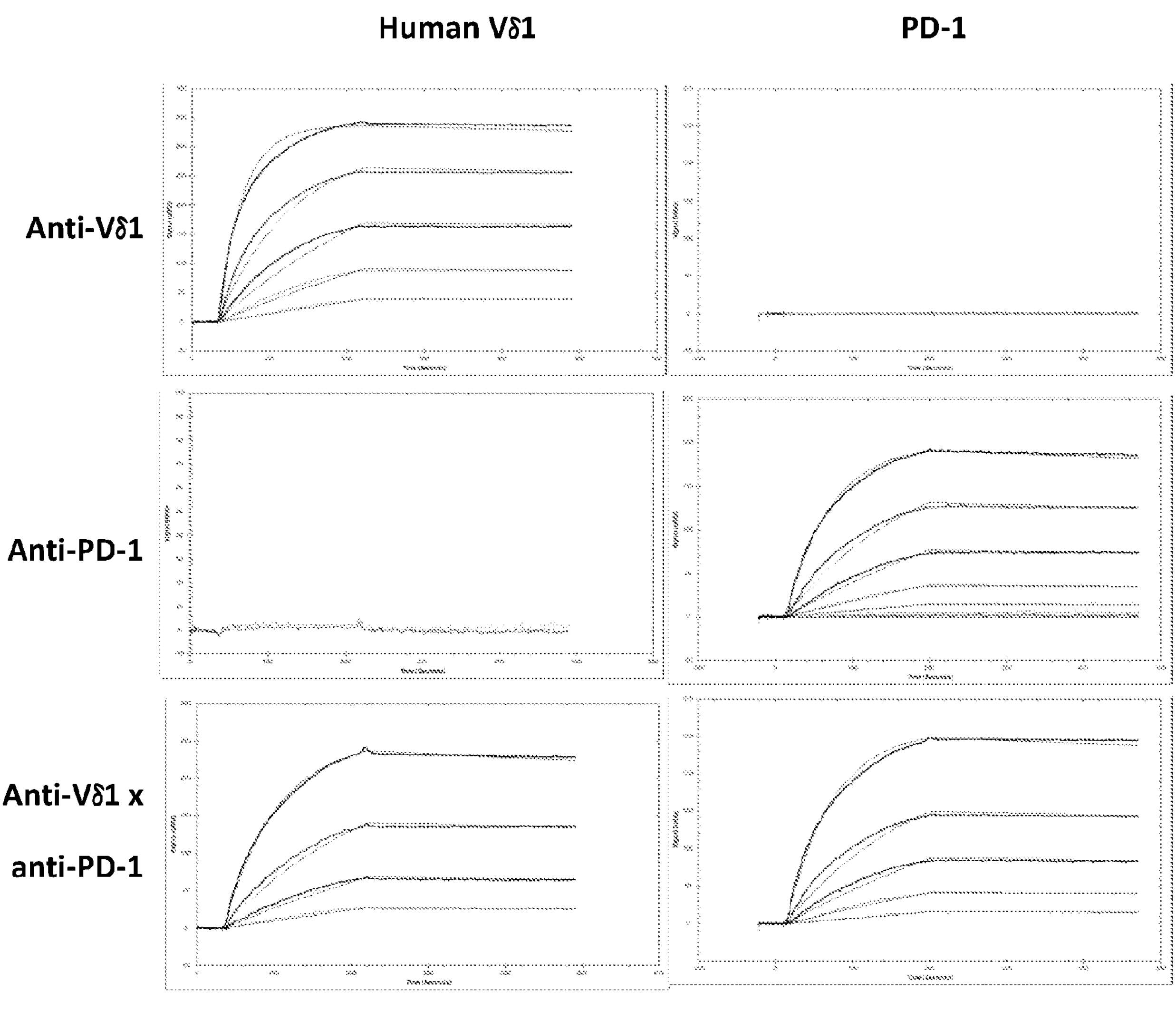

FIG 33 cont

B

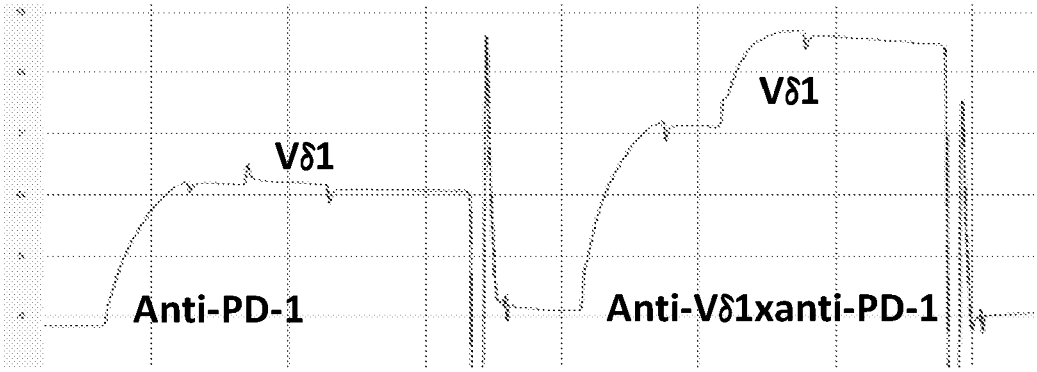


C

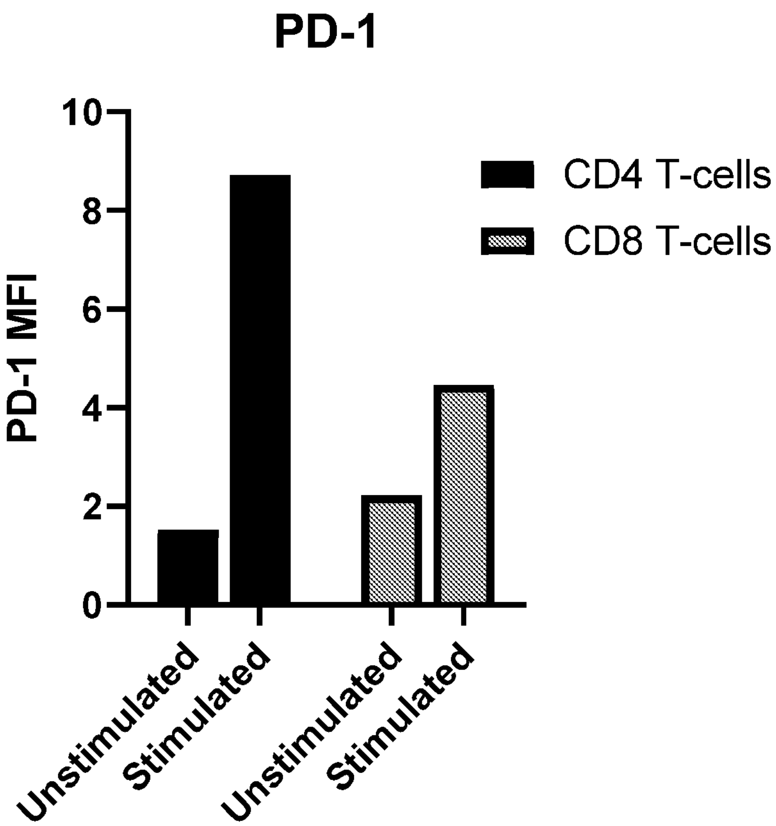

# FIG 33 cont

D

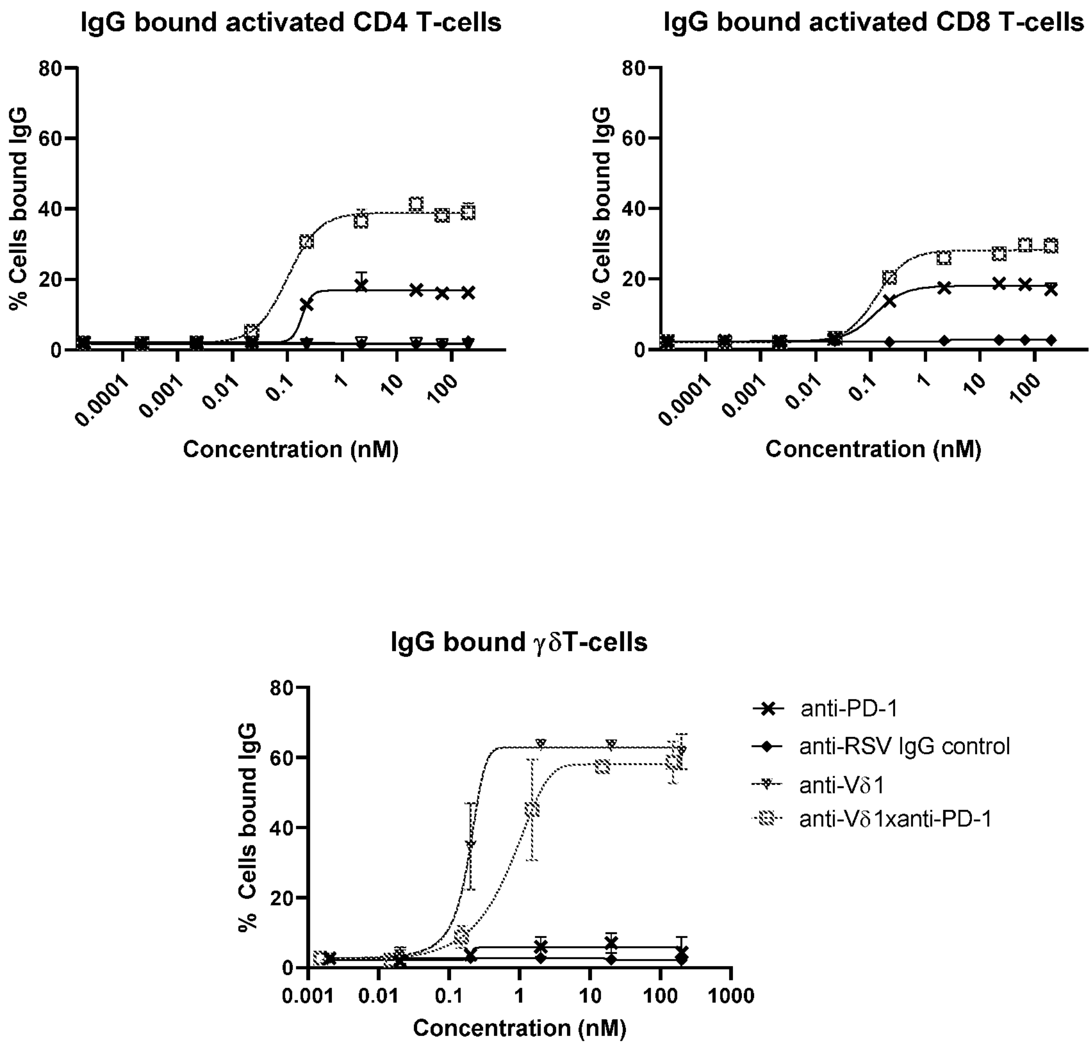
IgG bound activated CD4 T-cells
IgG bound activated CD8 T-cells
% Cells bound IgG
80
60
40
20
0
0.0001
0.001
0.01
0.1
1
10
100
Concentration (nM)
IgG bound γδT-cells
0.001 0.01 0.1 1 10 100 1000
anti-PD-1
anti-RSV IgG control
anti-Vδ1
anti-Vδ1xanti-PD-1

# FIG 33 cont

E

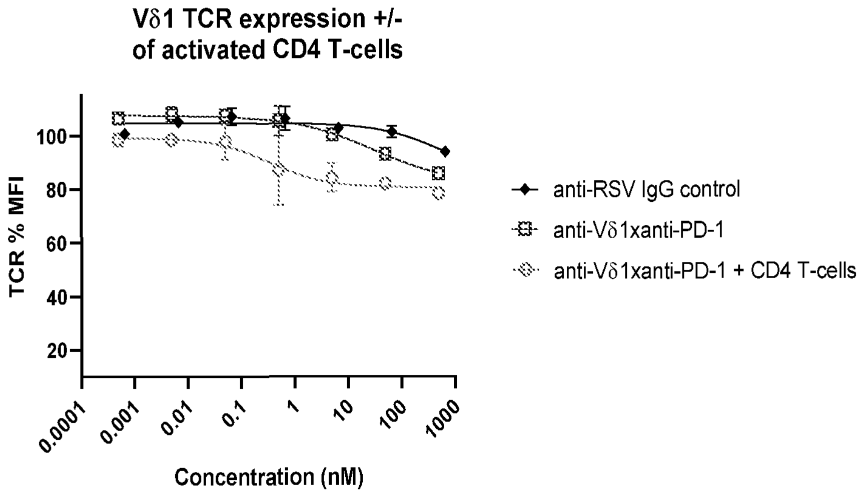


F

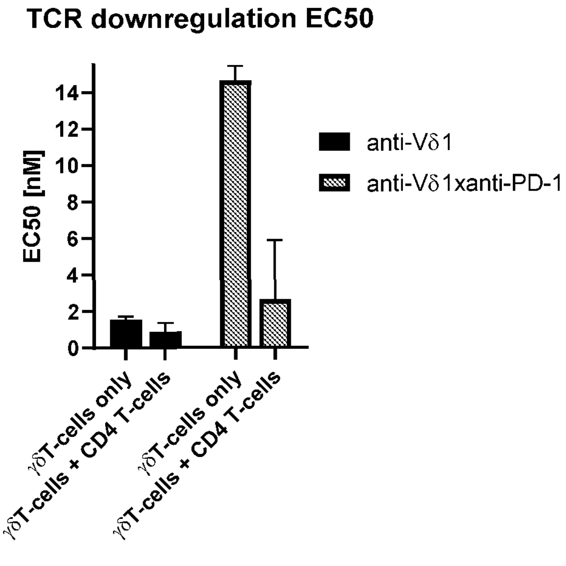


G

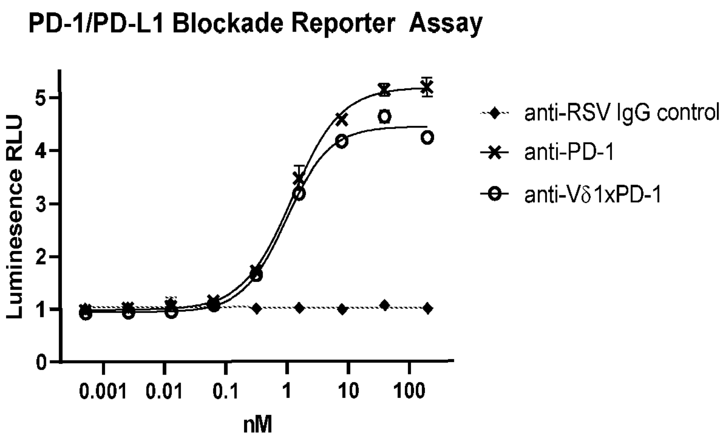

FIG 34

A

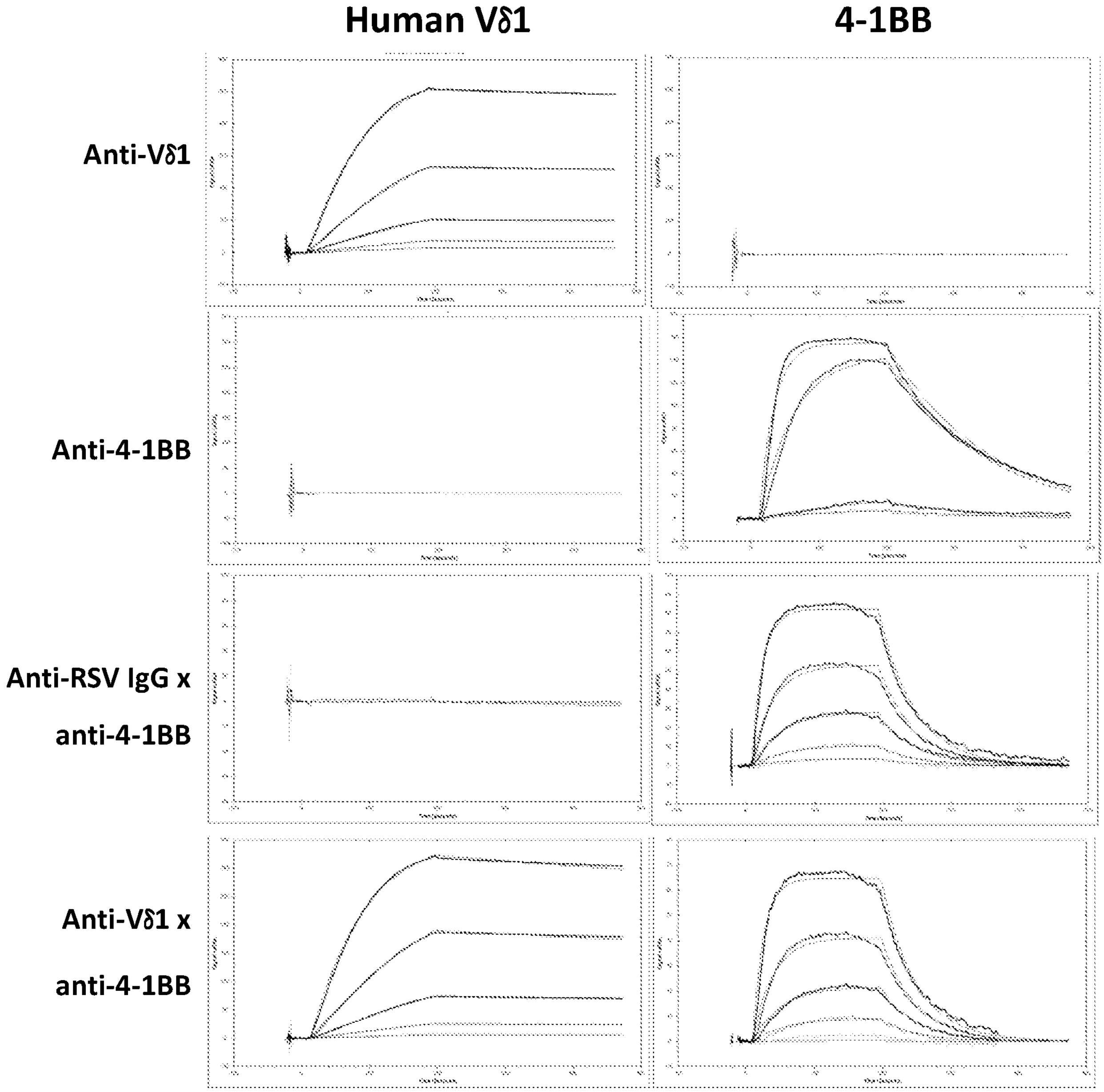
Human Vδ1
4-1BB
Anti-Vδ1
Anti-4-1BB
Anti-RSV IgG x
anti-4-1BB
Anti-Vδ1 x
anti-4-1BB

FIG 34 cont.

B

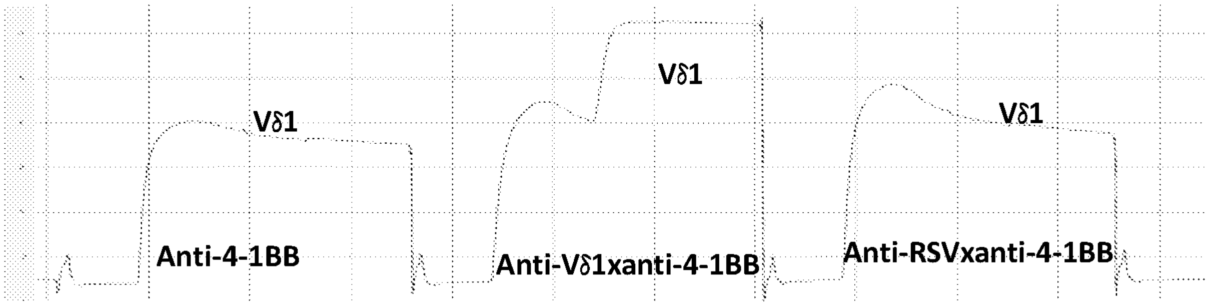


C

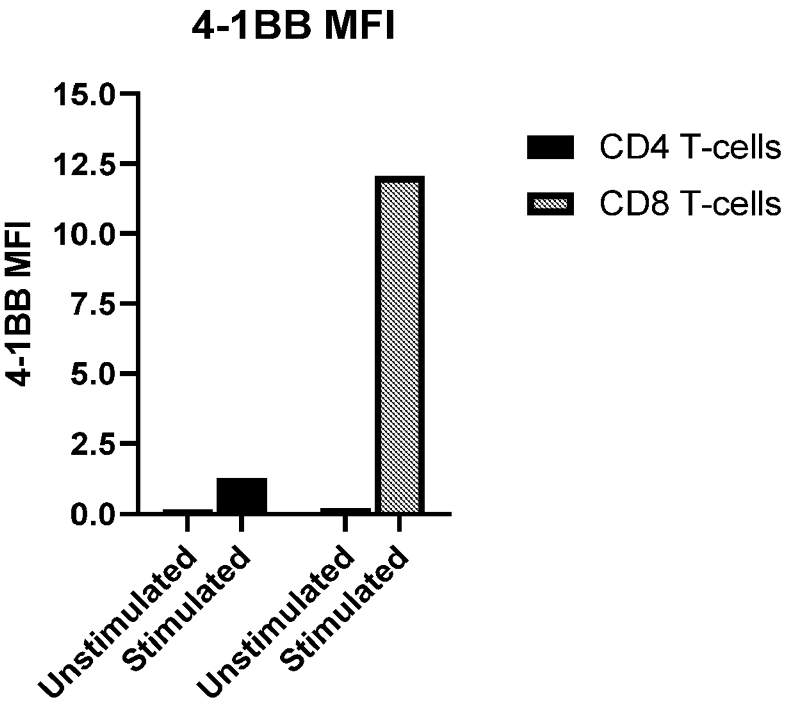

D

FIG 34 cont.

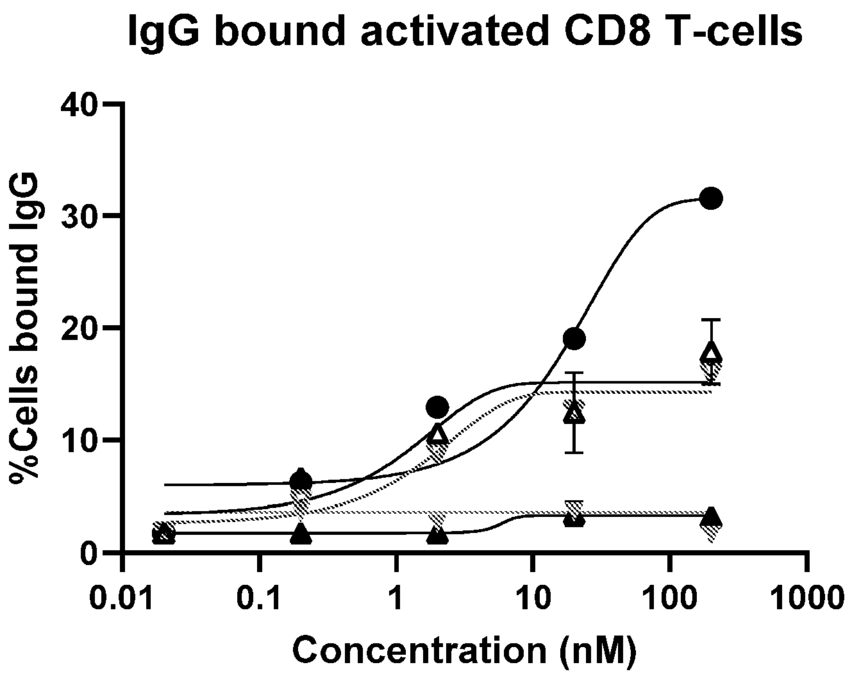
IgG bound activated CD8 T-cells
%Cells bound IgG
40
30
20
10
0
0.01
0.1
1
10
100
1000
Concentration (nM)

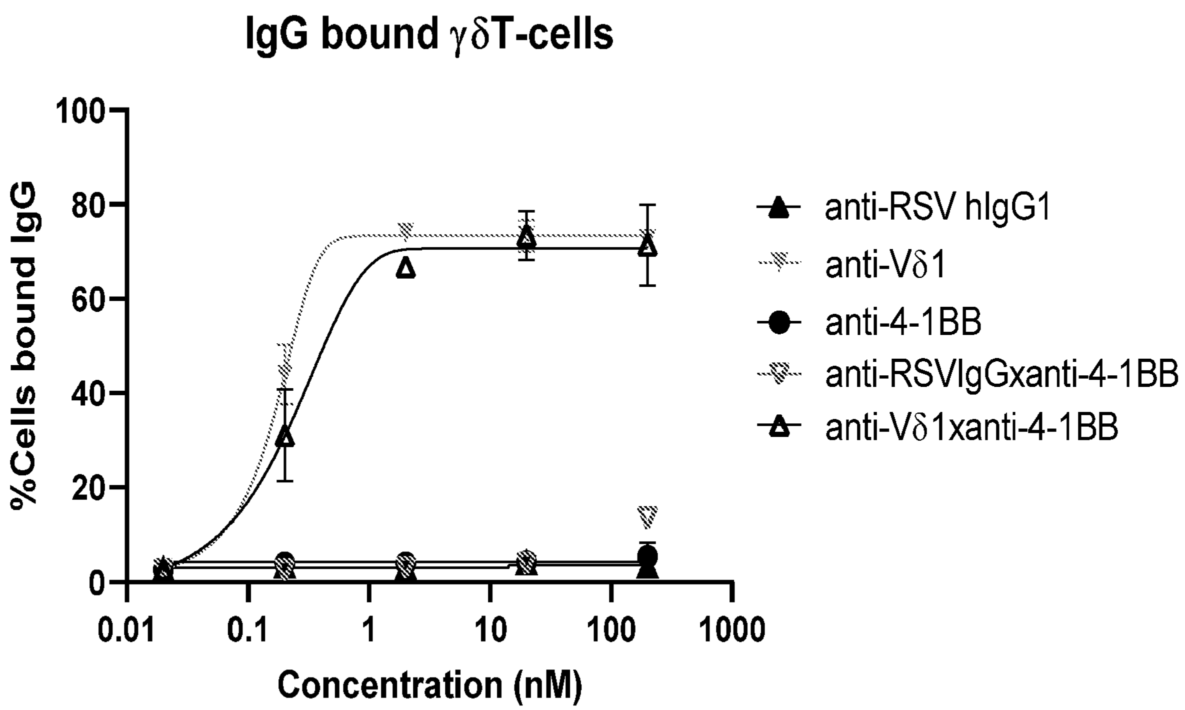
IgG bound γδT-cells
%Cells bound IgG
100
80
60
40
20
0
0.01
0.1
1
10
100
1000
Concentration (nM)
anti-RSV hIgG1
anti-Vδ1
anti-4-1BB
anti-RSVIgGxanti-4-1BB
anti-Vδ1xanti-4-1BB

**FIG 34 cont.**

**E**

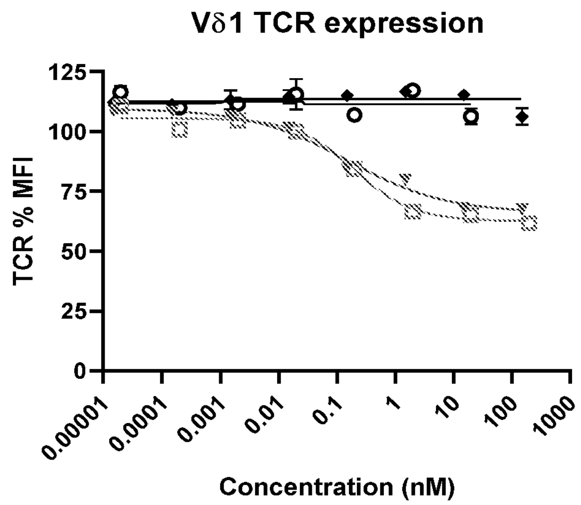


**F**

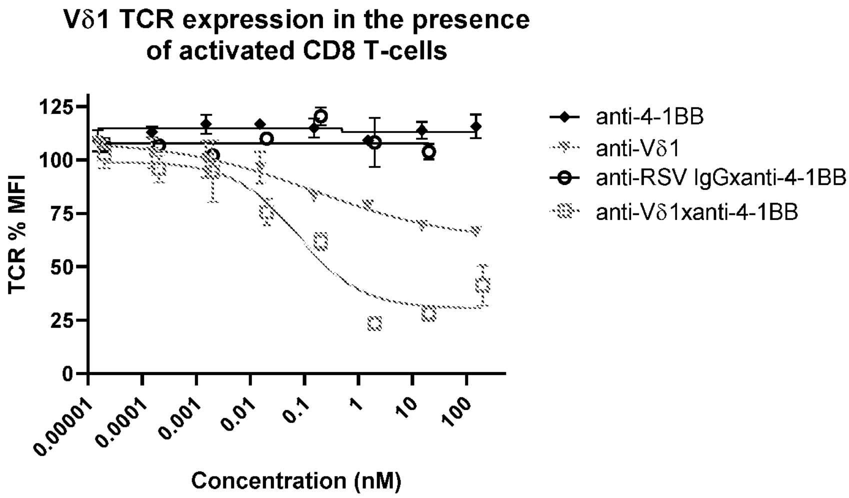


**G**

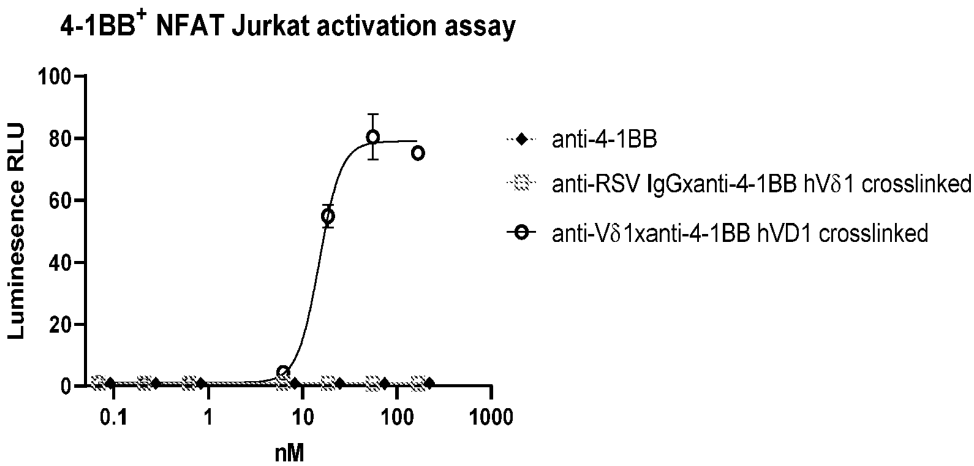

FIG 35

A

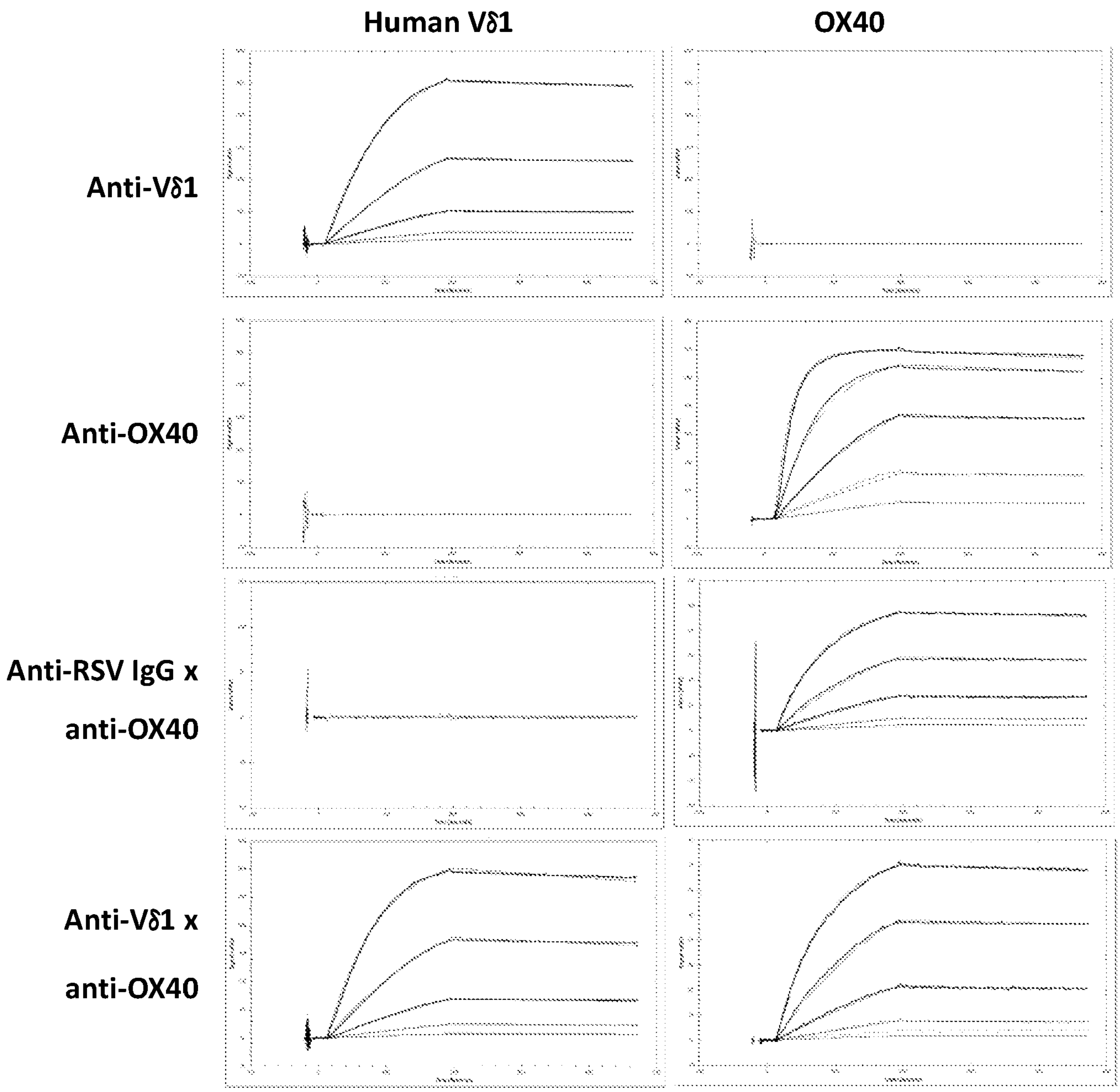
Human Vδ1
OX40
Anti-Vδ1
Anti-OX40
Anti-RSV IgG x
anti-OX40
Anti-Vδ1 x
anti-OX40

B

FIG 35 cont.

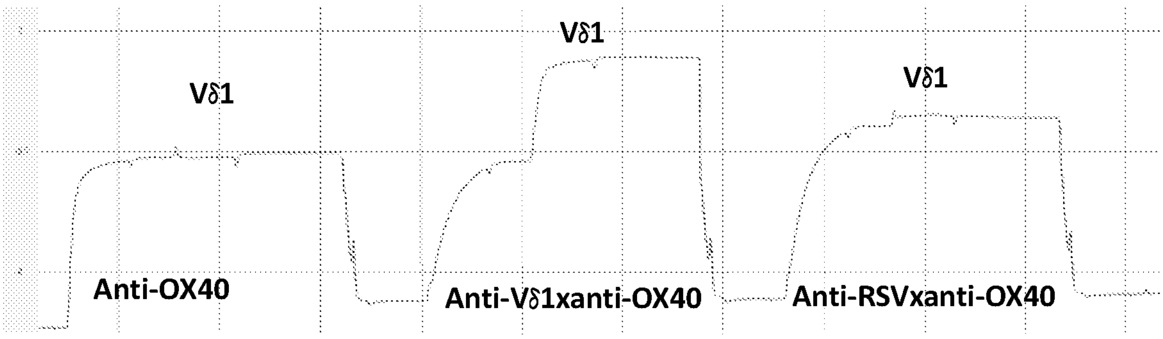


C

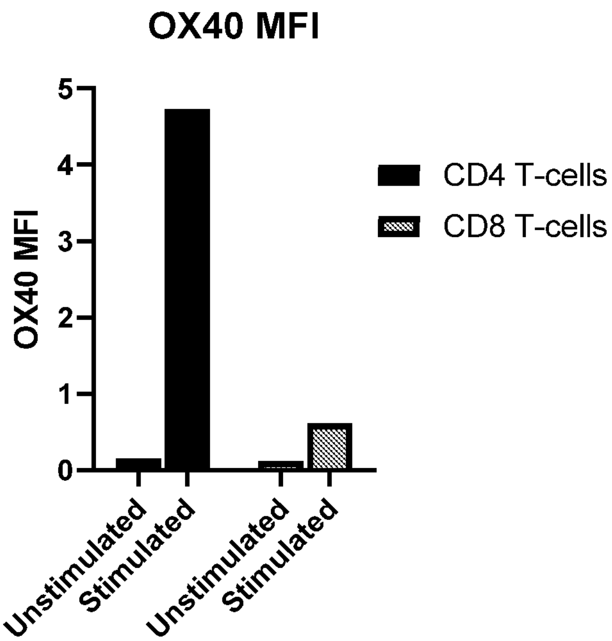

FIG 35 cont.

D

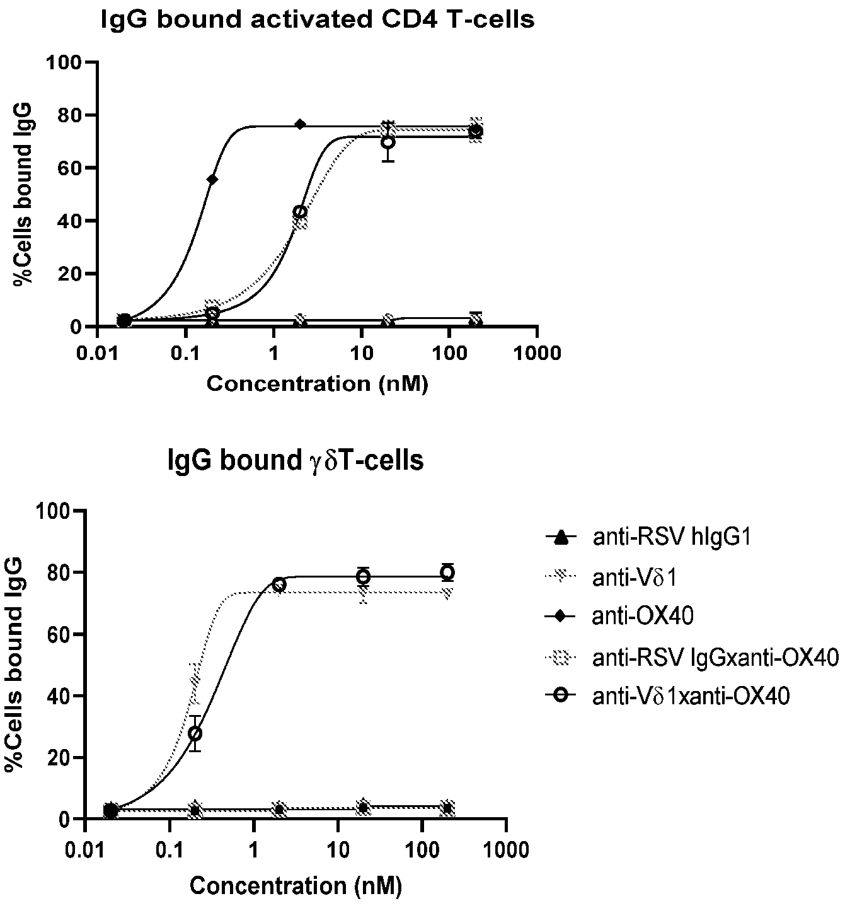


E

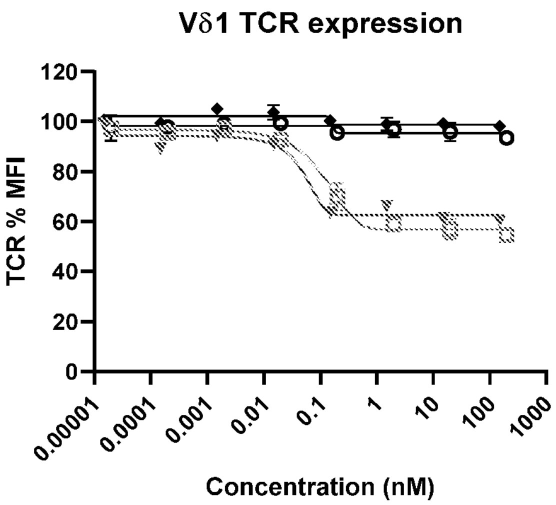

# FIG 35 cont.

F

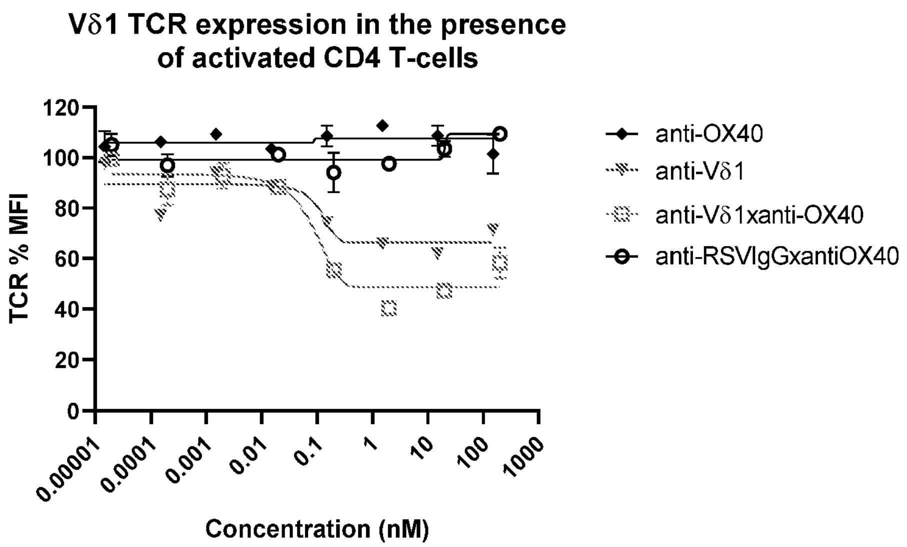


G

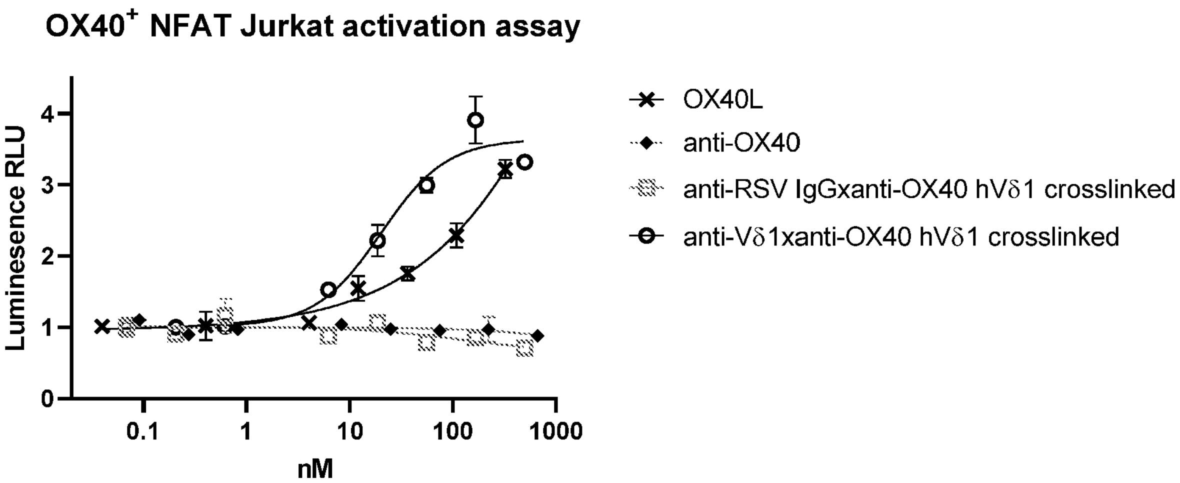

FIG 36

A

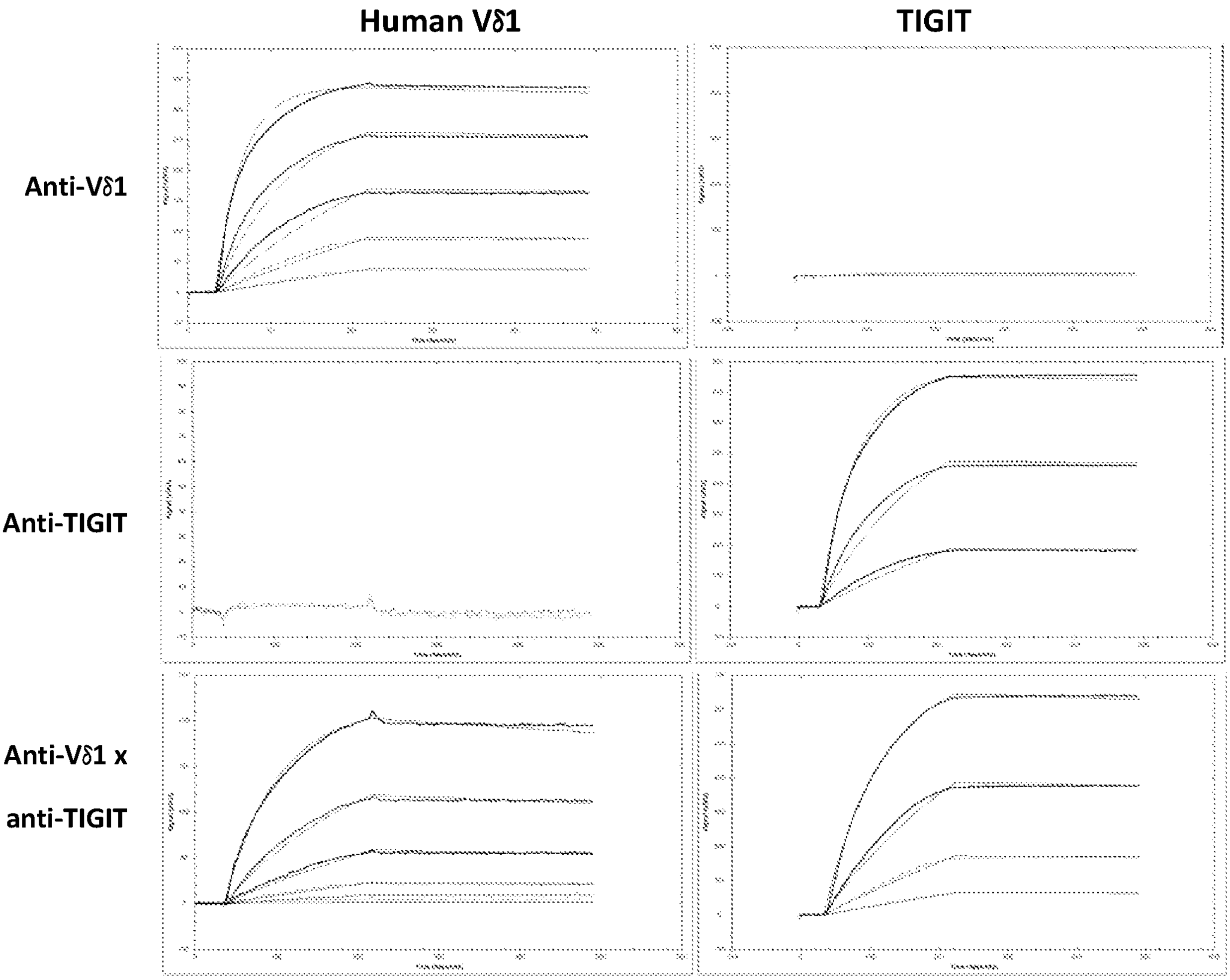

B

FIG 36 cont.

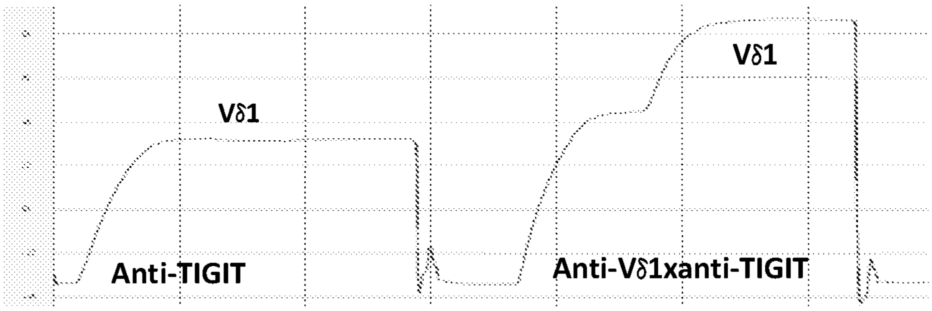


C

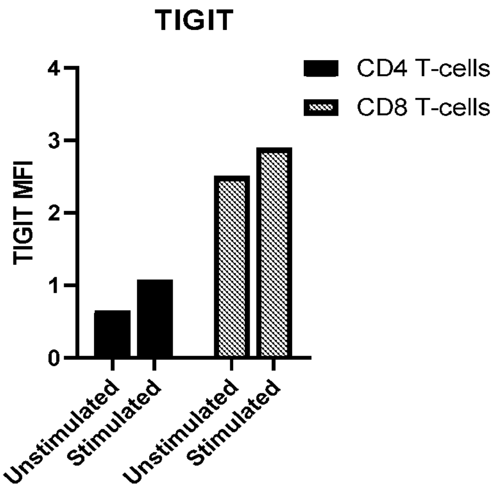

# FIG 36 cont.

D

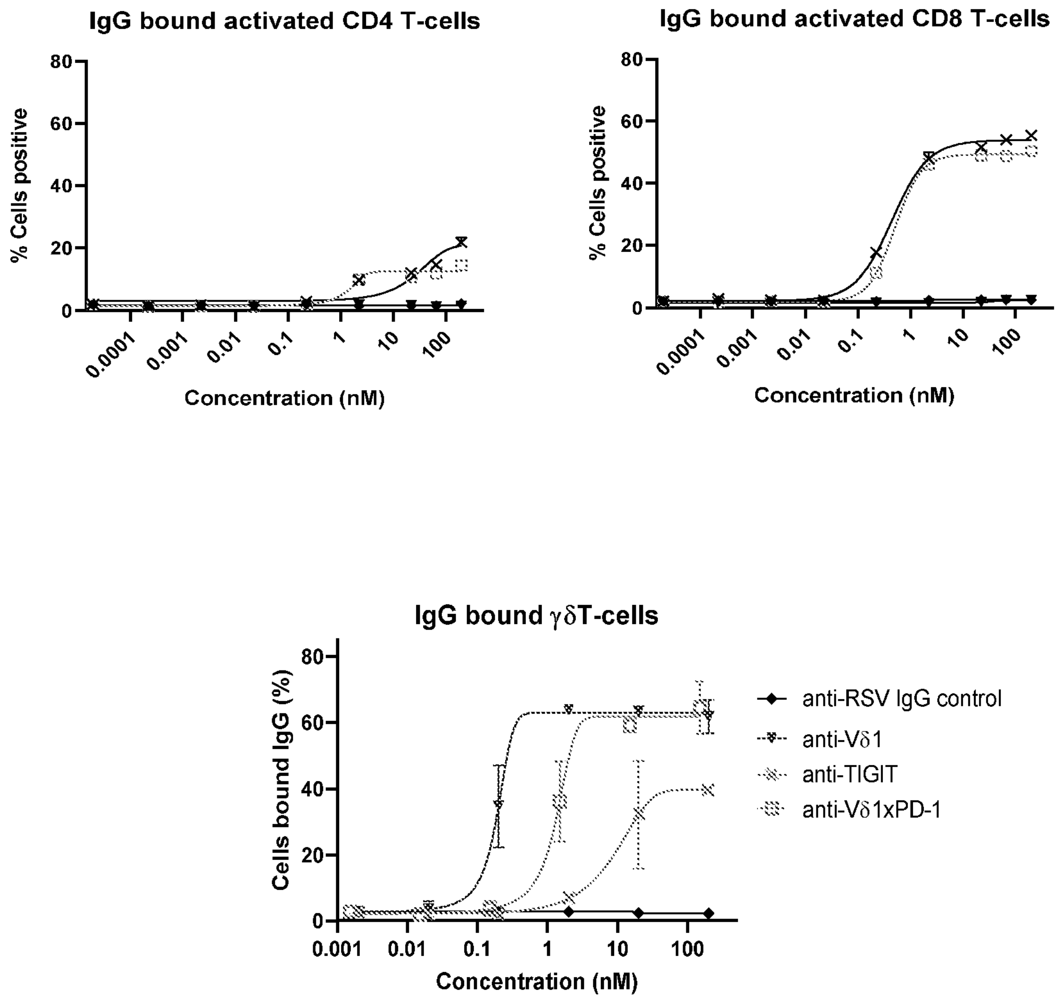
IgG bound activated CD4 T-cells
% Cells positive
80
60
40
20
0
0.0001
0.001
0.01
0.1
1
10
100
Concentration (nM)
IgG bound activated CD8 T-cells
% Cells positive
80
60
40
20
0
0.0001
0.001
0.01
0.1
1
10
100
Concentration (nM)
IgG bound γδT-cells
Cells bound IgG (%)
80
60
40
20
0
0.001
0.01
0.1
1
10
100
Concentration (nM)
anti-RSV IgG control
anti-Vδ1
anti-TIGIT
anti-Vδ1xPD-1

# FIG 36 cont.

E

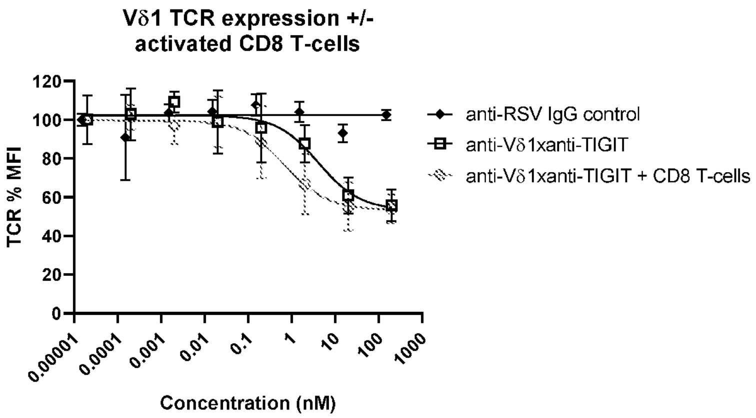


F

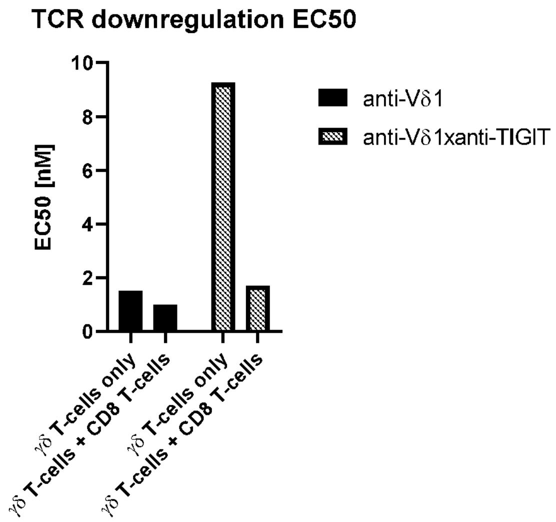


G

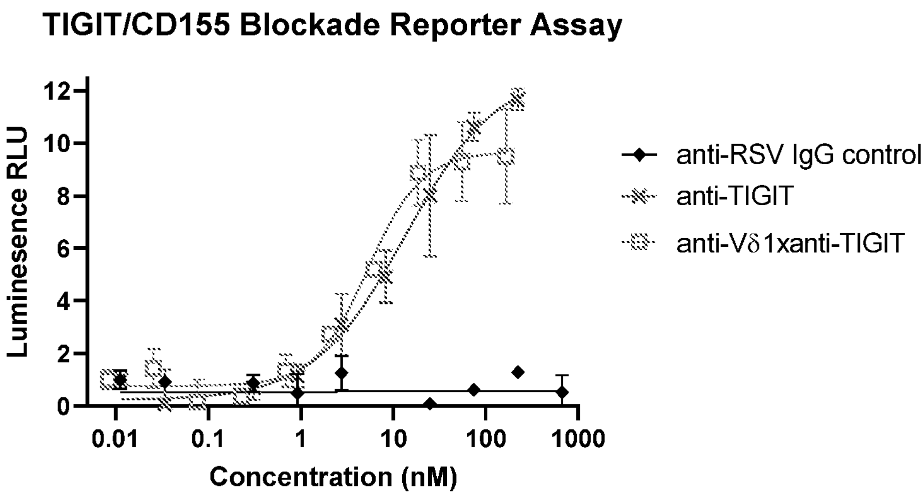

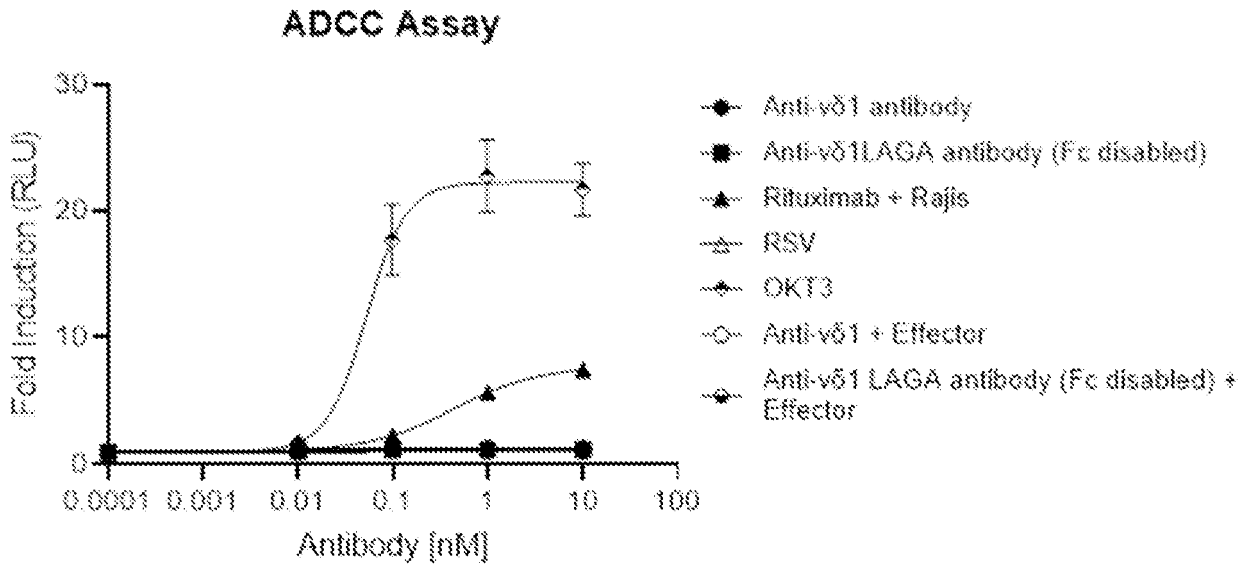
ADCC Assay
Fold Induction (RLU)
30
20
10
0
0.0001
0.001
0.01
0.1
1
10
100
Antibody [nM]
Anti-vδ1 antibody
Anti-vδ1LAGA antibody (Fc disabled)
Rituximab + Rajis
RSV
OKT3
Anti-vδ1 + Effector
Anti-vδ1 LAGA antibody (Fc disabled) + Effector

FIG 37

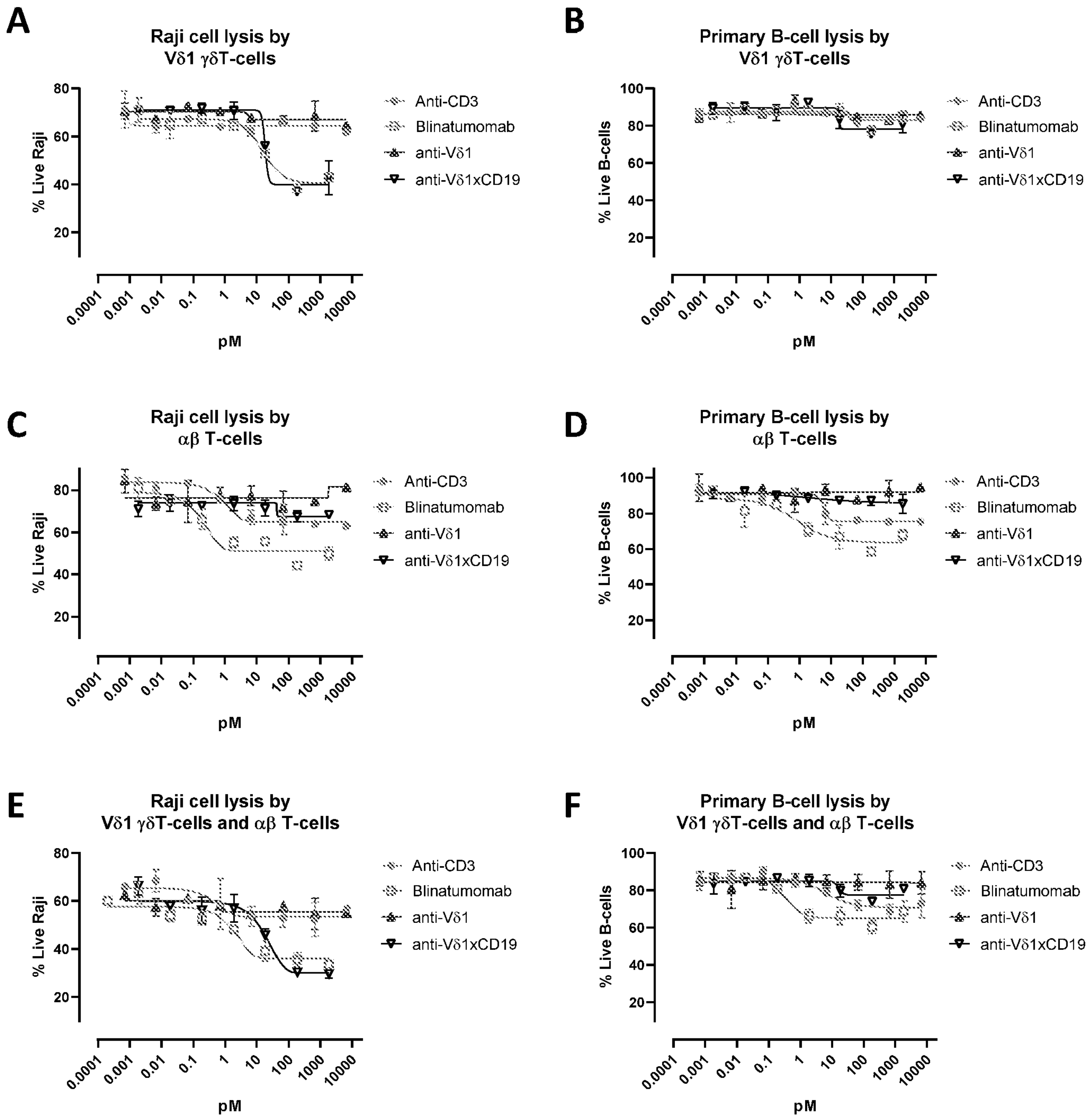
A
Raji cell lysis by
Vδ1 γδT-cells
% Live Raji
pM
Anti-CD3
Blinatumomab
anti-Vδ1
anti-Vδ1xCD19
B
Primary B-cell lysis by
Vδ1 γδT-cells
% Live B-cells
pM
C
Raji cell lysis by
αβ T-cells
D
Primary B-cell lysis by
αβ T-cells
E
Raji cell lysis by
Vδ1 γδT-cells and αβ T-cells
F
Primary B-cell lysis by
Vδ1 γδT-cells and αβ T-cells
0.0001
0.001
0.01
0.1
1
10
100
1000
10000

FIG 38

G

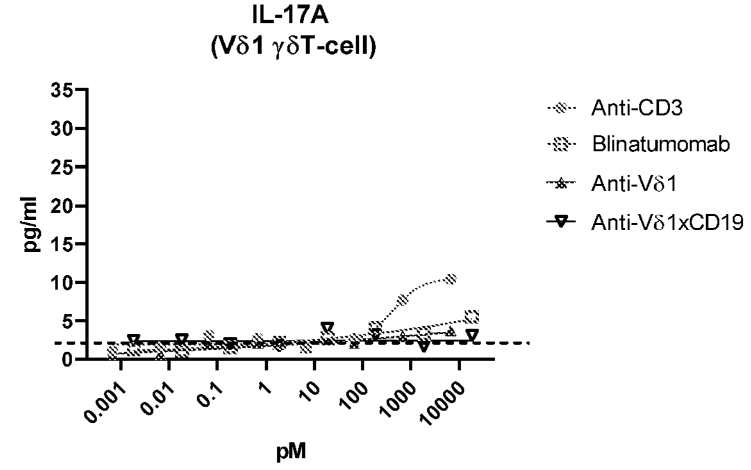


H

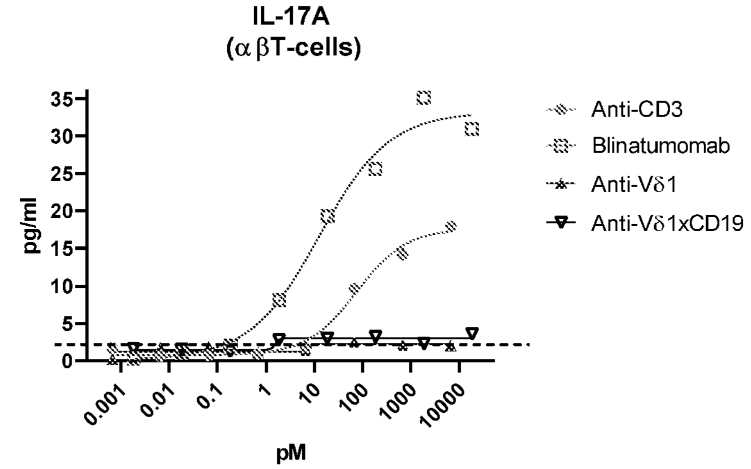


I

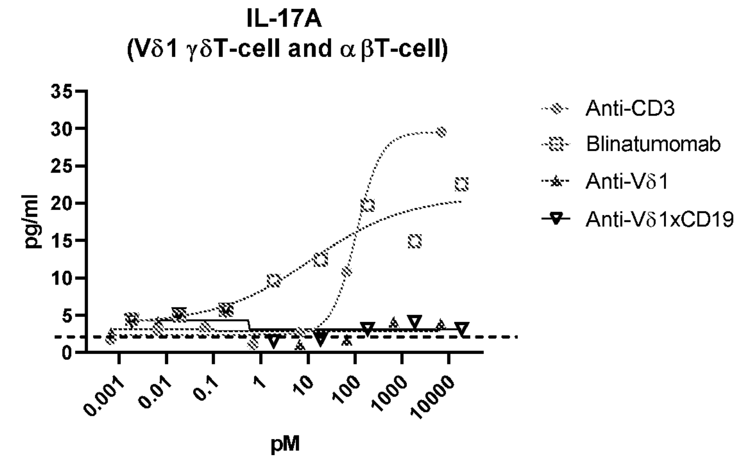


FIG 38 (cont)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019147735 A
- WO 2017072367 A
- WO 2018212808 A
- WO 2020060406 A
- WO 2020159368 A
- WO 2017197347 A
- EP 2546268 A1
- EP 3487885 A1
- WO 2016198480 A
- WO 2020095059 A


### Non-patent literature cited in the description


- **ADAMS et al.** *Cell Immunol.,* 2015, vol. 296, 30-40
- **MARKOWITZ et al.** *Kidney Int.,* 2003, vol. 64 (1), 281-289
- **LEJEUNE et al.** *Front Immunol.,* 2020, vol. 11, 762
- **QIN et al.** *Molecular cancer,* 2019, vol. 18, 155
- **PADLAN.** *Mol. Immunol.,* 1994, vol. 31, 169-217
- **KABAT et al.** Sequences of Proteins of Immunological Interest. U.S. Department of Health and Human Services, 1991
- **CHOTHIA et al.** *Nature,* 1989, vol. 342, 877-883
- **XINHUA WANG et al.** *Protein & Cell,* 2018, vol. 9, 63-73
- **PEREIRA et al.** *mAbs,* 2018, vol. 10 (5), 693-711
- **JEFFERIS ; LEFRANC.** *MAbs,* 2009, vol. 1 (4), 332-8
- **LUOMA et al.** *Immunity,* 2013, vol. 39, 1032-1042
- **XU et al.** *PNAS USA,* 2011, vol. 108 (6), 2414-2419
- Antigen Recognition by γδ T-Cells. **MING HENG ; MADALENE HENG.** Madame Curie Bioscience Database. Landes Bioscience, 2000
- **MAHVI et al.** *Cancer Imm. Immunother.,* 1993, vol. 37, 181-186
- **DUTTA I et al.** *Front. Immunol.,* 2017, vol. 8, 776
- **FERRARINI et al.** *Nat. Cancer Inst.,* 1996, vol. 88 (7), 436-441
- **MAEURER et al.** *JEM,* 1996, vol. 183 (4), 1681-1696
- **KITAYAMA.** *Clin Exp Imm,* 1993, vol. 93 (3), 442-7
- **WU et al.** *OncoImmunology,* 2015, vol. 4 (3), e992749
- **MIKULAK et al.** *JCI Insight.,* 2019, vol. 4 (24), e125884
- **GROH et al.** *PNAS,* 1999, vol. 96 (12), 6879-6884
- **FISHER et al.** *Clin Cancer Res,* 2014, vol. 20 (22), 5720-32
- **CORDOVA.** *Plos ONE,* 2012, vol. 7 (11), e49878
- **KNIGHT et al.** *Cytotherapy,* 2012, vol. 14 (9), 1110-1118
- **LORENZO et al.** *Canc Imm Res,* 2019, vol. 7 (4
- **ALMEIDA et al.** *Clin Can Res,* 2016, vol. 22 (23
- **STAFLIN et al.** *JCI Insight,* 2020, vol. 5 (7
- **SPIESS et al.** *Molecular Immunology,* 2015, vol. 67, 95-106
- **GENTLES et al.** *Nature Immunology,* 2015, vol. 21, 938-945
- **WU et al.** *Sci. Trans. Med.,* 2019, vol. 11 (513), 9364
- **CATELLANI et al.** *Blood,* 2007, vol. 109 (5), 2078-2085
- **ZHAO et al.** *Immunol. Res.,* 2018, vol. 2018, 5081634
- **VAN DORP et al.** *Biology of Blood and Marrow Transplantation,* 2011, vol. 17 (2), S217
- **POCCIA et al.** *J. Infect. Dis.,* 2006, vol. 193 (9), 1244-1249
- **ARUDA et al.** *Blood,* 2019, vol. 3 (21), 3436-3448
- **GODDER et al.** *Bone Marrow Transplantation,* 2007, vol. 39, 751-757
- **SHEN et al.** *Cell Prolif.,* 2018, vol. 51 (4), e12441
- **BONAVENTURA et al.** *Front. Immunol.,* 2019, vol. 10, 168
- **GREEN ; SAMBROOK.** Molecular Cloning: A Laboratory Manual. Cold Spring Harbour Laboratory Press, 2012

- **HOLLEVOET ; DECLERCK.** *J. Transl. Med.,* 2017, vol. 15 (1), 131
- **SCHOFIELD et al.** *Genome biology,* 2007, vol. 8 (11), R254
- **XU et al.** *PNAS,* 2011, vol. 108, 2414-2419
- **ENGLAND et al.** *J. Immunol.,* 1999, vol. 162, 2129-2136
- **MARTIN et al.** *BMC Biotechnol.,* 2006, vol. 6, 46
- **VON HORSTEN HH et al.** *Glycobiology,* 2010, vol. 20 (12), 1607-18